# EUROPEAN PATENT SPECIFICATION

(11) **EP 3 056 498 B1**
(45) Date of publication and mention of the grant of the patent: **19.09.2018**
(21) Application number: 15190459.6
(22) Date of filing: 19.10.2015
(51) Int. Cl.: C07D 519/00, H01L 51/00, H01L 51/50

(54) **DIAZADIBENZOFURANE AND DIAZADIBENZOTHIOPHENE DERIVATIVES AND THEIR USE IN ORGANIC OPTOELECTRONIC DEVICES**
DIAZADIBENZOFURAN UND DIAZADIBENZOTHIOPHEN DERIVATE UND IHRE VERWENDUNG IN ORGANISCHEN OPTOELEKTRONISCHEN VORRICHTUNGEN
DÉRIVÉS DE DIAZADIBENZOFURANE ET DIAZADIBENZOTHIOPHÈNE ET LEUR UTILISATION DANS UN DISPOSITIF OPTOÉLECTRONIQUE ORGANIQUE

(30) Priority: 13.02.2015 KR 20150022403
(43) Date of publication of application: 17.08.2016
(73) Proprietor: Samsung SDI Co., Ltd., Gyeonggi-do 17084 (KR)
(72) Inventor: Lee, Sang-Shin, 16678 Gyeonggi-do (KR); Kang, Dong-Min, 16678 Gyeonggi-do (KR); Kim, Young-Kwon, 16678 Gyeonggi-do (KR); Lui, Jin-Hyun, 16678 Gyeonngi-do (KR); Yu, Eun-Sun, 16678 Gyeonngi-do (KR); Jang, Yu-Na, 16678 Gyeonggi-do (KR); Han, Su-Jin, 16678 Gyeonggi-do (KR)
(74) Representative: Michalski Hüttermann & Partner Patentanwälte mbB

(56) References cited:
- EP-A1- 2 660 890
- EP-A1- 2 711 363
- WO-A1-2012/102967
- WO-A1-2013/180376
- WO-A1-2014/157599
- WO-A1-2015/182872
- JP-A- 2008 074 939
- JP-A- 2011 084 531
- JP-A- 2015 151 352
- KR-A- 20140 046 209
- KR-A- 20140 091 487
- US-A1- 2015 207 082

## Description

### CROSS-REFERENCE TO RELATED APPLICATION

This application claims priority to and the benefit of Korean Patent Application No. 10-2015-0022403 filed in the Korean Intellectual Property Office on February 13, 2015.

### BACKGROUND OF THE INVENTION

### (a) Field of the Invention

A compound for an organic optoelectronic device, an organic optoelectronic device, and a display device are disclosed.

### (b) Description of the Related Art

An organic optoelectronic device is a device that converts electrical energy into photoenergy, and vice versa.

An organic optoelectronic device may be classified as follows in accordance with its driving principles. One is a photoelectric device where excitons generated by photoenergy are separated into electrons and holes and the electrons and holes are transferred to different electrodes respectively and electrical energy is generated, and the other is a light emitting device to generate photoenergy from electrical energy by supplying a voltage or a current to electrodes.

Examples of the organic optoelectronic device include an organic photoelectric device, an organic light emitting diode, an organic solar cell, and an organic photo-conductor drum, and the like.

Among them, the organic light emitting diode (OLED) has recently drawn attention due to an increase in demand for flat panel displays. The organic light emitting diode converts electrical energy into light by applying current to an organic light emitting material, and has a structure in which an organic is interposed between an anode and a cathode. Herein, the organic layer may include an emission layer and optionally an auxiliary layer, and the auxiliary layer may include at least one layer selected from, for example a hole injection layer, a hole transport layer, an electron blocking layer, an electron transport layer, an electron injection layer, and a hole blocking layer in order to improve efficiency and stability of an organic light emitting diode.

Performance of an organic light emitting diode may be affected by characteristics of the organic layer, and among them, may be mainly affected by characteristics of an organic material of the organic layer.

Particularly, development for an organic material being capable of increasing hole and electron mobility and simultaneously increasing electrochemical stability is needed so that the organic light emitting diode may be applied to a large-size flat panel display.

### SUMMARY OF THE INVENTION

One embodiment provides a compound for an organic optoelectronic device having high efficiency and a long life-span.

Another embodiment provides a display device including the compound for an organic optoelectronic device.

Compounds for optoelectronical devices are inter alia disclosed in the KR 2014 0091487, KR 2014 0046209, JP 2008 074939, WO 2014/157599, EP 266890 EP 2711363, JP 2011 0843531, WO 2012/102967 and WO 2013/180376.

Yet another embodiment provides a display device including the organic optoelectronic device.

According to one embodiment, a compound for an organic optoelectronic device represented by Chemical Formula I is provided

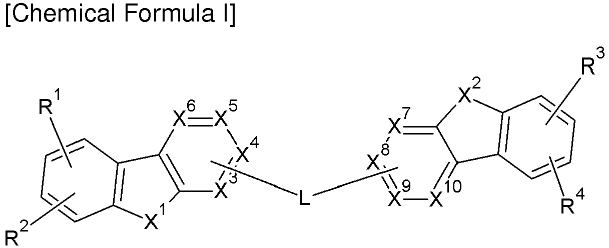

In Chemical Formula I,
X¹ and X² are each independently O or S,
X³ to X⁶ are each independently, C, CR^{a} or N,
at least two of X³ to X⁶ are N,
X⁷ to X¹⁰ are independently C, CR^{b} or N,
at least two of X⁷ to X¹⁰ are N,
L is a substituted or unsubstituted C6 to C30 arylene group, and
R¹ to R⁴, R^{a} and R^{b} are each independently hydrogen, deuterium, a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a substituted or unsubstituted C6 to C30 arylamine group, a substituted or unsubstituted C3 to C40 silyl group, a substituted or unsubstituted C1 to C30 alkylthiol group, a substituted or unsubstituted C6 to C30 arylthiol group, a halogen, a cyano group, a hydroxyl group, an amino group, a nitro group, or a combination thereof,
wherein "substituted" refers to that at least one hydrogen is replaced by deuterium, a halogen, a hydroxyl group, an amino group, a C1 to C30 amine group, a nitro group, a C1 to C40 silyl group, a C1 to C30 alkyl group, a C1 to C10 alkylsilyl group, a C3 to C30 cycloalkyl group, a C2 to C30 heterocycloalkyl group, a C6 to C30 aryl group, C2 to C30 heteroaryl group, a C1 to C20 alkoxy group, a fluoro group, a C1 to C10 trifluoroalkyl group, or a cyano group.

According to another embodiment, an organic optoelectronic device includes an anode and a cathode facing each other and at least one organic layer between the anode and the cathode, wherein the organic layer includes the compound for an organic optoelectronic device.

According to yet another embodiment, a display device including the organic optoelectronic device is provided.

An organic optoelectronic device having high efficiency long life-span may be realized.

### BRIEF DESCRIPTION OF THE DRAWINGS

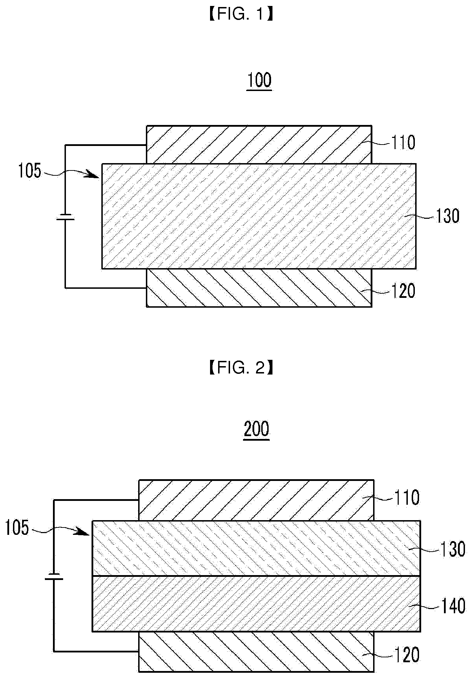

FIGS. 1 and 2 are cross-sectional views showing organic light emitting diodes according to one embodiment of the present invention.

### DETAILED DESCRIPTION

Hereinafter, embodiments of the present invention are described in detail. However, these embodiments are exemplary, the present invention is not limited thereto and the present invention is defined by the scope of claims.

As used herein, when a definition is not otherwise provided, the term "substituted" refers to one substituted with a deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C3 to C30 cycloalkyl group, a C3 to C30 heterocycloalkyl group, a C6 to C30 aryl group, a C6 to C30 heteroaryl group, a C1 to C20 alkoxy group, a fluoro group, a C1 to C10 trifluoroalkyl group such as a trifluoromethyl group, and the like, or a cyano group, instead of at least one hydrogen of a substituent or a compound.

In addition, two adjacent substituents of the substituted halogen, hydroxyl group, amino group, substituted or unsubstituted C1 to C30 amine group, nitro group, substituted or unsubstituted C1 to C40 silyl group, C1 to C30 alkyl group, C1 to C10 alkylsilyl group, C3 to C30 cycloalkyl group, C3 to C30 heterocycloalkyl group, C6 to C30 aryl group, C6 to C30 heteroaryl group, C1 to C20 alkoxy group, fluoro group, C1 to C10 trifluoroalkyl group such as trifluoromethyl group and the like, or cyano group may be fused with each other to form a ring. For example, the substituted C6 to C30 aryl group may be fused with another adjacent substituted C6 to C30 aryl group to form a substituted or unsubstituted fluorene ring.

In the present specification, when specific definition is not otherwise provided, the term "hetero" refers to one including 1 to 3 hetero atoms selected from N, O, S, P, and Si, and remaining carbons in one compound or substituent.

As used herein, when a definition is not otherwise provided, the term "alkyl group" may refer to an aliphatic hydrocarbon group. The alkyl group may refer to "a saturated alkyl group" without any double bond or triple bond.

The alkyl group may be a C1 to C30 alkyl group. More specifically, the alkyl group may be a C1 to C20 alkyl group or a C1 to C10 alkyl group. For example, a C1 to C4 alkyl group includes 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group, a cyclopropyl group, a cyclobutyl group, a cyclopentyl group, a cyclohexyl group, and the like.

As used herein, the term "aryl group" refers to a substituent including all element of the cycle having p-orbitals which form conjugation, and may be monocyclic, polycyclic or fused ring polycyclic (i.e., rings sharing adjacent pairs of carbon atoms) functional group, and also includes at least two aryl groups that are linked by a sigma bond directly such as a biphenyl group, a terphenyl group, quarterphenyl group, and the like.

As used herein, the term "heteroaryl group" may refer to aryl group including 1 to 3 hetero atoms selected from N, O, S, P, and Si and remaining carbons in one functional group. When the heterocyclic group is a fused ring, the entire ring or each ring of the heterocyclic group may include 1 to 3 heteroatoms, and include at least two heteroaryl groups that are linked by a sigma bond directly.

More specifically, the substituted or unsubstituted C6 to C30 aryl group and/or the substituted or unsubstituted C2 to C30 heteroaryl group may be a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted chrysenyl group, a substituted or unsubstituted triphenylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted fluorenyl group, a substituted or unsubstituted dibenzofuranyl group, a substituted or unsubstituted dibenzothiophenyl group, a substituted or unsubstituted carbazole group, or a combination thereof, but are not limited thereto.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electron formed in the cathode may be easily injected into the emission layer and transported in the emission layer due to conductive characteristics according to a lowest unoccupied molecular orbital (LUMO) level.

Hereinafter, a compound for an organic optoelectronic device according to one embodiment is described.

A compound for an organic optoelectronic device according to one embodiment is represented by Chemical Formula I.

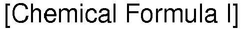

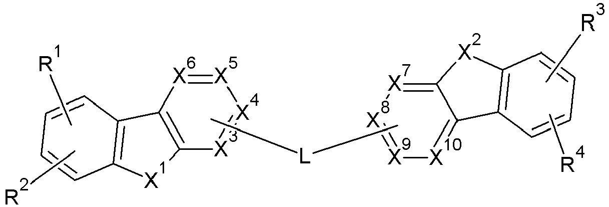

In Chemical Formula I,
X¹ and X² are each independently O or S,
X³ to X⁶ are independently, C, CR^{a} or N,
at least two of X³ to X⁶ are N,
X⁷ to X¹⁰ are independently, C, CR^{b} or N,
at least two of X⁷ to X¹⁰ are N,
L is a substituted or unsubstituted C6 to C30 arylene group, and
R¹ to R⁴, R^{a}, and R^{b} are each independently hydrogen, deuterium, a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a substituted or unsubstituted C6 to C30 arylamine group, a substituted or unsubstituted C3 to C40 silyl group, a substituted or unsubstituted C1 to C30 alkylthiol group, a substituted or unsubstituted C6 to C30 arylthiol group, a halogen, a cyano group, a hydroxyl group, an amino group, a nitro group, or a combination thereof,
wherein "substituted" refers to that at least one hydrogen is replaced by deuterium, a halogen, a hydroxyl group, an amino group, a C1 to C30 amine group, a nitro group, a C1 to C40 silyl group, a C1 to C30 alkyl group, a C1 to C10 alkylsilyl group, a C3 to C30 cycloalkyl group, a C2 to C30 heterocycloalkyl group, a C6 to C30 aryl group, C2 to C30 heteroaryl group, a C1 to C20 alkoxy group, a fluoro group, a C1 to C10 trifluoroalkyl group, or a cyano group.

The compound for an organic optoelectronic device represented by Chemical Formula I has a structure where two moieties selected from benzofuropyrimidine, benzofurotriazine, benzothienopyrimidine, and benzothienotriazine are linked by a linking group L.

The compound for an organic optoelectronic device has a effect of decreasing a driving voltage due to high electron mobility by strengthening a functional group transporting a charge and simultaneously, an effect of being suppressed from crystallization to have a small interaction among molecules due to steric hindrance by introducing a linking group or an additional expanding group, linking these two functional groups, and an functional group that is favorable to hole-transporting in a sterically different direction. Accordingly, the compound may have an effect of decreasing a driving voltage and thus lengthening current efficiency during manufacture of an organic optoelectronic device and also, increasing the life-span of the organic optoelectronic device.

In particular, the compound for an organic optoelectronic device represented by Chemical Formula I has a lower LUMO energy level than a compound including a moiety selected from benzofuropyridine respectively including N in X³ to X⁶ or X⁷ to X¹⁰ and benzothienylpyridine and thus excellent electron injection characteristics.

The compound for an organic optoelectronic device may be, for example represented by one of Chemical Formulae I-1 to I-8 depending on a bonding position of a linking group.

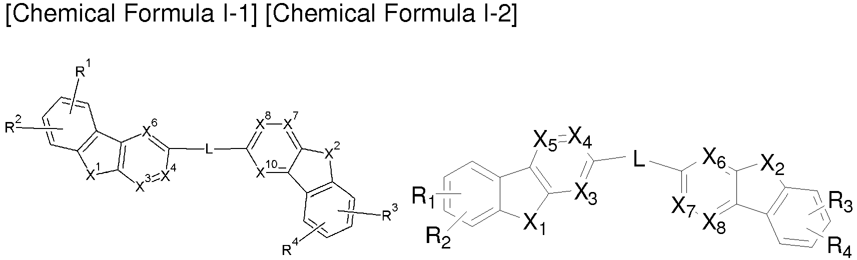

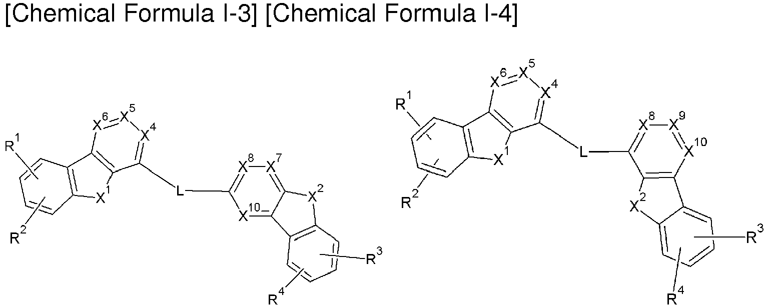

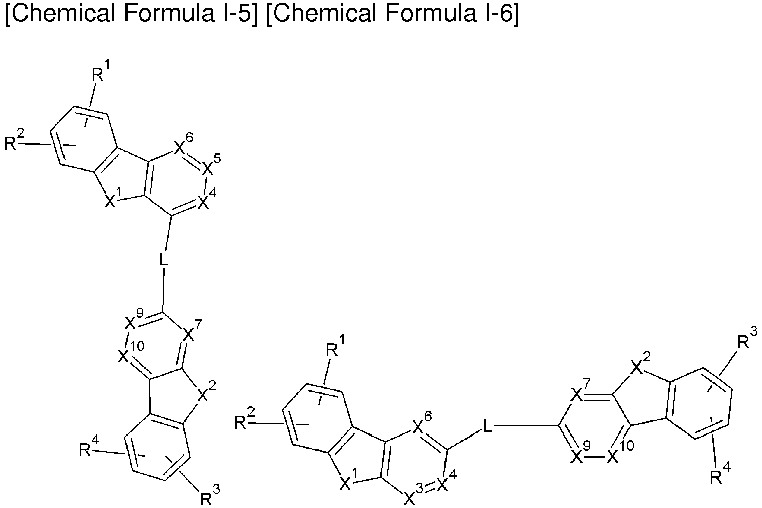

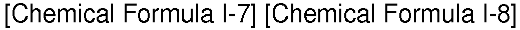

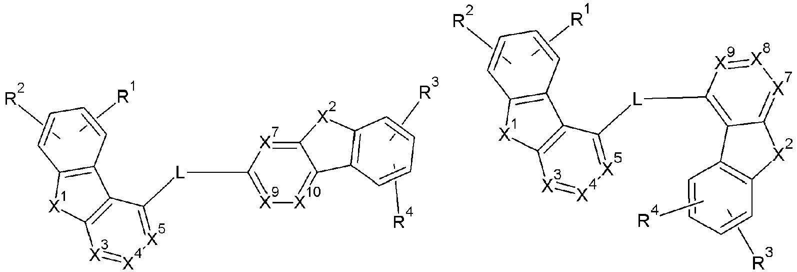

In Chemical Formulae I-1 to I-8, X¹ to X¹⁰ and R¹ to R⁴ and L are the same as described above.

The Chemical Formula I-1 may be represented by Chemical Formulae I-1a or I-1b.

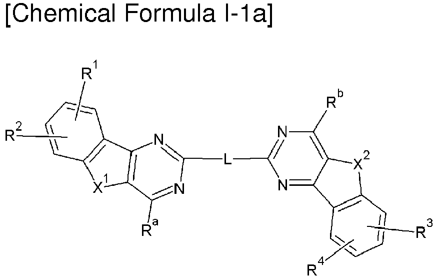

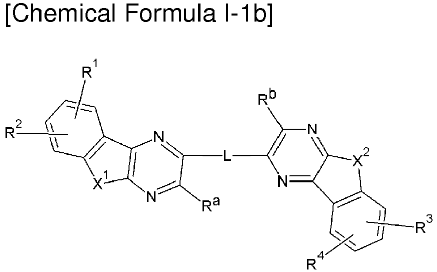

The Chemical Formula I-2 may be represented by Chemical Formula I-2a or I-2b.

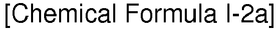

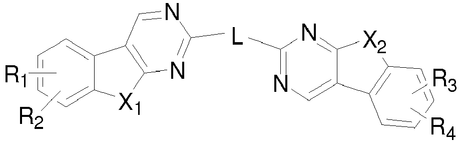

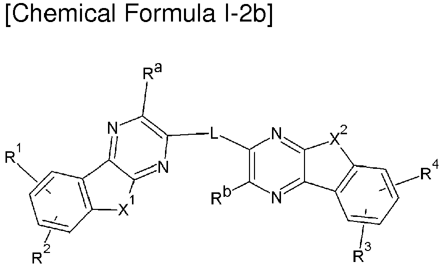

The Chemical Formula I-3 may be represented by Chemical Formula I-3a.

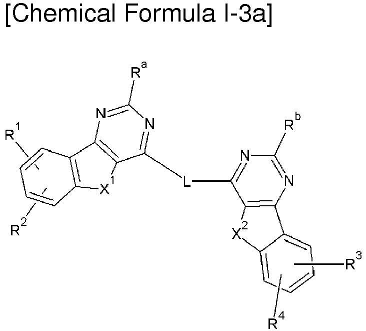

The Chemical Formula I-4 may be represented by Chemical Formula I-4a.

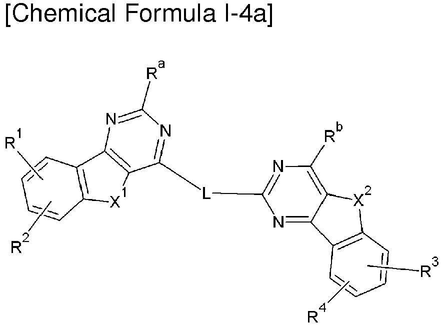

The Chemical Formula I-5 may be represented by Chemical Formula I-5a.

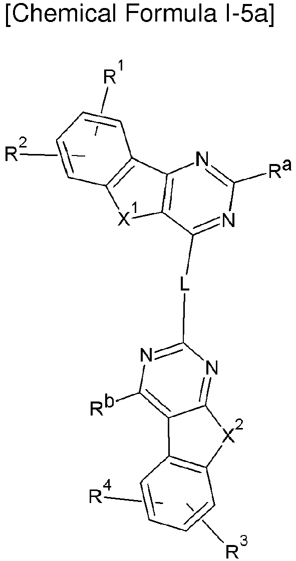

The Chemical Formula I-6 may be represented by Chemical Formula I-6a.

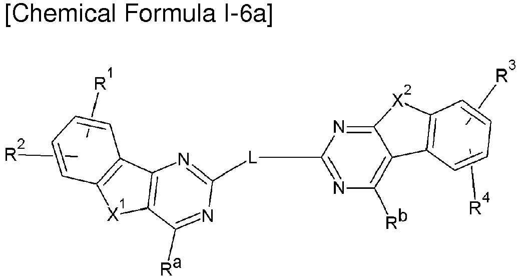

The Chemical Formula I-7 may be represented by Chemical Formula I-7a.

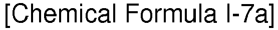

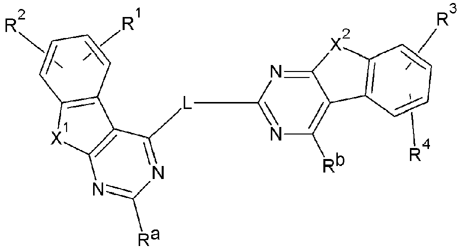

The Chemical Formula I-8 may be represented by Chemical Formula I-8a.

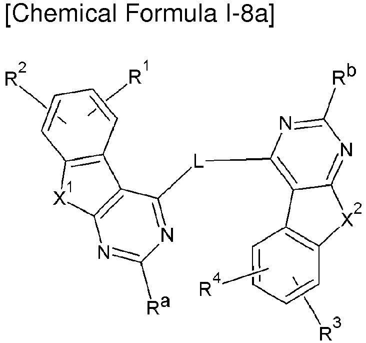

In Chemical Formulae I-1a to I-8a, I-1b and I-2b, X¹, X², L, and R¹ to R⁴ are the same as above,

R^{a} and R^{b} are each independently a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a substituted or unsubstituted C6 to C30 arylamine group, a substituted or unsubstituted C3 to C40 silyl group, a substituted or unsubstituted C1 to C30 alkylthiol group, a substituted or unsubstituted C6 to C30 arylthiol group, a halogen, a cyano group, a hydroxyl group, an amino group, a nitro group, or a combination thereof.

When R^{a} and R^{b} are substituted with the substituent except hydrogen, stability for excitons, holes, and charges may be improved.

In one embodiment, the linking group L bonded at a meta position may be, for example represented by Chemical Formula I-9.

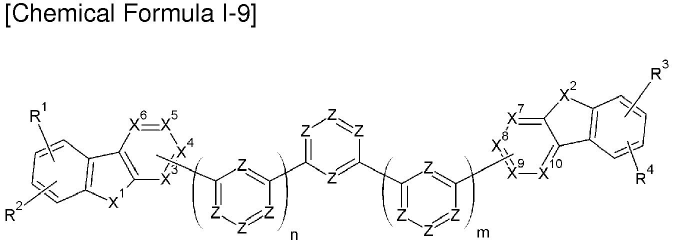

In another embodiment, the linking group L bonded at an ortho position may be, for example represented by Chemical Formula 1-10.

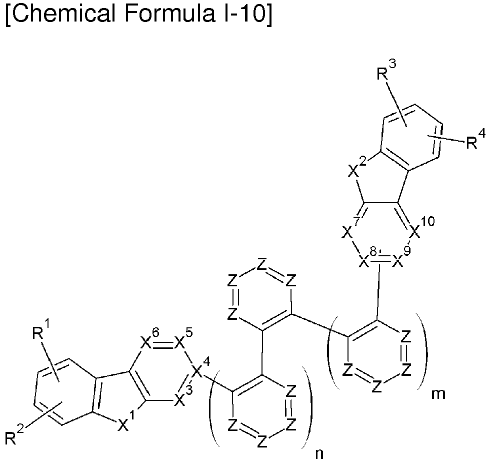

In another embodiment, the linking group L bonded at ortho and meta positions may be, for example represented by Chemical Formula 1-11.

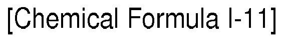

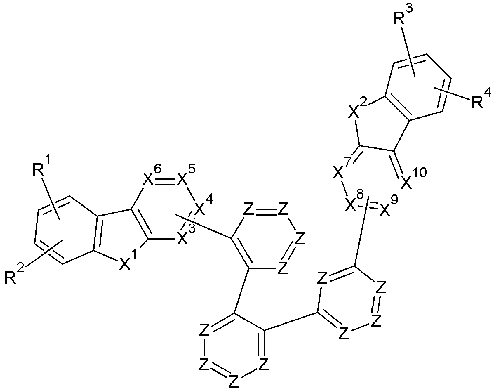

In another embodiment, the linking group L bonded at a para position may be, for example represented by Chemical Formula 1-12.

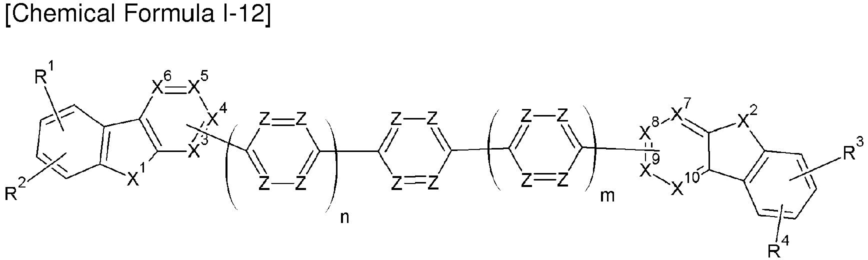

In another embodiment, the linking group L bonded at para and meta positions may be, for example represented by Chemical Formula 1-13.

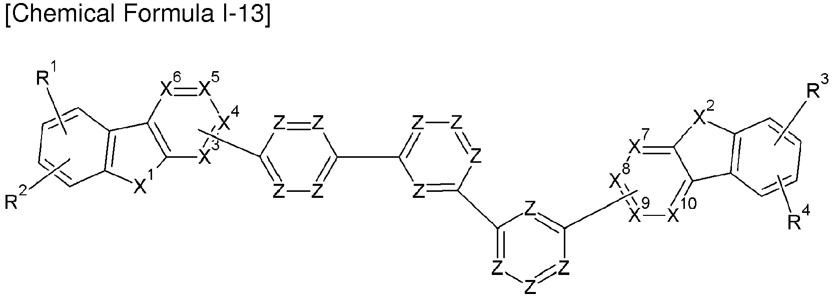

In Chemical Formulae I-9 to 1-13,
X¹ and X² are each independently O or S,
X³ to X⁶ are independently C, CR^{a} or N,
at least two of X³ to X⁶ are N,
X⁷ to X¹⁰ are independently, C, CR^{b} or N,
at least two of X⁷ to X¹⁰ are N,
Z is CR^{c}
R¹ to R⁴, R^{a}, R^{b} and R^{c} are each independently hydrogen, deuterium, a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a substituted or unsubstituted C6 to C30 arylamine group, a substituted or unsubstituted C3 to C40 silyl group, a substituted or unsubstituted C1 to C30 alkylthiol group, a substituted or unsubstituted C6 to C30 arylthiol group, a halogen, a cyano group, a hydroxyl group, an amino group, a nitro group, or a combination thereof, and
n and m are independently integers ranging from 0 to 2,
wherein "substituted" refers to that at least one hydrogen is replaced by deuterium, a halogen, a hydroxyl group, an amino group, a C1 to C30 amine group, a nitro group, a C1 to C40 silyl group, a C1 to C30 alkyl group, a C1 to C10 alkylsilyl group, a C3 to C30 cycloalkyl group, a C2 to C30 heterocycloalkyl group, a C6 to C30 aryl group, C2 to C30 heteroaryl group, a C1 to C20 alkoxy group, a fluoro group, a C1 to C10 trifluoroalkyl group, or a cyano group.

Specifically, at least one of n and m of the above Chemical Formula I-9 to 1-13 may be 1, and R^{a} and R^{b} of Chemical Formula I-9 to 1-13 are each independently a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a substituted or unsubstituted C6 to C30 arylamine group, a substituted or unsubstituted C3 to C40 silyl group, a substituted or unsubstituted C1 to C30 alkylthiol group, a substituted or unsubstituted C6 to C30 arylthiol group, a halogen, a cyano group, a hydroxyl group, an amino group, a nitro group, or a combination thereof.

According to one embodiment of the present invention, when two or more aryl groups and/or heteroaryl groups linked at a meta position are present as a linking group, a compound including the same is not easily crystallized due to excellent film characteristics and thus brings about stability during operation of a device.

In addition, when the linking group is substituted at an ortho position rather than the meta position according to another embodiment of the present invention, the linking group does not only suppress crystallization due to steric hindrance but also has an effect of improving processibility such as a deposition process, a solution process, and the like as a molecule size becomes smaller, and simultaneously, a glass transition temperature becomes higher and also, increases electron mobility, as functional groups playing a role of transporting electrons between linking groups becomes closer to each other.

The L is a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted quarterphenyl group, a substituted or unsubstituted chrysenyl group, a substituted or unsubstituted triphenylenyl group, a substituted or unsubstituted perylenyl group, a substituted or unsubstituted fluorenyl group, or a combination thereof. For example, L may be selected from the linking groups listed in Group 1.

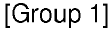

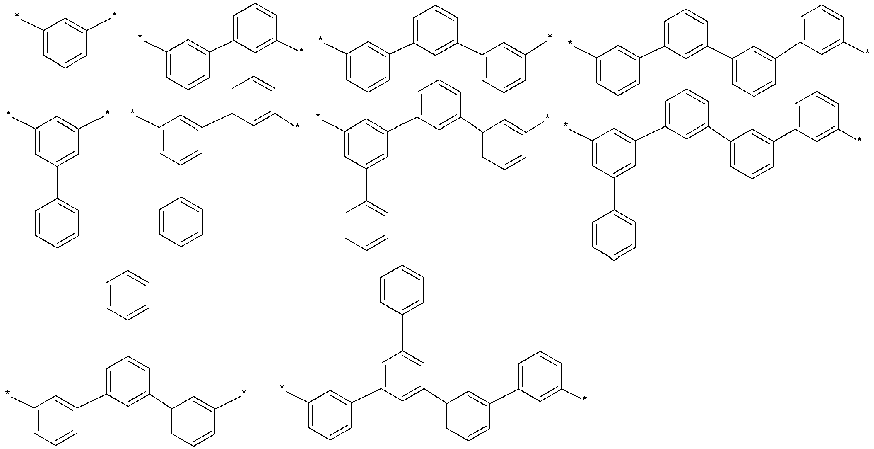

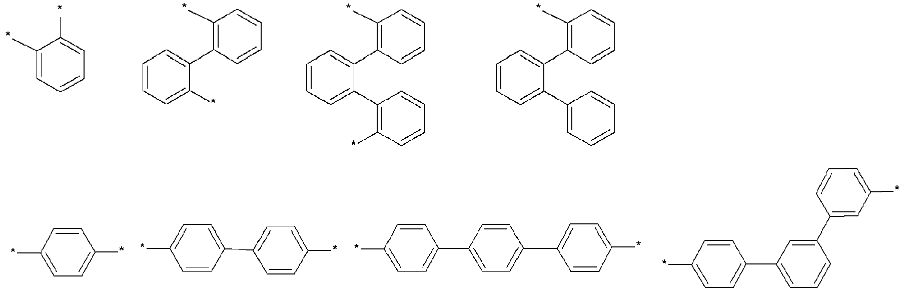

In Group 1, * is a linking point.

The compound for an organic optoelectronic device may be, for example compounds in Group 2, but is not limited thereto.

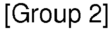

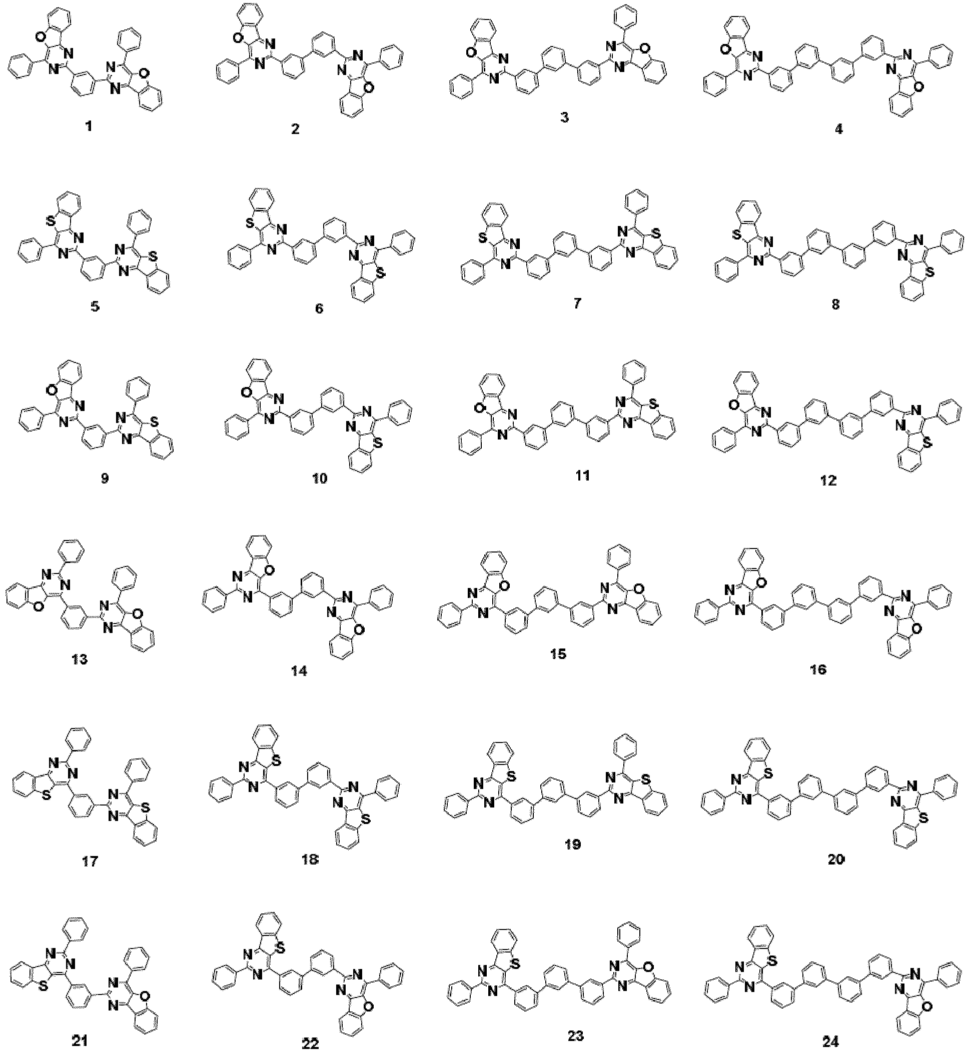

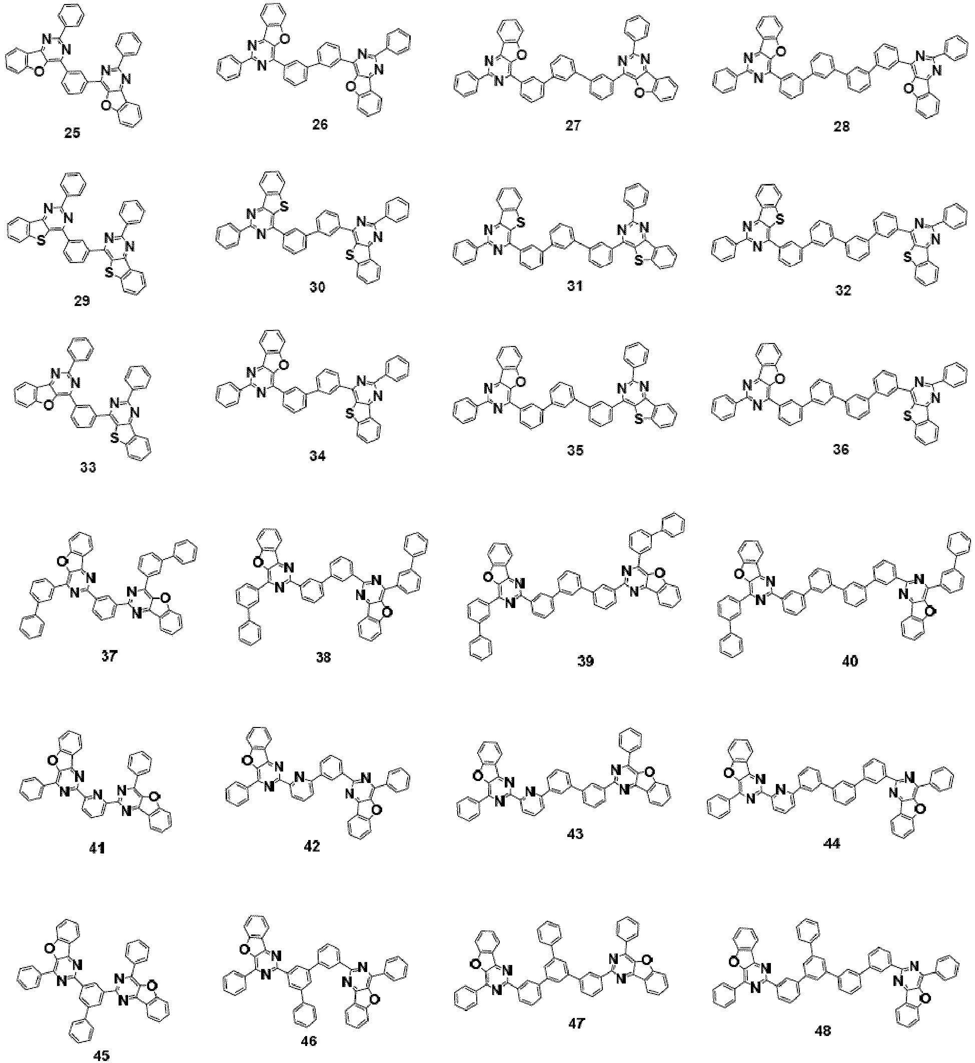

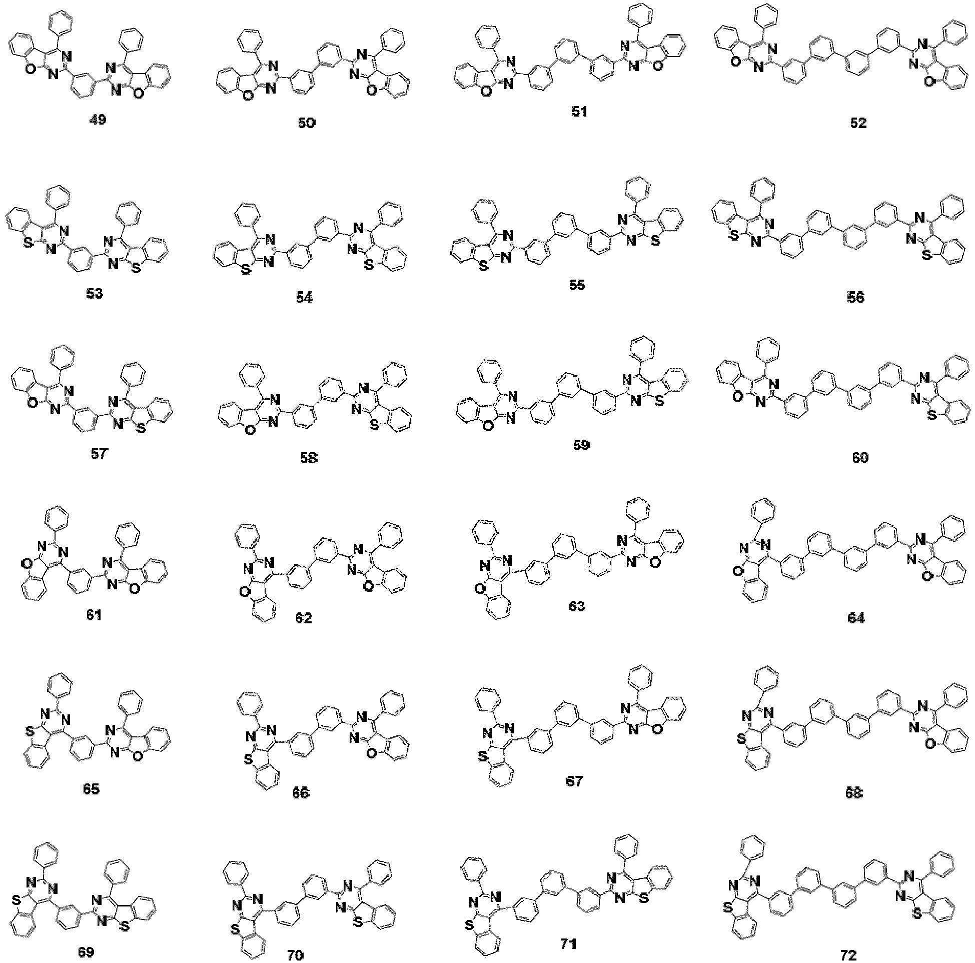

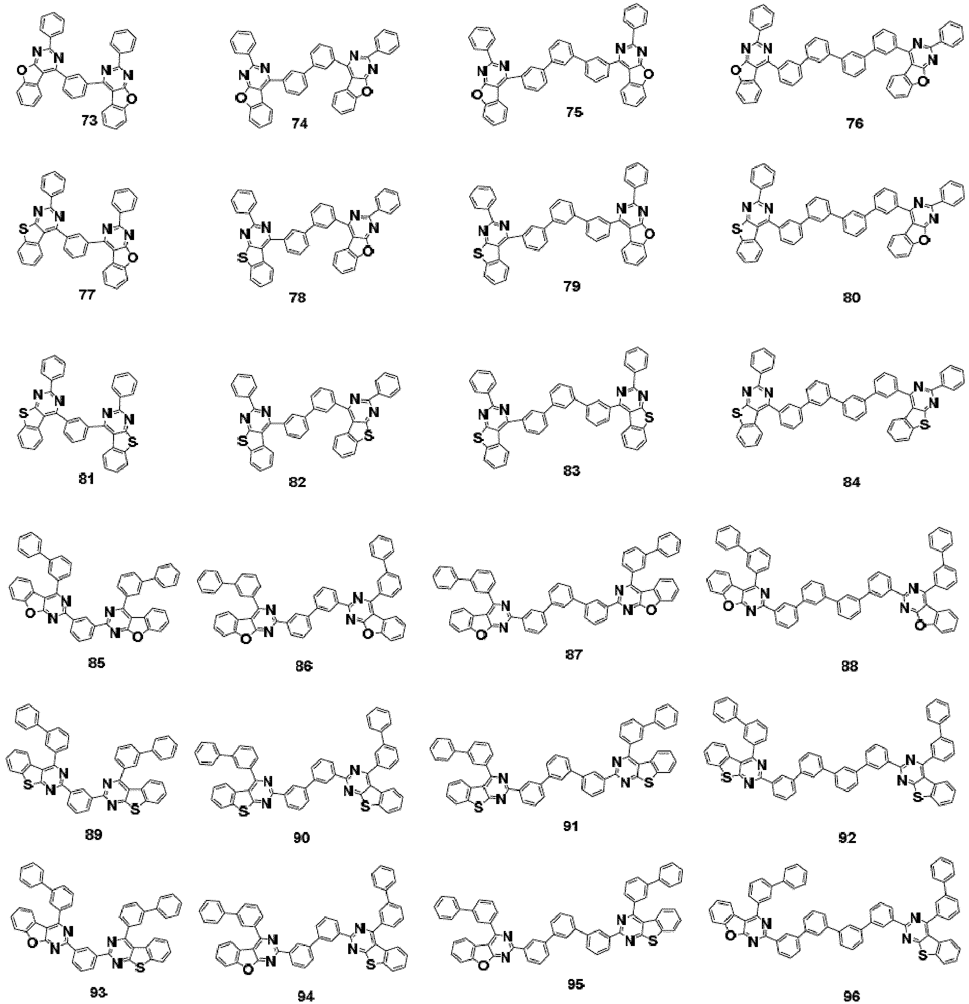

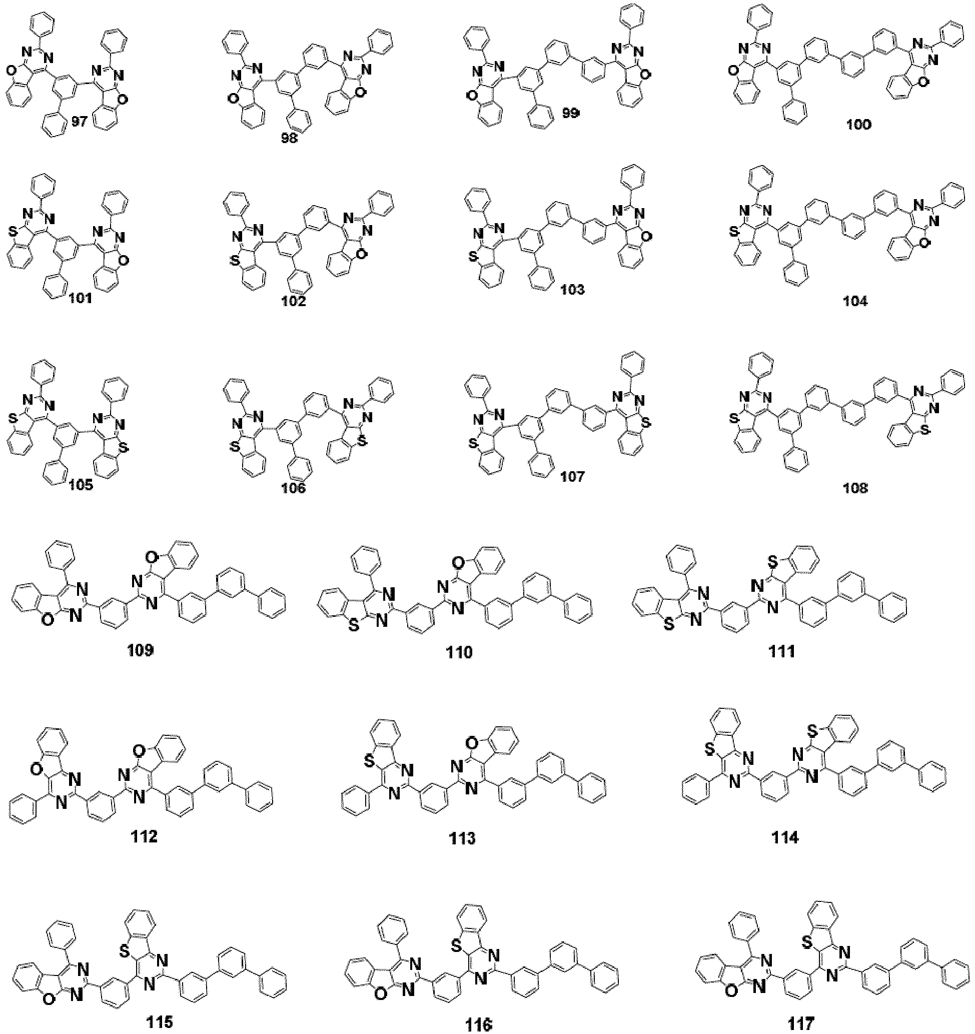

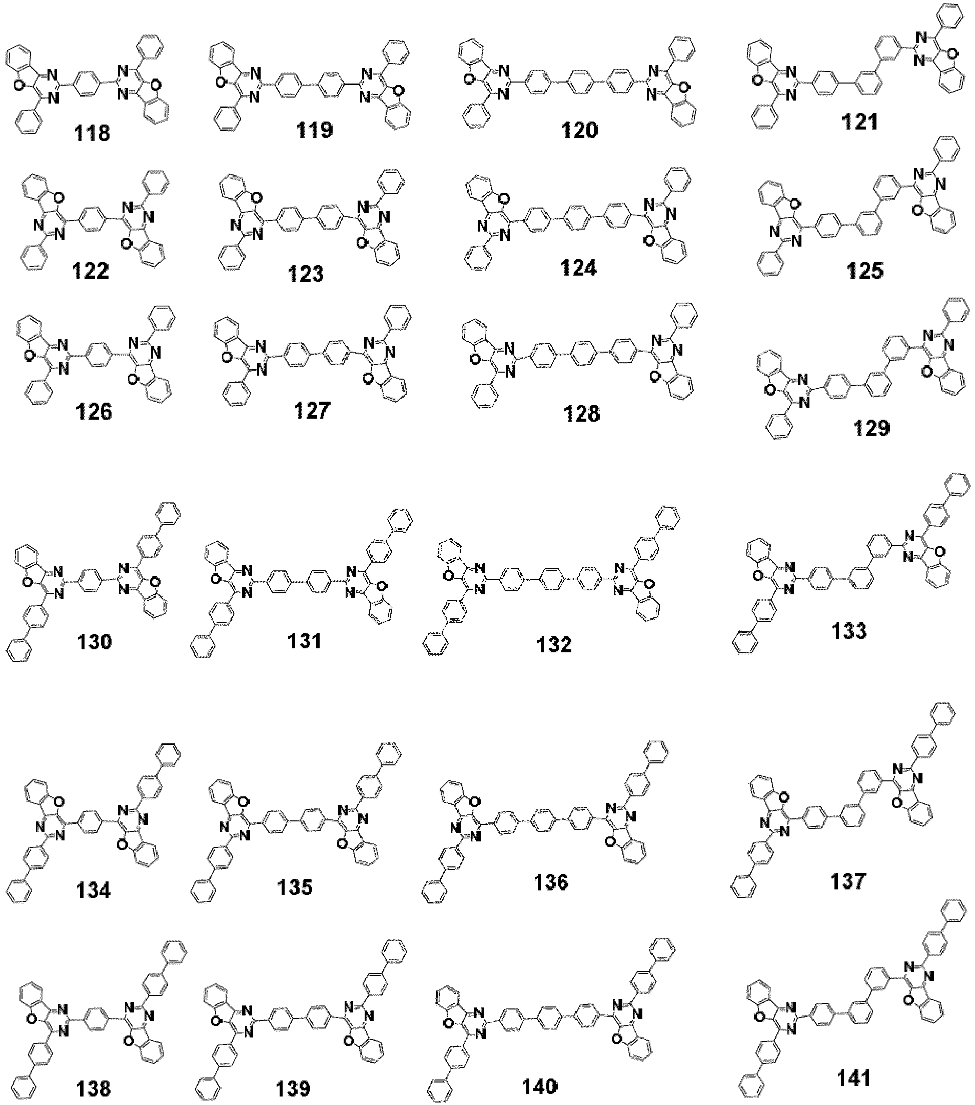

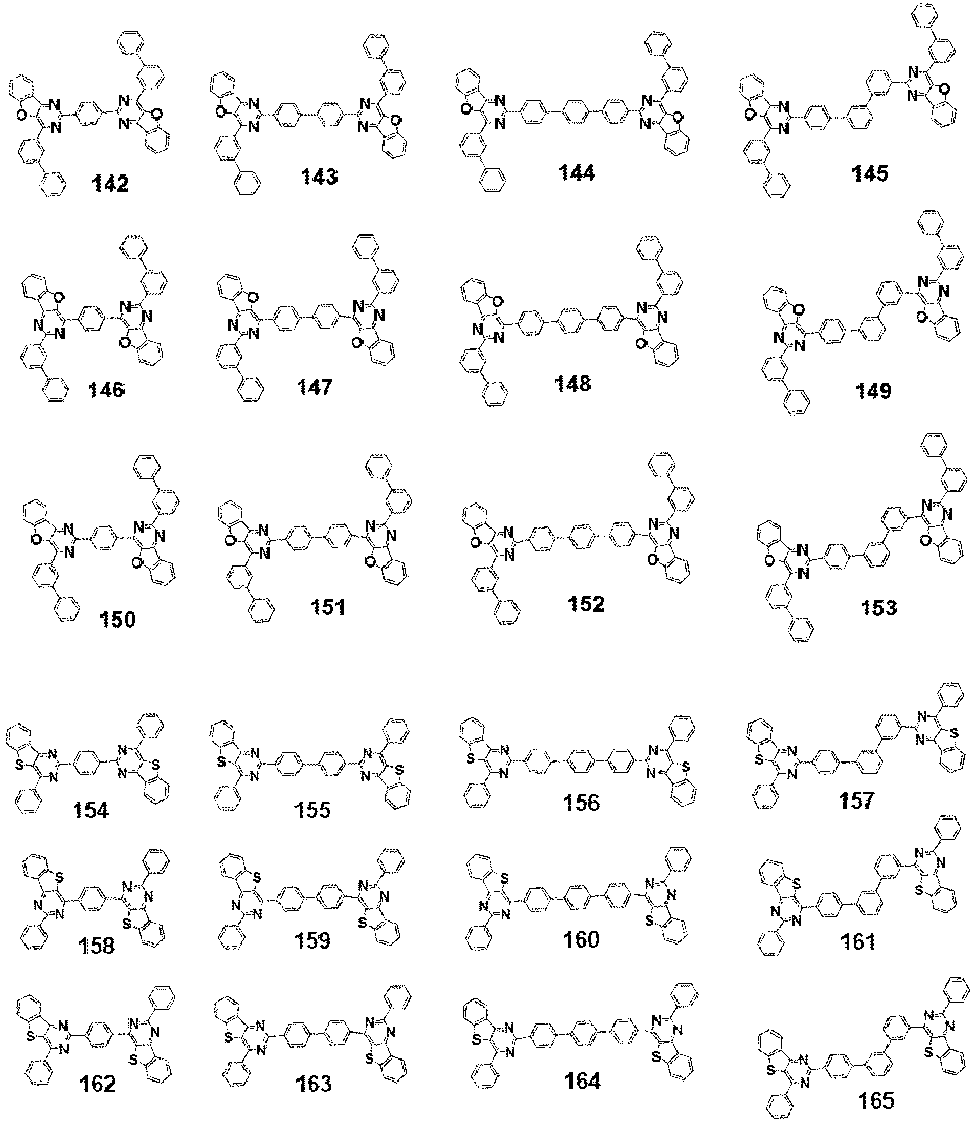

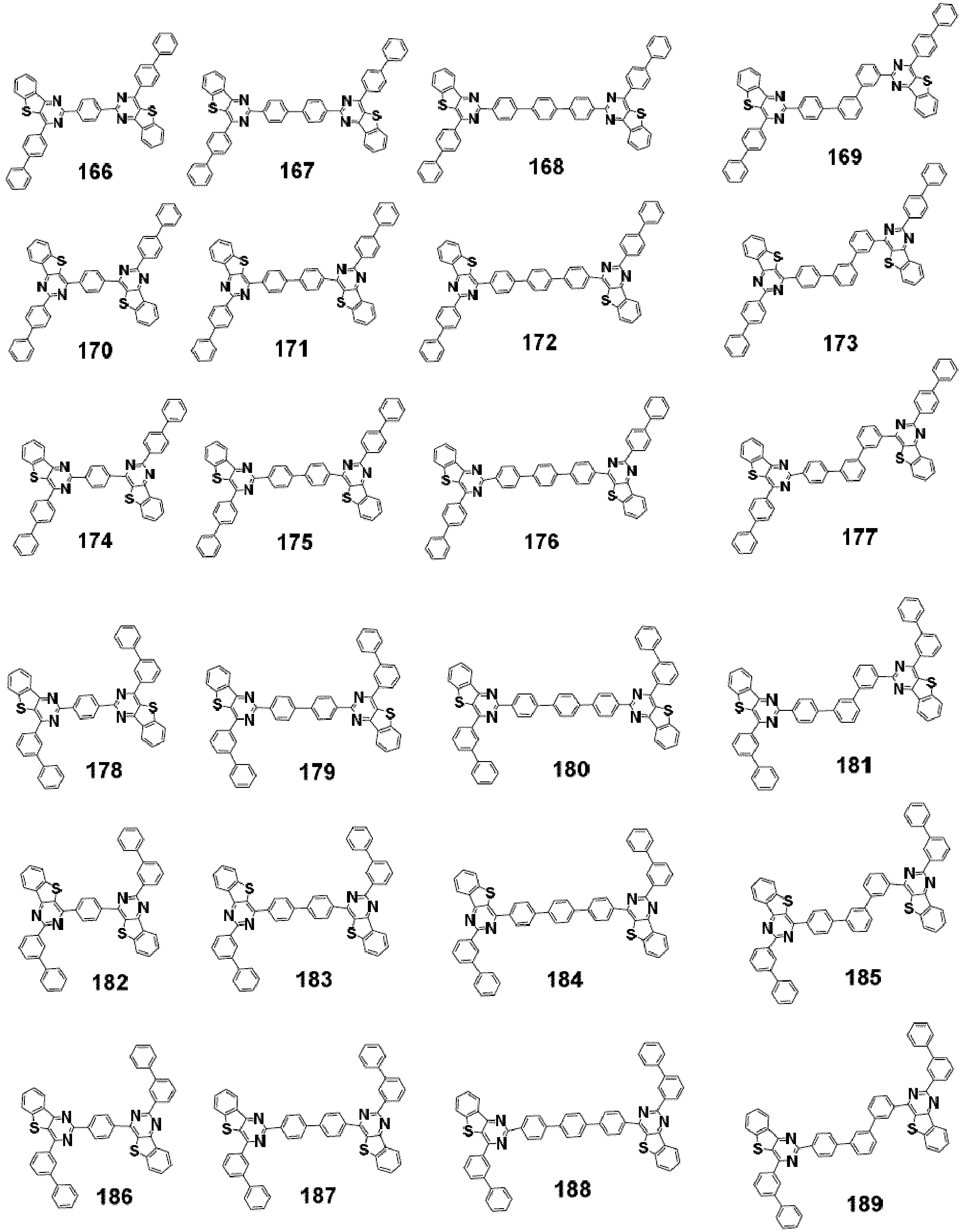

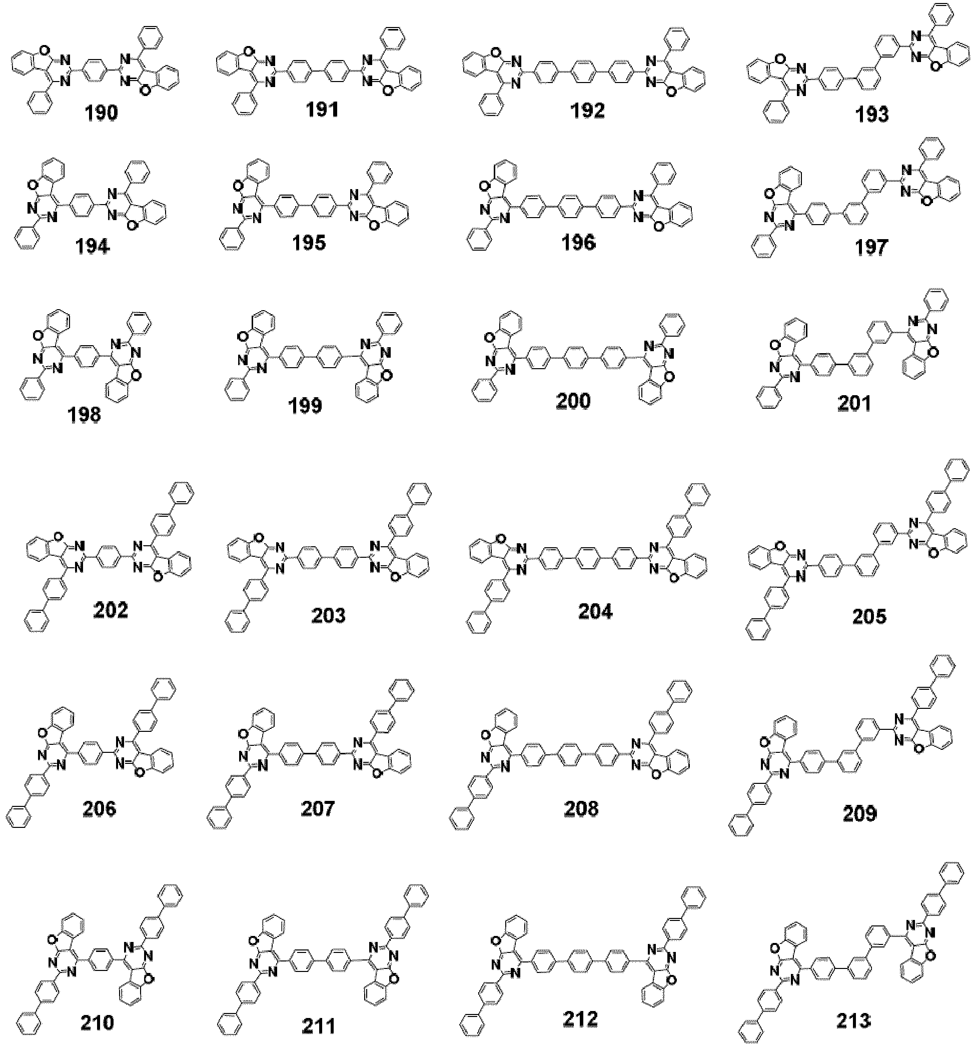

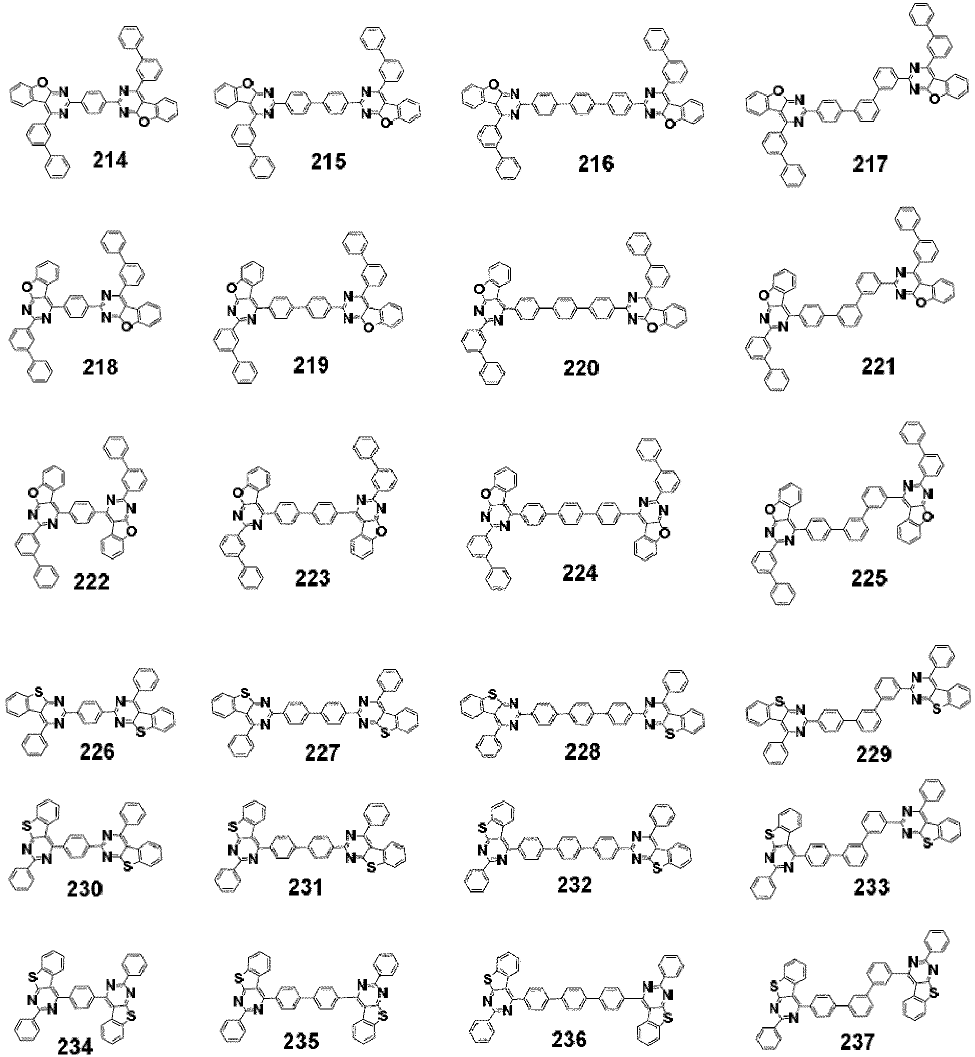

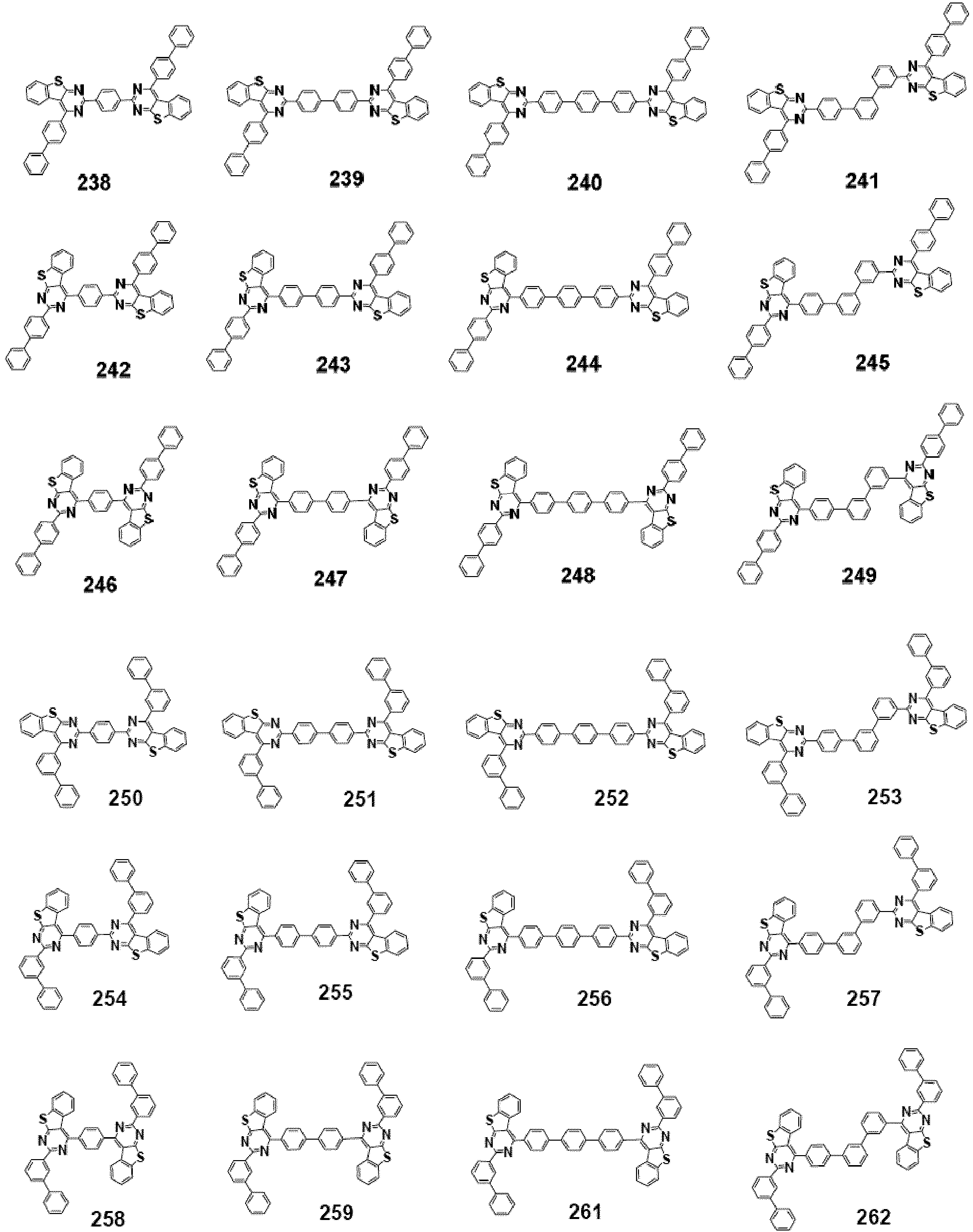

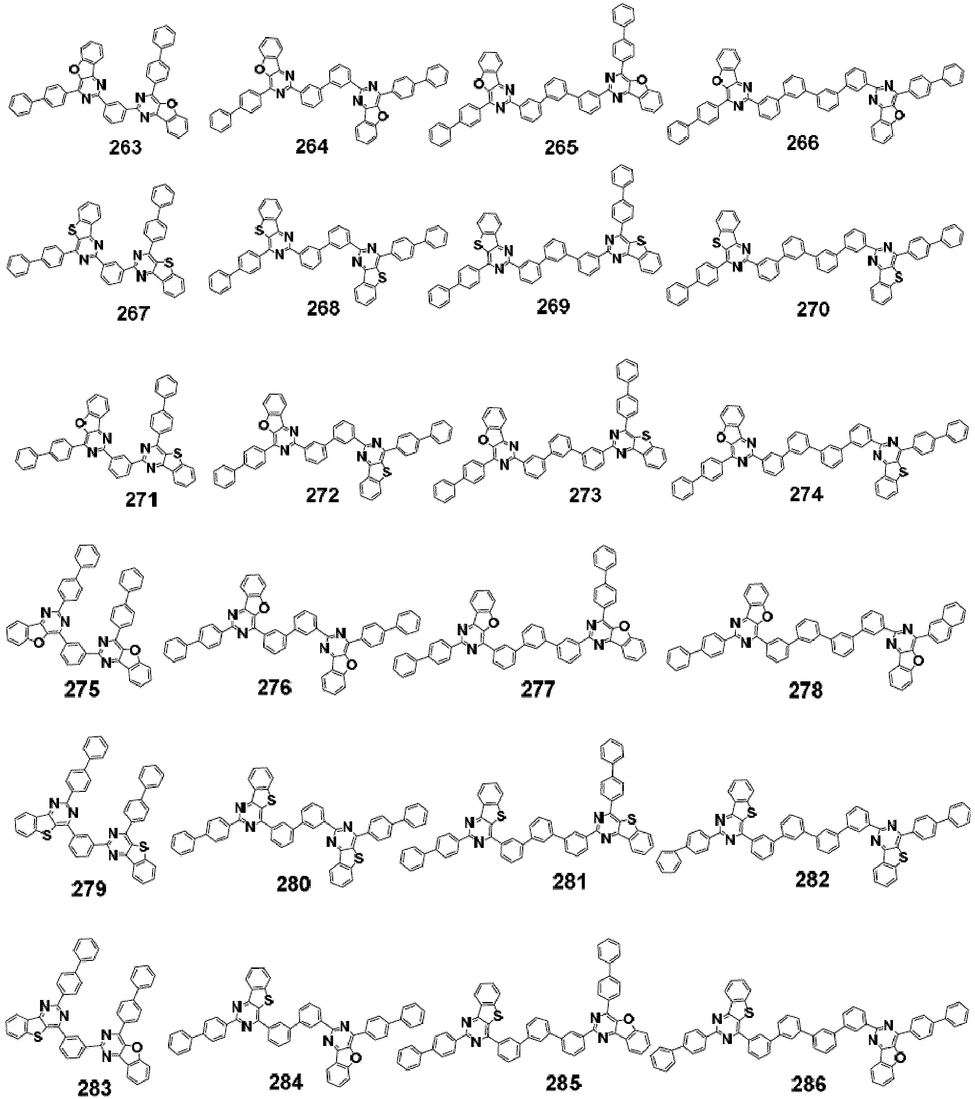

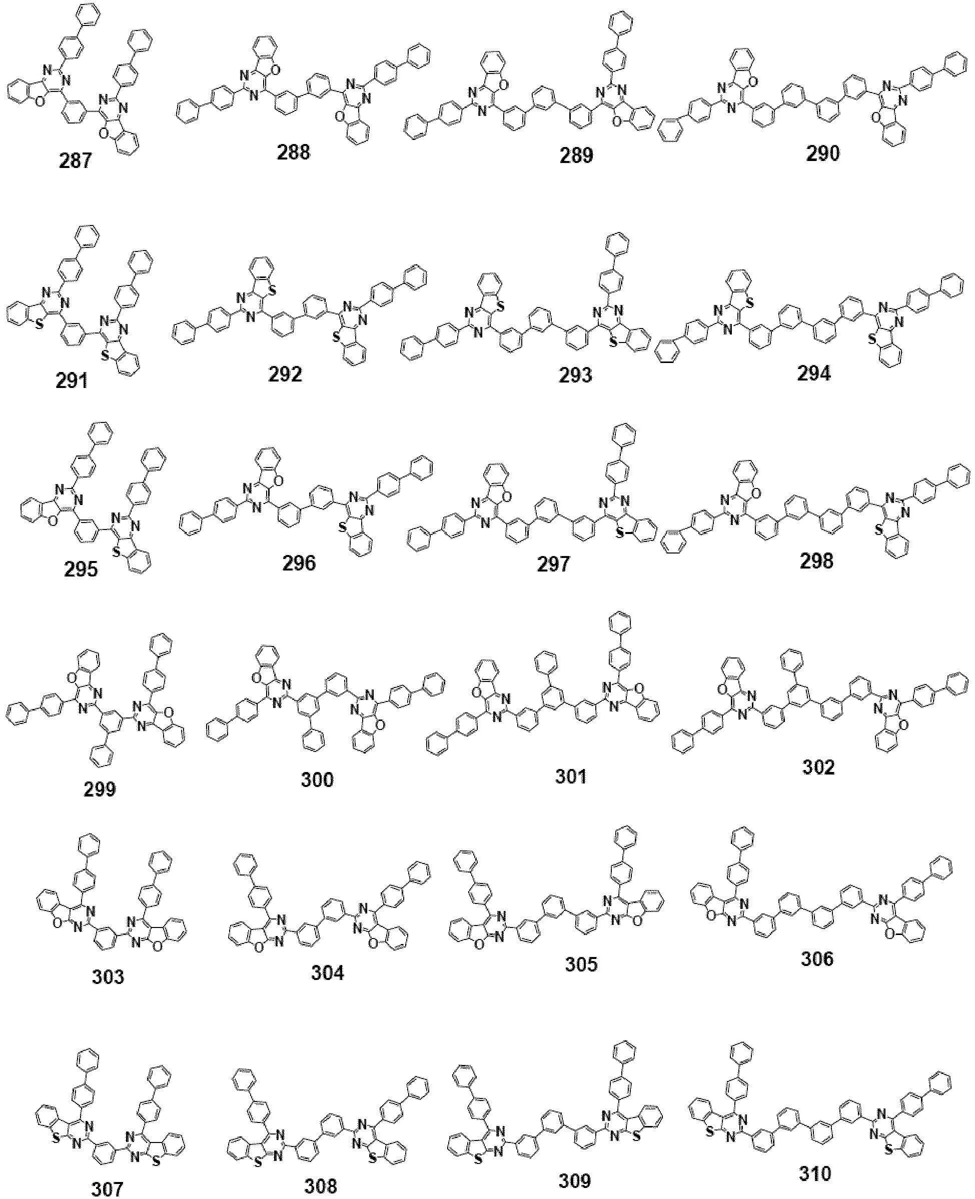

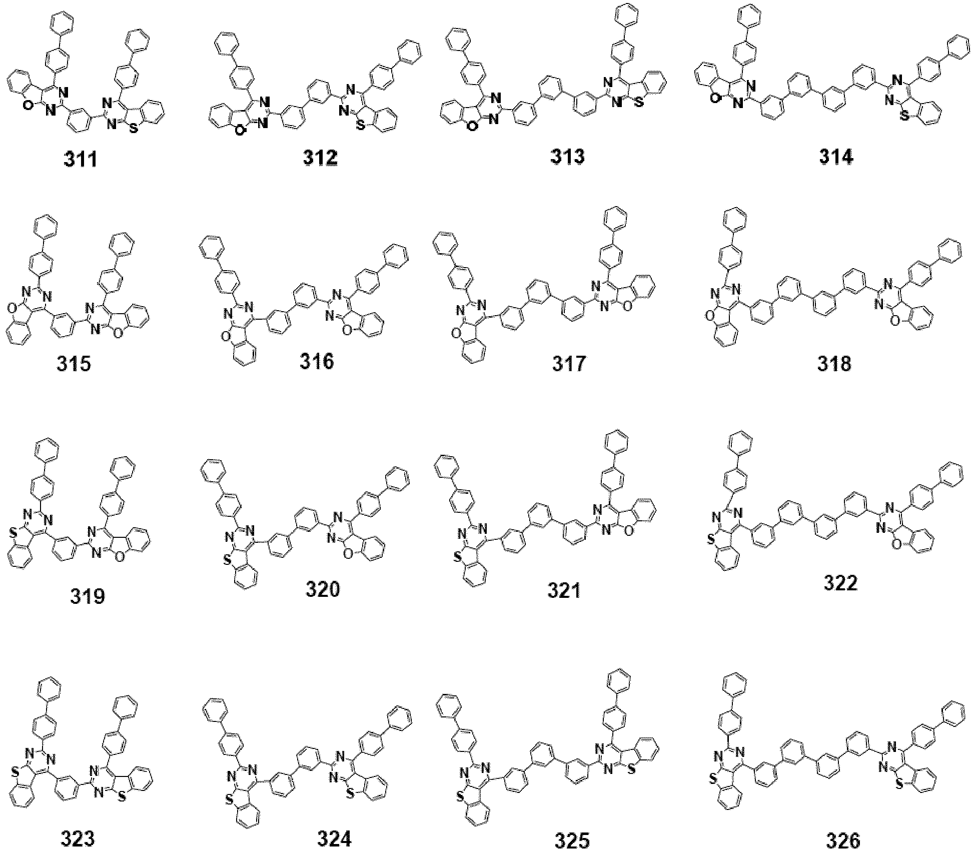

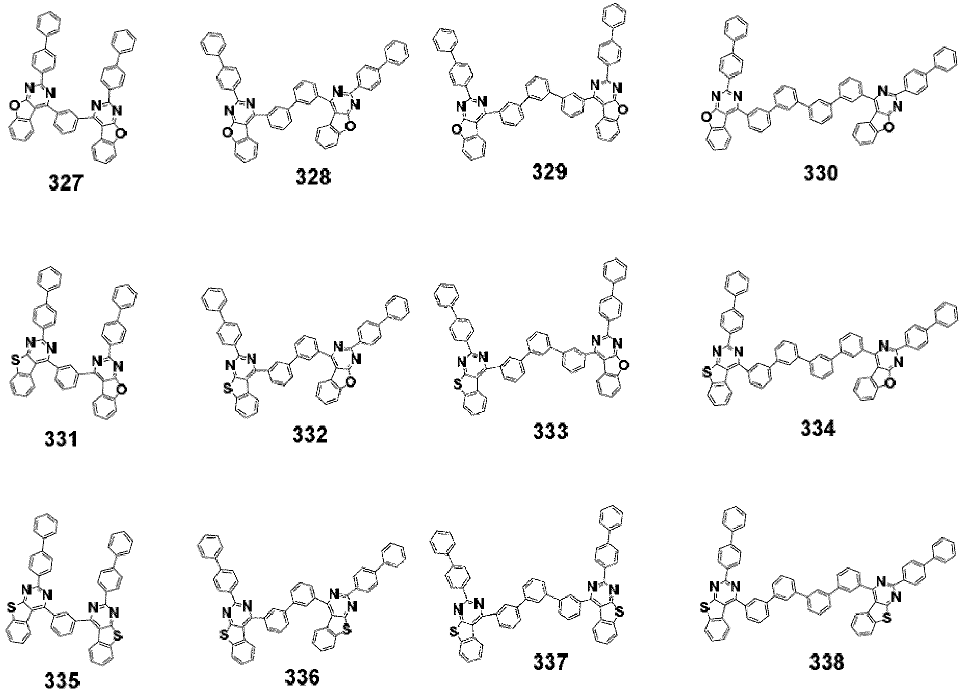

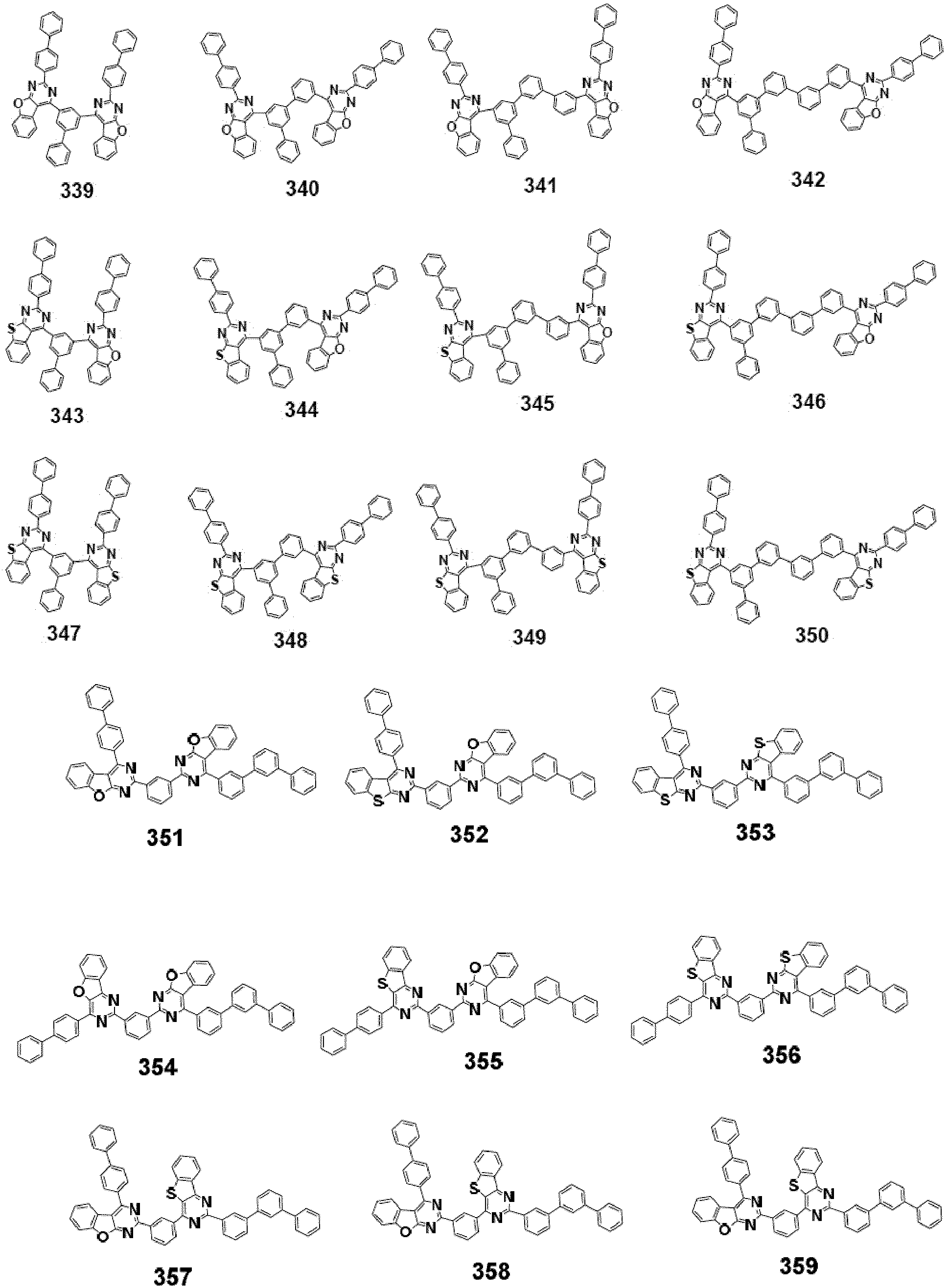

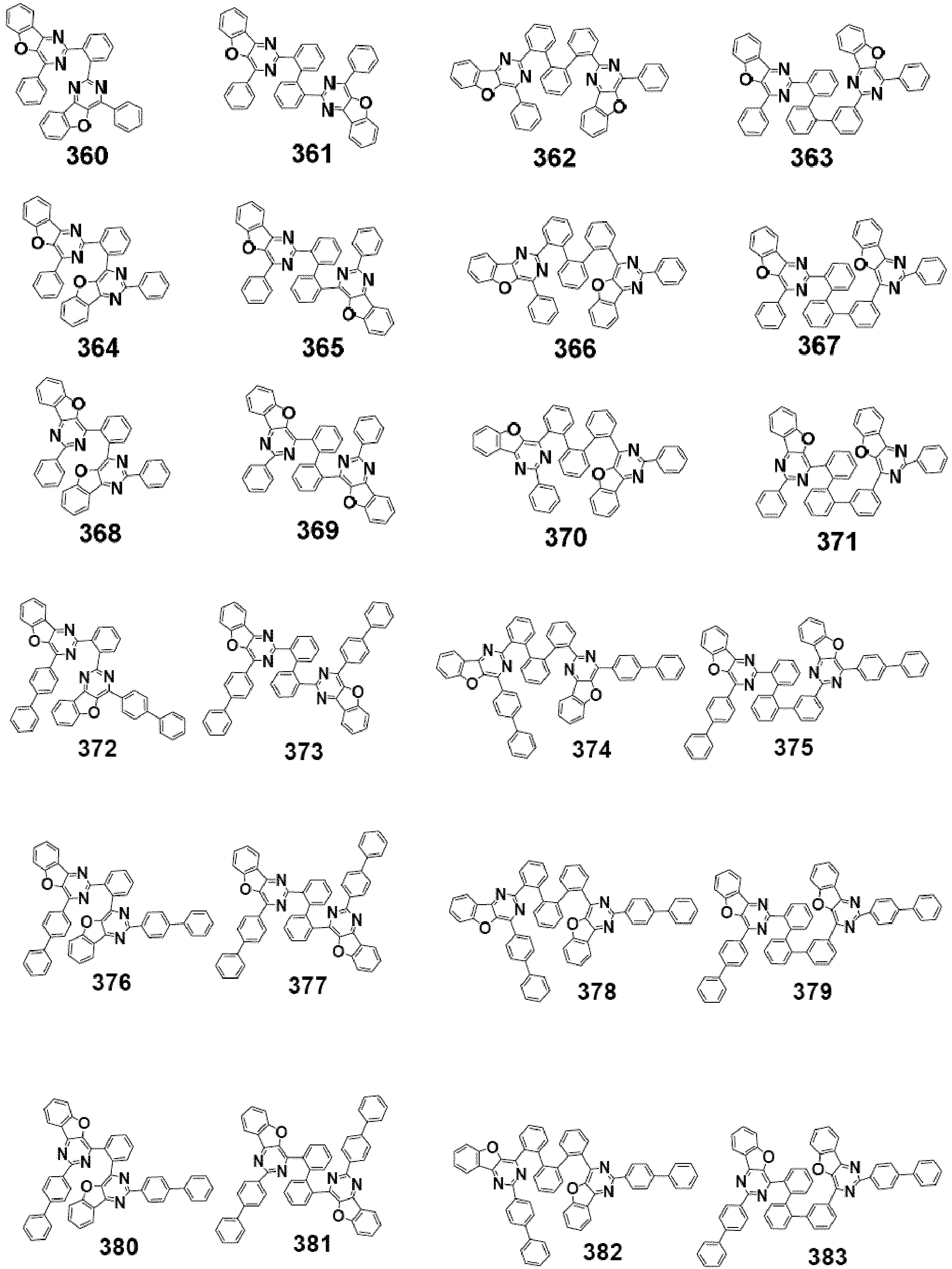

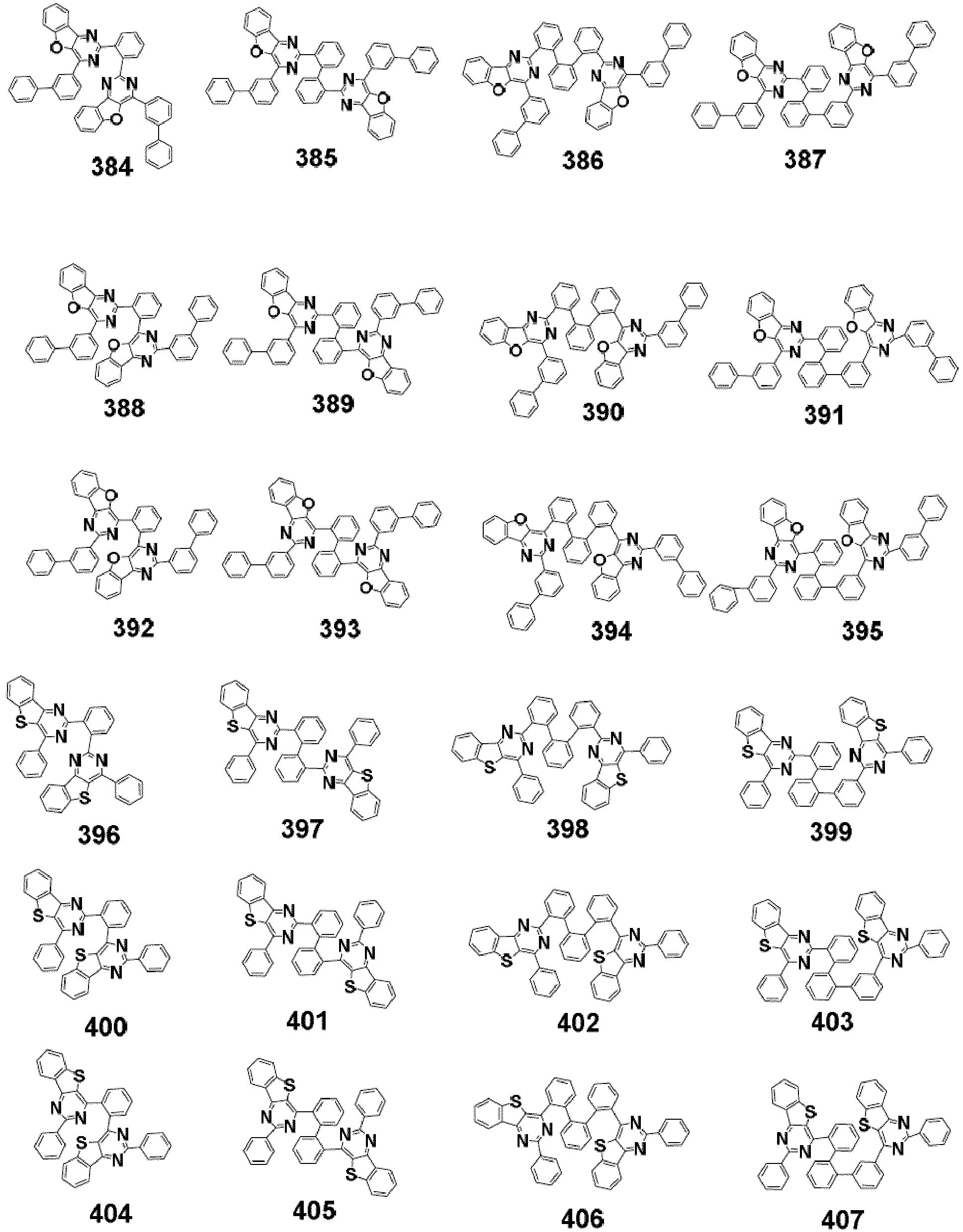

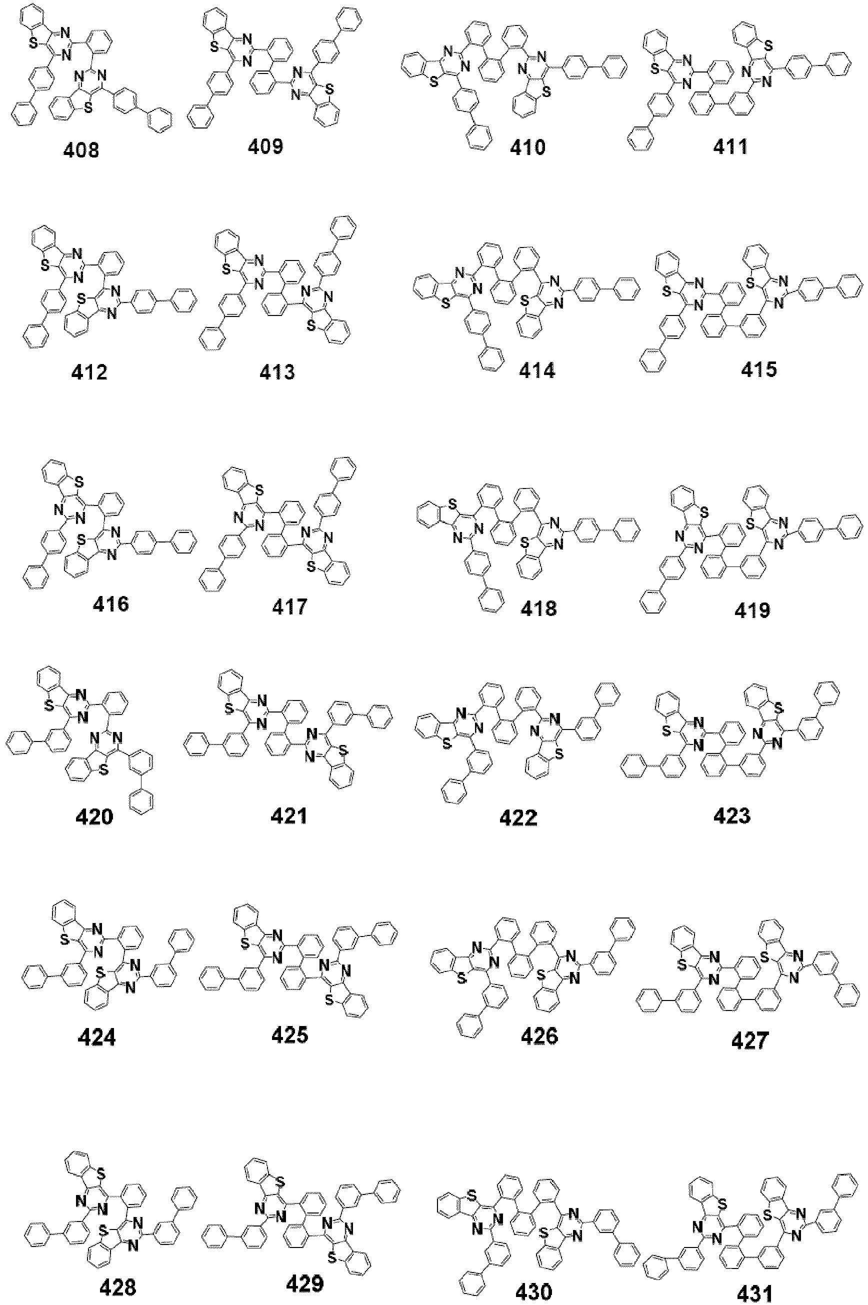

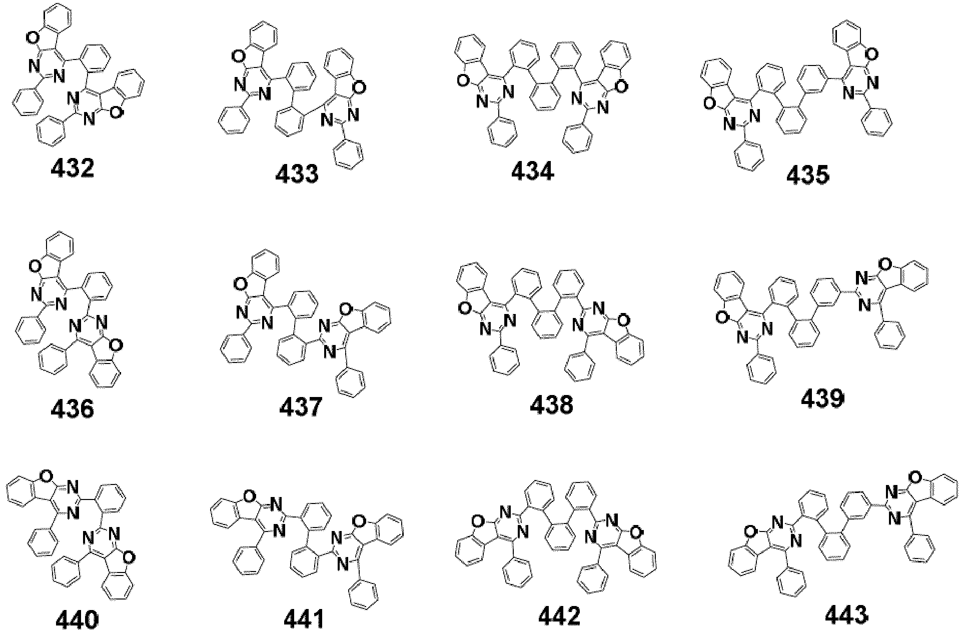

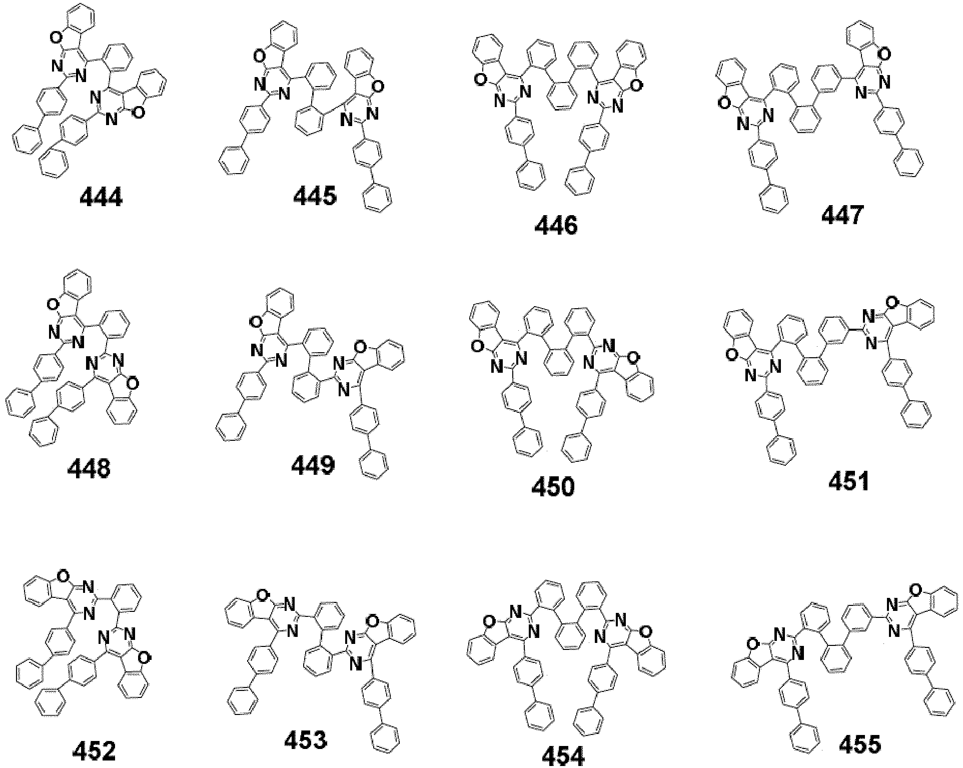

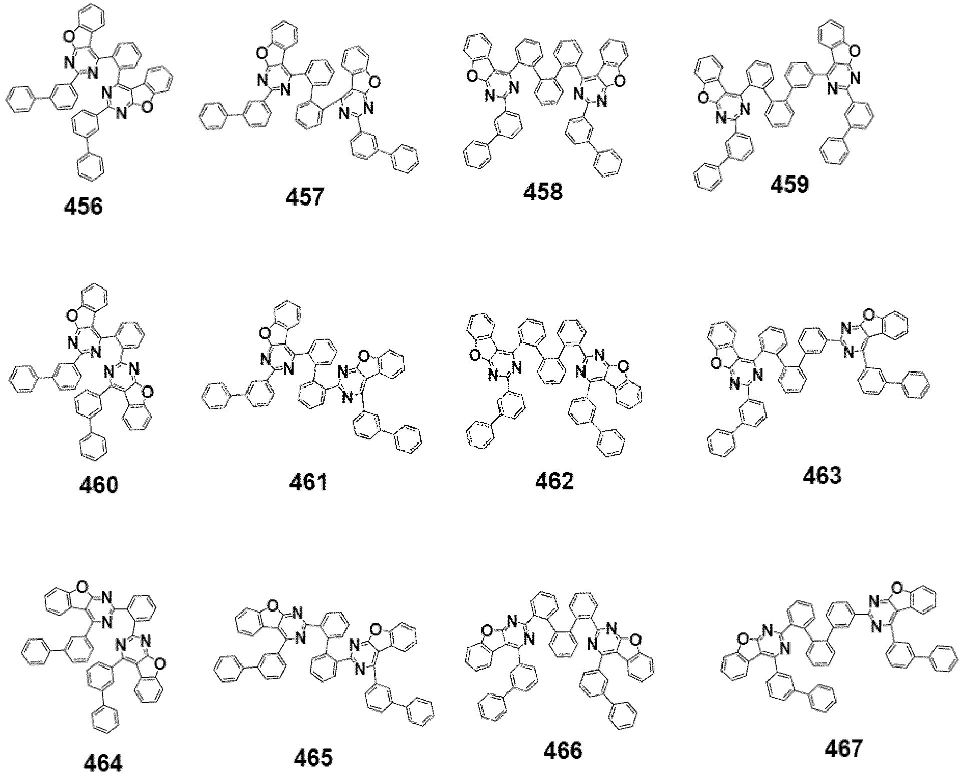

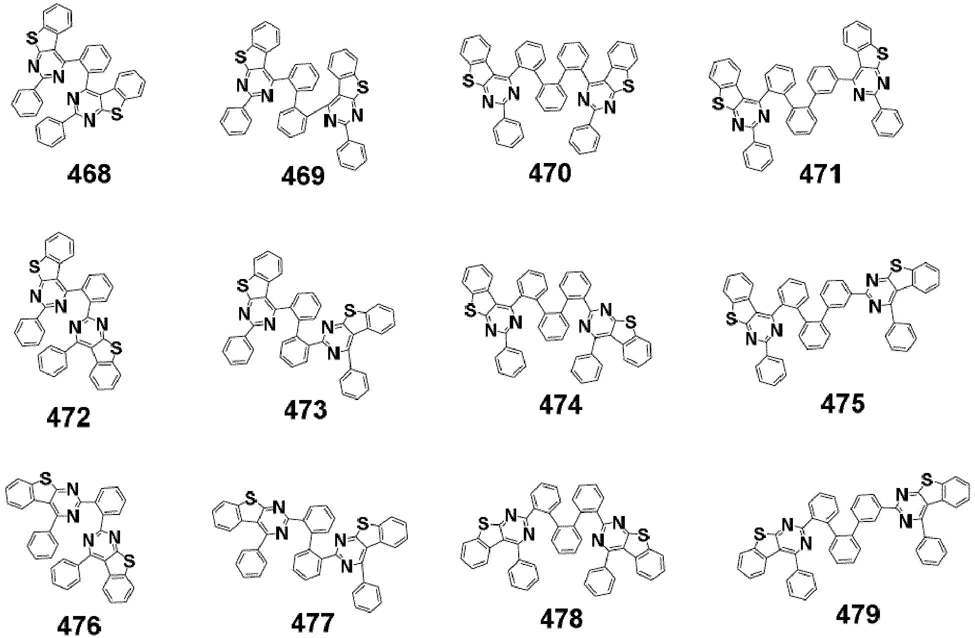

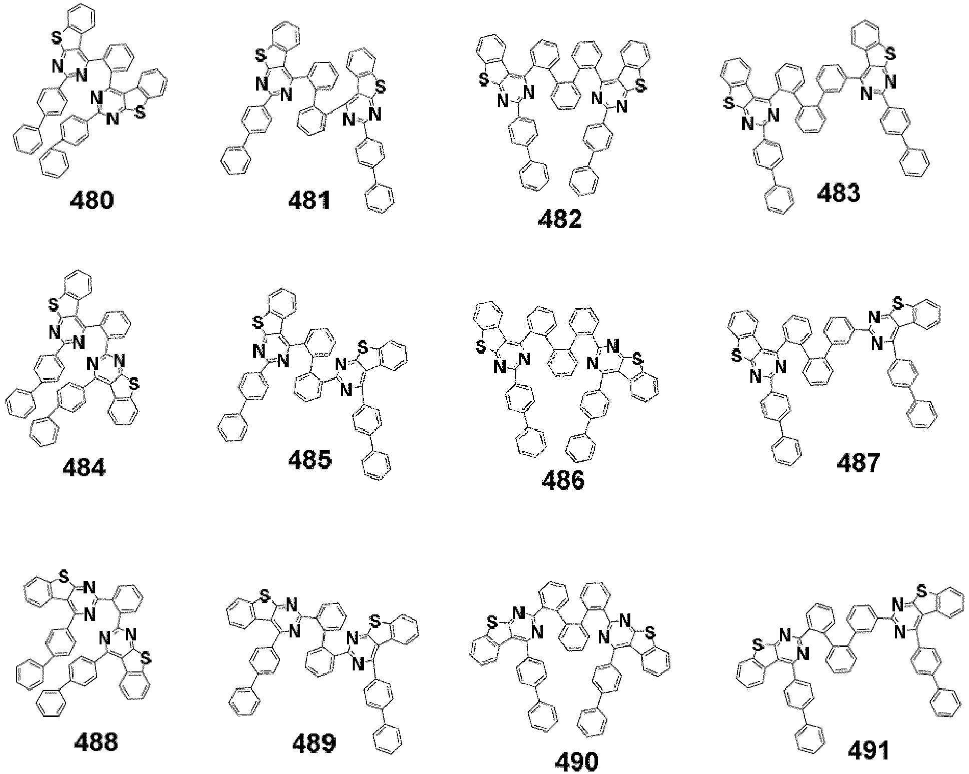

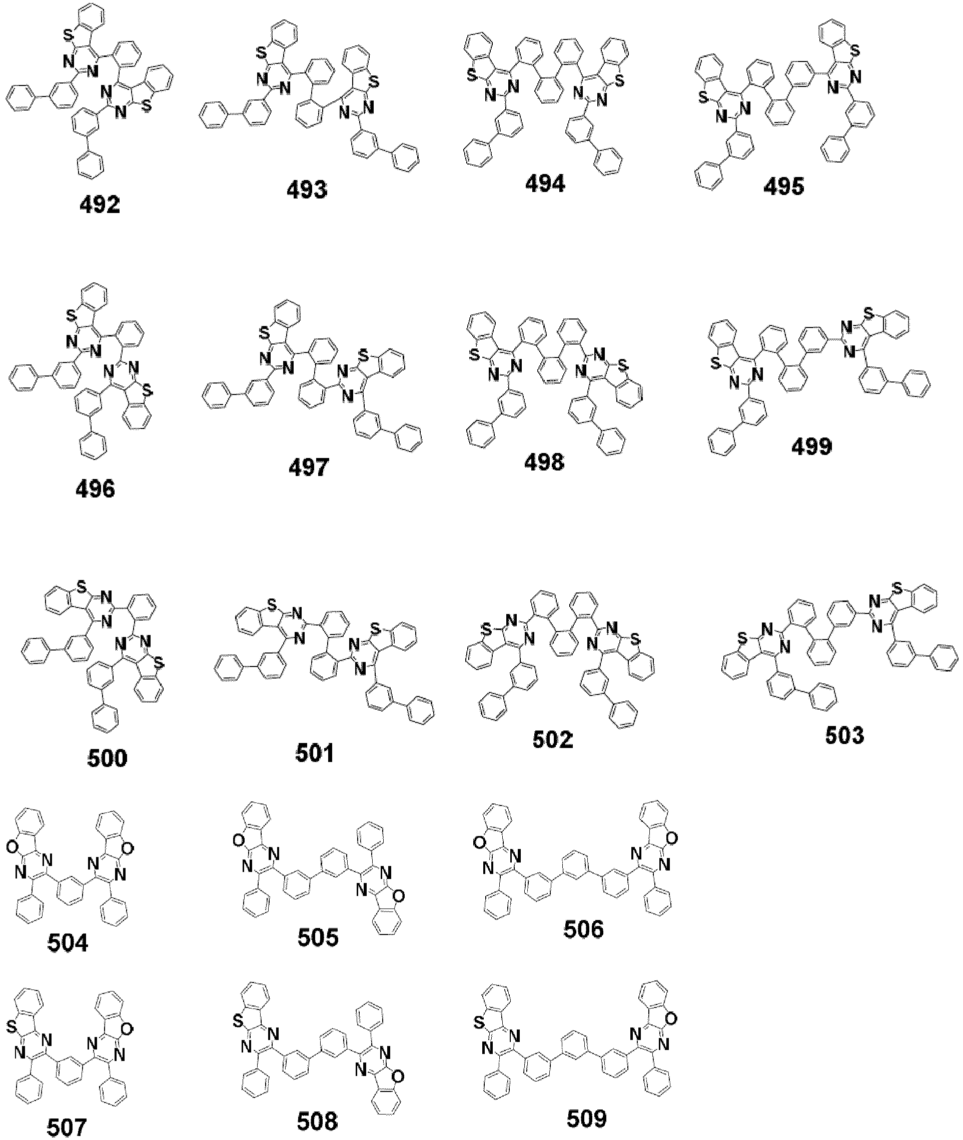

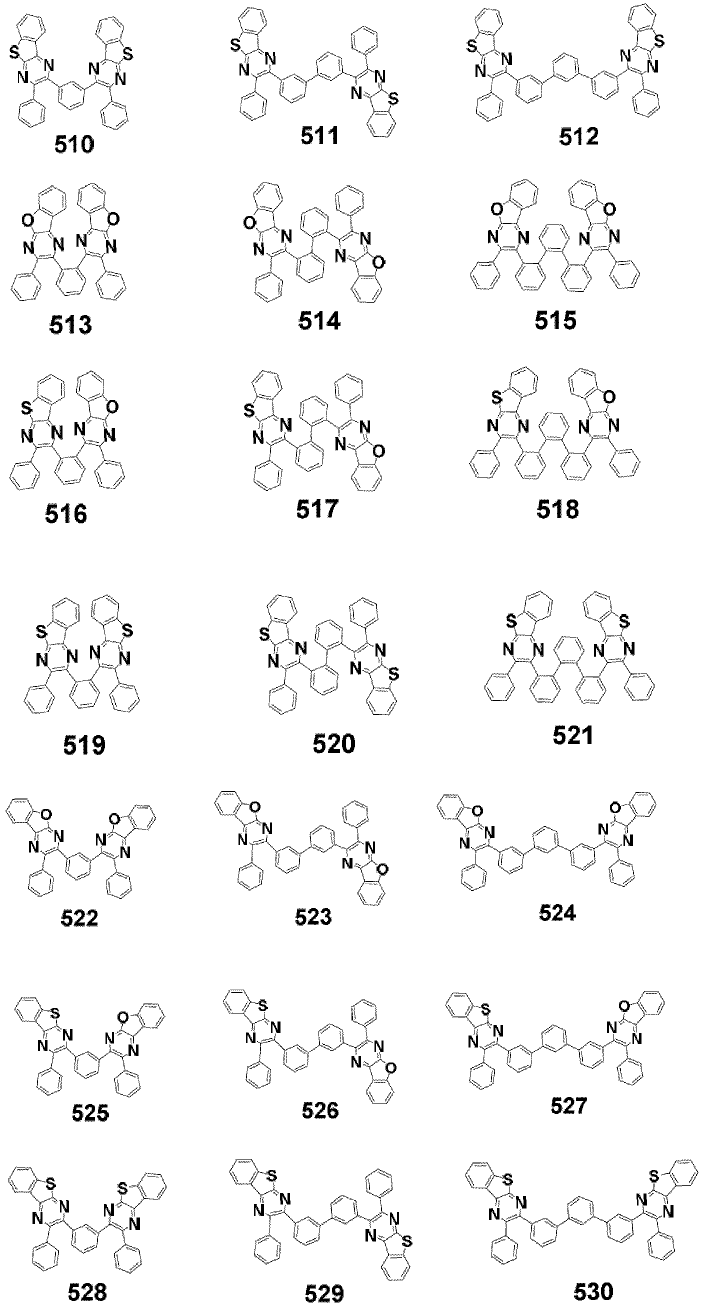

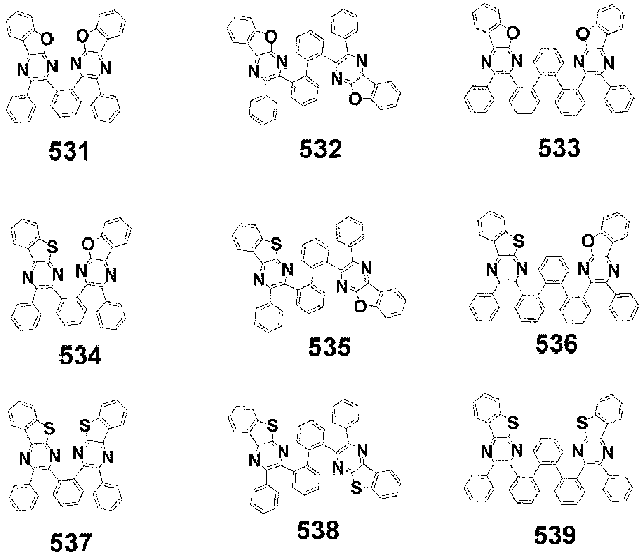

According to another aspect of the present invention, an organic light emitting diode includes the organic layer including i) a compound represented by Chemical Formula I and ii) at least one of a first compound represented by Chemical Formula 41 and a second compound represented by Chemical Formula 61.

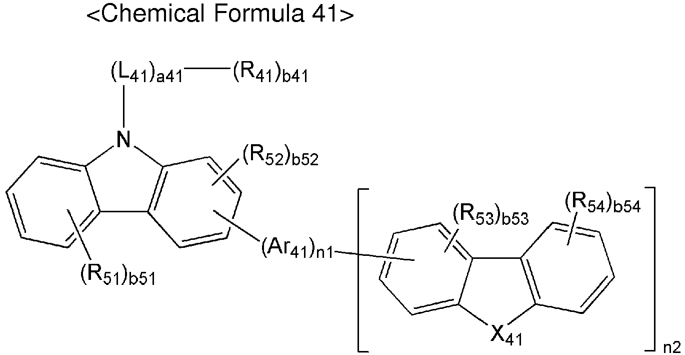

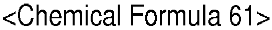

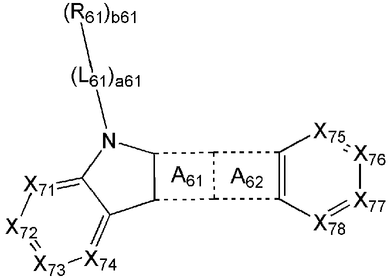

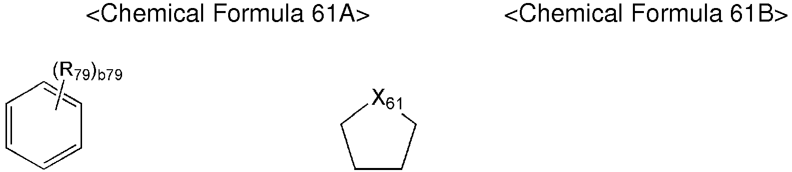

In Chemical Formula 41, X₄₁ is N-[(L₄₂)ₐ₄₂-(R₄₂)_{b42}], S, O, S(=O), S(=O)₂, C(=O), C(R₄₃)(R₄₄), Si(R₄₃)(R₄₄), P(R₄₃), P(=O)(R₄₃) or C=N(R₄₃);
in Chemical Formula 61, the ring A₆₁ is represented by Formula 61A;
in Chemical Formula 61, the ring A₆₂ is represented by Formula 61B;
X₆₁ is N-[(L₆₂)ₐ₆₂-(R₆₂)_{b62}], S, O, S(=O), S(=O)₂, C(=O), C(R₆₃)(R₆₄), Si(R₆₃)(R₆₄), P(R₆₃), P(=O)(R₆₃) or C=N(R₆₃);
X₇₁ is C(R₇₁) or N, X₇₂ is C(R₇₂) or N, X₇₃ is C(R₇₃) or N, X₇₄ is C(R₇₄) or N, X₇₅ is C(R₇₅) or N, X₇₆ is C(R₇₆) or N, X₇₇ is C(R₇₇) or N, and X₇₈ is C(R₇₈) or N;
Ar₄₁, L₄₁, L₄₂, L₆₁ and L₆₂ are each independently a substituted or unsubstituted C₃-C₁₀cycloalkylene group, a substituted or unsubstituted C₂-C₁₀heterocycloalkylene group, a substituted or unsubstituted C₃-C₁₀cycloalkenylene group, a substituted or unsubstituted C₂-C₁₀heterocycloalkenylene group, a substituted or unsubstituted C₆-C₆₀arylene group or a substituted or unsubstituted C₂-C₆₀heteroarylene group;
n1 and n2 are each independently an integer selected from 0 to 3;
a41, a42, a61 and a62 are each independently an integer selected from 0 to 5;
R₄₁ to R₄₄, R₅₁ to R₅₄, R₆₁ to R₆₄ and R₇₁ to R₇₉ are each independently hydrogen, deuterium, -F (a fluoro group), -Cl (a chloro group), -Br (a bromo group), -I (an iodo group), a hydroxyl group, a cyano group, an amino group, amidino group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₂-C₁₀heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group or a substituted or unsubstituted C₂-C₆₀ heteroaryl group; and
b41, b42, b51 to b54, b61, b62 and b79 are each independently an integer selected from 1 to 3.

The compound for an organic optoelectronic device may further include a dopant. The dopant may be a red, green, or blue dopant.

The dopant is mixed in a small amount to cause light emission, and may be generally a material such as a metal complex that emits light by multiple excitation into a triplet or more. The dopant may be, for example an inorganic, organic, or organic/inorganic compound, and one or more kinds thereof may be used.

The dopant may be a phosphorescent dopant, and examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example a compound represented by Chemical Formula Z, but is not limited thereto.

[Chemical Formula Z] L₂MX

In Chemical Formula Z, M is a metal, and L and X are the same or different, and are a ligand to form a complex compound with M.

The M may be, for example Ir, Pt, Os, Ti, Zr, Hf, Eu, Tb, Tm, Fe, Co, Ni, Ru, Rh, Pd or a combination thereof, and the L and X may be, for example a bidendate ligand.

Hereinafter, an organic optoelectronic device to which the compound for an organic optoelectronic device is applied is described.

The organic optoelectronic device includes an anode and a cathode facing each other and at least one organic layer between the anode and the cathode wherein the organic layer includes the compound for an organic optoelectronic device.

The organic layer includes an emission layer, and the emission layer includes the compound for an organic optoelectronic device.

Specifically, the compound for an organic optoelectronic device may be included as a host of the emission layer. The compound of the present invention may be used in an emission layer by mixing the host of the emission layer including at least one of a first compound represented by Chemical Formula 41 and a second compound represented by Chemical Formula 61.

In one embodiment of the present invention, the organic layer of the organic optoelectronic device may include at least one auxiliary layer selected from a hole injection layer (HIL), a hole transport layer (HTL), a hole transport auxiliary layer, an electron transport auxiliary layer, an electron transport layer (ETL), and an electron injection layer (EIL), and the auxiliary layer includes the compound for an organic optoelectronic device. For example, the compound of the present invention may be used in an electron transport auxiliary layer.

The organic optoelectronic device may be any device to convert electrical energy into photoenergy and vice versa without particular limitation, and may be, for example an organic photoelectric device, an organic light emitting diode, an organic solar cell, and an organic photo-conductor drum.

Herein, an organic light emitting diode as one example of an organic optoelectronic device is described referring to drawings.

FIGS. 1 and 2 are cross-sectional views of each organic light emitting diode according to one embodiment.

Referring to FIG. 1, an organic light emitting diode 100 according to one embodiment includes an anode 120 and a cathode 110 facing each other and an organic layer 105 interposed between the anode 120 and cathode 110.

The anode 120 may be made of a conductor having a large work function to help hole injection, and may be for example metal, metal oxide and/or a conductive polymer. The anode 120 may be, for example a metal such as nickel, platinum, vanadium, chromium, copper, zinc, and gold or an alloy thereof; metal oxide such as zinc oxide, indium oxide, indium tin oxide (ITO), indium zinc oxide (IZO), and the like; a combination of metal and oxide such as ZnO and Al or SnO₂ and Sb; a conductive polymer such as poly(3-methylthiophene), poly(3,4-(ethylene-1,2-dioxy)thiophene) (PEDT), polypyrrole, and polyaniline, but is not limited thereto.

The cathode 110 may be made of a conductor having a small work function to help electron injection, and may be for example metal, metal oxide and/or a conductive polymer. The cathode 110 may be for example a metal or an alloy thereof such as magnesium, calcium, sodium, potassium, titanium, indium, yttrium, lithium, gadolinium, aluminum silver, tin, lead, cesium, barium, and the like; a multi-layer structure material such as LiF/Al, LiO₂/Al, LiF/Ca, LiF/Al and BaF₂/Ca, but is not limited thereto.

The organic layer 105 includes an emission layer 130 including the compound for an organic optoelectronic device.

The emission layer 130 may include, for example the compound for an organic optoelectronic device at alone, or a mixture of at least two kinds and may include another compound different from the compound for an organic optoelectronic device. When the compound for an organic optoelectronic device is mixed with another compound, they may be, for example a host and a dopant, and the compound for an organic optoelectronic device may be, for example a host. The host may be, for example a phosphorescent host or fluorescent host, and may be, for example a phosphorescent host.

When the compound is a host, the dopant may be an inorganic, organic, or organic/inorganic compound, and may be selected from known dopants.

Referring to FIG. 2, an organic light emitting diode 200 further includes a hole auxiliary layer 140 in addition to an emission layer 130. The hole auxiliary layer 140 may improve hole injection and/or hole mobility between the anode 120 and the emission layer 130 and may block electrons. The hole auxiliary layer 140 may include, for example at least one of a hole transport layer, a hole injection layer and/or an electron blocking layer.

Even not shown in FIG. 1 or 2, the organic layer 105 may further include an electron injection layer, an electron transport layer, an auxiliary electron transport layer, a hole transport layer, an auxiliary hole transport layer, a hole injection layer or a combination thereof. The compound for an organic optoelectronic device may be included in the organic layer. The organic light emitting diodes 100 and 200 may be manufactured by forming an anode or a cathode on a substrate, forming an organic layer in accordance with a dry coating method such as evaporation, sputtering, plasma plating, and ion plating or a wet coating method such as spin coating, dipping, and flow coating; and forming a cathode or an anode thereon.

The organic light emitting diode may be applied to an organic light emitting diode (OLED) display.

Hereinafter, the embodiments are illustrated in more detail with reference to examples.

Hereinafter, a starting material and a reaction material used in Examples and Synthesis Examples were purchased from Sigma-Aldrich Co. Ltd. or TCI Inc. unless there was particularly mentioned.

### (Preparation of Compound for Organic Optoelectronic Device)

A compound was synthesized through the following steps as specific examples of a compound according to the present invention.

### Synthesis of Intermediate

### 1. Synthesis of methyl 3-amino-2-benzothiophenecarboxylate

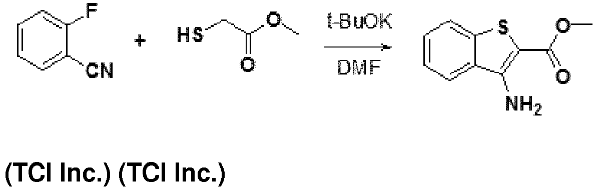

### 2. Synthesis of methyl 3-aminobenzofuran-2-carboxylate

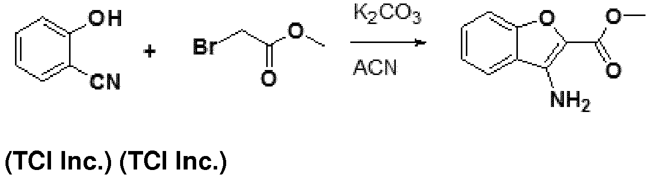

### 3. Synthesis of Intermediate C-0

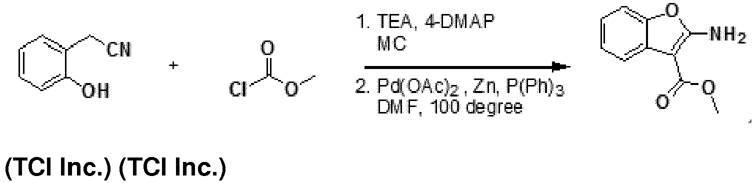

### 4. Synthesis of Intermediate D-1

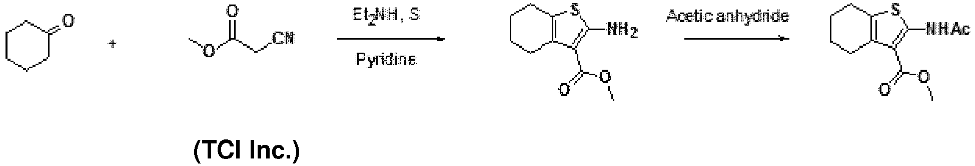

### (Daejung Chemicals and Materials Co., Ltd.)

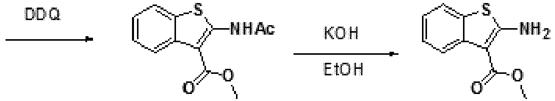

### Synthesis Example 1: Synthesis of Intermediate A

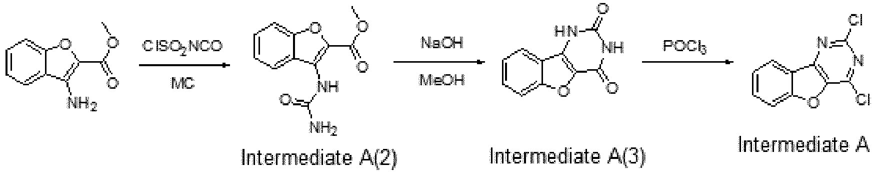

### Synthesis of Intermediate A (2) (benzo-methyl 3-ureidofuran-2-carboxylate)

Chlorosulfonyl isocyanate (ClSO₂NCO, 33.4 ml, 0.38 mol) was added to methyl 3-aminobenzofuran-2-carboxylate (49.0 g, 0.25 mol, refer to Synthesis of Intermediate 2) solution in dichloromethane (1000 ml) in a dropwise fashion in a 2000 mL round flask at - 78° C. The reactant was slowly heated up to room temperature and agitated for 2 hours. The reactant was concentrated, Conc. HCl (100 ml) was added to the residue, and the mixture was agitated at 100° C for 1 hour. The reaction mixture was cooled down to room temperature and neutralized with a saturated sodium bicarbonate aqueous solution. The produced solid was filtered, obtaining an intermediate A (2) (benzo-methyl 3-ureidofuran-2-carboxylate) (52.1 g, 87 %) as a beige solid.
calcd. C₁₁H₁₀N₂O₄: C, 56.41; H, 4.30; N, 11.96; O, 27.33; found: C, 56.45; H, 4.28; N, 11.94; O, 27.32

### Synthesis of Intermediate A (3) (benzo-furo[3,2-d]pyrimidine-2,4-diol)

The intermediate A (2) (methyl 3-ureidobenzofuran-2-carboxylate) (50.0 g, 0.21 mol) was suspended in 1000 ml of methanol in a 2000 mL round flask, and 300 ml of 2 M NaOH was added thereto in a dropwise fashion. The reaction mixture was agitated under a reflux for 3 hours. The reaction mixture was cooled down to room temperature and acidized into pH 3 by using Conc. HCl. The mixture was concentrated, and methanol was slowly added to the residue in a dropwise fashion to precipitate a solid. The produced solid was filtered and dried, obtaining an intermediate A (3) (benzo-furo[3,2-d]pyrimidine-2,4-diol) (38.0 g, 88 %).
calcd. C₁₀H₆N₂O₃: C, 59.41; H, 2.99; N, 13.86; O, 23.74; found: C, 59.41; H, 2.96; N, 13.81; O, 23.75

### Synthesis of Intermediate A (benzo-2,4-dichlorofuro[3,2-d]pyrimidine)

The intermediate A (3) (benzo-furo[3,2-d]pyrimidine-2,4-diol) (37.2 g, 0.18mol) was dissolved in phosphorous oxychloride (500 ml) in a 1000 mL round flask. The mixture was cooled down to -30° C, and N,N-diisopropylethylamine (52 ml, 0.36 mol) was slowly added thereto. The reactant was agitated under a reflux for 36 hours and cooled down to room temperature. The reactant was poured into ice/water and extracted with ethyl acetate. Then, an organic layer produced therein was washed with a saturated sodium bicarbonate aqueous solution and dried by using magnesium sulfate. The obtained organic layer was concentrated, obtaining an intermediate (A) (benzo-2,4-dichlorofuro[3,2-d]pyrimidine) (20.4 g, 46 %).

The atom analysis of the intermediate A is as follows.
calcd. C₁₀H₄Cl₂N₂O: C, 50.24; H, 1.69; Cl, 29.66; N, 11.72; O, 6.69; found: C, 50.18; H, 1.79; Cl, 29.69; N, 11.69; O, 6.70;

### Synthesis Example 2: Synthesis of Intermediate B

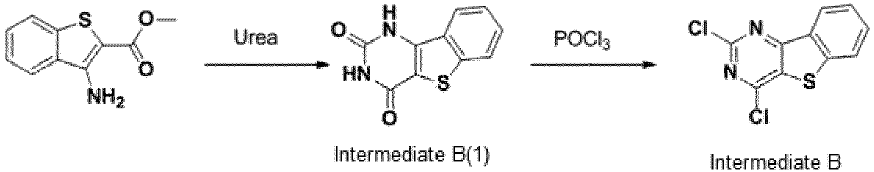

### Synthesis of Intermediate B (1) (benzo-1H-thieno[3,2-d]pyrimidine-2,4-dione)

A mixture of methyl 3-amino-2-benzothiophenecarboxylate (237.5 g, 1.15 mol, refer to Synthesis of Intermediate 1) and urea (397.0 g, 5.75 mol) was agitated at 200° C for 2 hours in a 2 L round flask. The reaction mixture at a high temperature was cooled down to room temperature and poured into a sodium hydroxide solution, and a precipitate obtained by acidizing the reactant (HCl, 2N) after filtering and removing impurities was dried, obtaining an intermediate B (1) (175 g, 75 %).
calcd. C₁₀H₆N₂O₂S: C, 55.04; H, 2.77; N, 12.84; O, 14.66; S, 14.69; found: C, 55.01; H, 2.79; N, 12.81; O, 14.69; S, 14.70

### Synthesis of Intermediate B (benzo-2,4-dichloro-thieno[3,2-d]pyrimidine)

A mixture of the intermediate B (1) (benzo-1H-thieno[3,2-d]pyrimidine-2,4-dione) (175 g, 0.80 mol) and phosphorous oxychloride (1000 mL) was agitated under a reflux for 8 hours in 3000 mL round flask. The reaction mixture was cooled down to room temperature and poured into ice/water while agitated, producing a precipitate. The obtained reactant was filtered, obtaining an intermediate B (benzo-2,4-dichloro-thieno[3,2-d]pyrimidine) (175 g, 85 %, a white solid). The atom analysis result of the intermediate B is as follows.
calcd. C₁₀H₄Cl₂N₂S: C, 47.08; H, 1.58; Cl, 27.79; N, 10.98; S, 12.57; found: C, 47.03; H, 1.61; Cl, 27.81; N, 10.98; S, 12.60

### Synthesis Example 3: Synthesis of Intermediate C

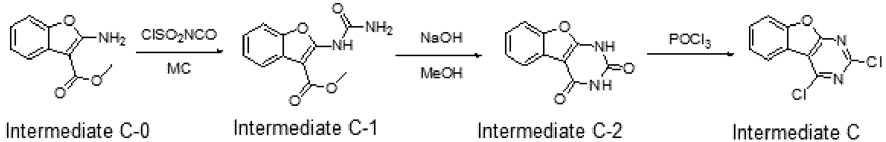

### Synthesis of Intermediate C-1

Chlorosulfonyl isocyanate (23.7 ml, 274.6 mmol) was added to the intermediate C-0 (35.0 g, 183.1 mmol, refer to Synthesis of Intermediate 3) solution in dichloromethane (1000 mL) in a dropwise fashion at - 78 °C in a 2000 mL round flask. The reactant was slowly heated up to room temperature and agitated for 2 hours. The reactant was concentrated, 6N HCl(300 ml) was added to the residue, and the mixture was agitated at 10 °C for 1 hour. The reaction mixture was cooled down to room temperature and neutralized with a saturated NaHCO3 aqueous solution. The produced solid was filtered, obtaining an intermediate C-1 (43.2 g, 88 %) as a beige solid.
calcd. C10H9NO3: C, 62.82; H, 4.74; N, 7.33; O, 25.11; found: C, 62.82; H, 4.74; N, 7.33; O, 25.11

### Synthesis of Intermediate C-2

The intermediate C-1 (40.0 g, 0.19 mol) was suspended in 1000 ml of methanol in a 1000 mL round flask, and 300 ml of 2 M NaOH was added thereto in a dropwise fashion. The reaction mixture was agitated under a reflux for 3 hours. The reaction mixture was cooled down to room temperature and acidized into pH 3 by using Conc. HCl. The mixture was concentrated, and methanol was added to the residue in a dropwise fashion, precipitating a solid. The produced solid was filtered and dried, obtaining an intermediate C-2 (39.0 g, 85 %).
calcd. C11H10N2O4: C, 56.41; H, 4.30; N, 11.96; O, 27.33; found: C, 56.40; H, 4.20; N, 11.92; O, 27.31

### Synthesis of Intermediate C

200 mL of a mixture of the intermediate C-2 (39.0 g, 191.0 mmol) and oxychloride was agitated under a reflux in a 500 mL round flask for 8 hours. The reaction mixture was cooled down to room temperature and poured into ice/water while fervently agitated, producing a precipitate. The obtained reactant was filtered, obtaining an intermediate C. (40.7 g, 89 %, a white solid)
calcd. C10H4Cl2N2O: C, 50.24; H, 1.69; Cl, 29.66; N, 11.72; O, 6.69; found: C, 50.21; H, 1.65; Cl, 29.63; N, 11.64; O, 6.62

### Synthesis Example 4: Synthesis of Intermediate D

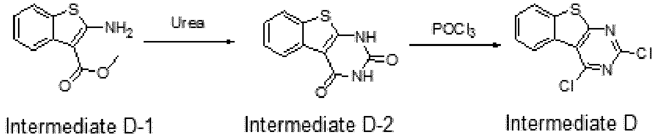

### Synthesis of Intermediate D-2

A mixture of the intermediate D-1 (35.0 g, 0.17 mol, refer to Synthesis of Intermediate 4) and urea (50.7 g, 0.84 mol) was agitated at 200 °C for 2 hours in a 250 mL round flask. The reaction mixture at the high temperature was cooled down to room temperature and poured into a sodium hydroxide solution, impurities were filtered and removed therefrom, and the reactant was acidized (HCl, 2N), and the obtained precipitate therefrom was dried, obtaining an intermediate D-2 (18.9 g, 51%).
calcd. C10H6N2O2S: C, 55.04; H, 2.77; N, 12.84; O, 14.66; S, 14.69; found: C, 55.01; H, 2.77; N, 12.83; O, 14.65; S, 14.63

### Synthesis of Intermediate D

A mixture of the intermediate D-2 (18.9 g, 99.2 mmol) and phosphorous oxychloride (100 mL) was agitate under a reflux for 6 hours in a 250 mL round flask. The reaction mixture was cooled down to room temperature and then, poured into ice/water while fervently agitated, producing a precipitate. The obtained reactant was filtered, obtaining an intermediate D. (17.5 g, 85 %, a white solid)
calcd. C10H4Cl2N2S: C, 47.08; H, 1.58; Cl, 27.79; N, 10.98; S, 12.57; found: C, 47.04; H, 1.53; Cl, 27.74; N, 10.96; S, 12.44

### <Synthesis Example 1> Synthesis of Compound 25

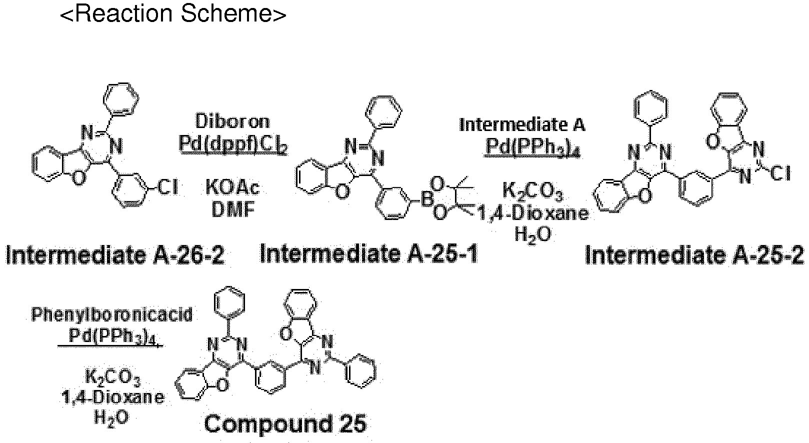

### Synthesis of Intermediate A-25-1

25 g (70.1 mmol) of the synthesized intermediate A-26-2 was put in a 250 mL flask, and 1.5 equivalent of diboron, 0.03 equivalent of dichloro diphenyl phosphinoferrocene palladium, and 2 equivalent of potassium acetate were suspended in 160 mL of dimethyl formamide. The reaction solution was heated and agitated at 140 °C for 20 hours. When the reaction was complete, the reaction solution was suspended in 500 mL of water for solidification, twice washed with 200 mL of water, and dried, obtaining 26.7 g of an intermediate A-25-1 (85 % of a yield). The intermediate itself was used in the following reaction without additional purification and analysis.

### Synthesis of Intermediate A-25-2

26.7 g (59.6 mmol) of the synthesized intermediate A-25-1 was put in a 250 mL flask, 1 equivalent of the synthesized intermediate A, 0.05 equivalent of tetrakis triphenyl phosphine palladium, and 3 equivalent of potassium carbonate were dissolved in 50 mL of water and 100 mL of dioxane, and the reaction solution was heated and agitated at 50 °C for 16 hours. When the reaction was complete, the reaction solution was added to 300 mL of methanol, and the mixture was agitated, and a precipitate produced therein was filtered. The filtered solid was recrystallized in 500 mL of toluene, obtaining 20.3 g of an intermediate A-25-2 (65 % of a yield).
MS calcd: C32H17ClN4O2 Exact Mass: 524.1040, found 524.09

### Synthesis of Compound 25

20.3 g (38.7 mmol) of the synthesized A-25-2 was put in a 250 mL flask, 1.2 equivalent of phenyl boronic acid, 0.05 equivalent of tetrakis triphenyl phosphine palladium, and 3 equivalent of potassium carbonate were dissolved in 50 mL of water and 100 mL of dioxane, and the reaction solution was heated and agitated at 110 °C for 18 hours. When the reaction was complete, the reaction solution was added to 300 mL of methanol, the mixture was agitated, and a product therein was precipitated and filtered. The filtered solid was recrystallized in 500 mL of xylene, obtaining 19.2 g of a compound 25 (88 % of a yield).
MS calcd: C38H22N4O2 Exact Mass: 566.1743, found: 566.20

### <Synthesis Example 2> Synthesis of Compound 26

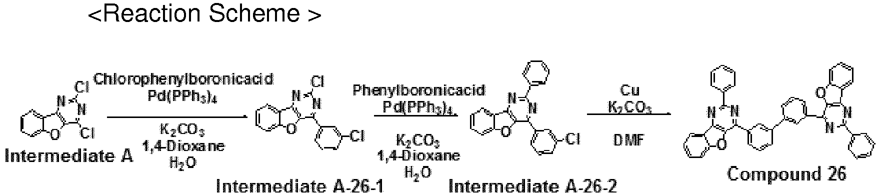

### Synthesis of Intermediate A-26-1

20 g (83.7 mmol) of the synthesized intermediate A was put in a 250 mL flask, 1 equivalent of chlorophenyl boronic acid, 0.05 equivalent of tetrakistriphenylphosphinepalladium, and 3 equivalent of potassium carbonate were dissolved in 50 mL of water and 100 mL of 1,4-dioxane, and the reaction solution was heated and agitated at 55 °C under a nitrogen stream for 18 hours. When the reaction was complete, the reaction solution was suspended in 500 mL of methanol, agitated, and filtered, and the obtained product was heated and agitated with 300 mL of toluene for recrystallization, obtaining 20.3 g of an intermediate A-26-1 (77 % of a yield).
MS calcd: C10H4Cl2N2O Exact Mass: 237.9701, found 237.92

### Synthesis of Intermediate A-26-2

20 g (63.5 mmol) of the synthesized intermediate A-25-1 was put in a 250 mL flask, 1.2 equivalent of phenyl boronic acid, 0.05 equivalent of tetrakis triphenyl phosphine palladium, and 3 equivalent of potassium carbonate were dissolved in 50 mL of water and 100 mL of 1,4-dioxane, and the reaction solution was heated and agitated under a nitrogen stream for 18 hours at 100 °C. When the reaction was complete, the reaction solution was suspended in 500 mL of methanol, agitated, and filtered, and a product obtained therefrom was heated and agitated with 300 mL of toluene for recrystallization, obtaining 18 g of an intermediate A-26-2 (80 % of a yield).
MS calcd: C22H13ClN2O Exact Mass: 356.0716, found: 356.11

### Synthesis of Compound 26

18 g (50.8 mmol) of the synthesized intermediate A-25-2 was put in a 250 mL flask, 3 equivalent of copper powder and 2 equivalent of potassium carbonate were added thereto, and the mixture was heated and agitated in 150 mL of dimethyl formamide for 24 hours. When the reaction was complete, the reaction solution was added to 300 mL of water for solidification, and a solid therein was filtered. The filtered solid was collected and recrystallized in 500 mL of dichloro benzene, obtaining 10.7 g of a compound 26 (75 % of a yield).
MS calcd: C44H26N4O2 Exact Mass: 642.2056, found: 642.23

### <Synthesis Example 3> Synthesis of Compound 27

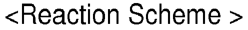

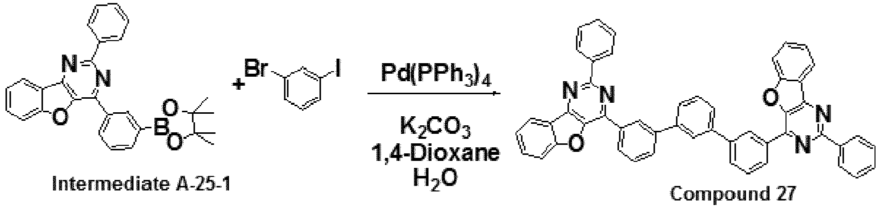

### Synthesis of Compound 27

12 g (26.8 mmol) of the synthesized intermediate A-25-1 was put in a 250 mL flask, 0.7 equivalent of 3-bromo-1-iodo benzene, 0.05 equivalent of tetrakis triphenyl phosphine palladium, and 3 equivalent of potassium carbonate were dissolved in 30 mL of water and 80 mL of 1,4-dioxane, and the reaction solution was heated and agitated under a nitrogen stream for 24 hours at 100 °C. When the reaction was complete, the reaction solution was suspended in 500 mL of methanol, agitated, and filtered, and a product therefrom was heated and recrystallized with 500 mL of dichloro benzene, obtaining 6.9 g of a compound 27 (72 % of a yield).
MS calcd: C50H30N4O2 Exact Mass: 718.2369, found: 718.25

### <Synthesis Example 4> Synthesis of Compound 2

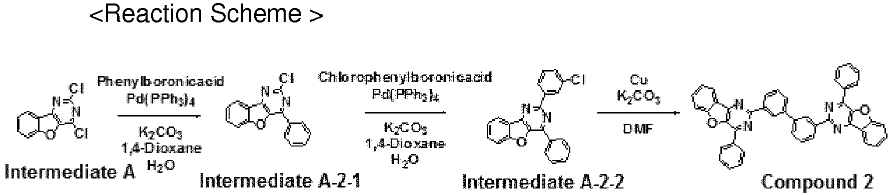

### Synthesis of Intermediate A-2-1

15 g (62.7 mmol) of the synthesized intermediate A was put in a 250 mL flask, 1 equivalent of phenyl boronic acid, 0.05 equivalent of tetrakis triphenyl phosphine palladium, and 3 equivalent of potassium carbonate were dissolved in 30 mL of water and 80 mL of 1,4-dioxane, and the reaction solution was heated and agitated under a nitrogen stream for 16 hours at 55 °C. When the reaction was complete, the reaction solution was suspended in 500 mL of methanol, agitated, and filtered, and a product therefrom was heated and recrystallized with 300 mL of toluene, obtaining 14.8 g of an intermediate A-2-1 (84 % of a yield).
MS calcd: C16H9ClN2O Exact Mass: 280.0403, found: 280.11

### Synthesis of Intermediate A-2-2

14.8 g (52.7 mmol) of the synthesized A-2-1 was put in a 250 mL flask, 1.2 equivalent of chloro phenyl boronic acid, 0.03 equivalent of tetrakis triphenyl phosphine palladium, and 3 equivalent of potassium carbonate were dissolved in 50 mL of water and 90 mL of 1,4-dioxane, and the reaction solution was heated and agitated under a nitrogen stream for 18 hours at 110 °C. When the reaction was complete, the reaction solution was suspended in 300 mL of methanol, agitated, and filtered, and a solid obtained therefrom was collected and then, heated and recrystallized with 500 mL of xylene, obtaining 16.2 g of an intermediate A-2-2 (86 % of a yield).
MS calcd: C22H13ClN2O Exact Mass: 356.0716, found 356.15

### Synthesis of Compound 2

16 g (44.8 mmol) of the synthesized intermediate A-2-2 was put in a 250 mL flask, and 5 equivalent of copper powder and 5 equivalent of potassium carbonate were suspended in 120 mL of dimethyl formamide. The reaction solution was heated and agitated at 140 °C for 24 hours. The reaction solution was cooled down and suspended by adding 400 mL of water thereto to precipitate a solid. The precipitated solid was filtered and recrystallized in 300 mL of dichloro benzene, obtaining 8.9 g of a compound 2 (62 % of a yield).
MS calcd: C44H26N4O2 Exact Mass: 642.2056, found 642.28

### <Synthesis Example 5> Synthesis of Compound 6

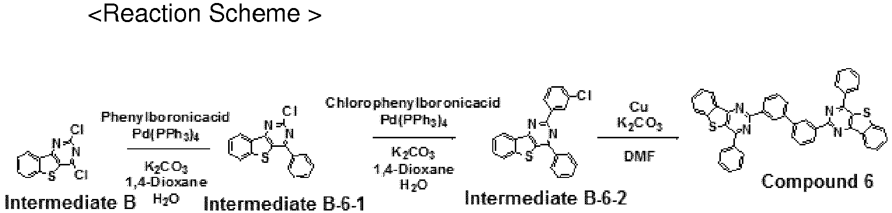

### Synthesis of Intermediate B-6-1

An intermediate B-6-1 was synthesized according to the same method as the intermediate A-2-1 of Synthesis Example 4 except for using the intermediate B as a starting material.
MS calcd: C16H9ClN2S, Exact Mass: 296.0175, found: 296.10

### Synthesis of Intermediate B-6-2

An intermediate B-6-2 was synthesized according to the same method as the intermediate A-2-2 of Synthesis Example 4 except for using the intermediate B-6-1 as a starting material.
MS calcd: C22H13ClN2S, Exact Mass: 372.0488, found: 372.02

### Synthesis of Compound 6

A compound 6 was synthesized according to the same method as the intermediate 2 of Synthesis Example 4 except for using the intermediate B-6-2 as a starting material.
MS calcd: C44H26N4S2, Exact Mass: 674.1599, found: 674.21

### <Synthesis Example 6> Synthesis of Compound 370

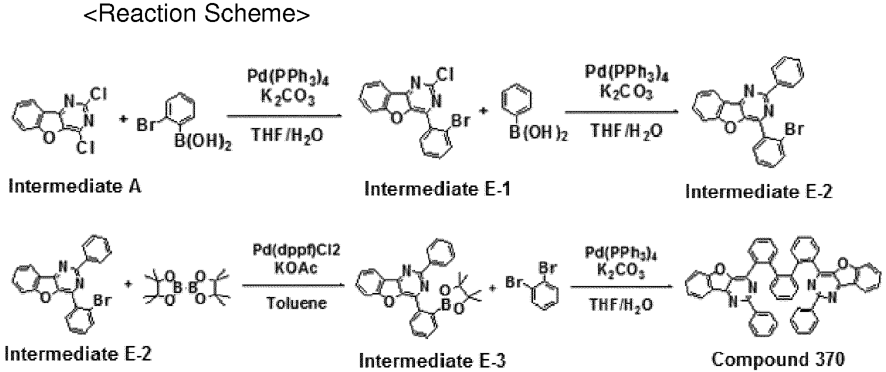

### Synthesis of Intermediate E-1

20 g (83.6 mmol) of the intermediate A, 1 equivalent of bromo phenyl boronic acid, 0.03 equivalent of tetrakis triphenyl phosphine palladium, and 2 equivalent of potassium carbonate were suspended in 150 mL of tetrahydrofuran and 50 mL of water in a 500 mL round-bottomed flask. The reaction solution was heated and agitated at 55 °C for 18 hours. The reaction solution was layer-separated, and the separated organic layer was concentrated. The concentrated residue was agitated in 500 mL of methanol and 50 mL of tetrahydrofuran for crystallization, filtered, and dried, obtaining 25 g of an intermediate E-1 (a yield of 85 %).
MS calcd: C16H8BrClN2O, Exact Mass: 357.9509, found: 357.99

### Synthesis of Intermediate E-2

25 g (71.1 mmol) of the intermediate E-1, 1.2 equivalent of phenyl boronic acid, 0.03 equivalent of tetrakis triphenyl phosphine palladium, and 2 equivalent of potassium carbonate were suspended in 150 mL of tetrahydrofuran and 50 mL of water in a 500 mL round-bottomed flask. The reaction solution was heated and agitated at 75 °C for 18 hours. The reaction solution was layer-separated, and the separated organic layer was concentrated. The concentrated residue was heated and agitated in 400 mL of an ethyl acetate solution for crystallization, obtaining 26.2 g of an intermediate E-2 (a yield of 92 %).
MS calcd: C22H13BrN2O Exact Mass: 400.0211 found: 400.11

### Synthesis of Intermediate E-3

26.2 g (65.41 mmol) of the intermediate E-2, 1.2 equivalent of bis pinacolato diboron, 0.03 equivalent of dichloro diphenylphosphine palladium, and 2 equivalent of potassium acetate were suspended in 200 mL of toluene in a 500 mL round-bottomed flask, and the reaction solution was heated and agitated at 110 °C for 16 hours. The reaction solution was suspended in 600 mL of methanol and filtered. The filtered solid was heated and dissolved in 500 mL of toluene, treated with activated carbon, cooled down, and crystallized, obtaining 25.7 g of an intermediate E-3 (a yield of 88 %).
MS calcd: C28H25BN2O3, Exact Mass: 448.1958 found: 448.20

### Synthesis of Compound 370

25.7 g (57.32 mmol) of the intermediate E-3, 0.5 equivalent of dibromo benzene, 0.03 equivalent of tetrakis triphenyl phosphine palladium, and 2 equivalent of potassium carbonate were suspended in 150 mL of tetrahydrofuran and 50 mL of water in a 500 mL round-bottomed flask. The reaction solution was heated and agitated at 75 °C for 18 hours. The reaction solution was suspended in 300 mL of methanol, filtered, and washed with methanol and water. The obtained solid was collected, heated and dissolved in 700 mL of toluene, treated with activated carbon, cooled down, and crystallized, obtaining 13.4 g of a compound 370 (a yield of 65 %).
MS calcd: C50H30N4O2, Exact Mass: 718.2369 found: 718.25

### <Synthesis Example 7> Synthesis of Compound 362

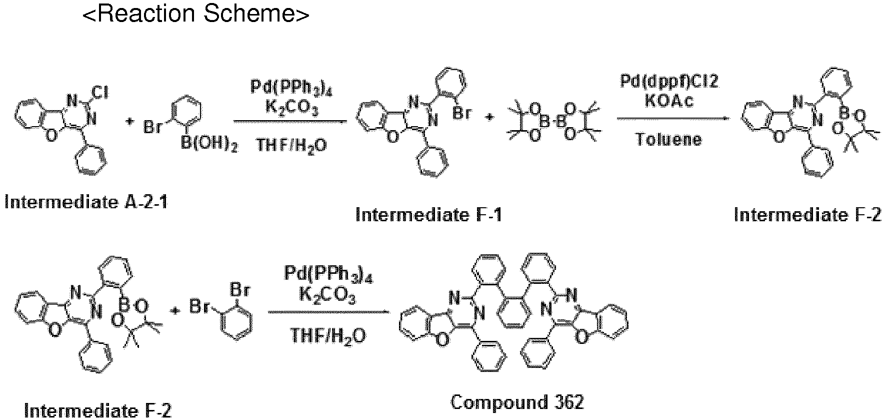

### Synthesis of Intermediate F-1

20 g (71.25 mmol) of the intermediate A-2-1, 1.1 equivalent of bromo phenyl boronic acid, 0.03 equivalent of tetrakis triphenyl phosphine palladium, and 2 equivalent of potassium carbonate were suspended in 100 mL of tetrahydrofuran and 30 mL of water in a 250 mL round-bottomed flask. The reaction solution was heated and agitated at 75 °C for 18 hours. The reaction solution was layer-separated, and the obtained organic layer was separated and concentrated. The concentrated residue was agitated in 500 mL of methanol and 50 mL of tetrahydrofuran for crystallization, filtered, and dried, obtaining 27.2 g of an intermediate F-1 (a yield of 95 %).

### Synthesis of Intermediate F-2

27.2 g (67.69 mmol) of the intermediate F-1, 1.2 equivalent of bispinacolato diboron, 0.03 equivalent of dichloro diphenylphosphine palladium, and 2 equivalent of potassium acetate were suspended in 200 mL of toluene in a 500 mL round-bottomed flask, and the reaction solution was heated and agitated at 110 °C for 16 hours. The reaction solution was suspended in 600 mL of methanol and filtered. The filtered solid was heated and dissolved in 500 mL of toluene, treated with activated carbon, and crystallized, obtaining 23.7 g of an intermediate F-2 (a yield of 78 %).
MS calcd: C28H25BN2O3, Exact Mass: 448.1958 found 448.22

### Synthesis of Compound 362

23.7 g (52.80 mmol) of the intermediate F-2, 0.5 equivalent of dibromo benzene, 0.03 equivalent of tetrakis triphenyl phosphine palladium, and 2 equivalent of potassium carbonate were suspended in 150 mL of tetrahydrofuran and 50 mL of water in a 500 mL round-bottomed flask. The reaction solution was heated and agitated at 75 °C for 18 hours. The reaction solution was suspended in 300 mL of methanol, filtered, and washed with methanol and water. The solid was collected and then, heated and dissolved in 700 mL of toluene, treated with activated carbon, cooled down, and crystallized, obtaining 14 g of a compound 362 (a yield of 74 %).

### <Synthesis Example 8> Synthesis of Compound 406

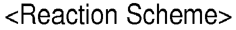

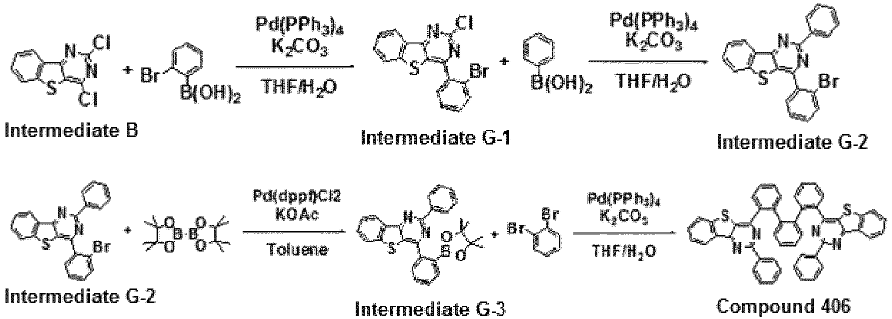

### Synthesis of Compound 406

A compound 406 was obtained according to the same method as Synthesis Example 6 except for using the intermediate B as a starting material.
MS calcd: C50H30N4S2, Exact Mass: 750.1912 found 750. 18

### <synthesis Example 9> Synthesis of Compound 398

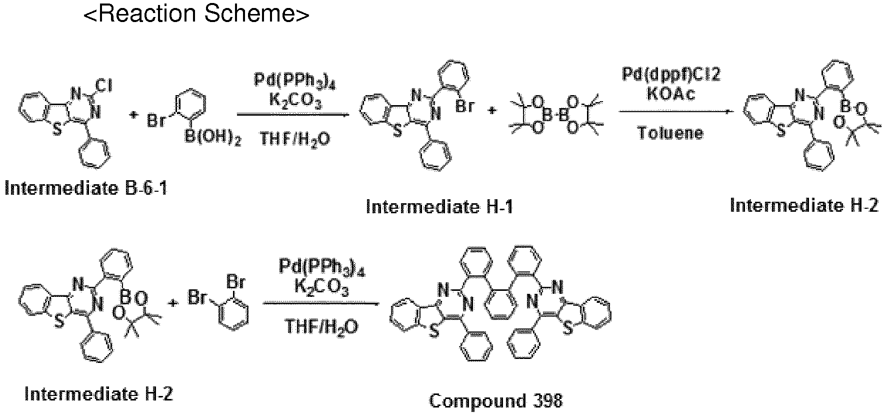

### Synthesis of Compound 398

A compound 398 was obtained according to the same method as Synthesis Example 7 except for using the intermediate B-6-1 as a starting material.
MS calcd: C50H30N4S2, Exact Mass: 750.1912 found 750.15

### (Comparative Example)

### Comparative Examples 1, Comparative Example 2

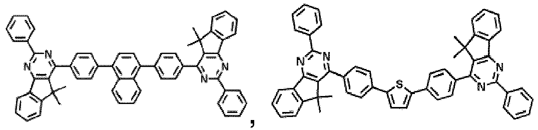

### Comparative Example 3, Comparative Example 4

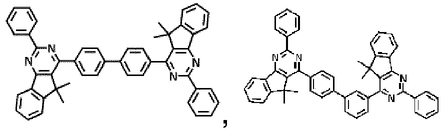

### Comparative Example 5, Comparative Example 6

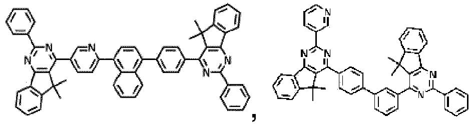

### Comparative Example 7, Comparative Example 8

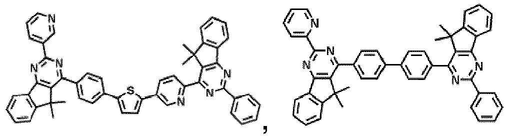

### (Comparison of Simulation Characteristics of Compounds for Organic Optoelectronic Device)

Energy level of each compound was calculated in a Gaussian 09 method by using a supercomputer GAIA (IBM power 6), and the results are provided in the following Table 1.

**[Table 1]**

| Compounds | HOMO (eV) | LUMO (eV) | T1 (eV) | S1 (eV) |
|---|---|---|---|---|
| compound 2 | -5.814 | -1.881 | 2.815 | 3.489 |
| compound 6 | -5.766 | -1.824 | 2.813 | 3.471 |
| compound 25 | -6.011 | -2.02 | 2.742 | 3.552 |
| compound 26 | -5.991 | -1.920 | 2.789 | 3.595 |
| compound 27 | -5.995 | -1.935 | 2.808 | 3.600 |
| Comparative Example 1 | -5.674 | -1.562 | 2.55 | 3.691 |
| Comparative Example 2 | -5.571 | -1.775 | 2.313 | 3.393 |
| Comparative Example 3 | -6.057 | -1.586 | 2.979 | 3.923 |
| Comparative Example 4 | -6.06 | -1.547 | 2.98 | 3.925 |
| Comparative Example 5 | -5.653 | -1.716 | 2.453 | 3.514 |
| Comparative Example 6 | -6.066 | -1.667 | 2.983 | 3.939 |
| Comparative Example 7 | -5.753 | -2.154 | 2.244 | 3.203 |
| Comparative Example 8 | -6.029 | -1.575 | 2.981 | 3.773 |

As shown in Table 1,
the compounds of Examples in general showed a lower LUMO than the compounds of Comparative Examples and had easy electron injection characteristics and thus an effect of decreasing a threshold voltage and simultaneously, showed appropriate T1 energy along with the low LUMO and thus had no energy surplus and deficiency in luminescence and may show characteristics of decreasing polaron extinction of a device.

### Manufacture of Organic Light Emitting Diode

### Device Example 1

A glass substrate having an ITO electrode was cut into a size of 50 mm x 50 mm x 0.5 mm, ultrasonic wave-cleaned in acetone isopropyl alcohol and pure water for 15 minutes respectively, and UV ozone-cleaned for 30 minutes.

On the ITO electrode, a 600 Å-thick hole injection layer (HIL) was formed by vacuum-depositing m-MTDATA at 1 Å/sec, and a 300 Å-thick hole transport layer (HTL) was formed on the hole injection layer (HIL) by vacuum-depositing the α-NPB at 1 Å/sec. Subsequently, a 400 Å-thick emission layer was formed on the hole transport layer (HTL) by respectively codepositing Ir(ppy)₃ (a dopant) and the compound 25 at each speed of 0.1 Å/sec and 1 Å/sec. On the emission layer, a 50 Å-thick hole blocking layer was formed by vacuum-depositing BAlq at 1 Å/sec, and then, a 300 Å**-**thick electron transport layer (ETL) was formed on the hole blocking layer by vacuum-depositing Alq₃. On the electron transport layer (ETL), LiF and Al were sequentially deposited to form a 10 Å-thick electron injection layer (EIL) and a 2000 Å-thick cathode, manufacturing an organic light emitting diode.

### Device Example 2

An organic light emitting diode was manufactured according to the same method as Device Example 1 except for using the compound 26 instead of the compound 25 as a host for forming the emission layer.

### Device Example 3

An organic light emitting diode was manufactured according to the same method as Device Example 1 except for using the compound 27 instead of the compound 25 as a host for forming the emission layer.

### Device Example 4

An organic light emitting diode was manufactured according to the same method as Device Example 1 except for using the compound 2 instead of the compound 25 as a host for forming the emission layer.

### Device Example 5

An organic light emitting diode was manufactured according to the same method as Device Example 1 except for using the compound 6 instead of the compound 25 as a host for forming the emission layer.

### Device Comparative Example 1

An organic light emitting diode was manufactured according to the same method as Device Example 1 except for using Comparative Example A instead of the compound 25 as a host for forming the emission layer.

### <Comparative Example A>

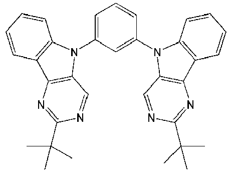

### Device Comparative Example 2

An organic light emitting diode was manufactured according to the same method as Device Example 1 except for using Comparative Example B instead of the compound 25 as a host for forming the emission layer.

### <Comparative Example B>

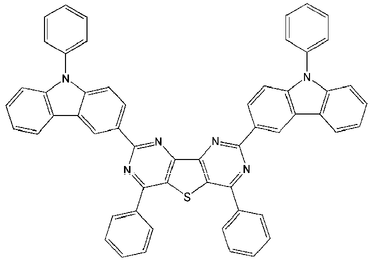

### Device Comparative Example 3

An organic light emitting diode was manufactured according to the same method as Device Example 1 except for using Comparative Example C instead of the compound 25 as a host for forming the emission layer.

### <Comparative Example C>

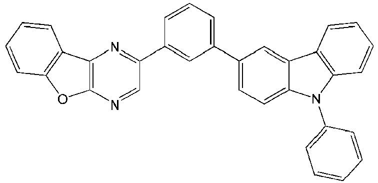

### Device Comparative Example 4

An organic light emitting diode was manufactured according to the same method as Device Example 1 except for using Comparative Example D instead of the compound 25 as a host for forming the emission layer.

### <Comparative Example D>

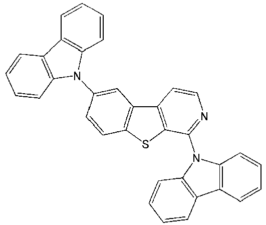

### (Performance Measurement of Organic Light Emitting Diode)

Current density change, luminance change, and luminous efficiency of each organic light emitting diode according to Device Examples 1 to 5 and Device Comparative Examples 1 to 4 were measured.

Specific measurement methods are as follows, and the results are shown in the following Table 2.

### (1) Measurement of Current Density Change Depending on Voltage Change

The obtained organic light emitting diodes were measured for current value flowing in the unit device while increasing the voltage from 0 V to 10 V using a current-voltage meter (Keithley smu 236), and the measured current value was divided by area to provide the result.

### (2) Measurement of Luminance Change Depending on Voltage Change

Luminance was measured by using a luminance meter (Minolta Cs-1000A), while the voltage of the organic light emitting diodes was increased from 0 V to 10 V.

### (3) Measurement of Luminous Efficiency

Current efficiency (cd/A) at the same current density (10 mA/cm²) were calculated by using the luminance, current density, and voltages (V) from the items (1) and (2).

**[Table 2]**

| | Host | Dopant | Driving voltage (V) | Current efficiency (cd/A) | Luminance (cd/m²) |
|---|---|---|---|---|---|
| Device Example 1 | compound 25 | Ir(ppy)3 | 4.4 | 39 | 6000 |
| Device Example 2 | compound 26 | Ir(ppy)3 | 4.5 | 44 | 6000 |
| Device Example 3 | compound 27 | Ir(ppy)3 | 4.6 | 42 | 6000 |
| Device Example 4 | compound 2 | Ir(ppy)3 | 4.3 | 45 | 6000 |
| Device Example 5 | compound 6 | Ir(ppy)3 | 4.2 | 39 | 6000 |
| Device Comparative Example 1 | compound A | Ir(ppy)3 | 5.0 | 38 | 6000 |
| Device Comparative Example 2 | compound B | Ir(ppy)3 | 5.1 | 29 | 6000 |
| Device Comparative Example 3 | compound C | Ir(ppy)3 | 4.8 | 34 | 6000 |
| Device Comparative Example 4 | compound D | Ir(ppy)3 | 4.8 | 31 | 6000 |

As shown in Table 2, the compound used in the present invention showed excellent driving voltage and current efficiency based on the same luminance compared with a material having a similar structure. The reason is that the compound has a double structure by using a functional group having a hole transport effect as a linking group or an additional expanding group and thus maximizing the effect of a functional group having a charge transport effect.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims. Therefore, the aforementioned embodiments should be understood to be exemplary of the present invention.

## Claims

1. A compound for an organic optoelectronic device represented by Chemical Formula I: 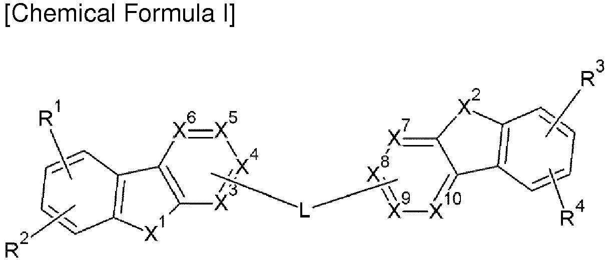 wherein, in Chemical Formula I,
X¹ and X² are each independently O or S,
X³ to X⁶ are each independently C, CR^{a} or N,
at least two of X³ to X⁶ are N,
X⁷ to X¹⁰ are each independently, C, CR^{b} or N,
at least two of X⁷ to X¹⁰ are N,
L is a substituted or unsubstituted C6 to C30 arylene group, and
R¹ to R⁴ and R^{a} and R^{b} are each independently hydrogen, deuterium, a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a substituted or unsubstituted C6 to C30 arylamine group, a substituted or unsubstituted C3 to C40 silyl group, a substituted or unsubstituted C1 to C30 alkylthiol group, a substituted or unsubstituted C6 to C30 arylthiol group, a halogen, a cyano group, a hydroxyl group, an amino group, a nitro group, or a combination thereof,
wherein "substituted" refers to that at least one hydrogen is replaced by deuterium, a halogen, a hydroxyl group, an amino group, a C1 to C30 amine group, a nitro group, a C1 to C40 silyl group, a C1 to C30 alkyl group, a C1 to C10 alkylsilyl group, a C3 to C30 cycloalkyl group, a C2 to C30 heterocycloalkyl group, a C6 to C30 aryl group, C2 to C30 heteroaryl group, a C1 to C20 alkoxy group, a fluoro group, a C1 to C10 trifluoroalkyl group, or a cyano group.

2. The compound for an organic optoelectronic device of claim 1, which is represented by one of Chemical Formulae I-1 to I-8: 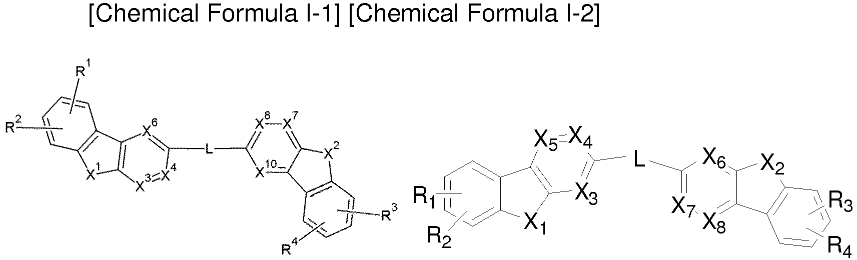 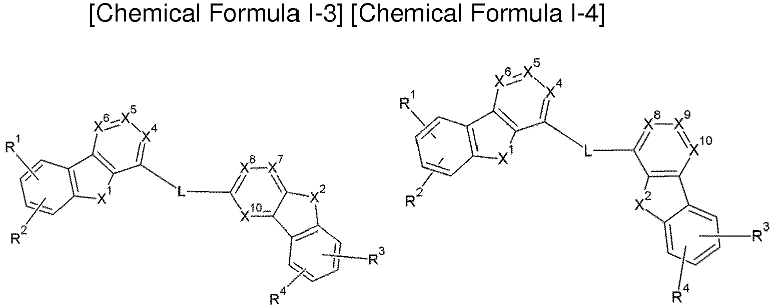 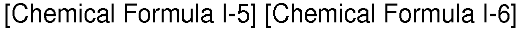 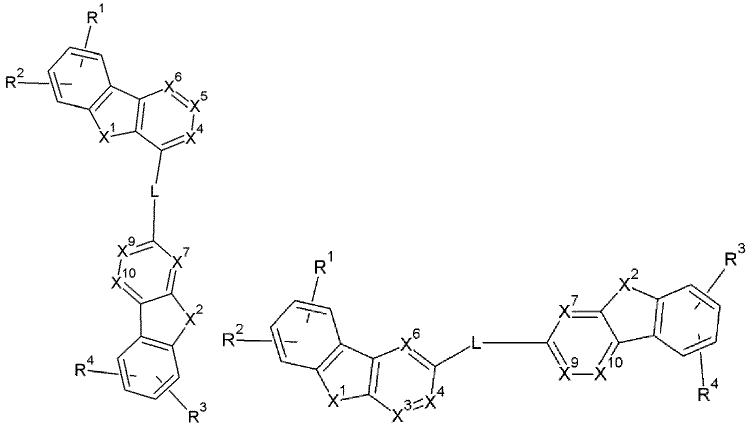 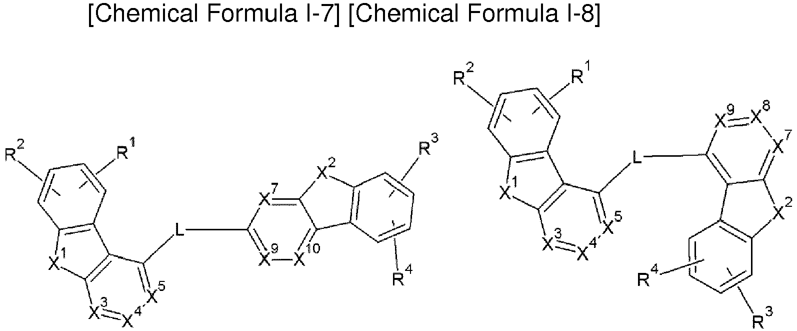 wherein, in Chemical Formulae I-1 to I-8,
X¹ and X² are each independently O or S,
X³ to X⁶ are independently, CR^{a} or N,
at least two of X³ to X⁶ are N,
X⁷ to X¹⁰ are independently, CR^{b} or N,
At least two of X⁷ to X¹⁰ are N,
L is a substituted or unsubstituted C6 to C30 arylene group, and
R¹ to R⁴ and R^{a} and R^{b} are each independently hydrogen, deuterium, a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a substituted or unsubstituted C6 to C30 arylamine group, a substituted or unsubstituted C3 to C40 silyl group, a substituted or unsubstituted C1 to C30 alkylthiol group, a substituted or unsubstituted C6 to C30 arylthiol group, a halogen, a cyano group, a hydroxyl group, an amino group, a nitro group, or a combination thereof,
wherein "substituted" refers to that at least one hydrogen is replaced by deuterium, a halogen, a hydroxyl group, an amino group, a C1 to C30 amine group, a nitro group, a C1 to C40 silyl group, a C1 to C30 alkyl group, a C1 to C10 alkylsilyl group, a C3 to C30 cycloalkyl group, a C2 to C30 heterocycloalkyl group, a C6 to C30 aryl group, C2 to C30 heteroaryl group, a C1 to C20 alkoxy group, a fluoro group, a C1 to C10 trifluoroalkyl group, or a cyano group.

3. The compound for an organic optoelectronic device of claim 2, which is represented by one of Chemical Formulae I-1a to I-8a, I-1b and I-2b: 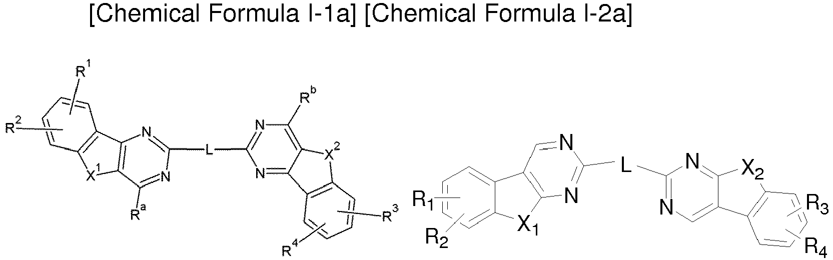 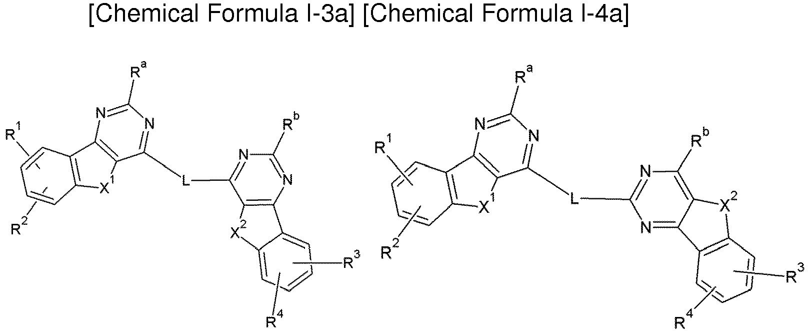 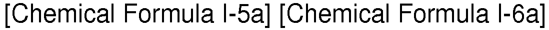 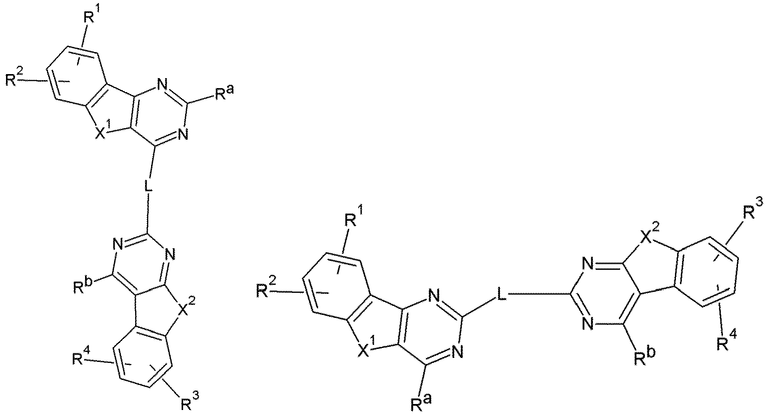 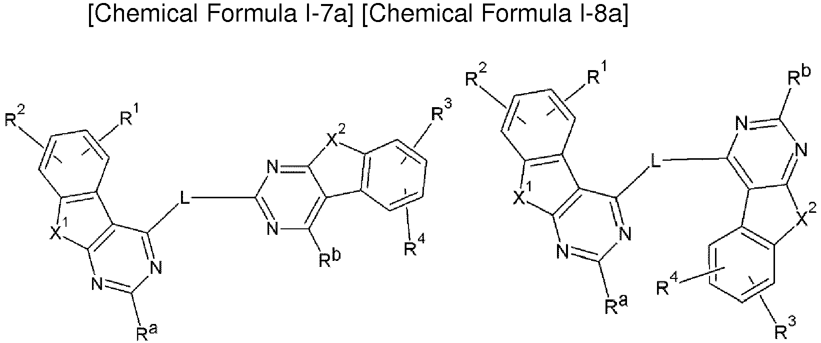 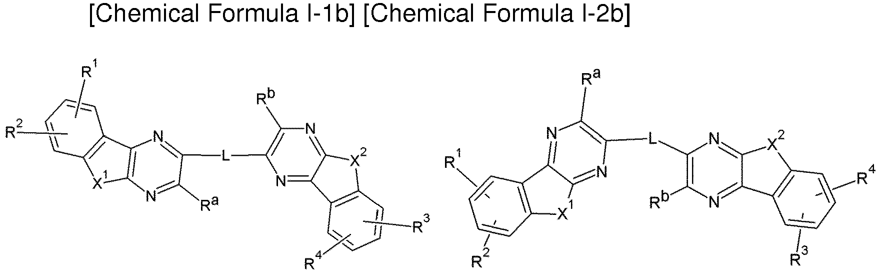 wherein, in Chemical Formulae I-1a to I-8a, I-1b, and I-2b,
X¹ and X² are each independently O or S,
R^{a} and R^{b} are each independently a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a substituted or unsubstituted C6 to C30 arylamine group, a substituted or unsubstituted C3 to C40 silyl group, a substituted or unsubstituted C1 to C30 alkylthiol group, a substituted or unsubstituted C6 to C30 arylthiol group, a halogen, a cyano group, a hydroxyl group, an amino group, a nitro group, or a combination thereof,
L is a substituted or unsubstituted C6 to C30 arylene group, and
R¹ to R⁴ are each independently hydrogen, deuterium, a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a substituted or unsubstituted C6 to C30 arylamine group, a substituted or unsubstituted C3 to C40 silyl group, a substituted or unsubstituted C1 to C30 alkylthiol group, a substituted or unsubstituted C6 to C30 arylthiol group, a halogen, a cyano group, a hydroxyl group, an amino group, a nitro group, or a combination thereof,
wherein "substituted" refers to that at least one hydrogen is replaced by deuterium, a halogen, a hydroxyl group, an amino group, a C1 to C30 amine group, a nitro group, a C1 to C40 silyl group, a C1 to C30 alkyl group, a C1 to C10 alkylsilyl group, a C3 to C30 cycloalkyl group, a C2 to C30 heterocycloalkyl group, a C6 to C30 aryl group, C2 to C30 heteroaryl group, a C1 to C20 alkoxy group, a fluoro group, a C1 to C10 trifluoroalkyl group, or a cyano group.

4. The compound for an organic optoelectronic device of claim 2, which is represented by one of Chemical Formulae I-9 to I-13: 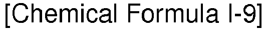 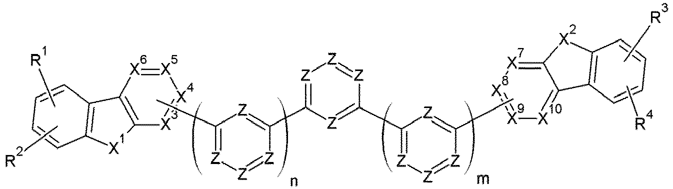 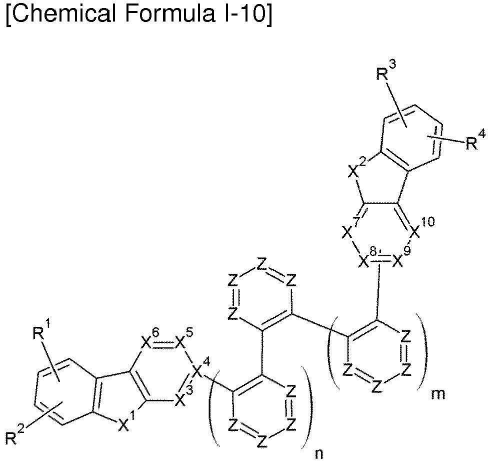 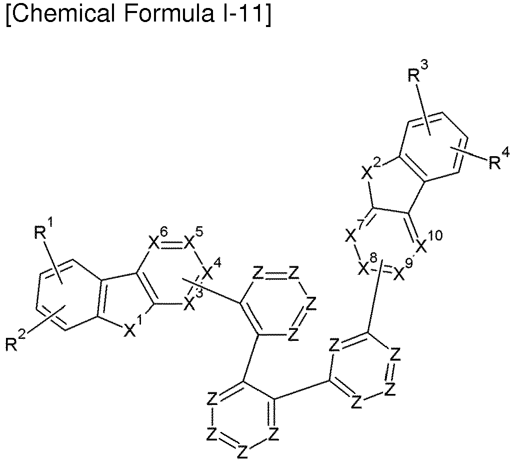 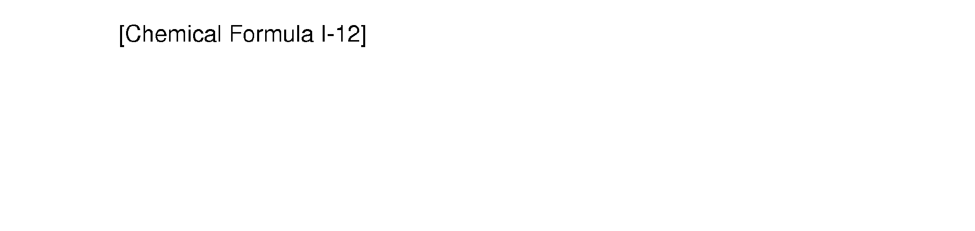 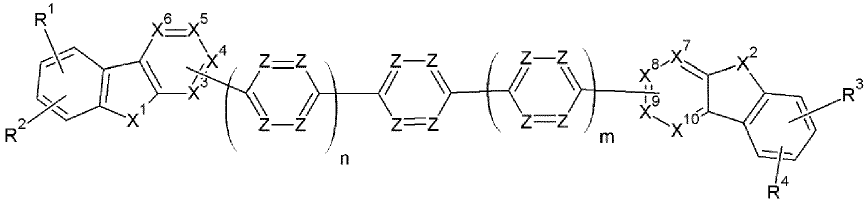 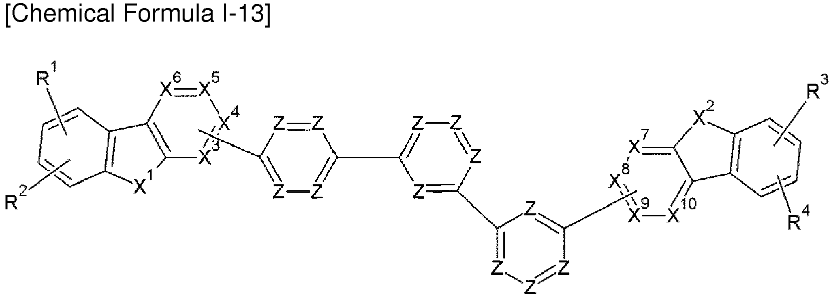 wherein, in Chemical Formulae I-9 to I-13,
X¹ and X² are each independently O or S,
X³ to X⁶ are each independently C, CR^{a} or N,
at least two of X³ to X⁶ are N,
X⁷ to X¹⁰ are each independently, C, CR^{b} or N,
at least two of X⁷ to X¹⁰ are N,
Z is CR^{c},
R¹ to R⁴, R^{a}, R^{b}, and R^{c} are each independently hydrogen, deuterium, a substituted or unsubstituted C1 to C30 alkyl group, and a substituted or unsubstituted C6 to C30 aryl group, and
n and m are independently integers ranging from 0 to 2,
wherein "substituted" refers to that at least one hydrogen is replaced by deuterium, a halogen, a hydroxyl group, an amino group, a C1 to C30 amine group, a nitro group, a C1 to C40 silyl group, a C1 to C30 alkyl group, a C1 to C10 alkylsilyl group, a C3 to C30 cycloalkyl group, a C2 to C30 heterocycloalkyl group, a C6 to C30 aryl group, C2 to C30 heteroaryl group, a C1 to C20 alkoxy group, a fluoro group, a C1 to C10 trifluoroalkyl group, or a cyano group.

5. The compound for an organic optoelectronic device of claim 4, wherein at least one of n and m is 1.

6. The compound for an organic optoelectronic device of claim 1, wherein the L is a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted quarterphenyl group, a substituted or unsubstituted chrysenyl group, a substituted or unsubstituted triphenylenyl group, a substituted or unsubstituted perylenyl group, a substituted or unsubstituted fluorenyl group, or a combination thereof.

7. The compound for an organic optoelectronic device of claim 6, wherein the L is selected from the linking groups listed in Group 1: 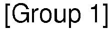 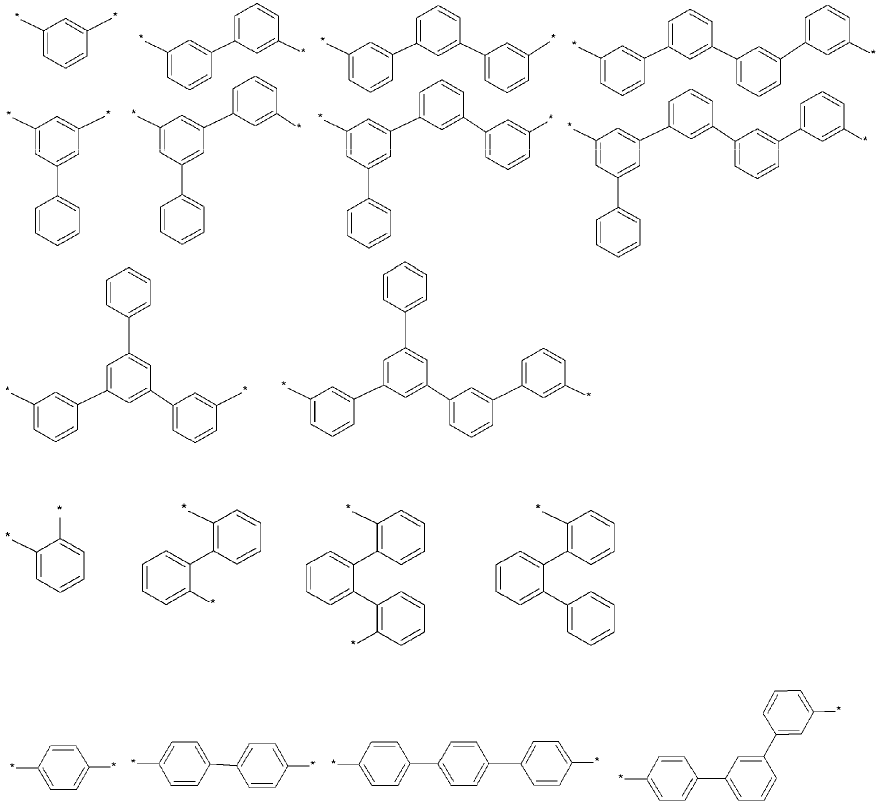 wherein, in Group 1, * is a linking point.

8. The compound for an organic optoelectronic device of claim 6, wherein the compound is compounds in Group 2:

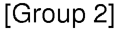

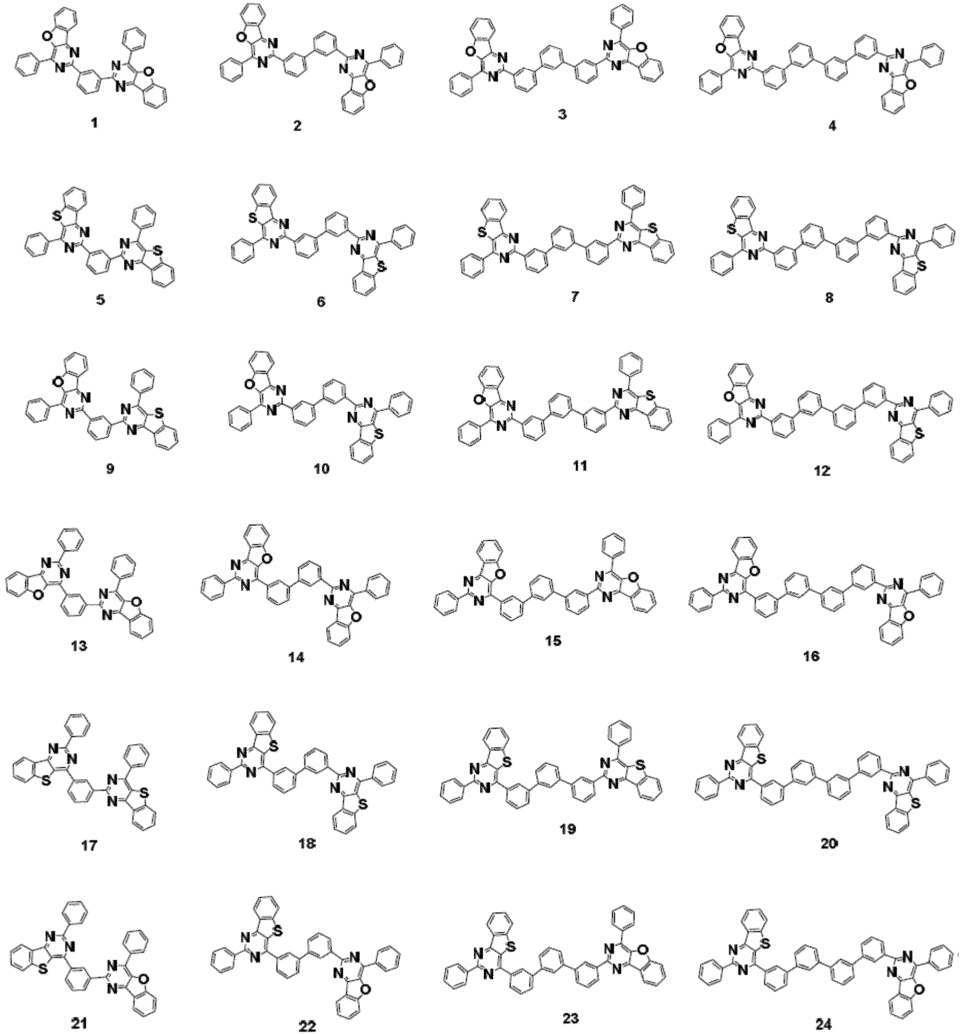

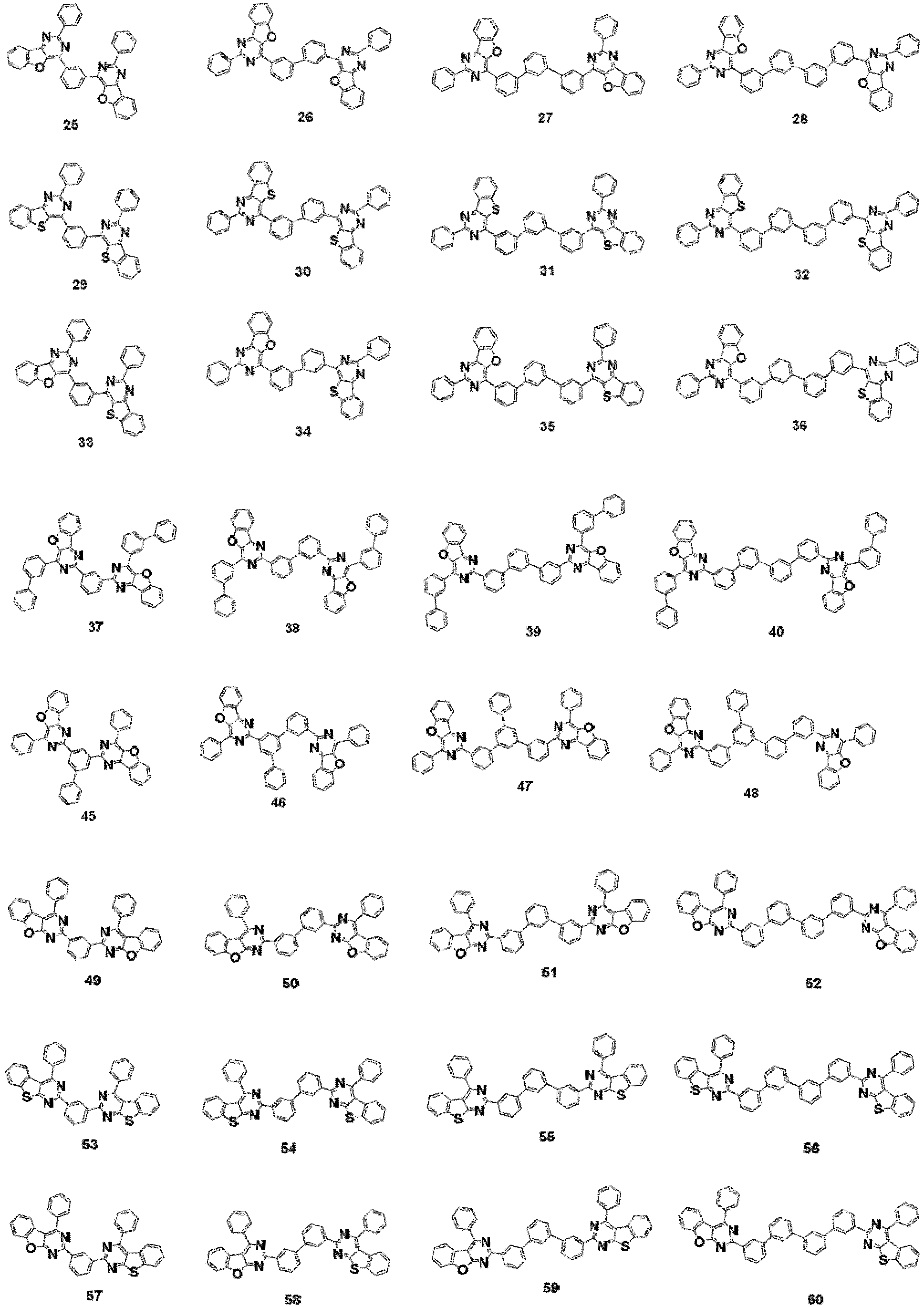

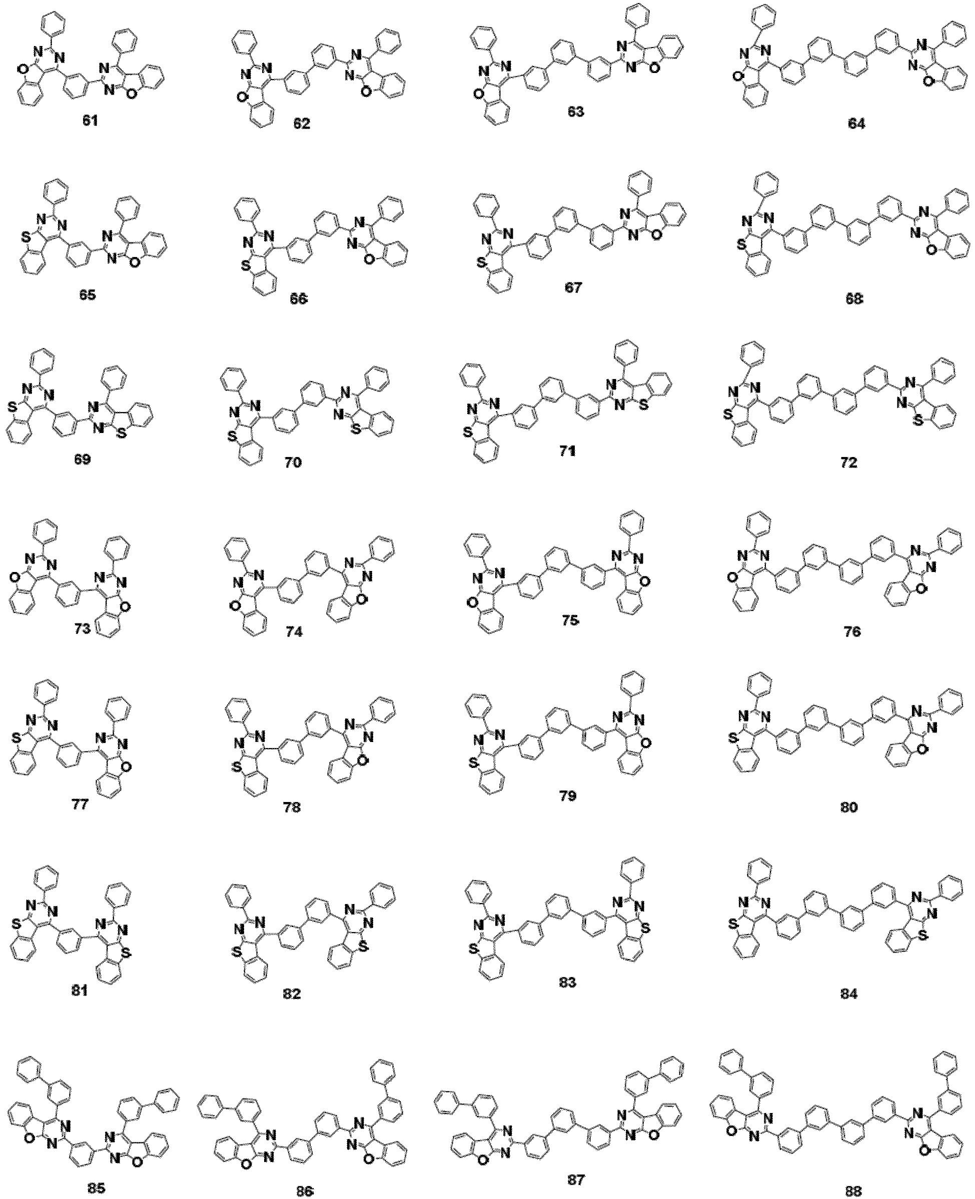

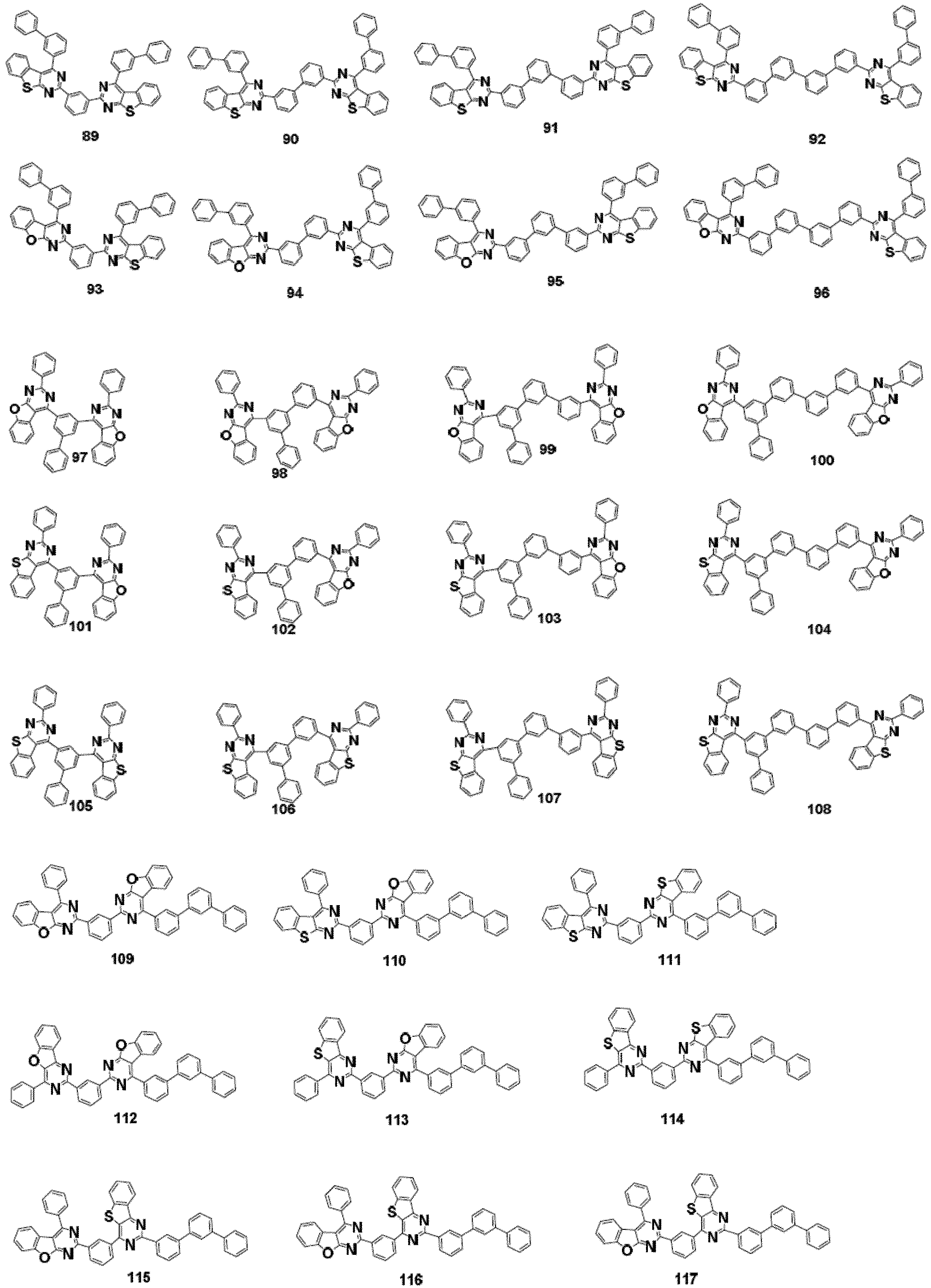

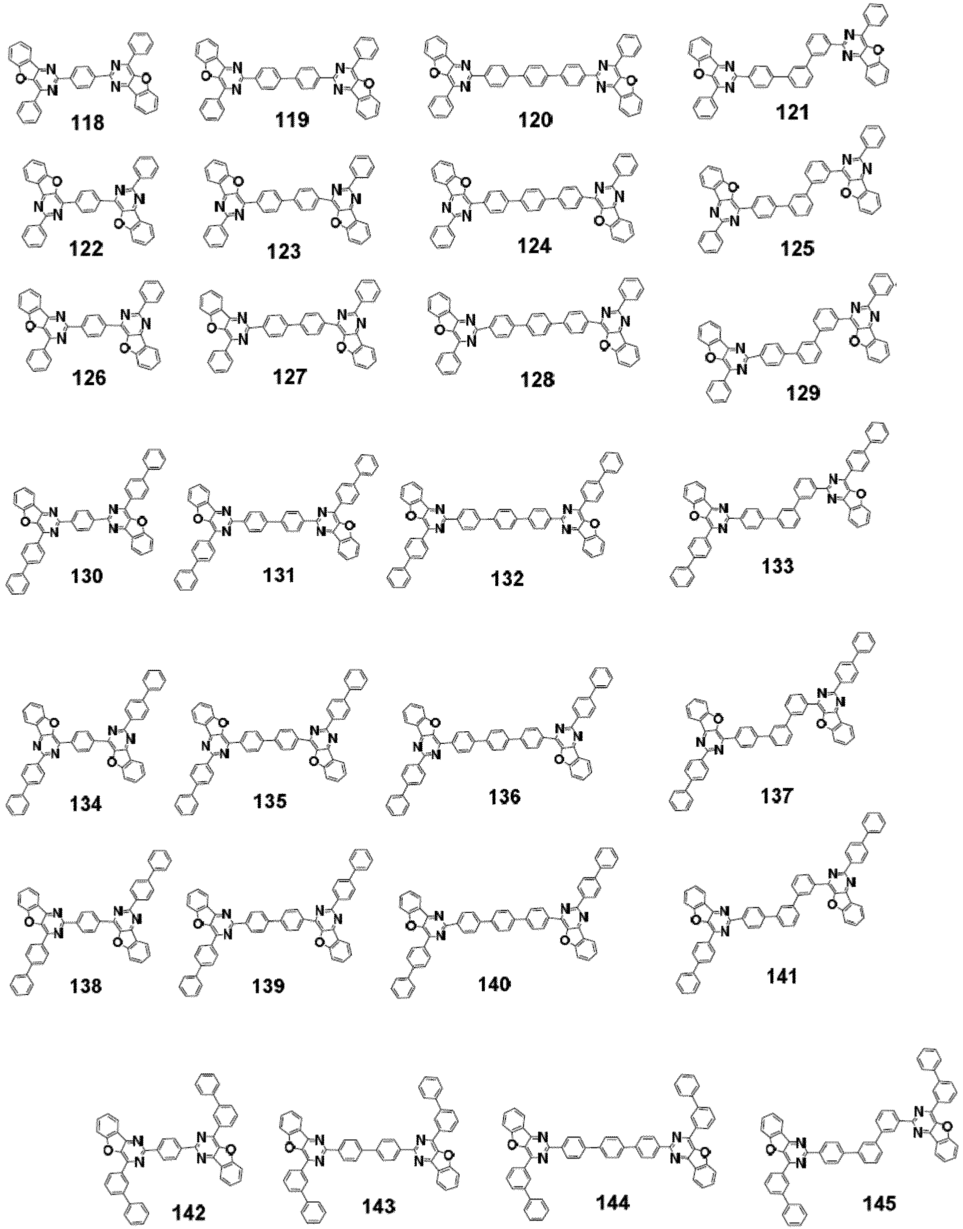

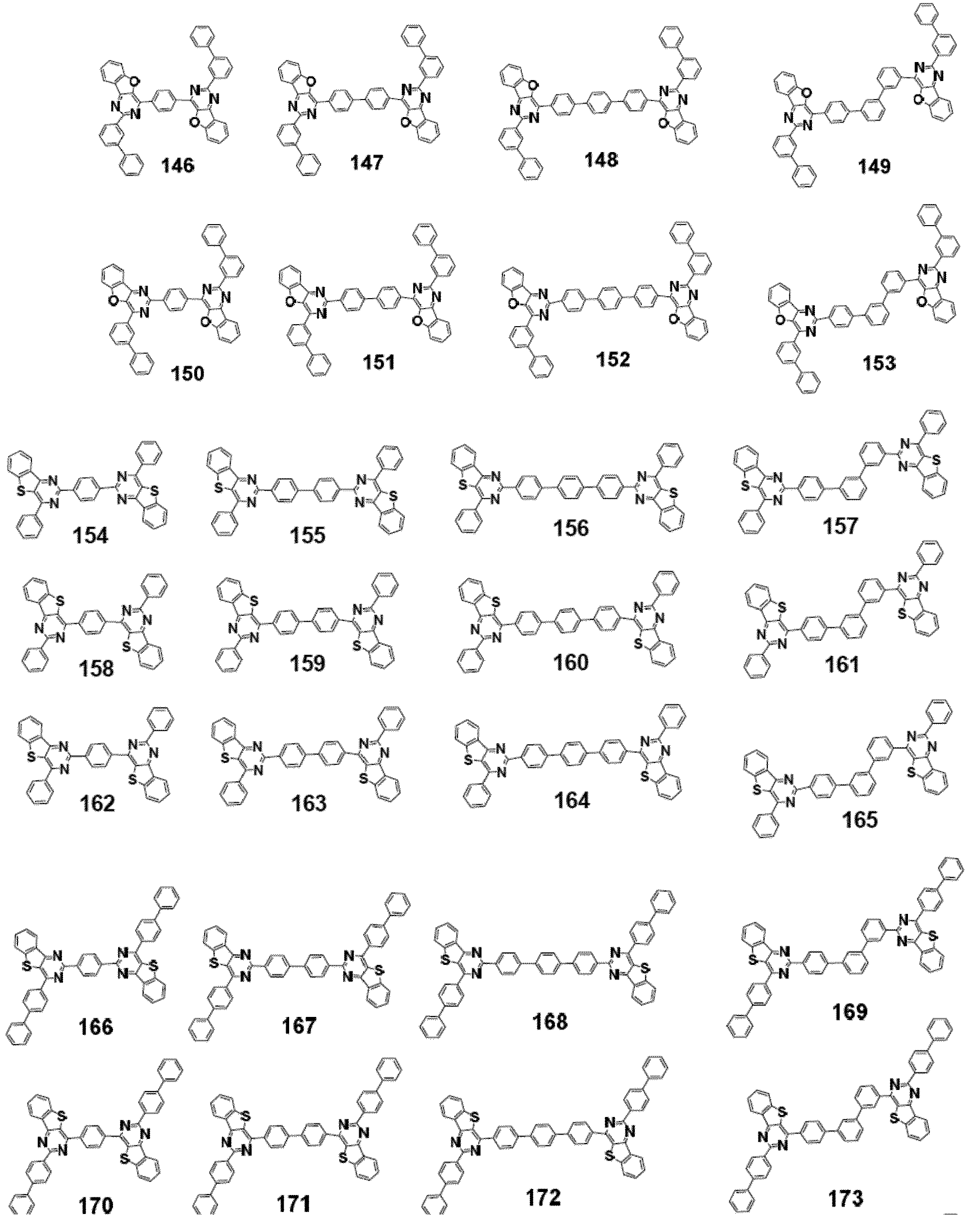

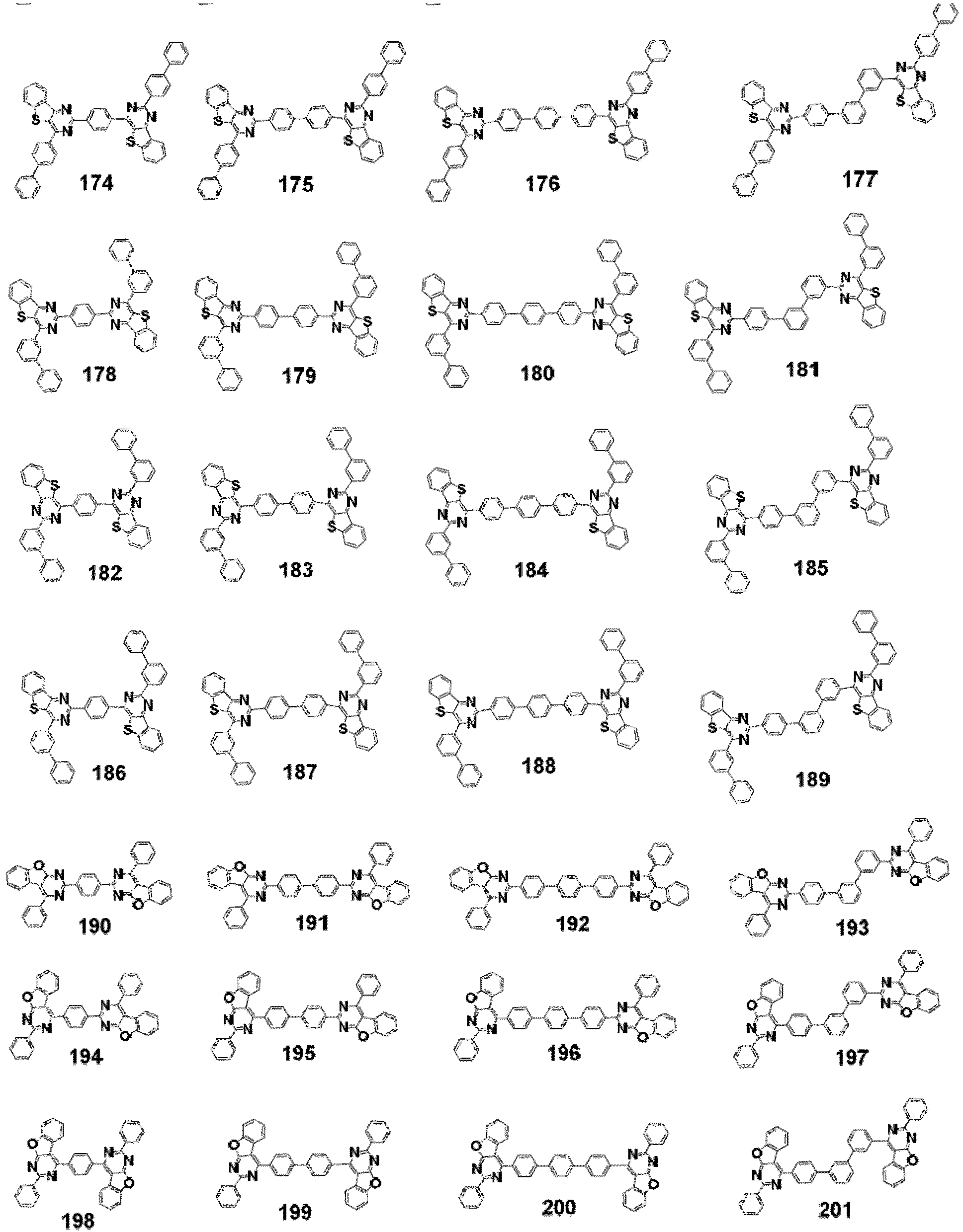

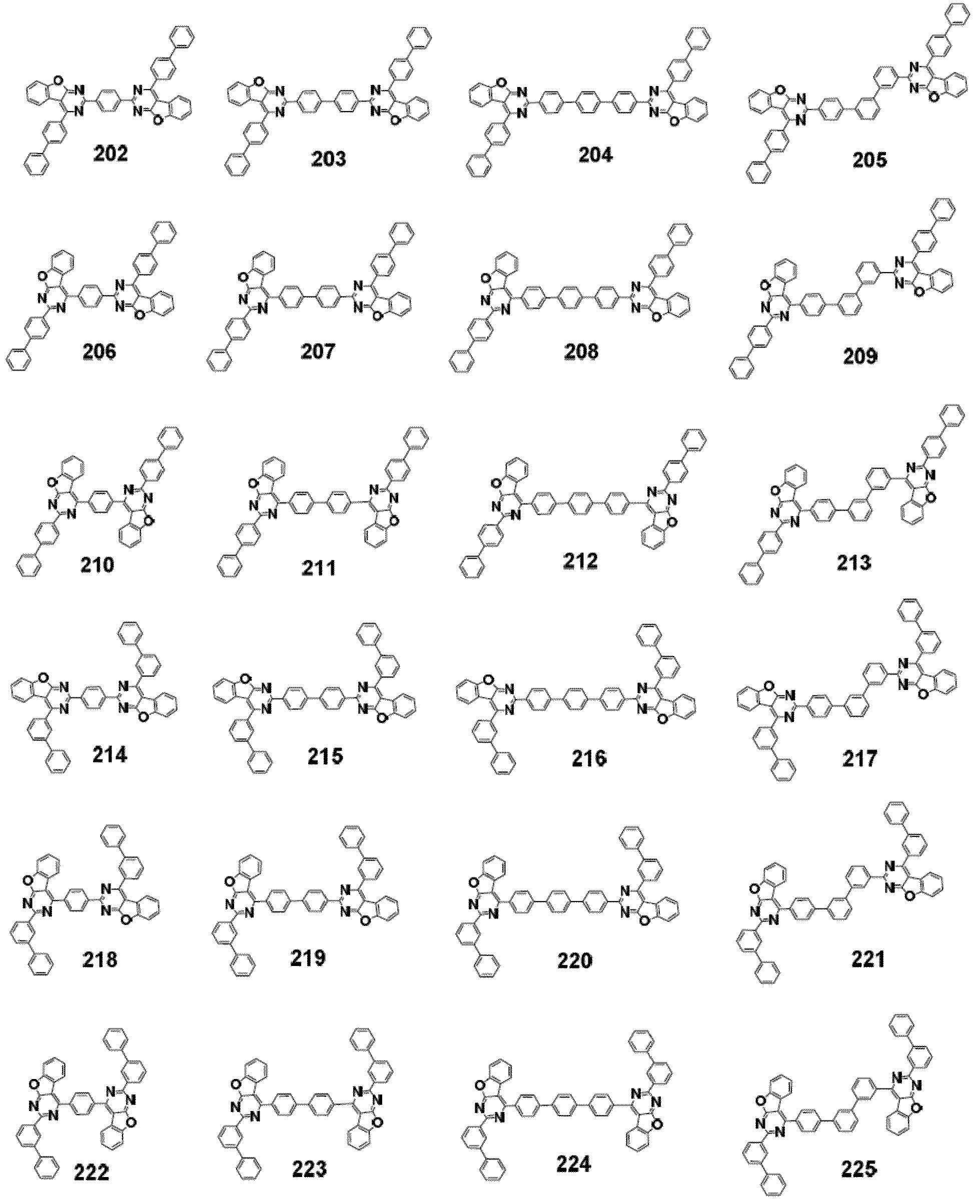

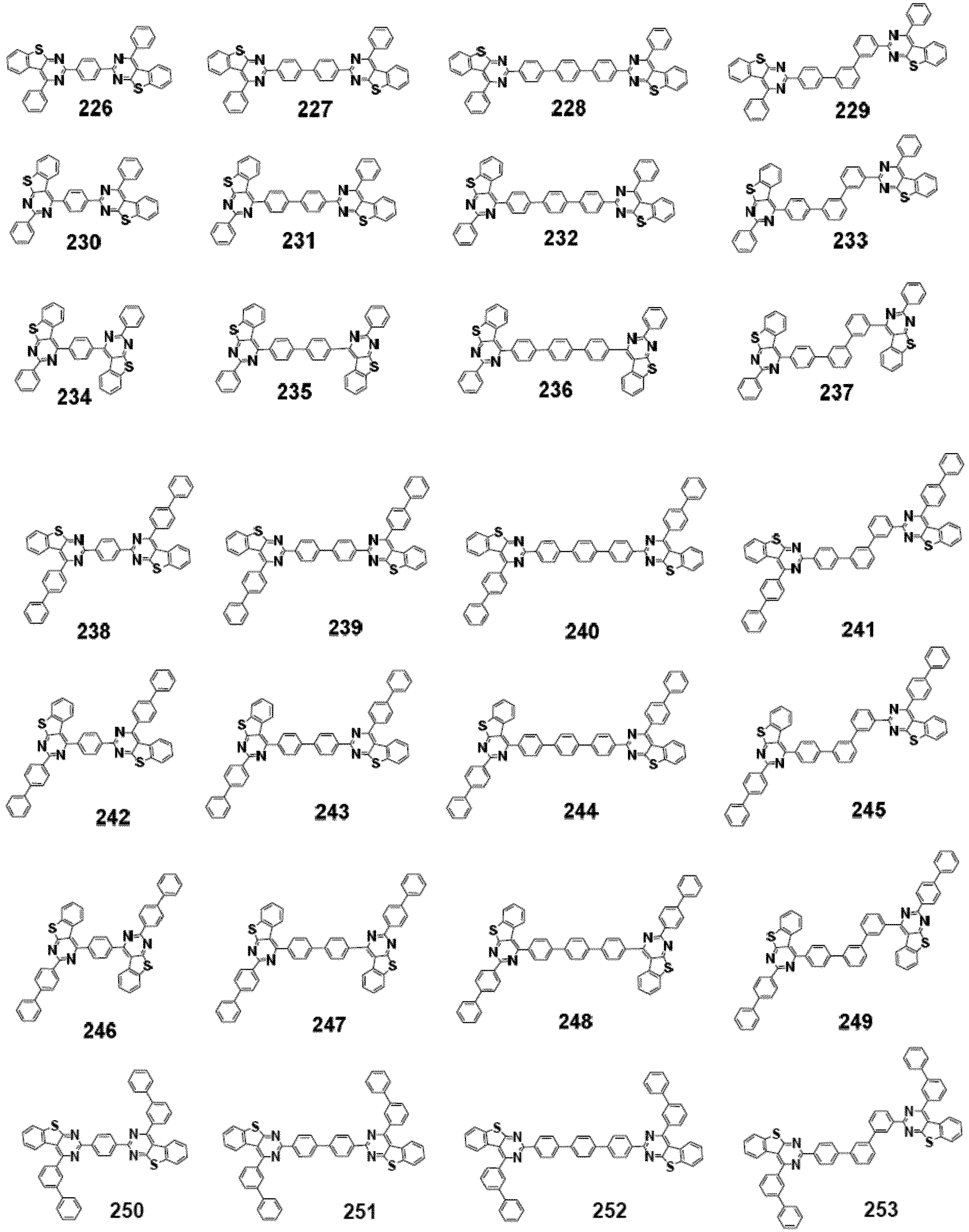

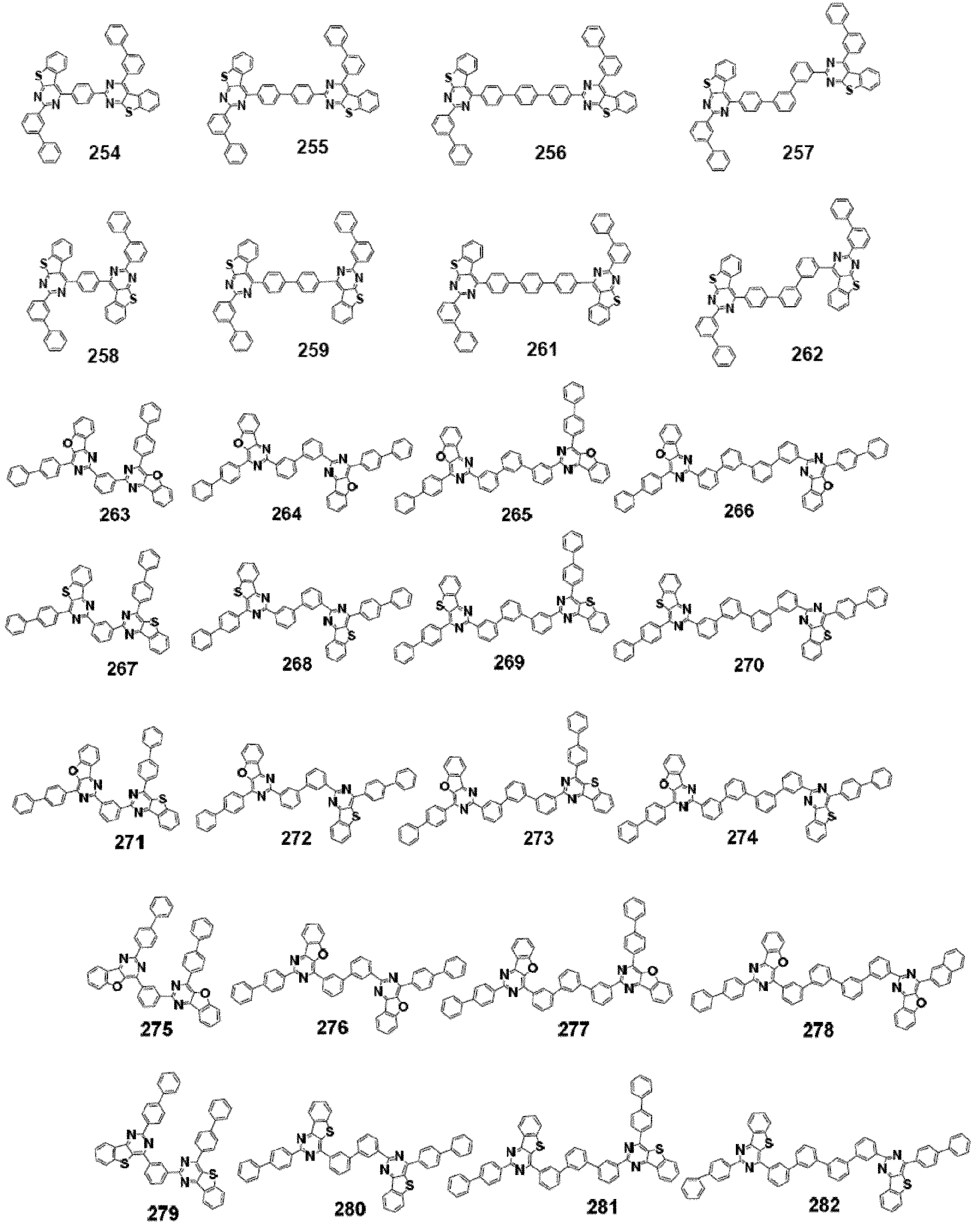

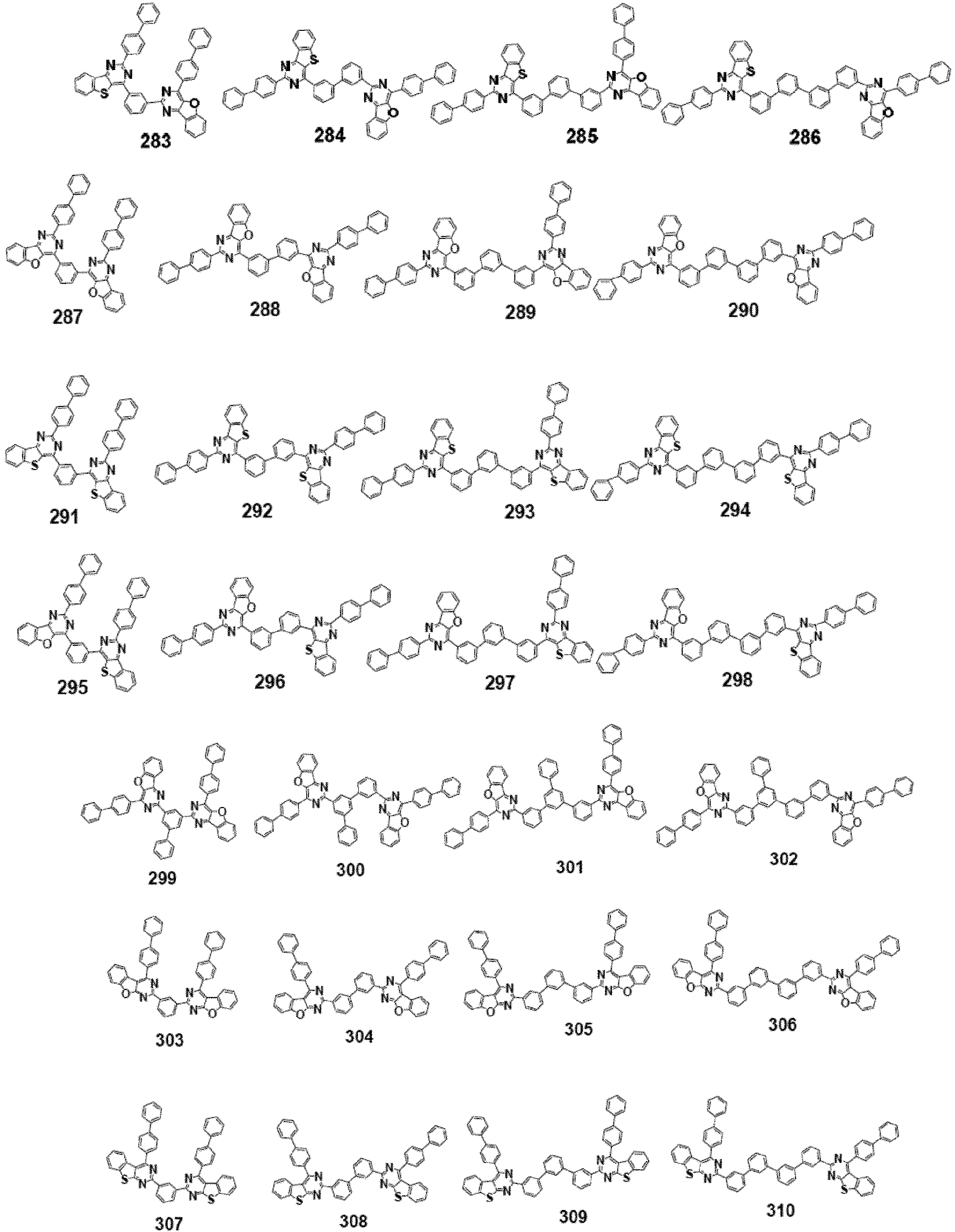

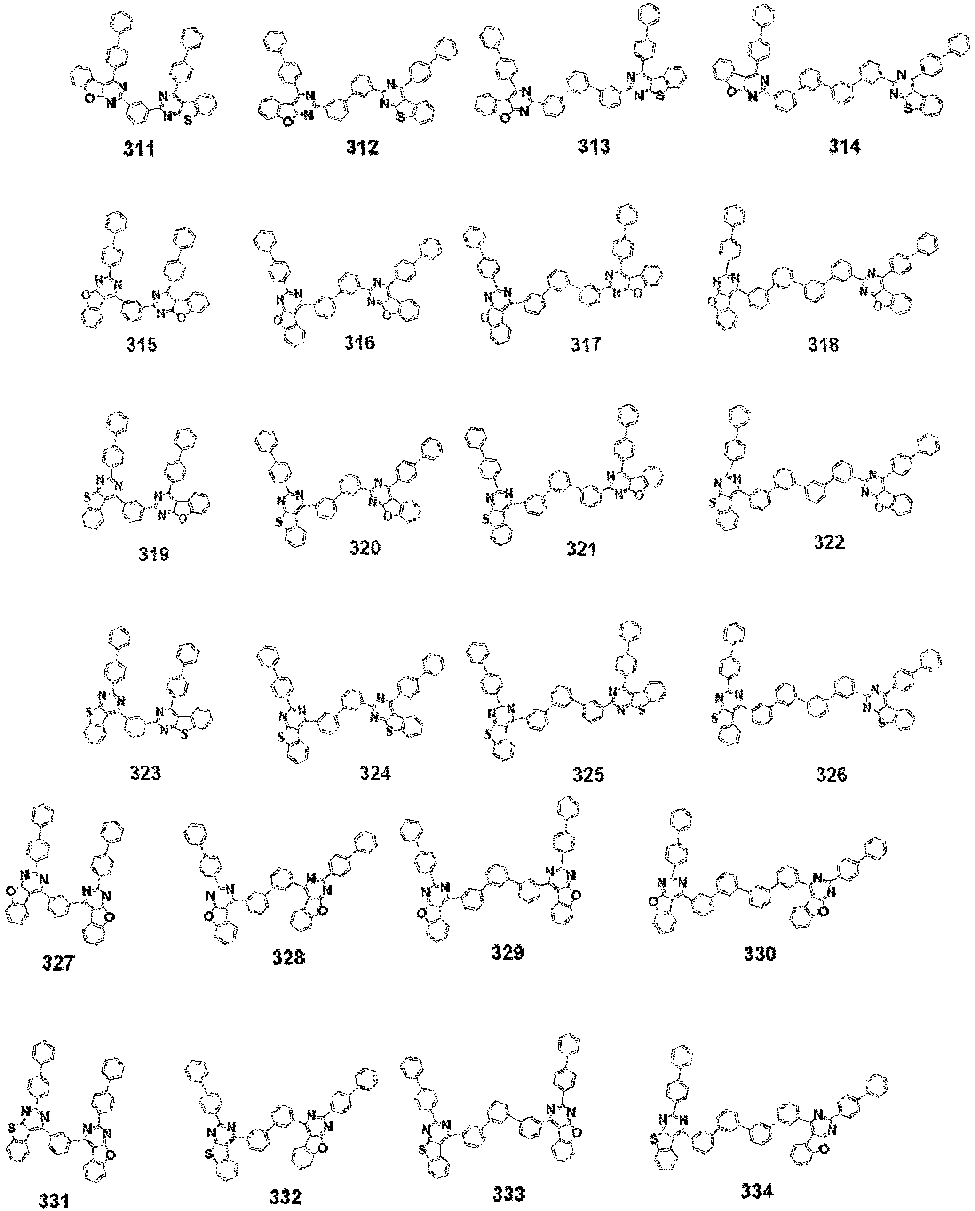

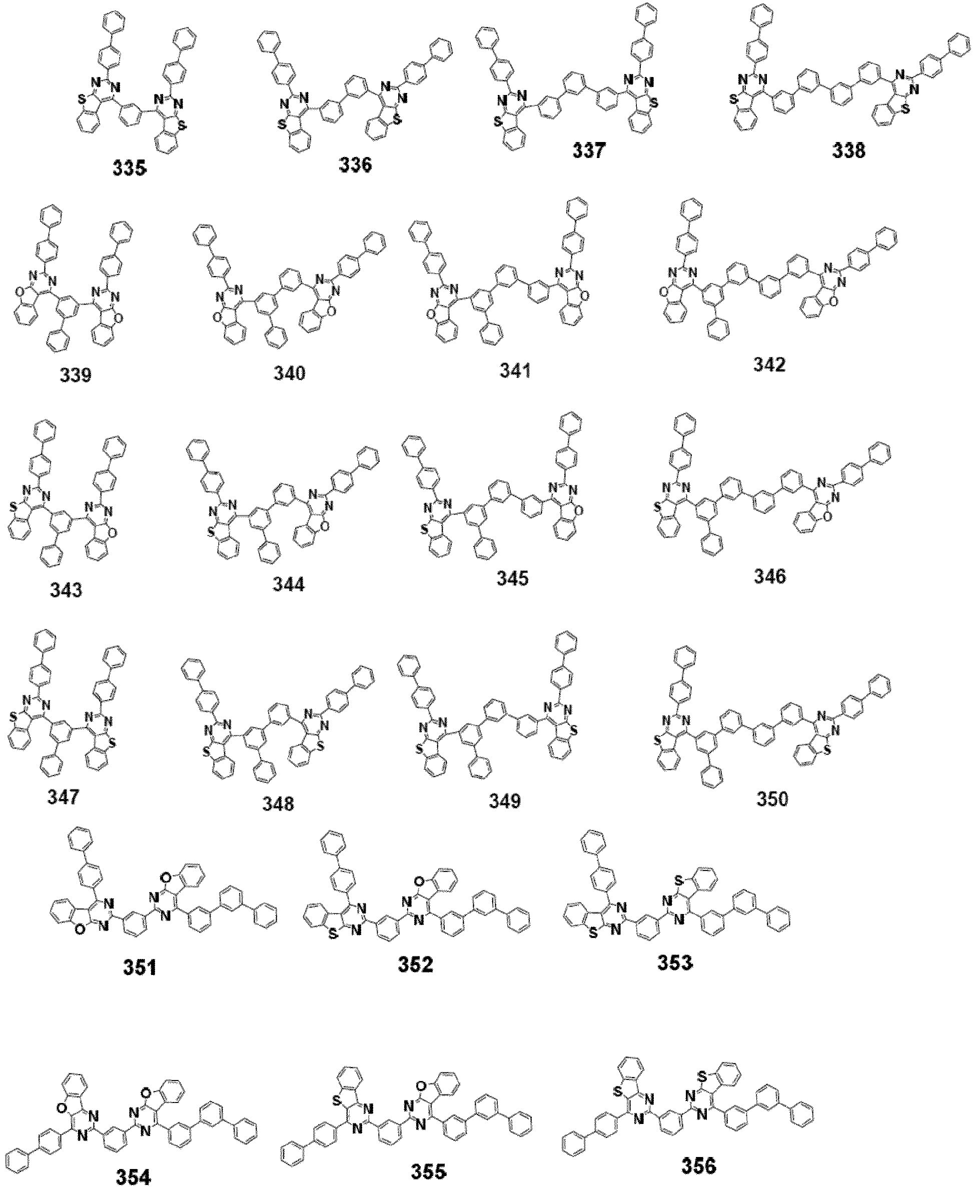

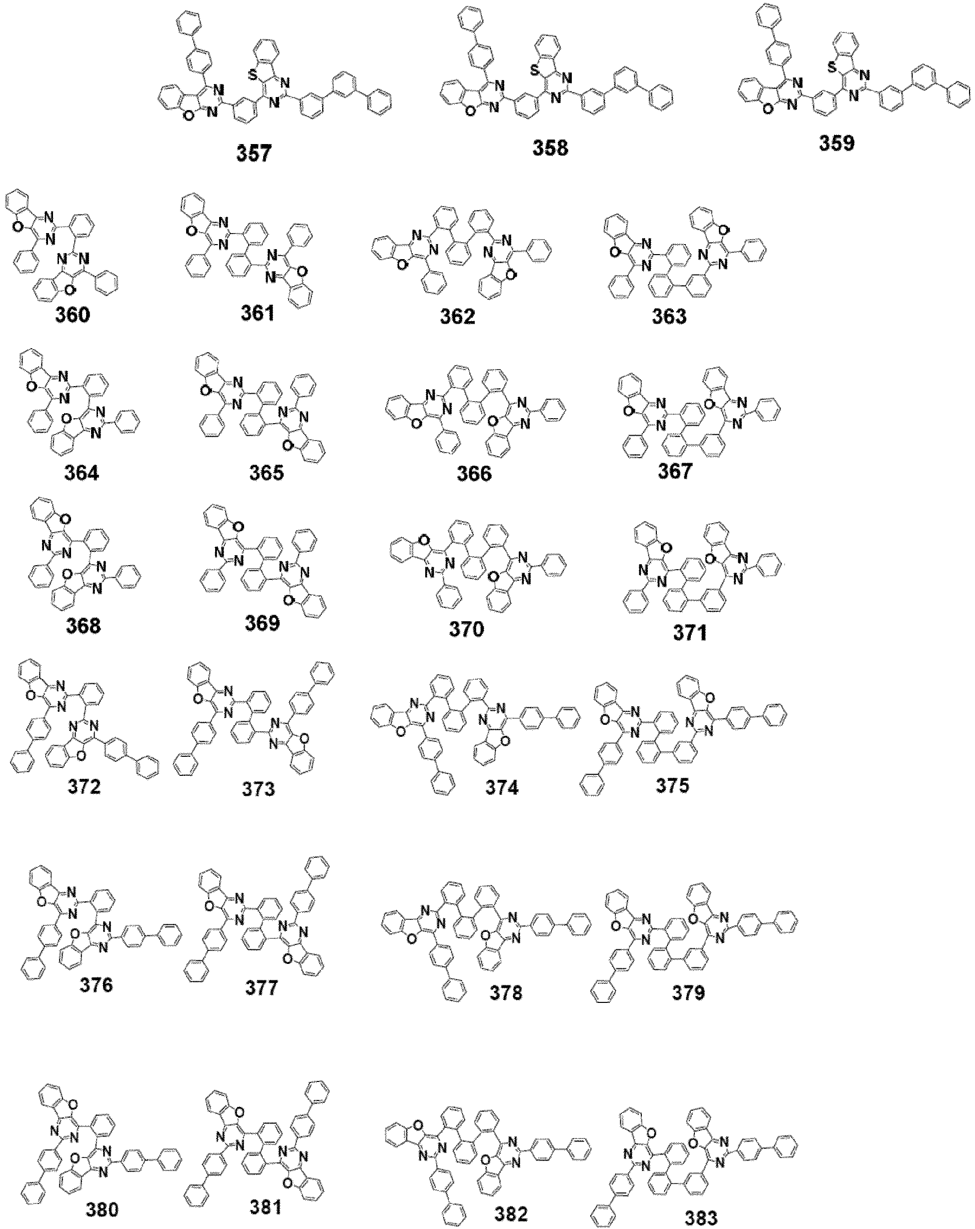

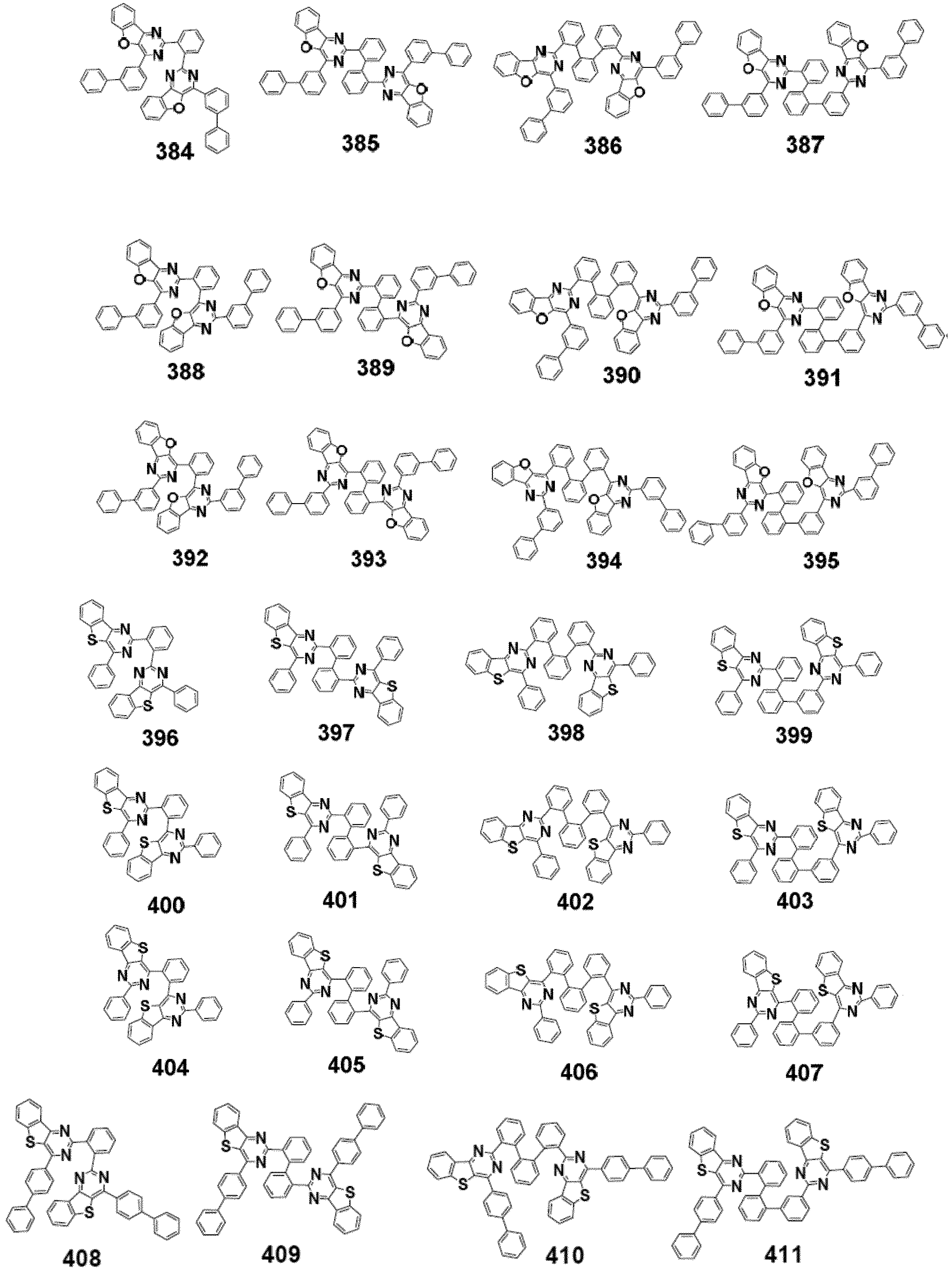

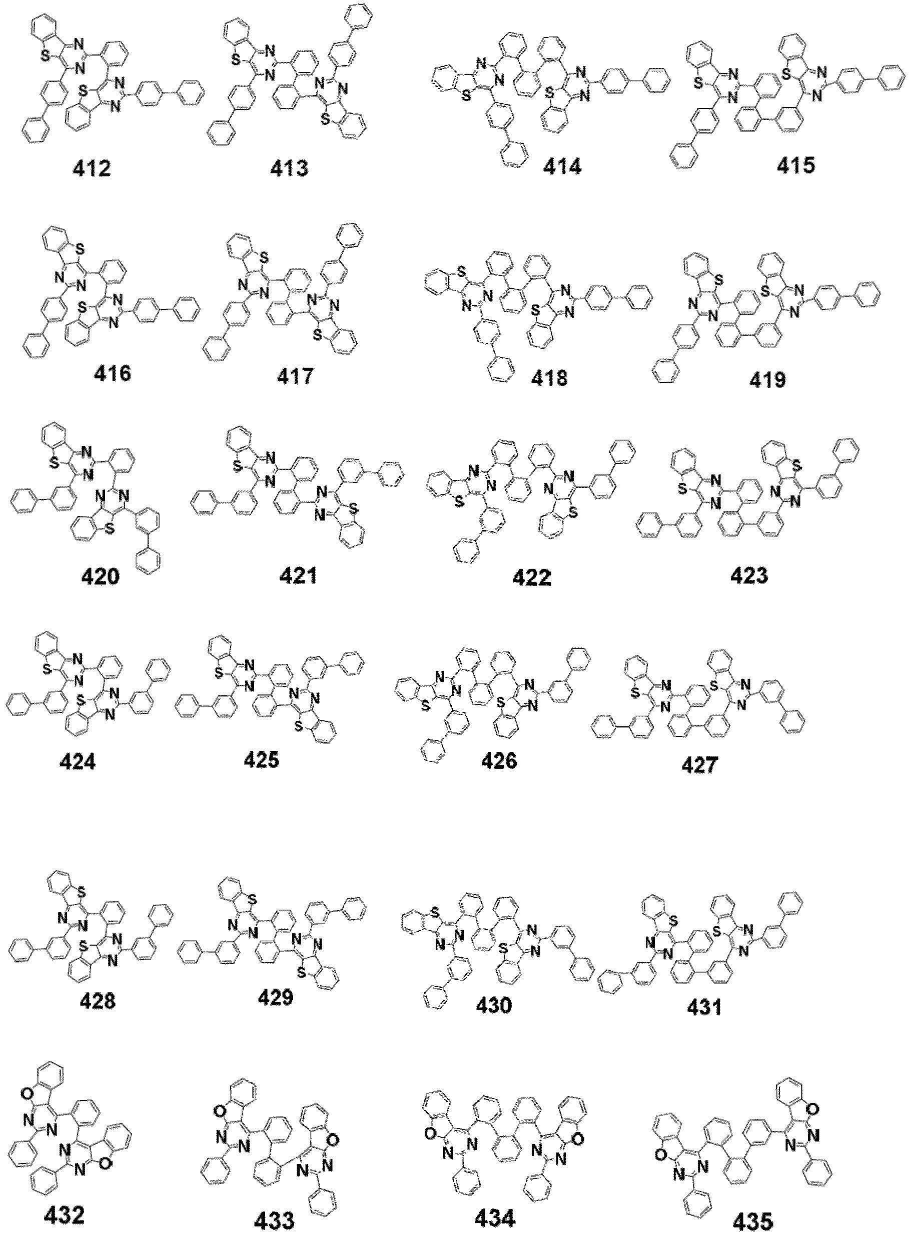

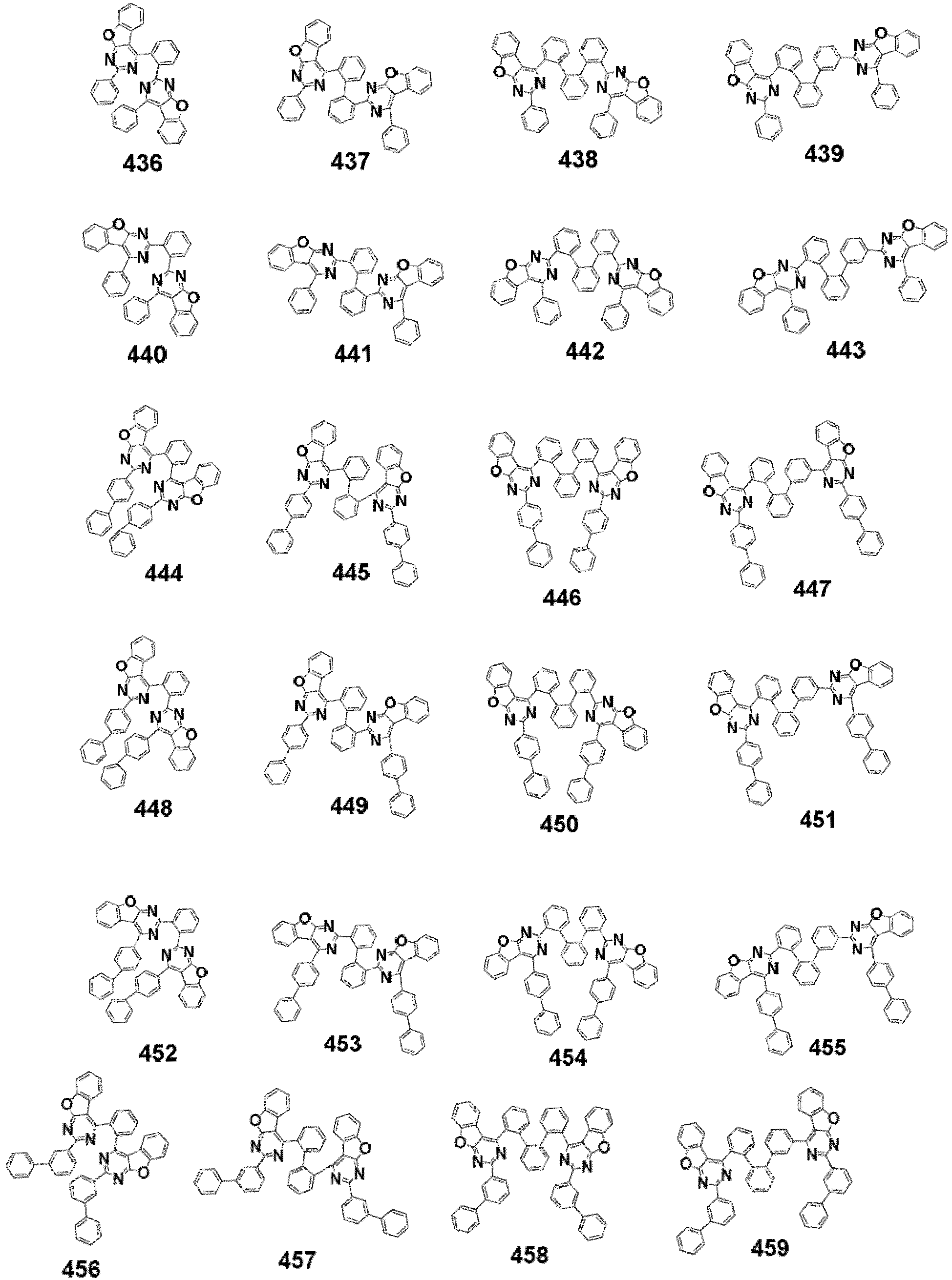

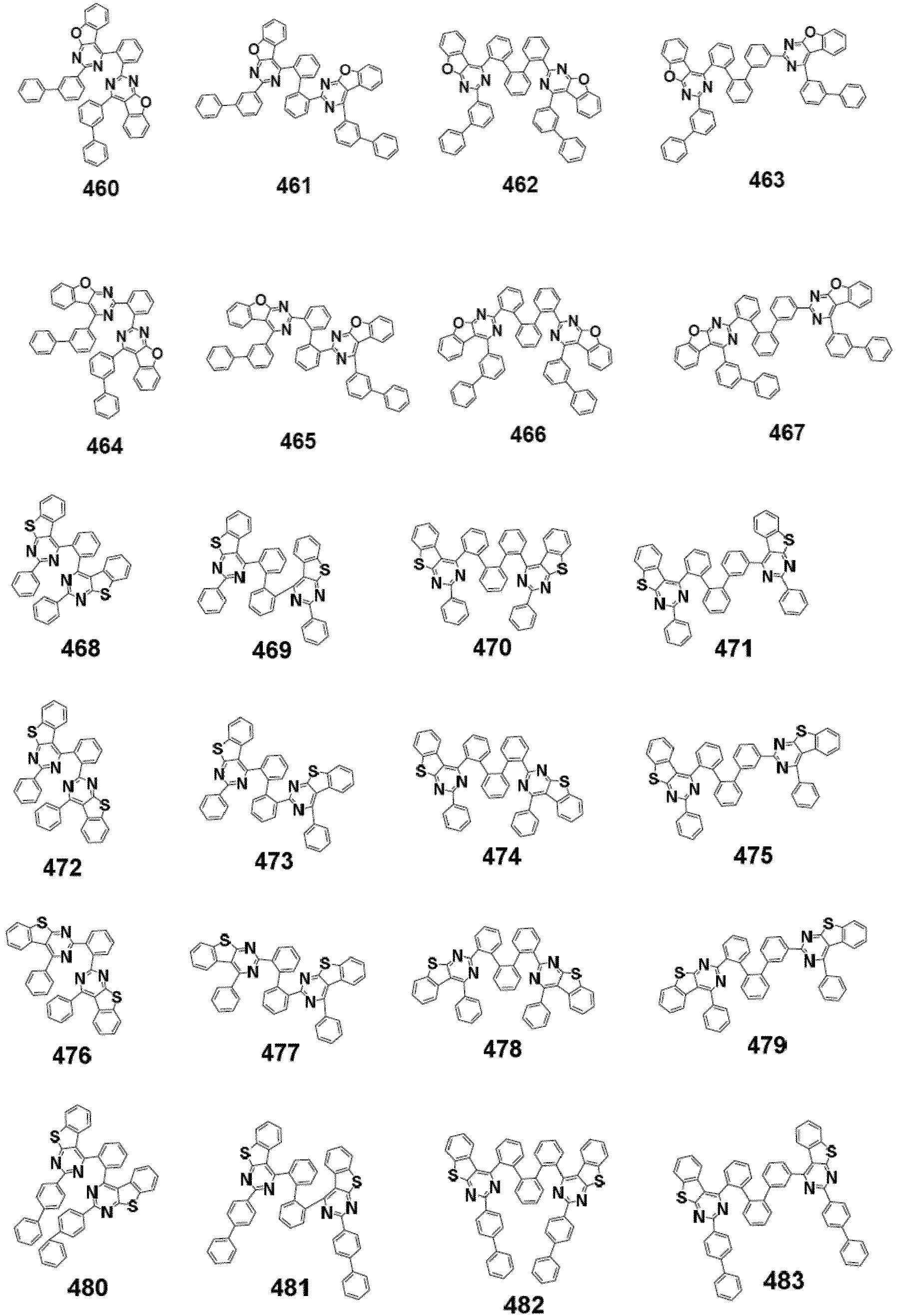

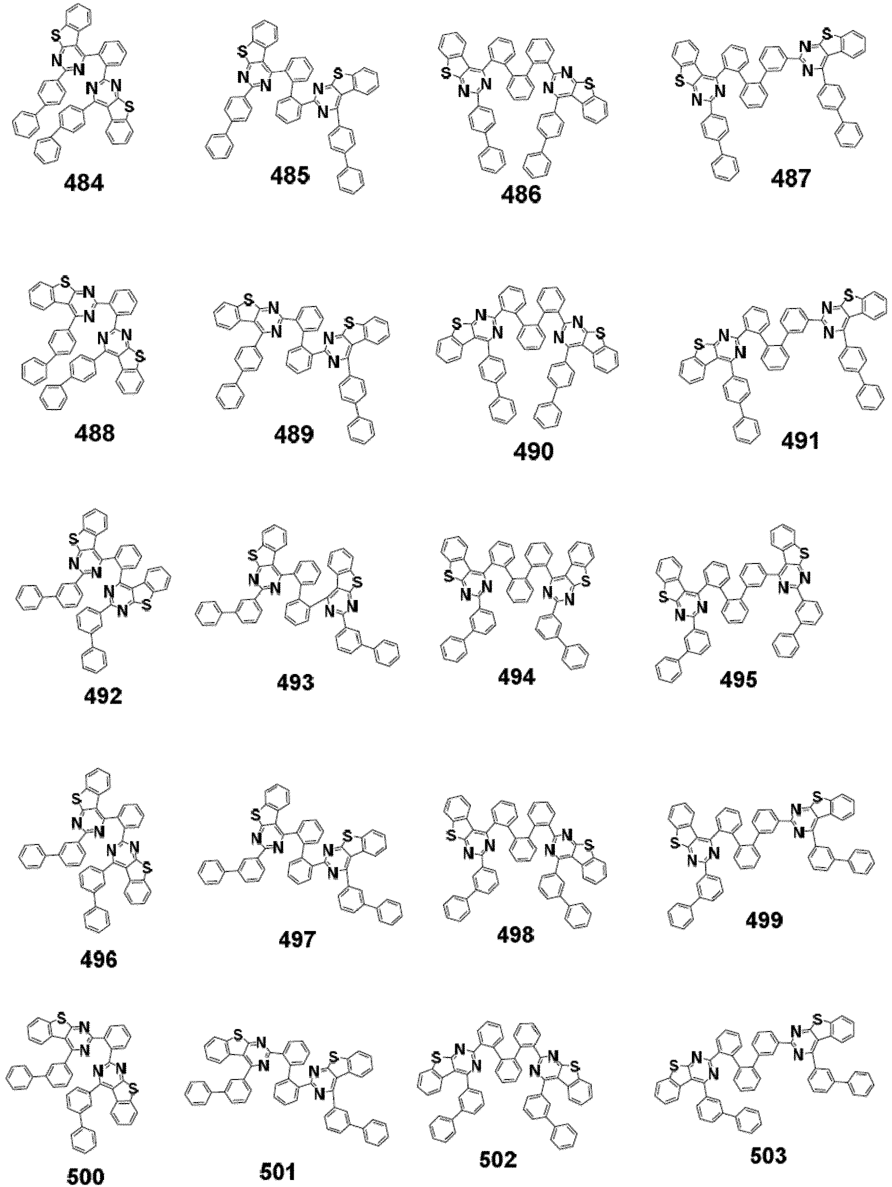

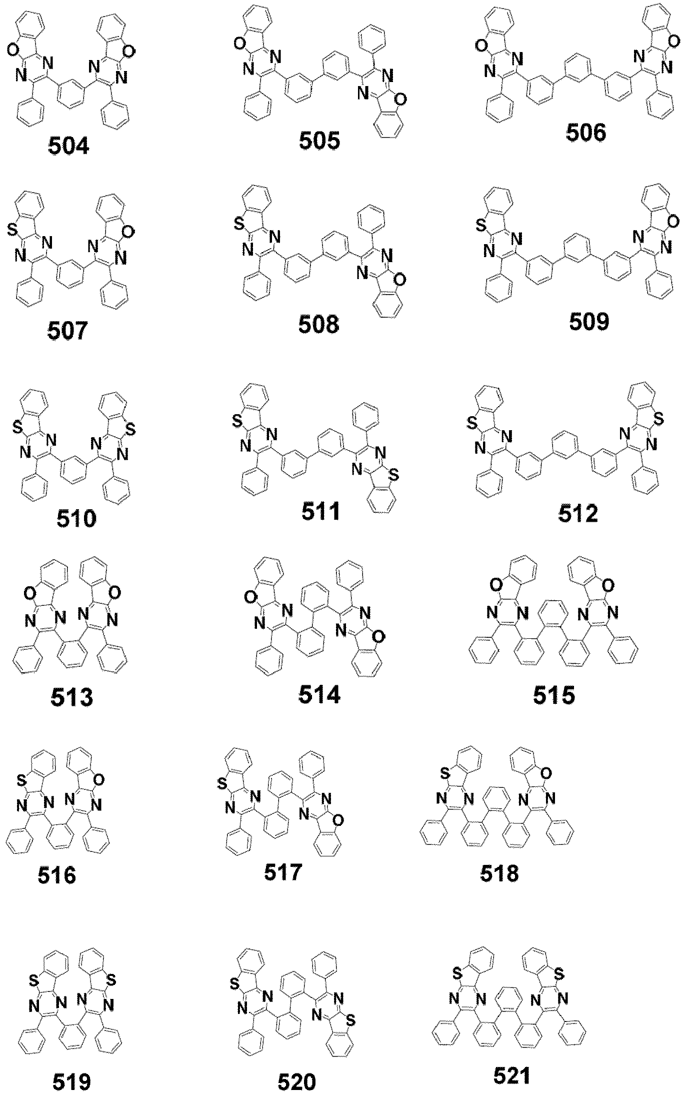

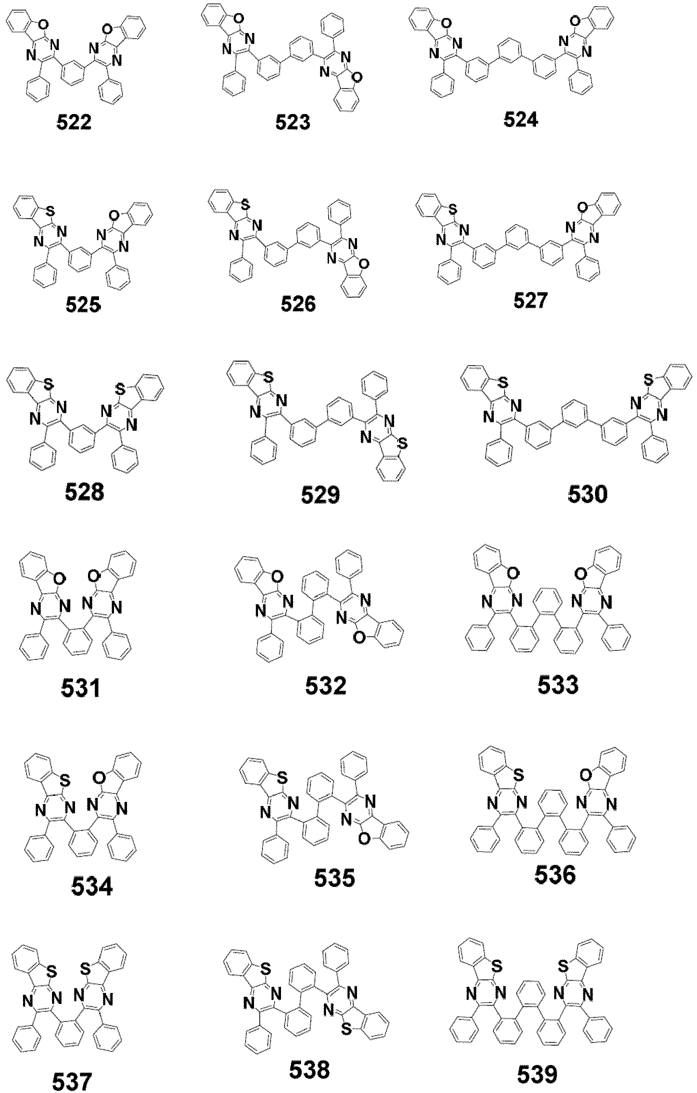

9. An organic optoelectronic device comprising
an anode and a cathode facing each other, and
at least one organic layer between the anode and the cathode,
wherein the organic layer comprises the compound for an organic optoelectronic device of any one of claim 1 to claim 8.

10. The organic optoelectronic device of claim 9, wherein the organic layer comprises an emission layer, and
the emission layer comprises the compound for an organic optoelectronic device.

11. The organic optoelectronic device of claim 10, wherein the compound for an organic optoelectronic device is included as a host in the emission layer.

12. The organic optoelectronic device of claim 9, wherein the organic layer comprises at least one auxiliary layer selected from a hole injection layer (HIL), a hole transport layer (HTL), a hole transport auxiliary layer, an electron transport auxiliary layer, an electron transport layer (ETL), and an electron injection layer (EIL), and
the auxiliary layer comprises the compound for an organic optoelectronic device.

13. A display device comprising the organic optoelectronic device of claim 9.

## Patentansprüche

1. Verbindung für ein organisches optoelektronisches Bauelement, die dargestellt ist durch die chemische Formel I: 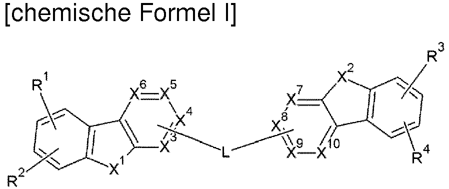 wobei in der chemischen Formel I
X¹ und X² jeweils unabhängig O oder S sind,
X³ bis X⁶ jeweils unabhängig C, CR^{a} oder N sind,
wobei mindestens zwei von X³ bis X⁶ N sind,
X⁷ bis X¹⁰ jeweils unabhängig C, CR^{b} oder N sind,
wobei mindestens zwei von X⁷ bis X¹⁰ N sind,
L eine substituierte oder unsubstituierte C6- bis C30-Arylengruppe ist, und
R¹ bis R⁴ und R^{a} und R^{b} jeweils unabhängig Wasserstoff, Deuterium, eine substituierte oder unsubstituierte C1- bis C30-Alkylgruppe, eine substituierte oder unsubstituierte C3- bis C30-Cycloalkylgruppe, eine substituierte oder unsubstituierte C6- bis C30-Arylgruppe, eine substituierte oder unsubstituierte C2- bis C30-Heteroarylgruppe, eine substituierte oder unsubstituierte C6- bis C30-Arylamingruppe, eine substituierte oder unsubstituierte C3-bis C40-Silylgruppe, eine substituierte oder unsubstituierte C1- bis C30-Al-kylthiolgruppe, eine substituierte oder unsubstituierte C6- bis C30-Arylthi-olgruppe, ein Halogen, eine Cyanogruppe, eine Hydroxylgruppe, eine Aminogruppe, eine Nitrogruppe oder eine Kombination davon sind,
wobei "substituiert" bedeutet, dass mindestens ein Wasserstoff durch Deuterium, ein Halogen, eine Hydroxylgruppe, eine Aminogruppe, eine C1-bis C30-Amingruppe, eine Nitrogruppe, eine C1- bis C40-Silylgruppe, eine C1- bis C30-Alkylgruppe, eine C1- bis C10-Alkylsilylgruppe, eine C3- bis C30-Cycloalkylgruppe, eine C2- bis C30-Heterocycloalkylgruppe, eine C6-bis C30-Arylgruppe, eine C2- bis C30-Heteroarylgruppe, eine C1- bis C20-Alkoxygruppe, eine Fluorgruppe, eine C1- bis C10-Trifluoralkylgruppe oder eine Cyanogruppe ersetzt ist.

2. Verbindung nach Anspruch 1, die durch eine der chemischen Formeln I-1 bis I-8 dargestellt ist: 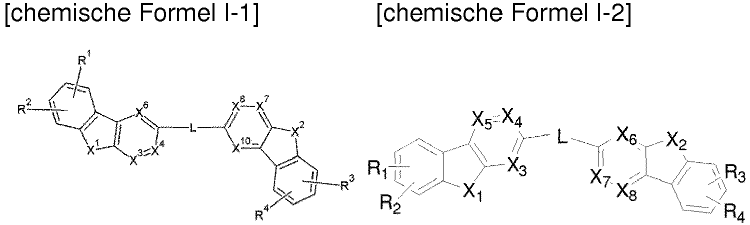 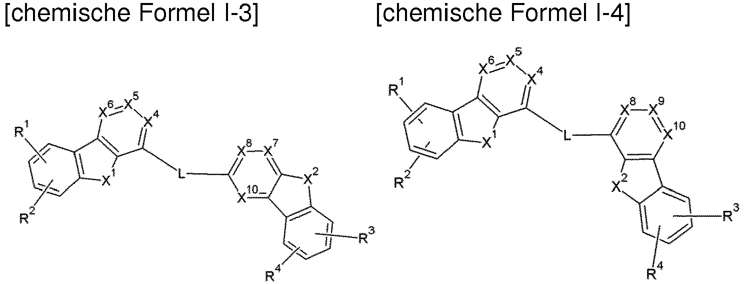 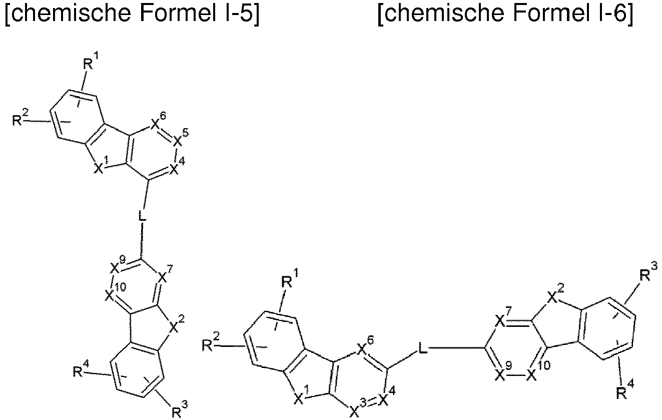 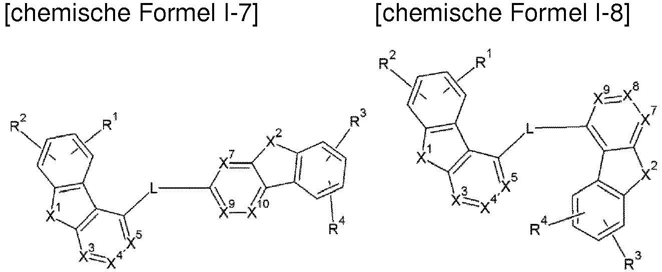 wobei in den chemischen Formeln I-1 bis I-8
X¹ und X² jeweils unabhängig O oder S sind,
X³ bis X⁶ jeweils unabhängig CR^{a} oder N sind,
wobei mindestens zwei X³ bis X⁶ N sind,
X⁷ bis X¹⁰ jeweils unabhängig CR^{b} oder N sind,
wobei mindestens zwei X⁷ bis X¹⁰ N sind,
L eine substituierte oder unsubstituierte C6- bis C30-Arylengruppe ist, und
R¹ bis R⁴ und R^{a} und R^{b} jeweils unabhängig Wasserstoff, Deuterium, eine substituierte oder unsubstituierte C1- bis C30-Alkylgruppe, eine substituierte oder unsubstituierte C3- bis C30-Cycloalkylgruppe, eine substituierte oder unsubstituierte C6- bis C30-Arylgruppe, eine substituierte oder unsubstituierte C2- bis C30-Heteroarylgruppe, eine substituierte oder unsubstituierte C6- bis C30-Arylamingruppe, eine substituierte oder unsubstituierte C3-bis C40-Silylgruppe, eine substituierte oder unsubstituierte C1- bis C30-Al-kylthiolgruppe, eine substituierte oder unsubstituierte C6- bis C30-Arylthi-olgruppe, ein Halogen, eine Cyanogruppe, eine Hydroxylgruppe, eine Aminogruppe, eine Nitrogruppe oder eine Kombination davon sind,
wobei "substituiert" bedeutet, dass mindestens ein Wasserstoff durch Deuterium, ein Halogen, eine Hydroxylgruppe, eine Aminogruppe, eine C1-bis C30-Amingruppe, eine Nitrogruppe, eine C1- bis C40-Silylgruppe, eine C1- bis C30-Alkylgruppe, eine C1- bis C10-Alkylsilylgruppe, eine C3- bis C30-Cycloalkylgruppe, eine C2- bis C30-Heterocycloalkylgruppe, eine C6-bis C30-Arylgruppe, eine C2- bis C30-Heteroarylgruppe, eine C1- bis C20-Alkoxygruppe, eine Fluorgruppe, eine C1- bis C10-Trifluoralkylgruppe oder eine Cyanogruppe ersetzt ist.

3. Verbindung nach Anspruch 2, die durch eine der chemischen Formeln I-1a bis l-8a, I-1b und I-2b dargestellt ist: 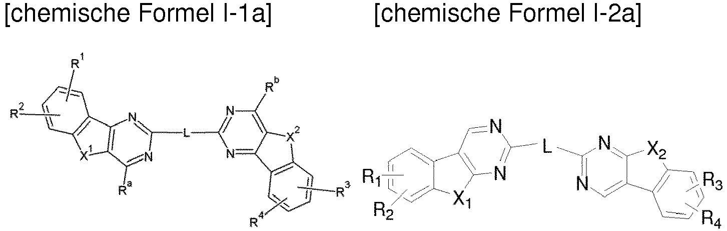 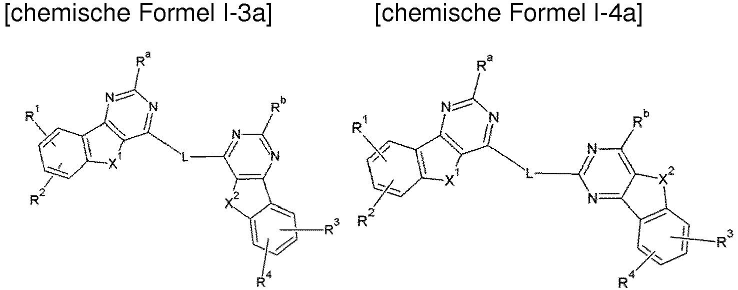 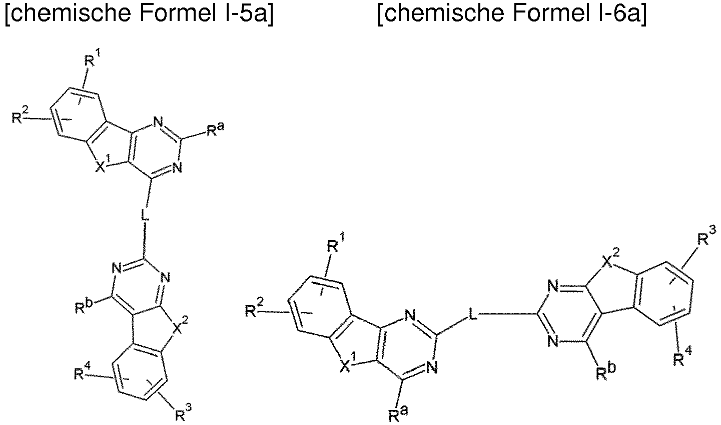 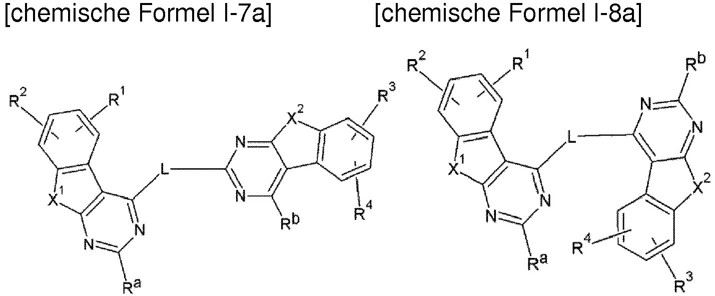 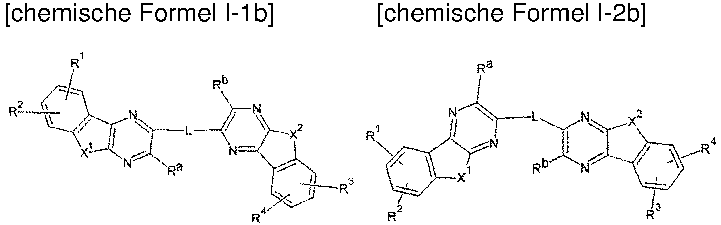 wobei in den chemischen Formeln I-1a bis I-8a. I-1b und I-2b
X¹ und X² jeweils unabhängig O oder S sind,
R^{a} und R^{b} jeweils unabhängig eine substituierte oder unsubstituierte C1- bis C30-Alkylgruppe, eine substituierte oder unsubstituierte C3- bis C30-Cycloalkylgruppe, eine substituierte oder unsubstituierte C6- bis C30-Arylgruppe, eine substituierte oder unsubstituierte C2- bis C30-Heteroa-rylgruppe, eine substituierte oder unsubstituierte C6- bis C30-Arylamin-gruppe, eine substituierte oder unsubstituierte C3- bis C40-Silylgruppe, eine substituierte oder unsubstituierte C1- bis C30-Alkylthiolgruppe, eine substituierte oder unsubstituierte C6- bis C30-Arylthiolgruppe, ein Halogen, eine Cyanogruppe, eine Hydroxylgruppe, eine Aminogruppe, eine Nitrogruppe oder eine Kombination davon sind,
L eine substituierte oder unsubstituierte C6- bis C30-Arylengruppe ist, und
R¹ bis R⁴ jeweils unabhängig Wasserstoff, Deuterium, eine substituierte oder unsubstituierte C1- bis C30-Alkylgruppe, eine substituierte oder unsubstituierte C3- bis C30-Cycloalkylgruppe, eine substituierte oder unsubstituierte C6- bis C30-Arylgruppe, eine substituierte oder unsubstituierte C2-bis C30-Heteroarylgruppe, eine substituierte oder unsubstituierte C6- bis C30-Arylamingruppe, eine substituierte oder unsubstituierte C3- bis C40-Silylgruppe, eine substituierte oder unsubstituierte C1- bis C30-Alkylthi-olgruppe, eine substituierte oder unsubstituierte C6- bis C30-Arylthiolgruppe, ein Halogen, eine Cyanogruppe, eine Hydroxylgruppe, eine Aminogruppe, eine Nitrogruppe oder eine Kombination davon sind,
wobei "substituiert" bedeutet, dass mindestens ein Wasserstoff durch Deuterium, ein Halogen, eine Hydroxylgruppe, eine Aminogruppe, eine C1-bis C30-Amingruppe, eine Nitrogruppe, eine C1- bis C40-Silylgruppe, eine C1- bis C30-Alkylgruppe, eine C1- bis C10-Alkylsilylgruppe, eine C3- bis C30-Cycloalkylgruppe, eine C2- bis C30-Heterocycloalkylgruppe, eine C6-bis C30-Arylgruppe, eine C2- bis C30-Heteroarylgruppe, eine C1- bis C20-Alkoxygruppe, eine Fluorgruppe, eine C1- bis C10-Trifluoralkylgruppe oder eine Cyanogruppe ersetzt ist.

4. Verbindung nach Anspruch 2, die durch eine der chemischen Formeln I-9 bis I-13 dargestellt ist: 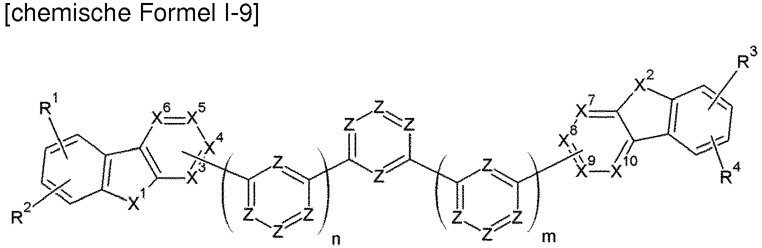 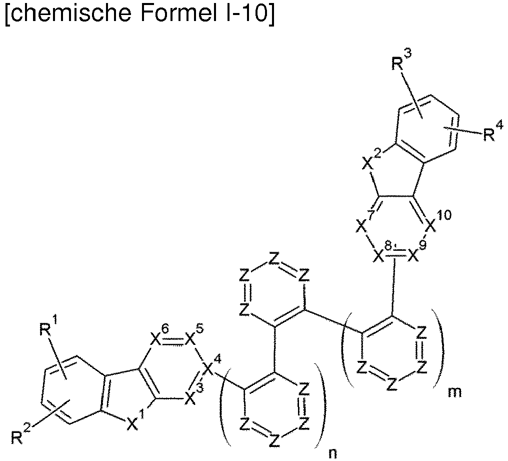 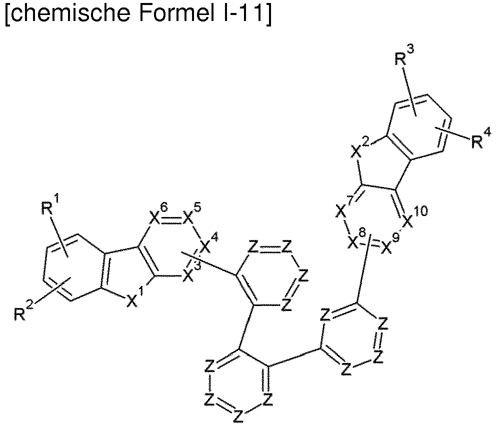 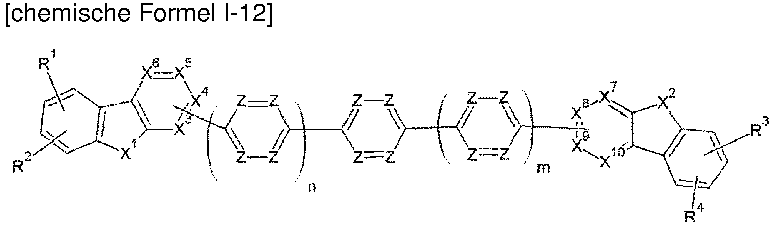 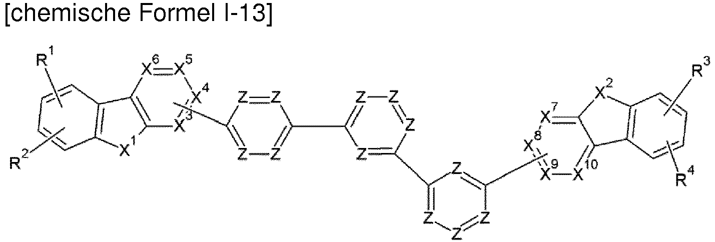 wobei in den chemischen Formeln I-9 bis I-13
X¹ und X² jeweils unabhängig O oder S sind,
X³ bis X⁶ jeweils unabhängig C, CR^{a} oder N sind,
wobei mindestens zwei X³ bis X⁶ N sind,
X⁷ bis X¹⁰ jeweils unabhängig C, CR^{b} oder N sind,
wobei mindestens zwei X⁷ bis X¹⁰ N sind,
Z CR^{c} ist,
R¹ bis R⁴ und R^{a} und R^{b} jeweils unabhängig Wasserstoff, Deuterium, eine substituierte oder unsubstituierte C1- bis C30-Alkylgrupp oder eine substituierte oder unsubstituierte C3- bis C30-Cycloalkylgruppe sind, und
n und m unabhängig ganze Zahlen im Bereich von 0 bis 2 sind, wobei "substituiert" bedeutet, dass mindestens ein Wasserstoff durch Deuterium, ein Halogen, eine Hydroxylgruppe, eine Aminogruppe, eine C1-bis C30-Amingruppe, eine Nitrogruppe, eine C1- bis C40-Silylgruppe, eine C1- bis C30-Alkylgruppe, eine C1- bis C10-Alkylsilylgruppe, eine C3- bis C30-Cycloalkylgruppe, eine C2- bis C30-Heterocycloalkylgruppe, eine C6-bis C30-Arylgruppe, eine C2- bis C30-Heteroarylgruppe, eine C1- bis C20-Alkoxygruppe, eine Fluorgruppe, eine C1- bis C10-Trifluoralkylgruppe oder eine Cyanogruppe ersetzt ist.

5. Verbindung nach Anspruch 4, wobei mindestens eine der ganzen Zahlen n und m 1 beträgt.

6. Verbindung nach Anspruch 1, wobei L eine substituierte oder unsubstituierte Phenylgruppe, eine substituierte oder unsubstituierte Naphthylgruppe, eine substituierte oder unsubstituierte Anthracenylgruppe, eine substituierte oder unsubstituierte Phenanthrylgruppe, eine substituierte oder unsubstituierte Naphthacenylgruppe, eine substituierte oder unsubstituierte Pyrenylgruppe, eine substituierte oder unsubstituierte Biphenylgruppe, eine substituierte oder unsubstituierte p-Terphenylgruppe, eine substituierte oder unsubstituierte m-Terphenylgruppe, eine substituierte oder unsubstituierte Quarterphenylgruppe, eine substituierte oder unsubstituierte Chrysenylgruppe, eine substituierte oder unsubstituierte Triphenylenylgruppe, eine substituierte oder unsubstituierte Perylenylgruppe, eine substituierte oder unsubstituierte Fluorenylgruppe oder eine Kombination davon ist.

7. Verbindung nach Anspruch 6, wobei L ausgewählt ist aus den in Gruppe 1 aufgeführten Bindungsgruppen: 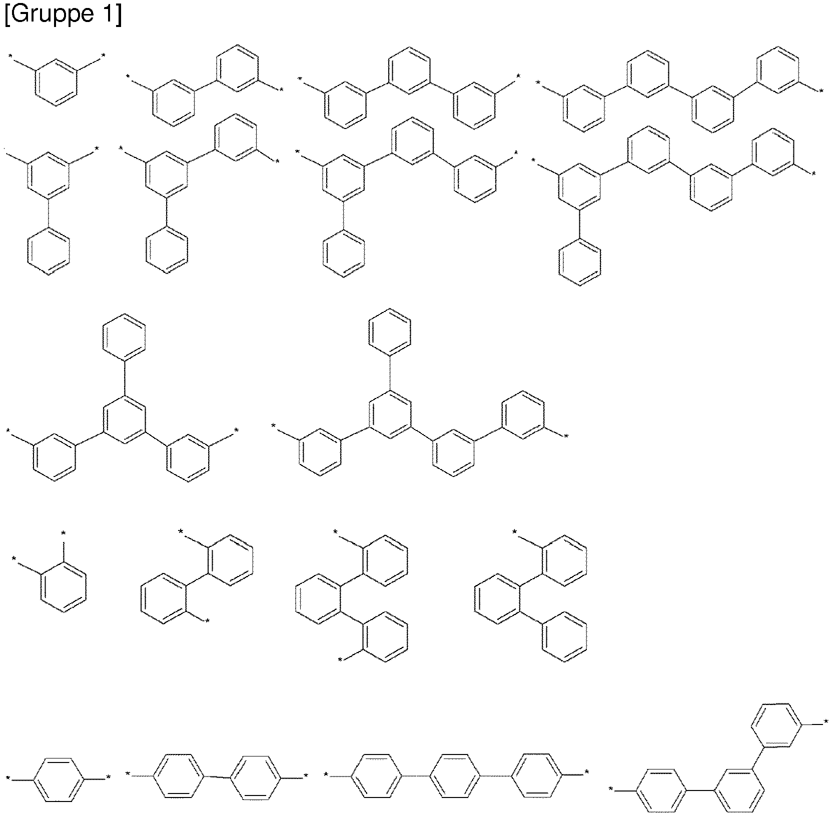 wobei in Gruppe 1 * eine Bindungsstelle darstellt.

8. Verbindung nach Anspruch 6, wobei die Verbindung eine Verbindung der Gruppe 2 ist:

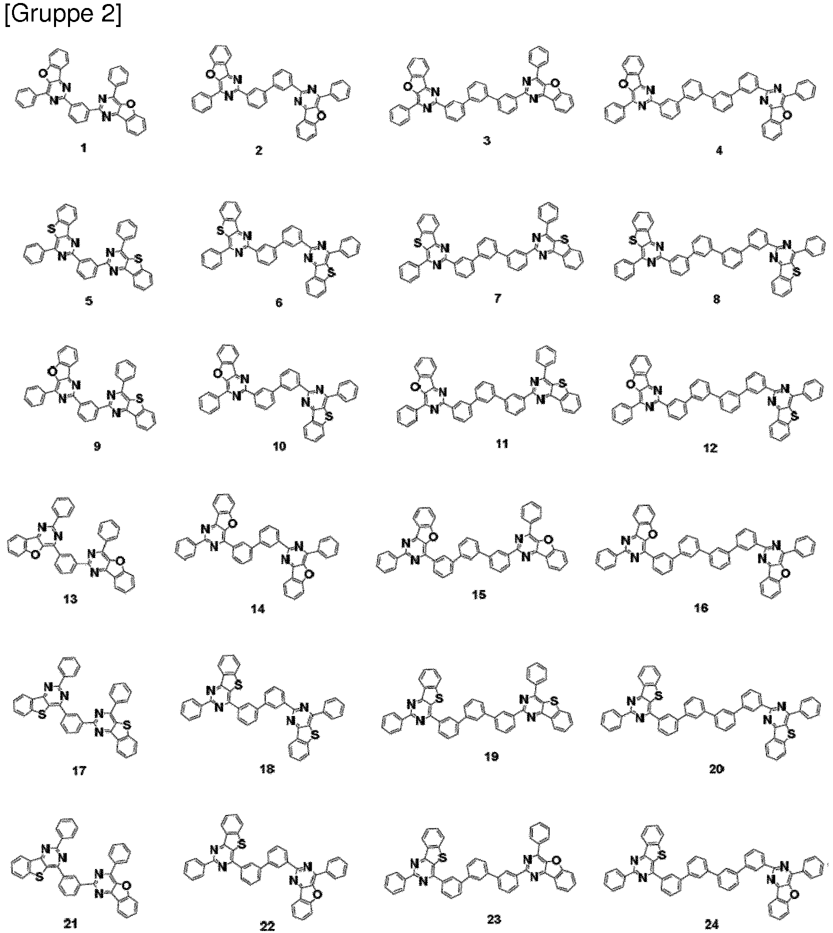

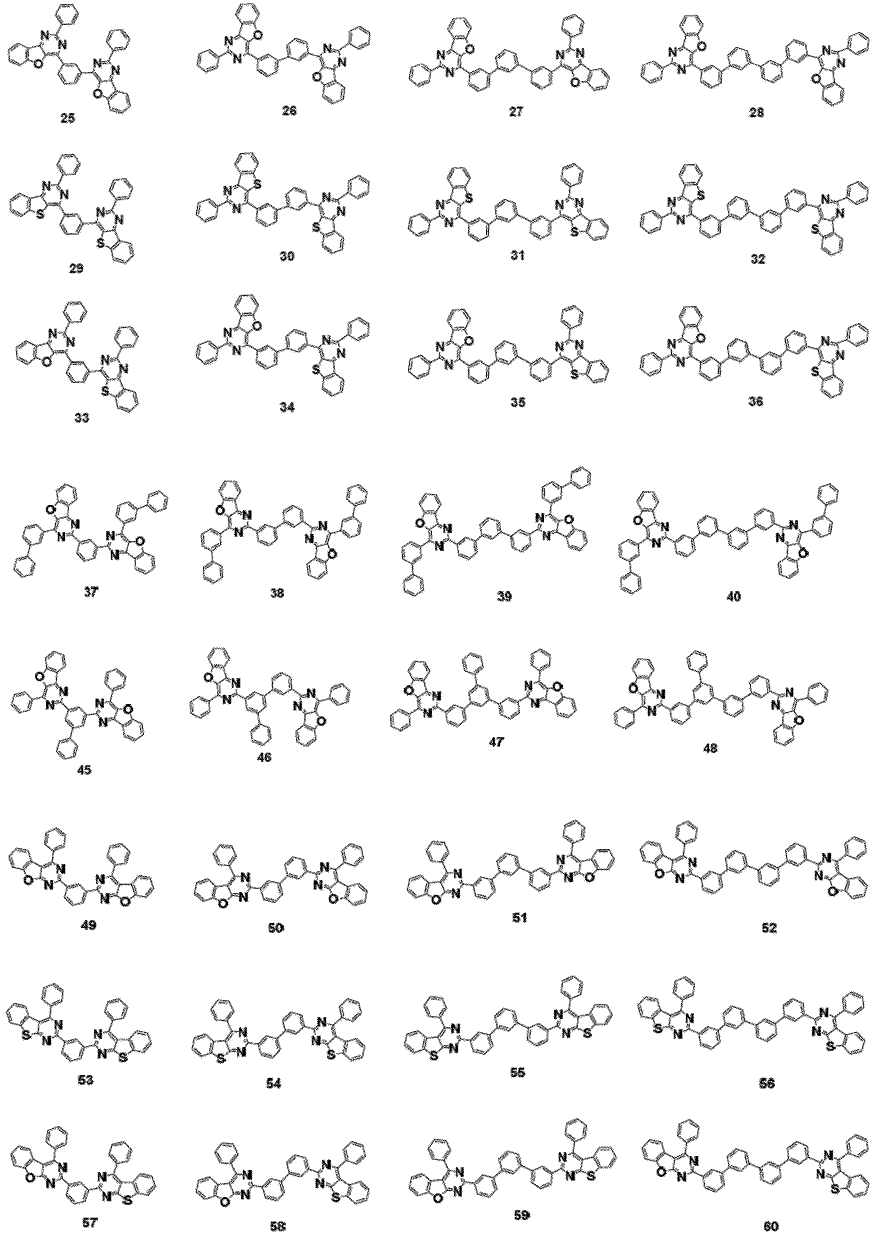

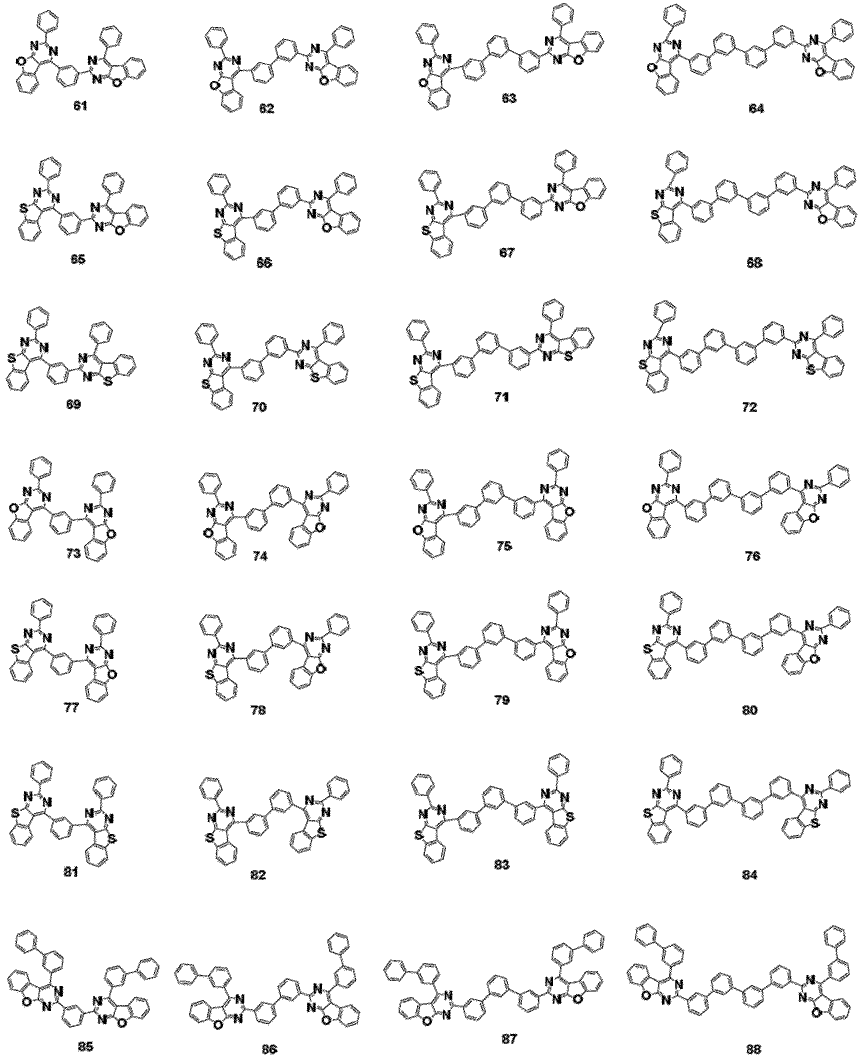

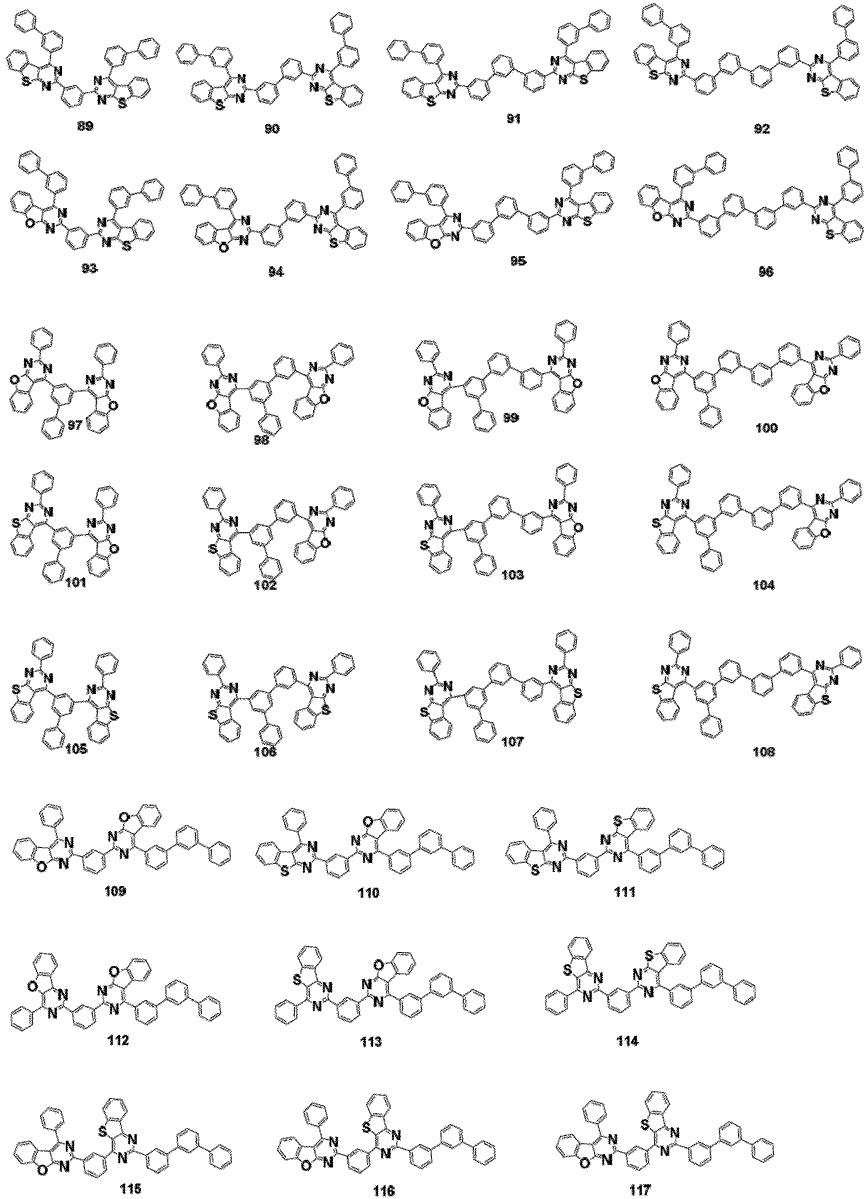

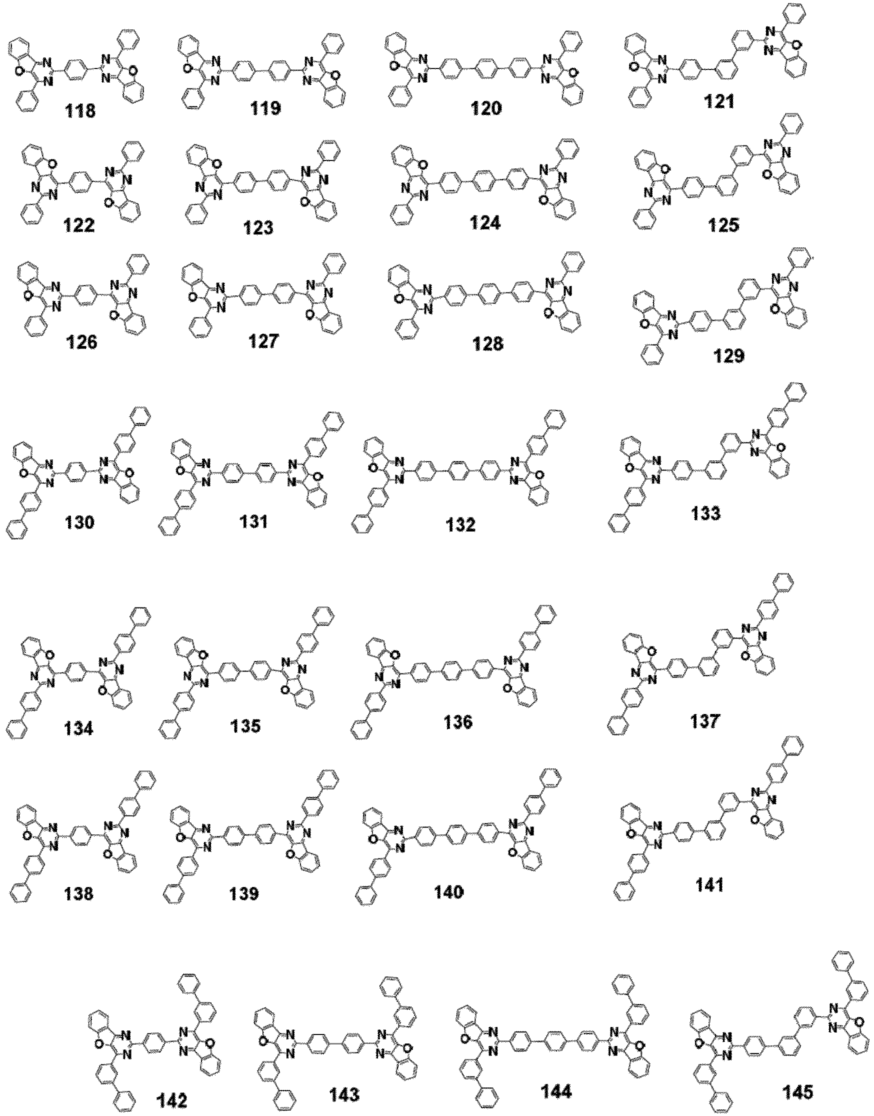

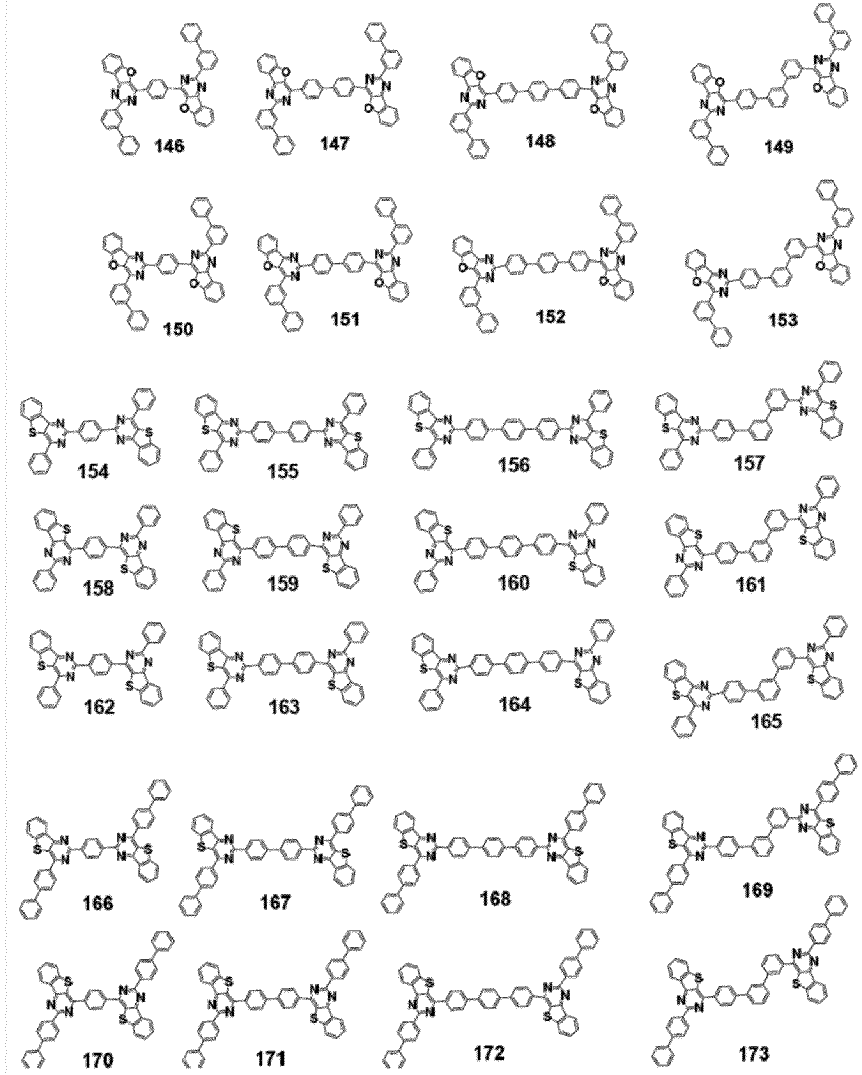

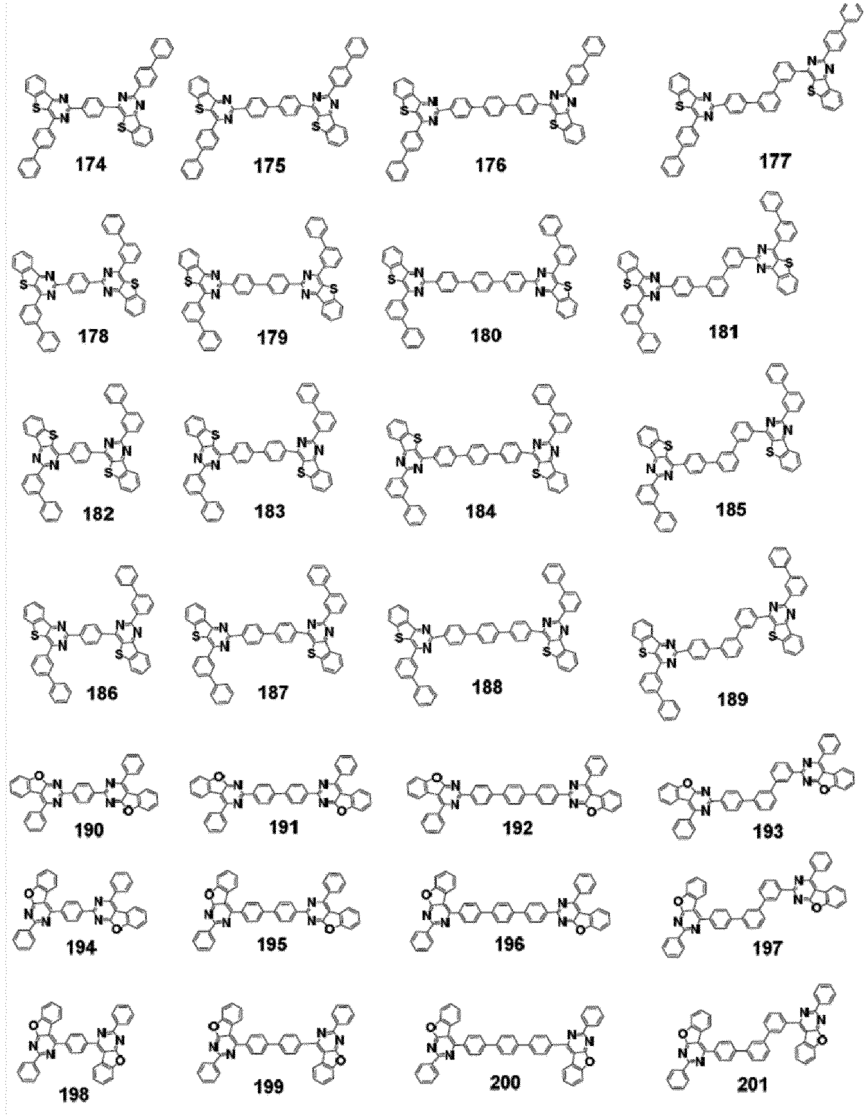

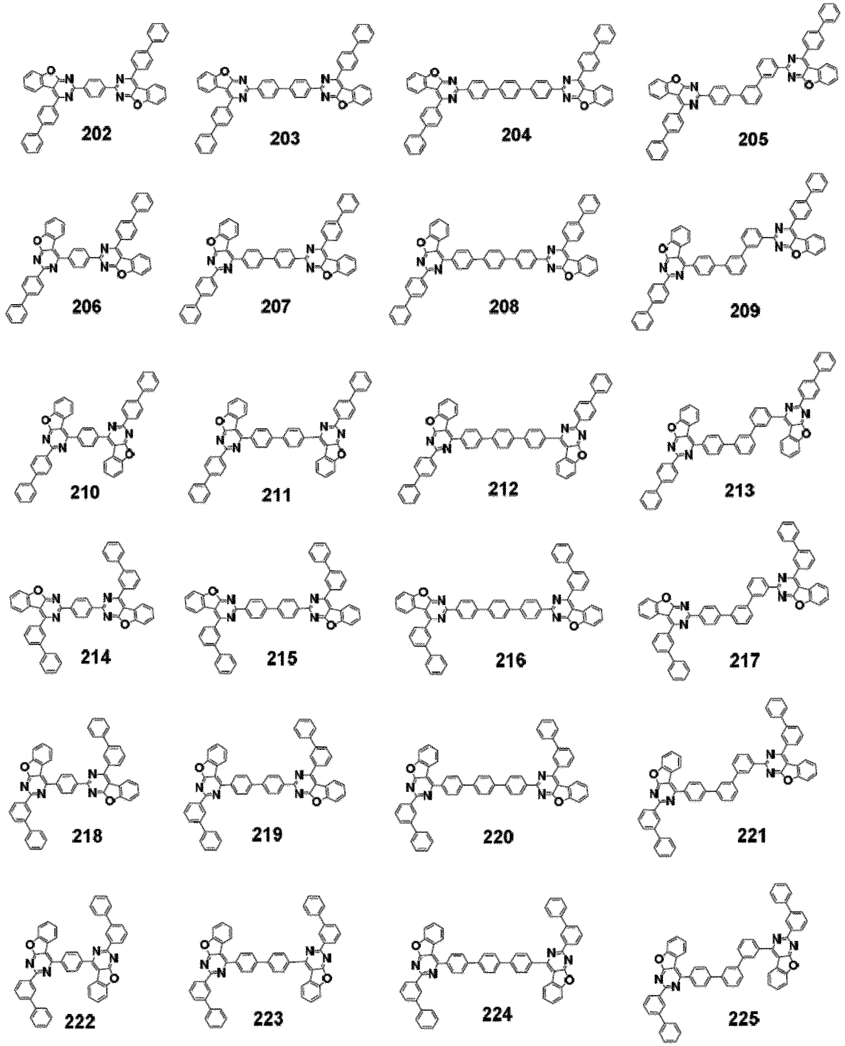

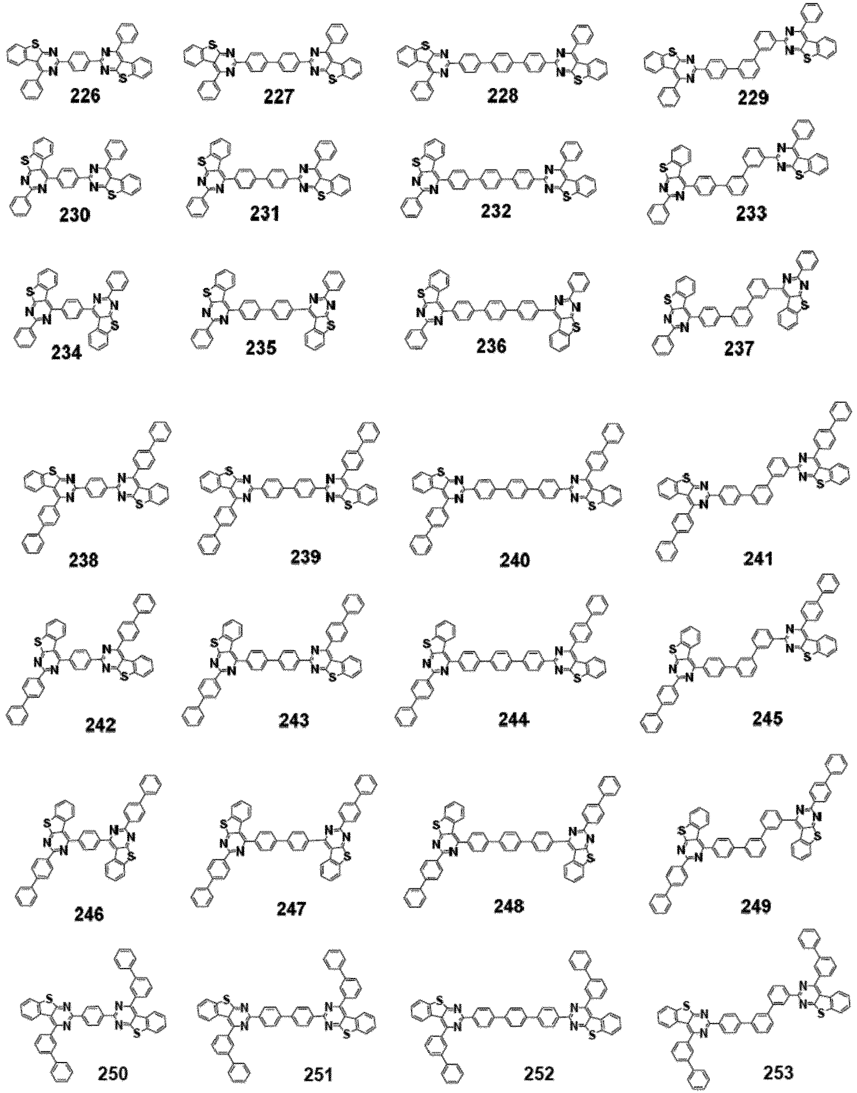

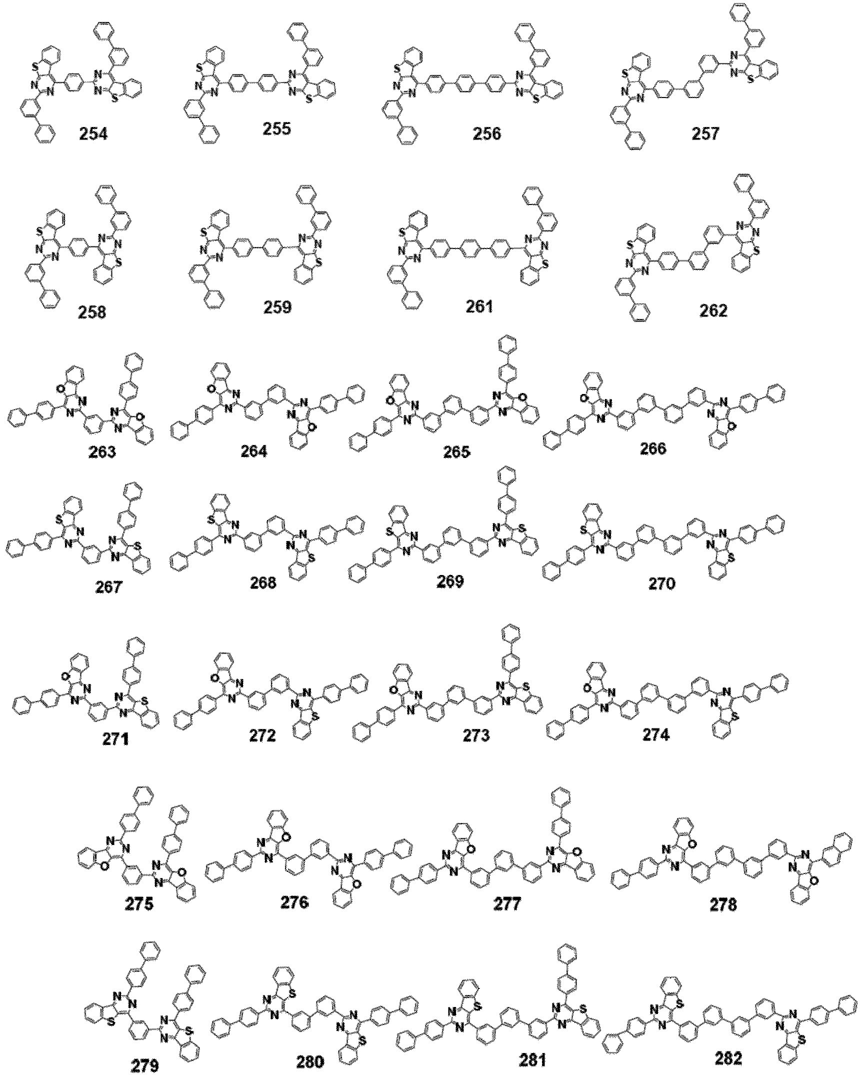

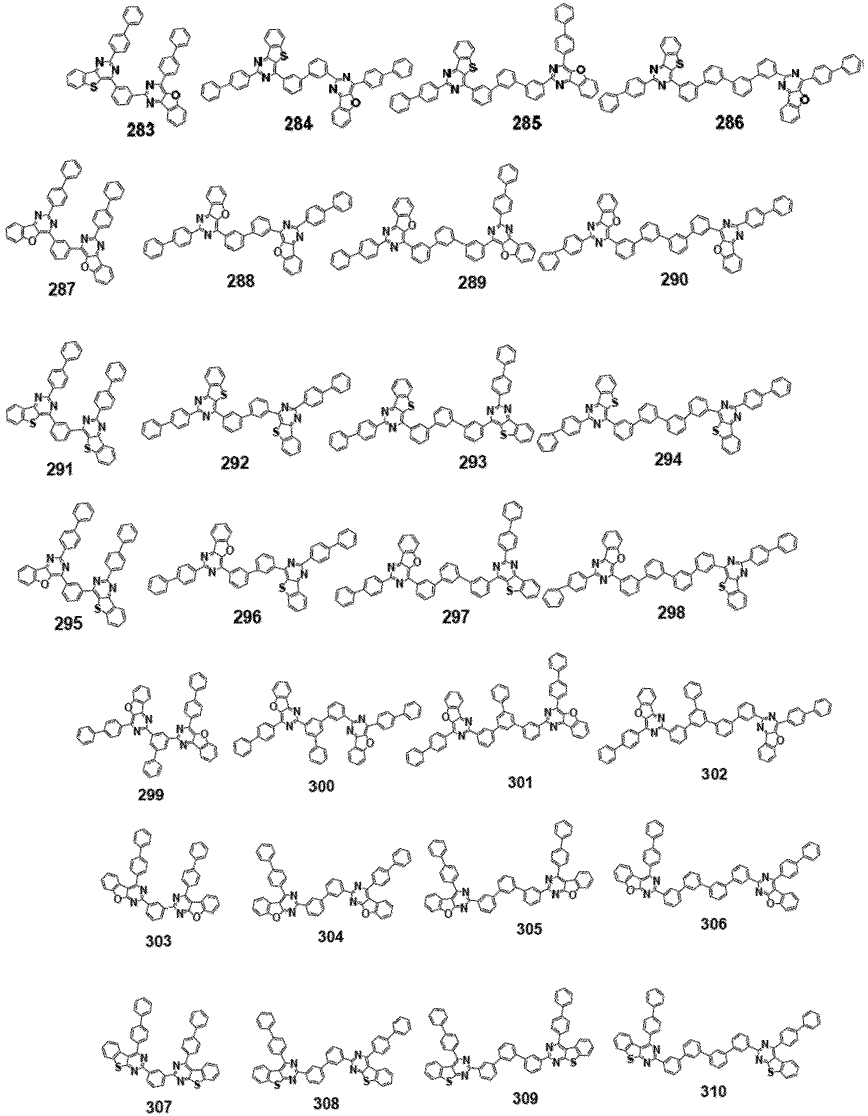

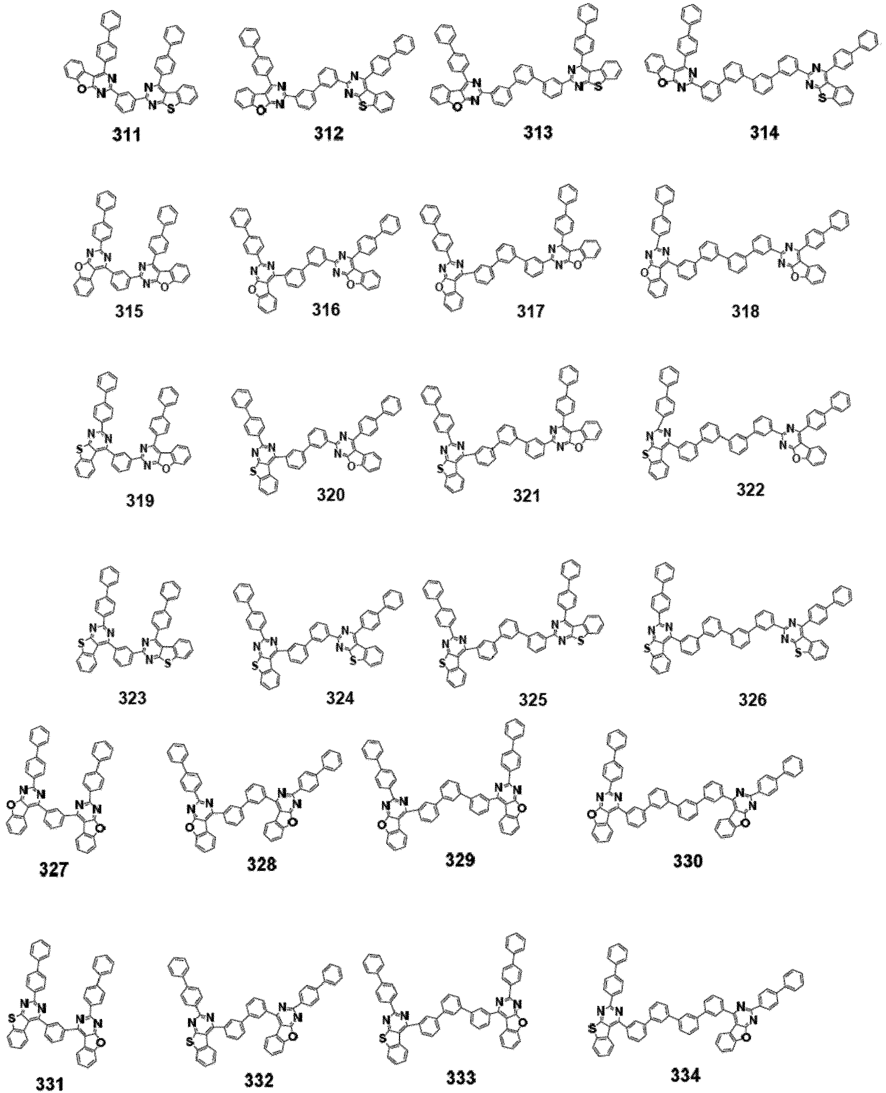

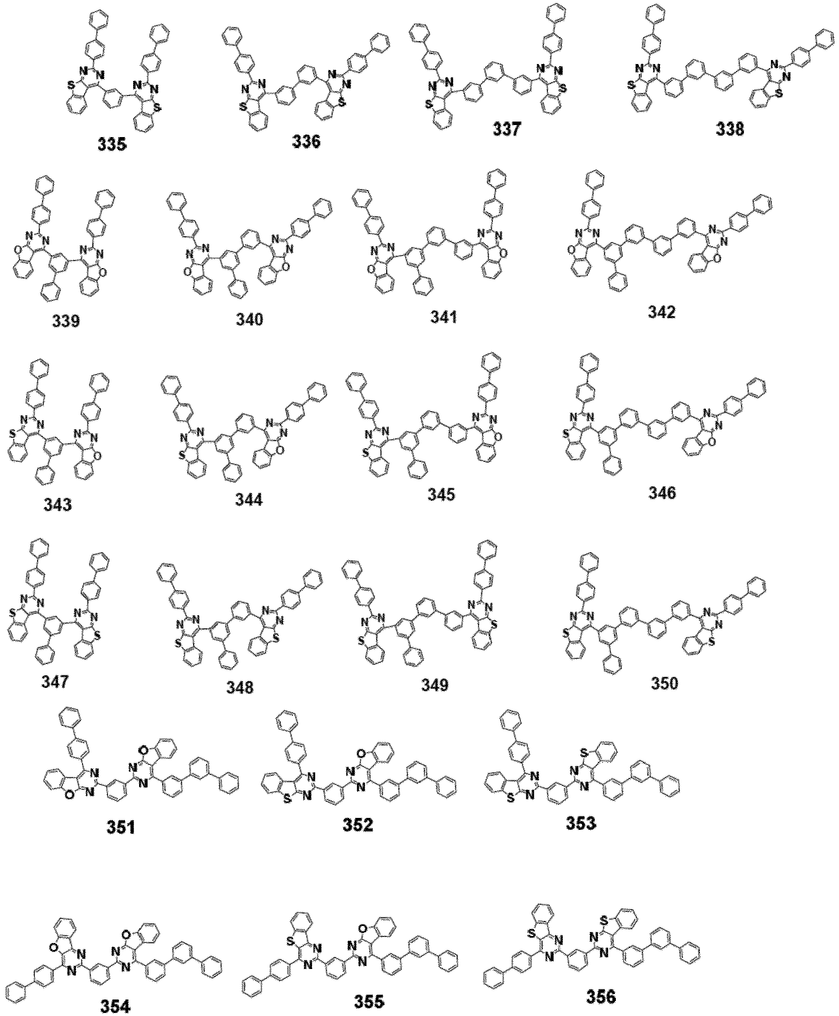

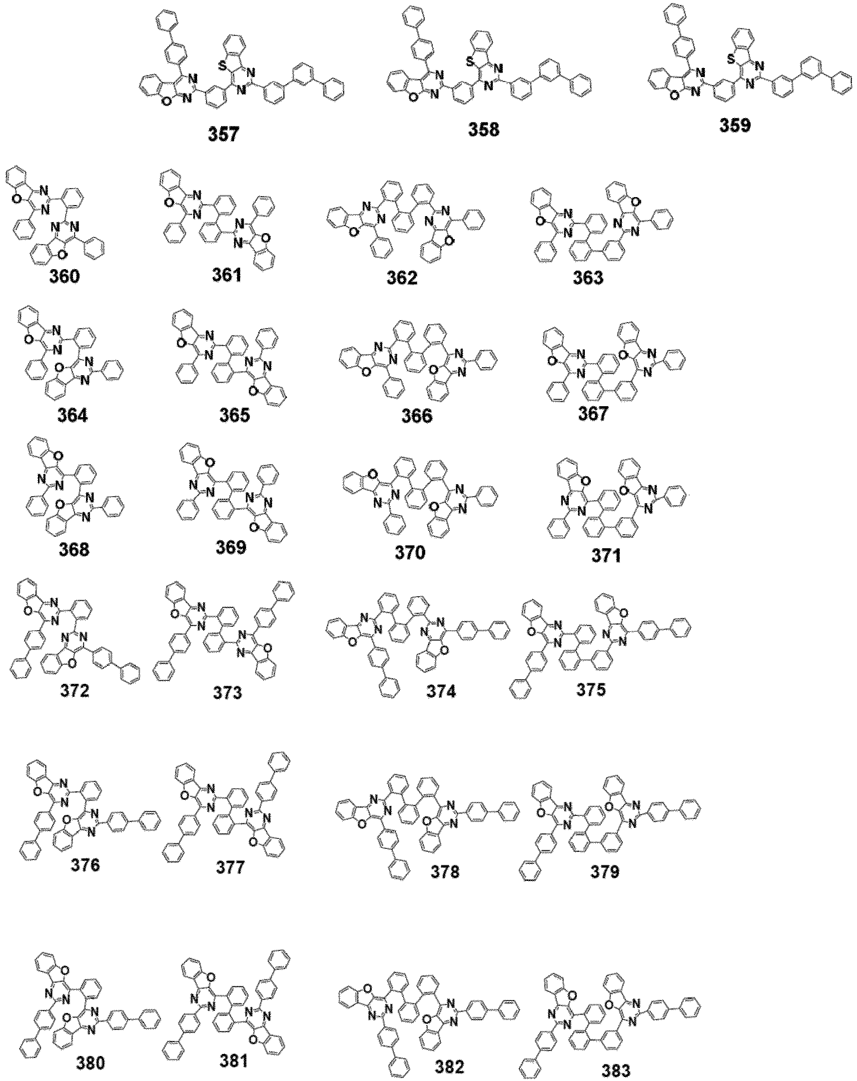

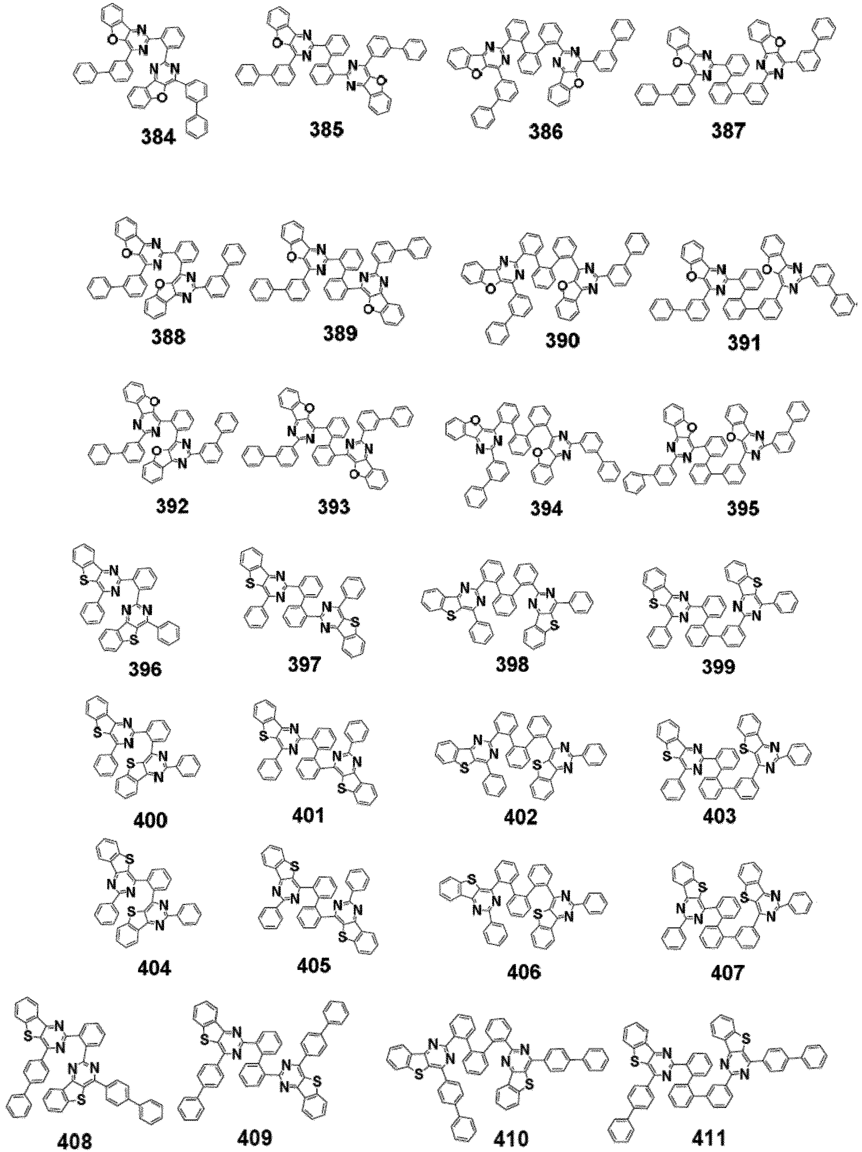

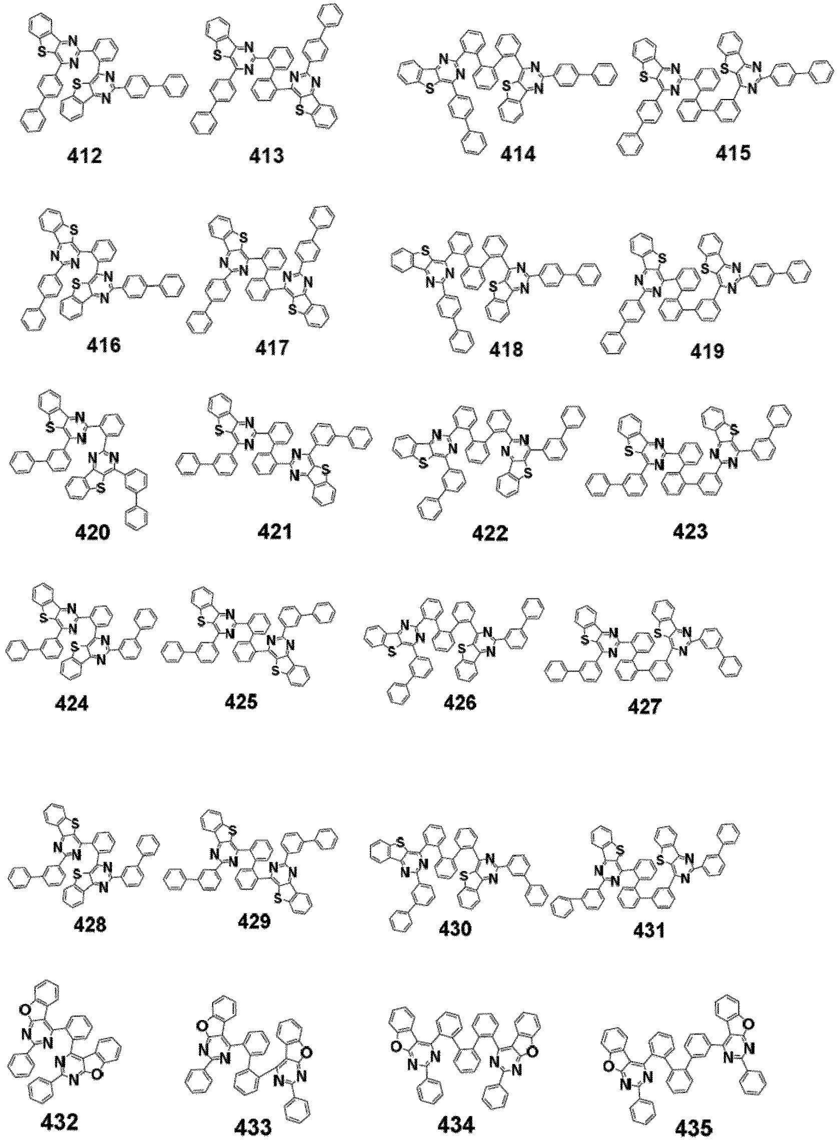

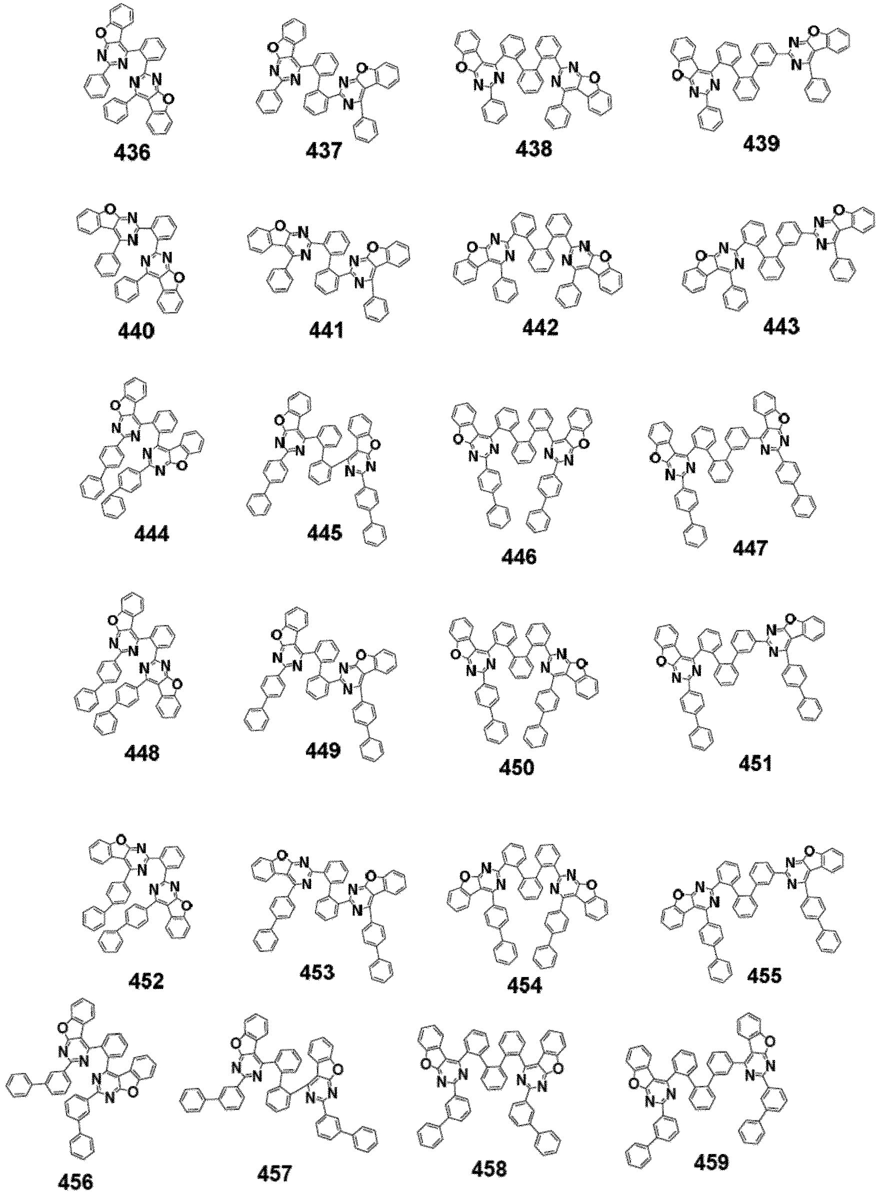

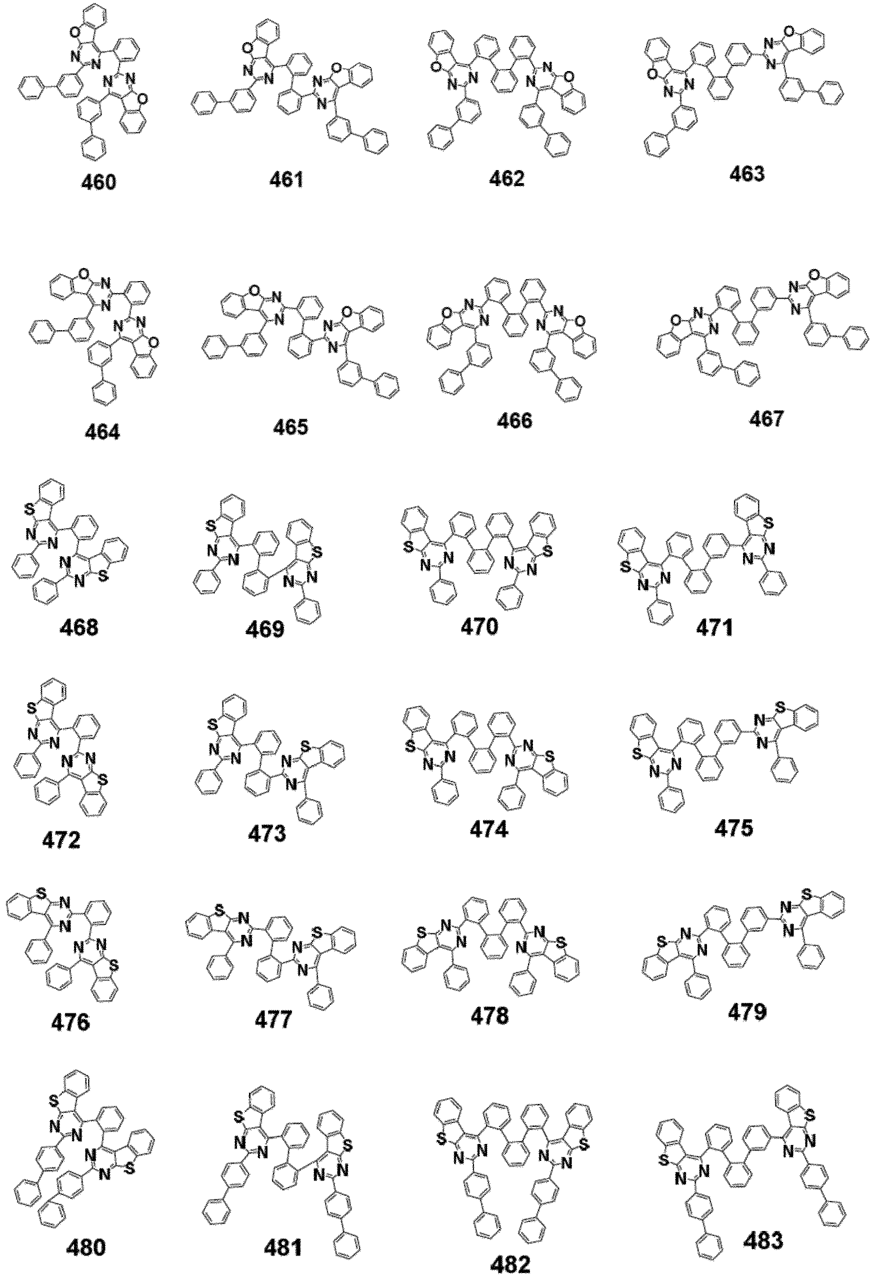

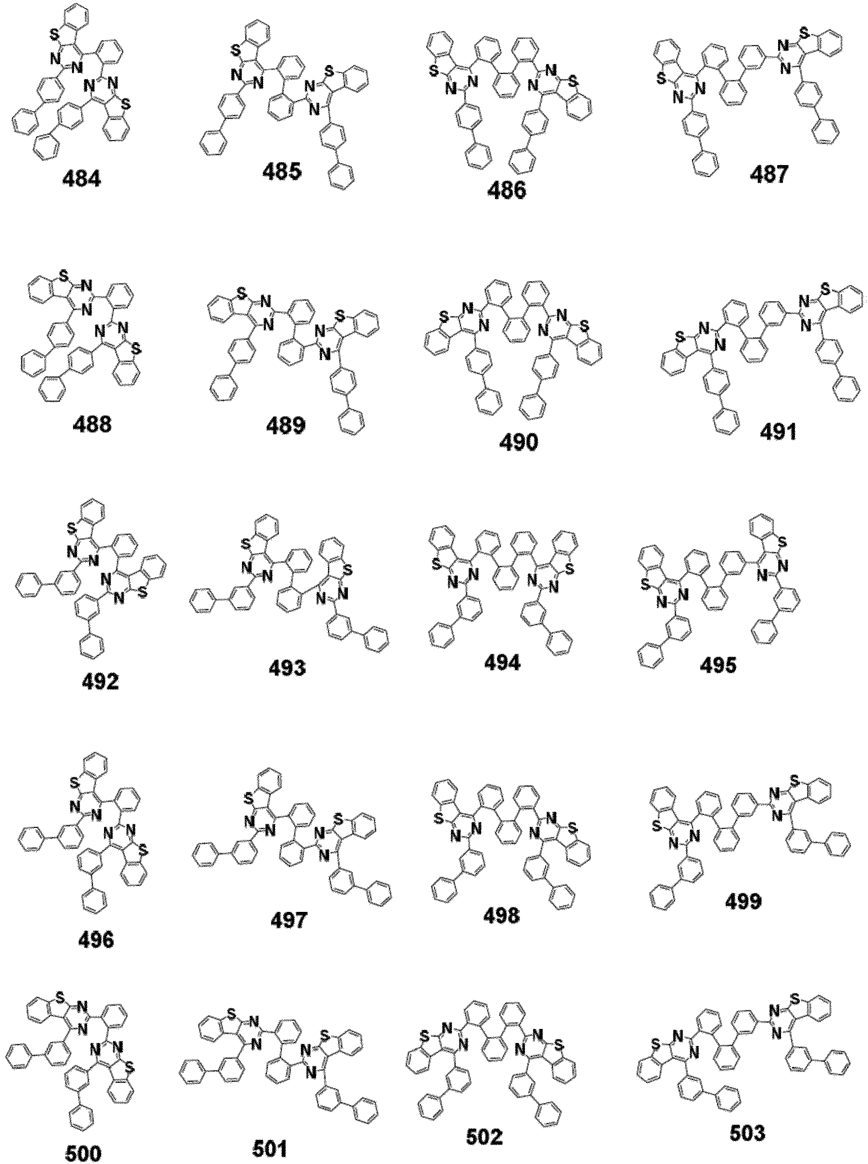

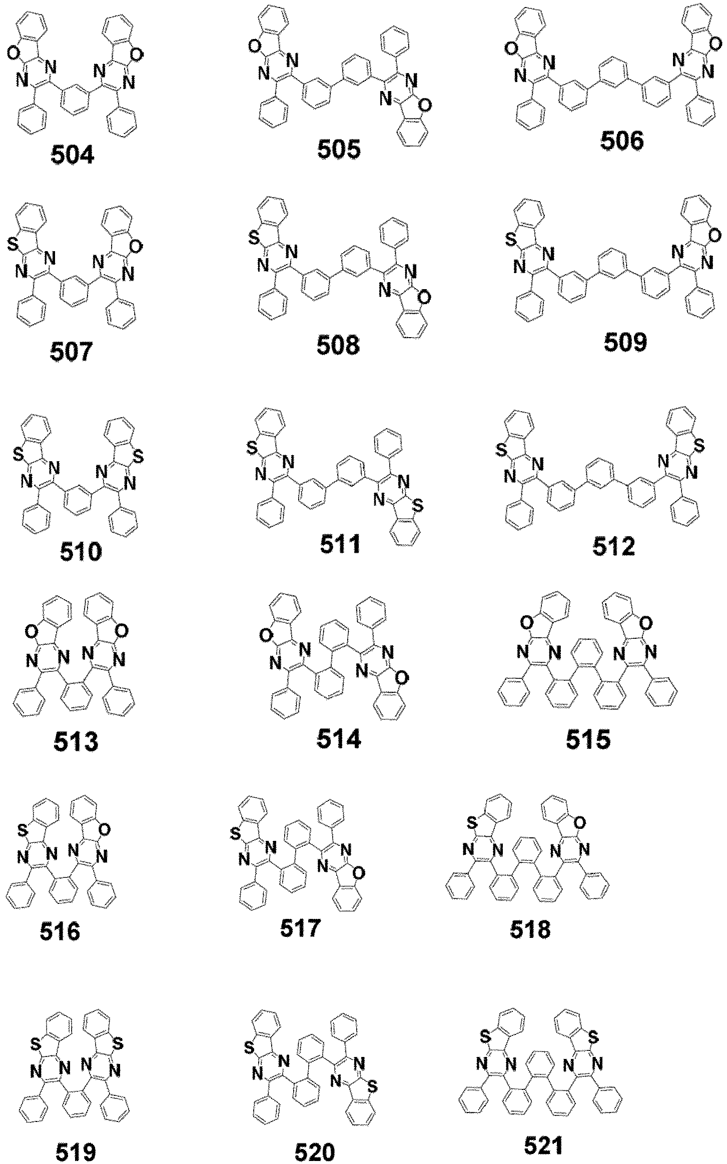

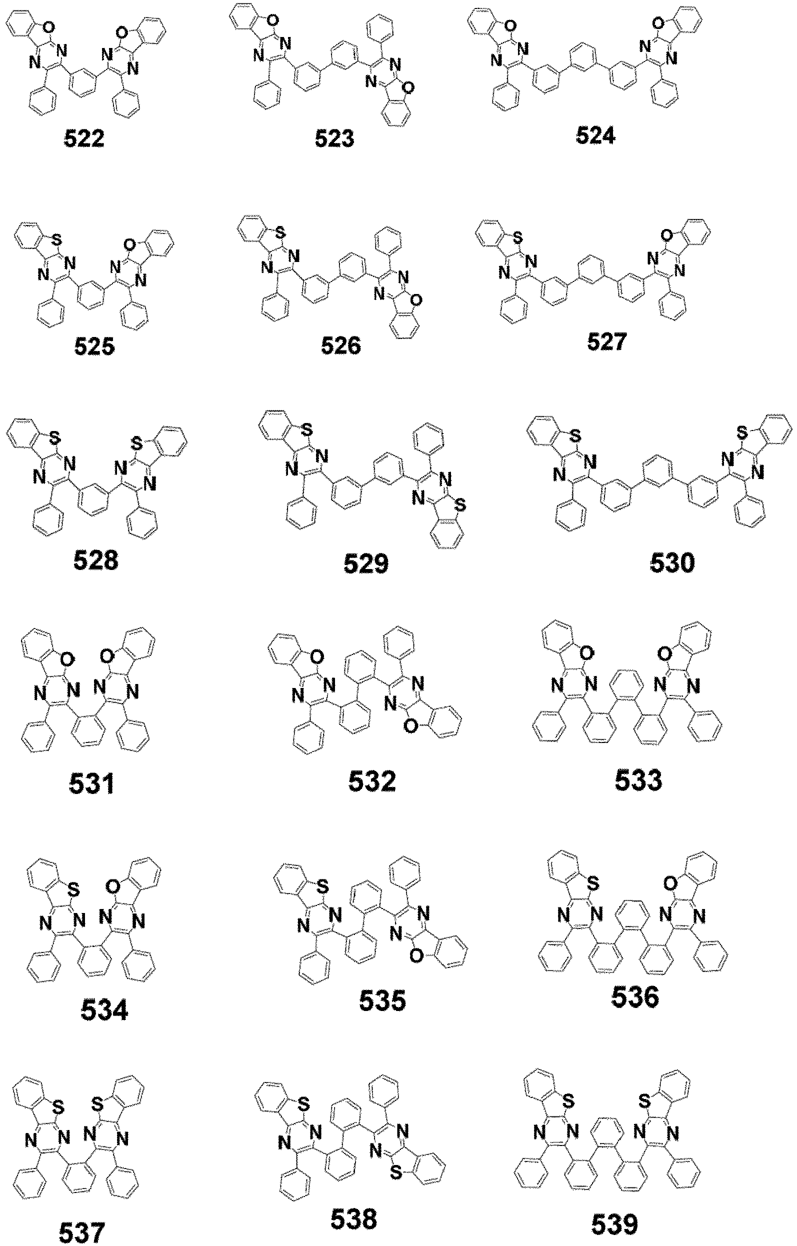

9. Organisches optoelektronisches Bauelement, mit:
einer Anode und einer Kathode, die einander zugewandt sind; und
mindestens einer organischen Schicht zwischen der Anode und der Kathode,
wobei die organische Schicht die Verbindung für ein organisches optoelektronisches Bauelement nach einem der Ansprüche 1 bis 8 aufweist.

10. Organisches optoelektronisches Bauelement nach Anspruch 9, wobei die organische Schicht eine Emissionsschicht aufweist, und
wobei die Emissionsschicht die Verbindung für ein organisches optoelektronisches Bauelement aufweist.

11. Organisches optoelektronisches Bauelement nach Anspruch 10, wobei die Verbindung für ein organisches optoelektronisches Bauelement als Host-Material in der Emissionsschicht enthalten ist.

12. Organisches optoelektronisches Bauelement nach Anspruch 9, wobei die organische Schicht mindestens eine Hilfsschicht umfasst, die aus einer Lochinjektionsschicht (HIL), einer Lochtransportschicht (HTL), einer Lochtransporthilfsschicht, einer Elektronentransporthilfsschicht, einer Elektronentransportschicht (ETL) und einer Elektroneninjektionsschicht (EIL) ausgewählt ist, und
wobei die Hilfsschicht die Verbindung für ein organisches optoelektronisches Bauelement aufweist.

13. Anzeigevorrichtung mit dem organischen optoelektronischen Bauelement nach Anspruch 9.

## Revendications

1. Composé pour un dispositif optoélectronique organique représenté par la Formule Chimique I : 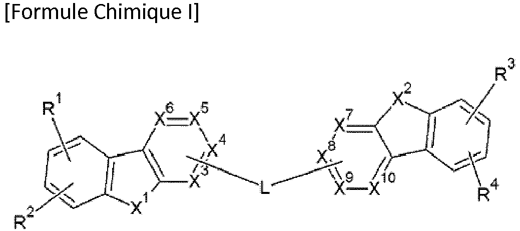 dans laquelle,
X¹ et X² sont chacun indépendamment O ou S,
X³ à X⁶ sont chacun indépendamment C, CR^{a} ou N,
au moins deux de X³ à X⁶ sont N,
X⁷à X¹⁰ sont chacun indépendamment, C, CR^{b} ou N,
au moins deux de X⁷ à X¹⁰ sont N,
L est un groupement arylène substitué ou non-substitué en C₆ à C₃₀, et
R¹ à R⁴ et R^{a} et R^{b} sont chacun indépendamment un hydrogène, un deutérium, un groupement alkyle substitué ou non-substitué en C₁ à C₃₀, un groupement cycloalkyle substitué ou non-substitué en C₃ à C₃₀, un groupement aryle substitué ou non-substitué en C₆ à C₃₀, un groupement hétéroaryle substitué ou non-substitué en C₂ à C₃₀, un groupement arylamine substitué ou non-substitué en C₆ à C₃₀, un groupement silyle substitué ou non-substitué en C₃ à C₄₀, un groupement alkylthiol substitué ou non-substitué en C₁ à C₃₀, un groupement arylthiol substitué ou non-substitué en C₆ à C₃₀, un halogène, un groupement cyano, un groupement hydroxyl, un groupement amino, un groupement nitro, ou une combinaison de ceux-ci,
dans lequel « substitué » se réfère au fait qu'au moins un hydrogène est remplacé par un deutérium, un halogène, un groupement hydroxyle, un groupement amino, un groupement amine en C₁ à C₃₀, un groupement nitro, un groupement silyle en C₁ à C₄₀, un groupement alkyle en C₁ à C₃₀, un groupement alkylsilyle en C₁ à C₁₀, un groupement cycloalkyle en C₃ à C₃₀, un groupement hétérocycloalkyle en C₂ à C₃₀, un groupement aryle en C₆ à C₃₀, un groupement hétéroaryle en C₂ à C₃₀, un groupement alcoxy en C₁ à C₂₀, un groupement fluoro, un groupement trifluoroalkyle en C₁ à C₁₀, ou un groupement cyano.

2. Composé pour un dispositif optoélectronique organique selon la revendication 1, représenté par une des Formules Chimiques I-1 à I-8 : 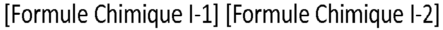 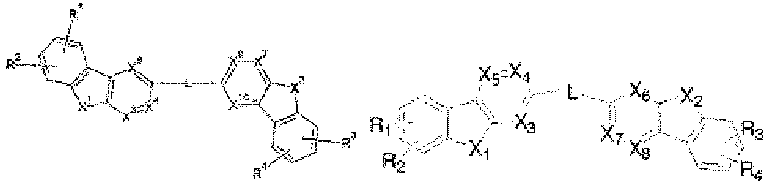 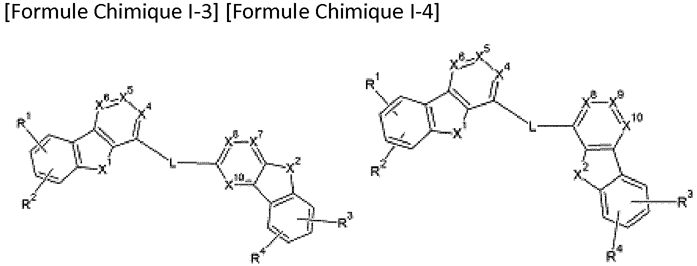 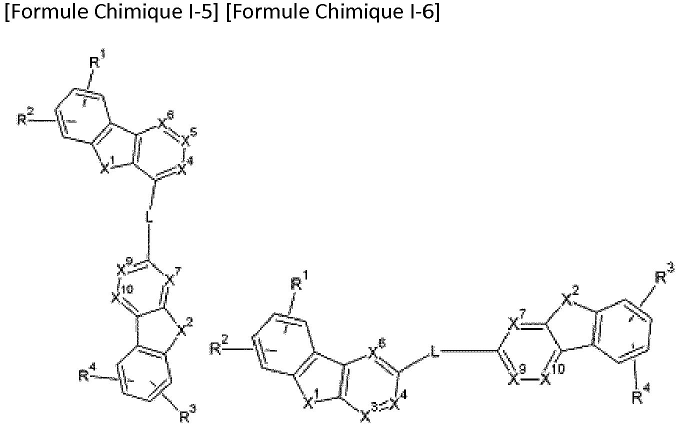 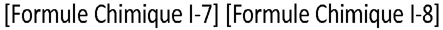 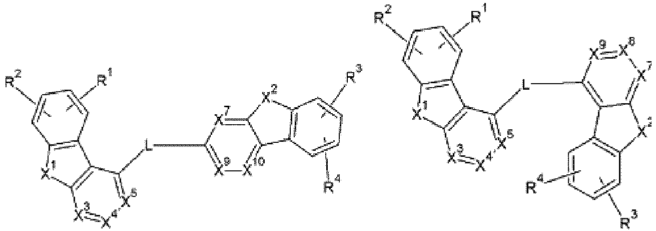 où, dans les Formules Chimiques I-1 à I-8,
X¹ et X² sont chacun indépendamment O ou S,
X³ à X⁶ sont chacun indépendamment C, CR^{a} ou N,
au moins deux de X³ à X⁶ sont N,
X⁷à X¹⁰ sont chacun indépendamment, C, CR^{b} ou N,
au moins deux de X⁷ à X¹⁰ sont N,
L est un groupement arylène substitué ou non-substitué en C₆ à C₃₀, et
R¹ à R⁴ et R^{a} et R^{b} sont chacun indépendamment un hydrogène, un deutérium, un groupement alkyle substitué ou non-substitué en C₁ à C₃₀, un groupement cycloalkyle substitué ou non-substitué en C₃ à C₃₀, un groupement aryle substitué ou non-substitué en C₆ à C₃₀, un groupement hétéroaryle substitué ou non-substitué en C₂ à C₃₀, un groupement arylamine substitué ou non-substitué en C₆ à C₃₀, un groupement silyle substitué ou non-substitué en C₃ à C₄₀, un groupement alkylthiol substitué ou non-substitué en C₁ à C₃₀, un groupement arylthiol substitué ou non-substitué en C₆ à C₃₀, un halogène, un groupement cyano, un groupement hydroxyl, un groupement amino, un groupement nitro, ou une combinaison de ceux-ci,
dans lequel « substitué » se réfère au fait qu'au moins un hydrogène est remplacé par un deutérium, un halogène, un groupement hydroxyle, un groupement amino, un groupement amine en C₁ à C₃₀, un groupement nitro, un groupement silyle en C₁ à C₄₀, un groupement alkyle en C₁ à C₃₀, un groupement alkylsilyle en C₁ à C₁₀, un groupement cycloalkyle en C₃ à C₃₀, un groupement hétérocycloalkyle en C₂ à C₃₀, un groupement aryle en C₆ à C₃₀, un groupement hétéroaryle en C₂ à C₃₀, un groupement alcoxy en C₁ à C₂₀, un groupement fluoro, un groupement trifluoroalkyle en C₁ à C₁₀, ou un groupement cyano.

3. Composé pour un dispositif optoélectronique organique selon la revendication 2, représenté par une des Formules Chimiques I-1 à I-8, I-1b et I-2b : 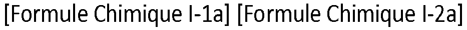 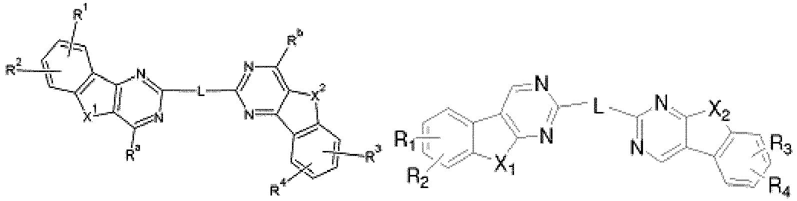 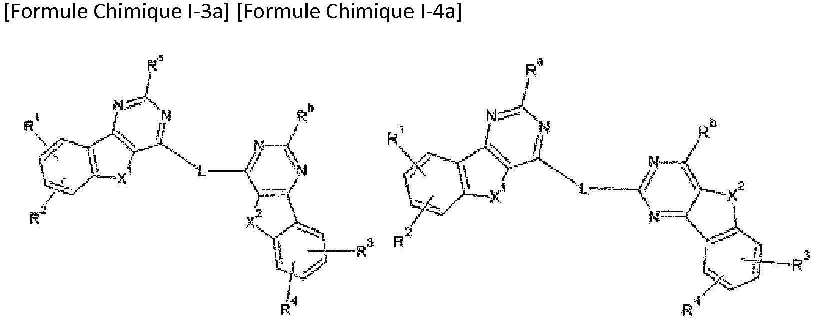 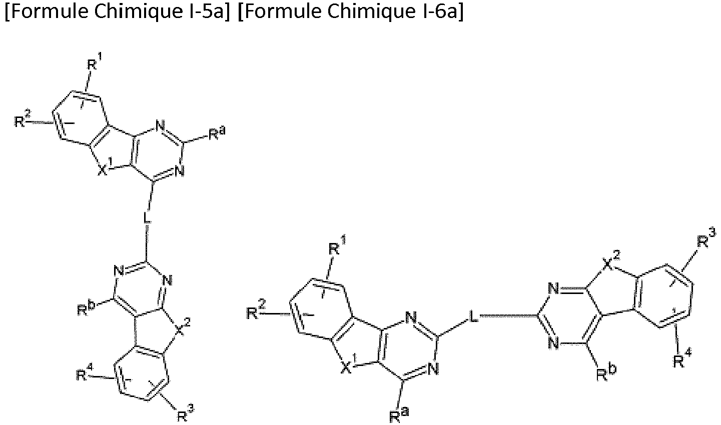 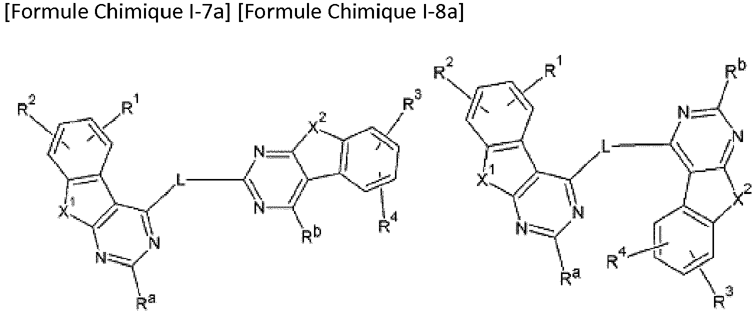 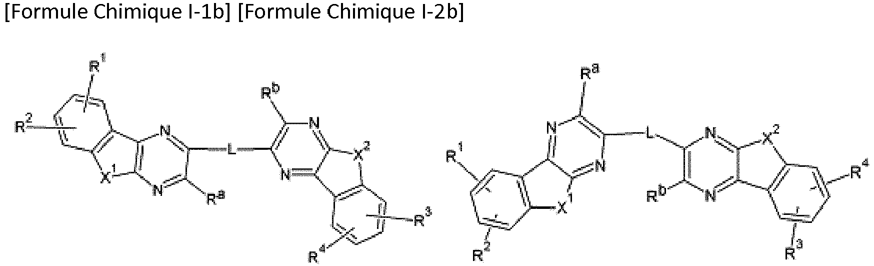 où, dans les Formules Chimiques I-1a à I-8a, I-1b, et I-2b,
X¹ et X² sont chacun indépendamment O ou S,
R^{a} et R^{b} sont chacun indépendamment un groupement alkyle substitué ou non-substitué en C₁ à C₃₀, un groupement cycloalkyle substitué ou non-substitué en C₃ à C₃₀, un groupement aryle substitué ou non-substitué en C₆ à C₃₀, un groupement hétéroaryle substitué ou non-substitué en C₂ à C₃₀, un groupement arylamine substitué ou non-substitué en C₆ à C₃₀, un groupement silyle substitué ou non-substitué en C₃ à C₄₀, un groupement alkylthiol substitué ou non-substitué en C₁ à C₃₀, un groupement arylthiol substitué ou non-substitué en C₆ à C₃₀, un halogène, un groupement cyano, un groupement hydroxyl, un groupement amino, un groupement nitro, ou une combinaison de ceux-ci,
L est un groupement arylène substitué ou non-substitué en C₆ à C₃₀, et
R¹ à R⁴ sont chacun indépendamment un hydrogène, un deutérium, un groupement alkyle substitué ou non-substitué en C₁ à C₃₀, un groupement cycloalkyle substitué ou non-substitué en C₃ à C₃₀, un groupement aryle substitué ou non-substitué en C₆ à C₃₀, un groupement hétéroaryle substitué ou non-substitué en C₂ à C₃₀, un groupement arylamine substitué ou non-substitué en C₆ à C₃₀, un groupement silyle substitué ou non-substitué en C₃ à C₄₀, un groupement alkylthiol substitué ou non-substitué en C₁ à C₃₀, un groupement arylthiol substitué ou non-substitué en C₆ à C₃₀, un halogène, un groupement cyano, un groupement hydroxyl, un groupement amino, un groupement nitro, ou une combinaison de ceux-ci,
dans lequel « substitué » se réfère au fait qu'au moins un hydrogène est remplacé par un deutérium, un halogène, un groupement hydroxyle, un groupement amino, un groupement amine en C₁ à C₃₀, un groupement nitro, un groupement silyle en C₁ à C₄₀, un groupement alkyle en C₁ à C₃₀, un groupement alkylsilyle en C₁ à C₁₀, un groupement cycloalkyle en C₃ à C₃₀, un groupement hétérocycloalkyle en C₂ à C₃₀, un groupement aryle en C₆ à C₃₀, un groupement hétéroaryle en C₂ à C₃₀, un groupement alcoxy en C₁ à C₂₀, un groupement fluoro, un groupement trifluoroalkyle en C₁ à C₁₀, ou un groupement cyano.

4. Composé pour un dispositif optoélectronique organique selon la revendication 2, représenté par une des Formules Chimiques I-9 à I-13 : 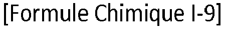 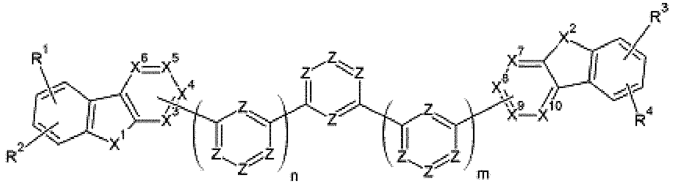 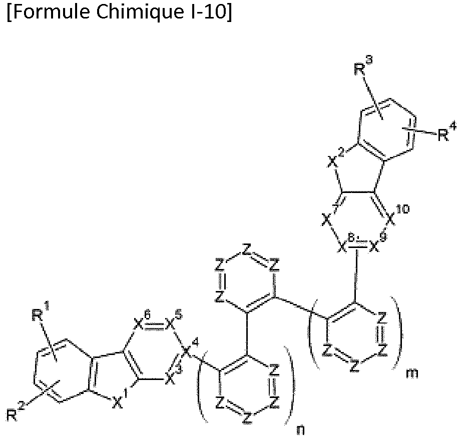 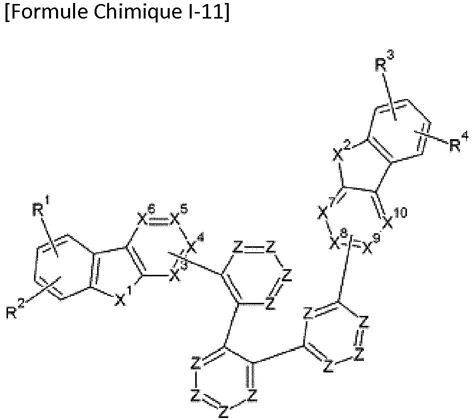 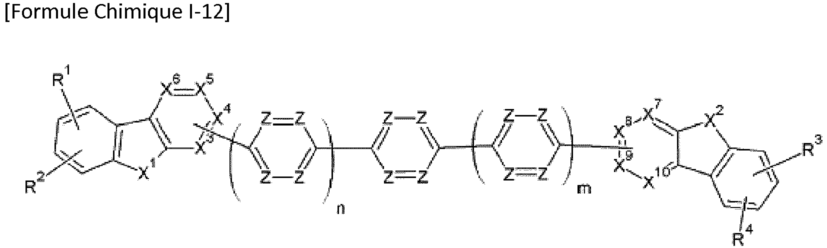 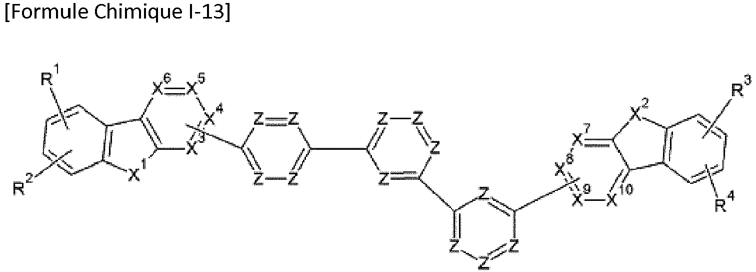 où, dans les Formules Chimiques I-9 à I-13,
X¹ et X² sont chacun indépendamment O ou S,
X³ à X⁶ sont chacun indépendamment C, CR^{a} ou N,
au moins deux de X³ à X⁶ sont N,
X⁷à X¹⁰ sont chacun indépendamment, C, CR^{b} ou N,
au moins deux de X⁷ à X¹⁰ sont N,
Z est CR^{c},
L est un groupement arylène substitué ou non-substitué en C₆ à C₃₀, et
R¹ à R⁴, R^{a}, R^{b} et R^{c} sont chacun indépendamment un hydrogène, un deutérium, un groupement alkyle substitué ou non-substitué en C₁ à C₃₀, un groupement aryle substitué ou non-substitué en C₆ à C₃₀, et
n et m sont indépendamment des nombres entiers allant de 0 à 2,
dans lequel « substitué » se réfère au fait qu'au moins un hydrogène est remplacé par un deutérium, un halogène, un groupement hydroxyle, un groupement amino, un groupement amine en C₁ à C₃₀, un groupement nitro, un groupement silyle en C₁ à C₄₀, un groupement alkyle en C₁ à C₃₀, un groupement alkylsilyle en C₁ à C₁₀, un groupement cycloalkyle en C₃ à C₃₀, un groupement hétérocycloalkyle en C₂ à C₃₀, un groupement aryle en C₆ à C₃₀, un groupement hétéroaryle en C₂ à C₃₀, un groupement alcoxy en C₁ à C₂₀, un groupement fluoro, un groupement trifluoroalkyle en C₁ à C₁₀, ou un groupement cyano.

5. Composé pour un dispositif optoélectronique organique selon la revendication 4, dans lequel au moins un de n et m est égal à 1.

6. Composé pour un dispositif optoélectronique organique selon la revendication 1, dans lequel le L est un groupement phényle substitué ou non-substitué, un groupement naphtyle substitué ou non-substitué, un groupement anthracényle substitué ou non-substitué, un groupement phénanthryle substitué ou non-substitué, un groupement pyrényle substitué ou non-substitué, un groupement biphényle substitué ou non-substitué, un groupement p-terphényle substitué ou non-substitué, un groupement m-terphényle substitué ou non-substitué, un groupement quaterphényle substitué ou non-substitué, un groupement chrysényle substitué ou non-substitué, un groupement triphénylényle substitué ou non-substitué, un groupement pérylényle substitué ou non-substitué, un groupement fluorényle substitué ou non-substitué, ou une combinaison de ceux-ci.

7. Composé pour un dispositif optoélectronique organique selon la revendication 6, dans lequel le L est sélectionné parmi les groupements de liaison listés dans le Groupe 1 : 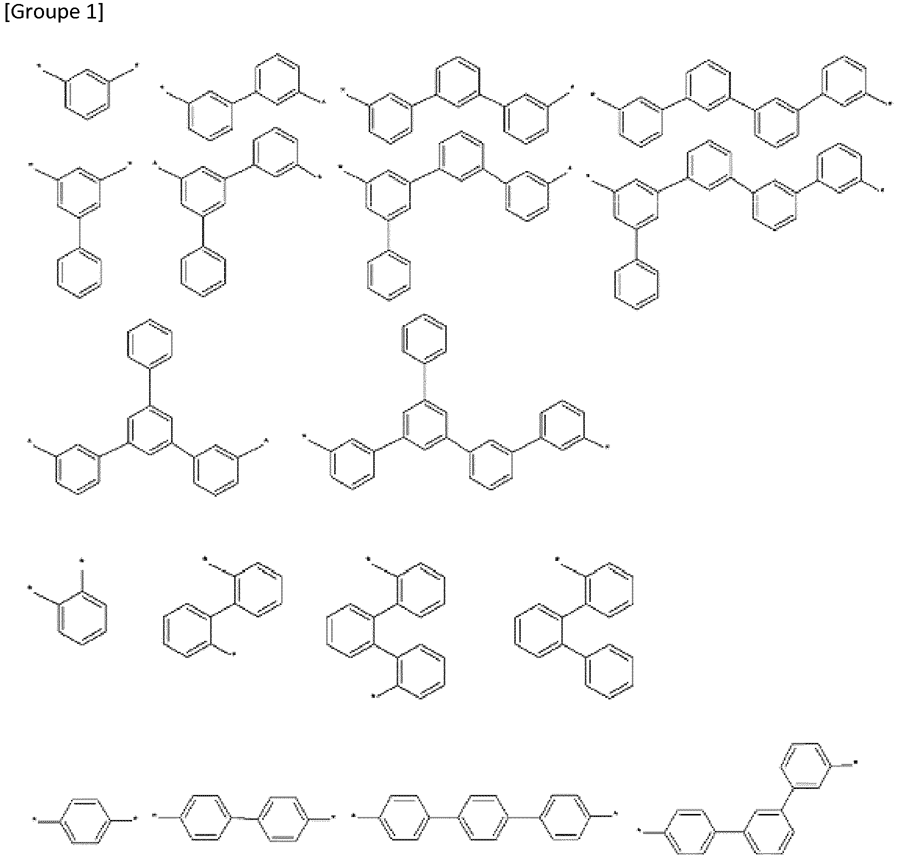 où, dans le Groupe 1, * est un point de liaison.

8. Composé pour un dispositif optoélectronique organique selon la revendication 6, dans lequel le composé est compris dans le Groupe 2 :

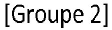

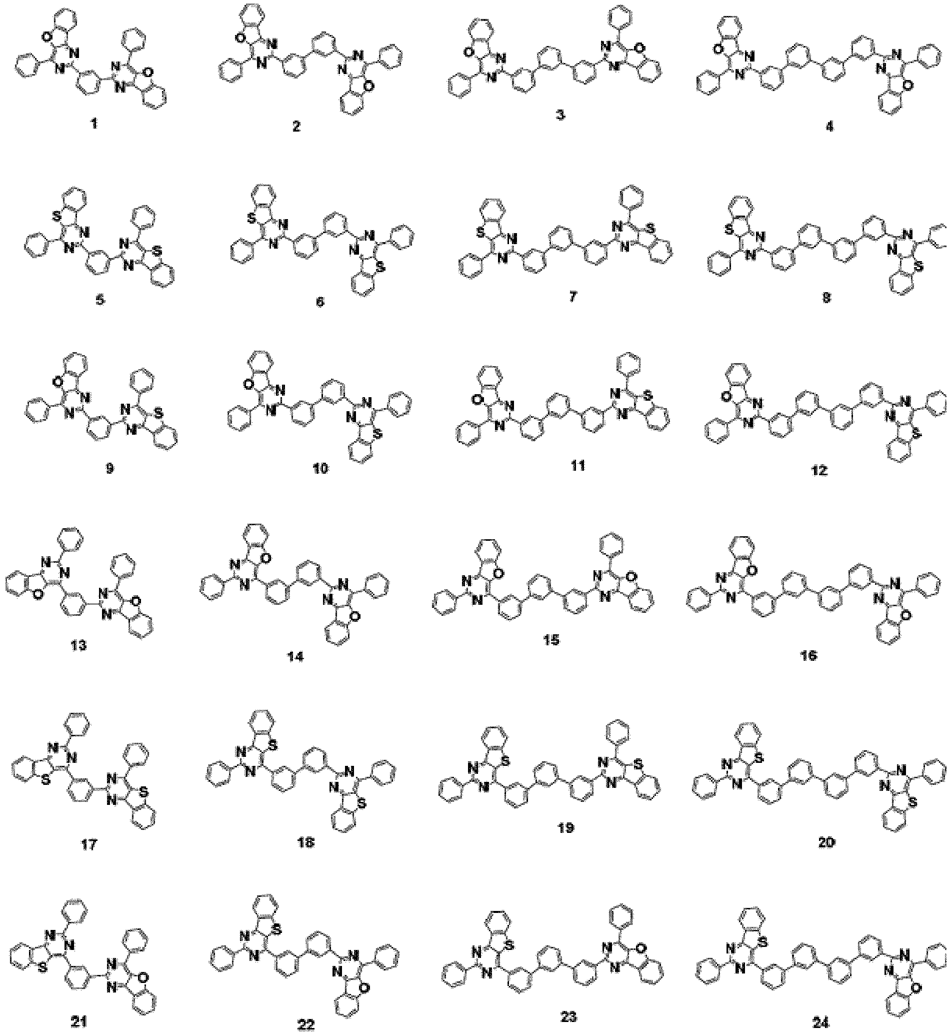

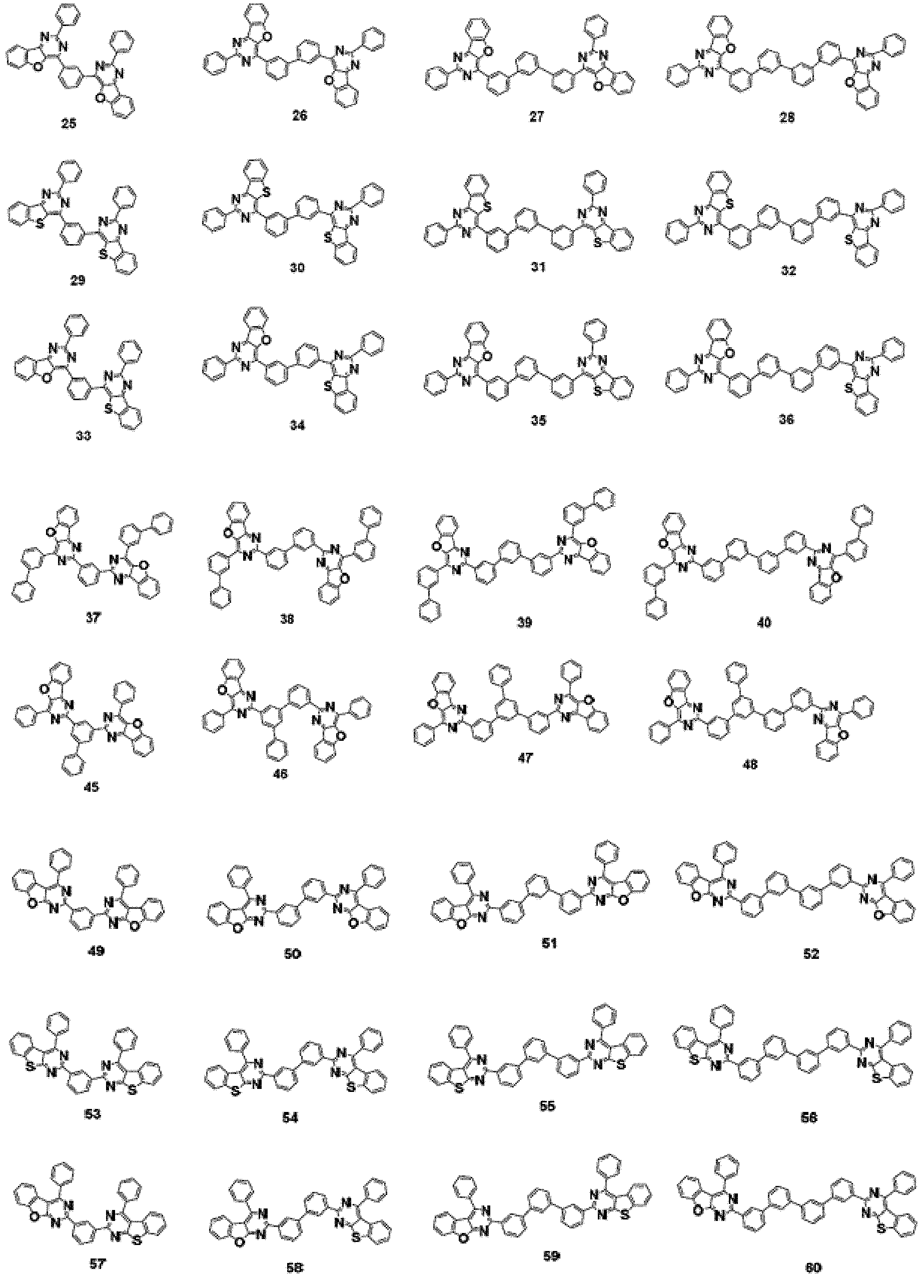

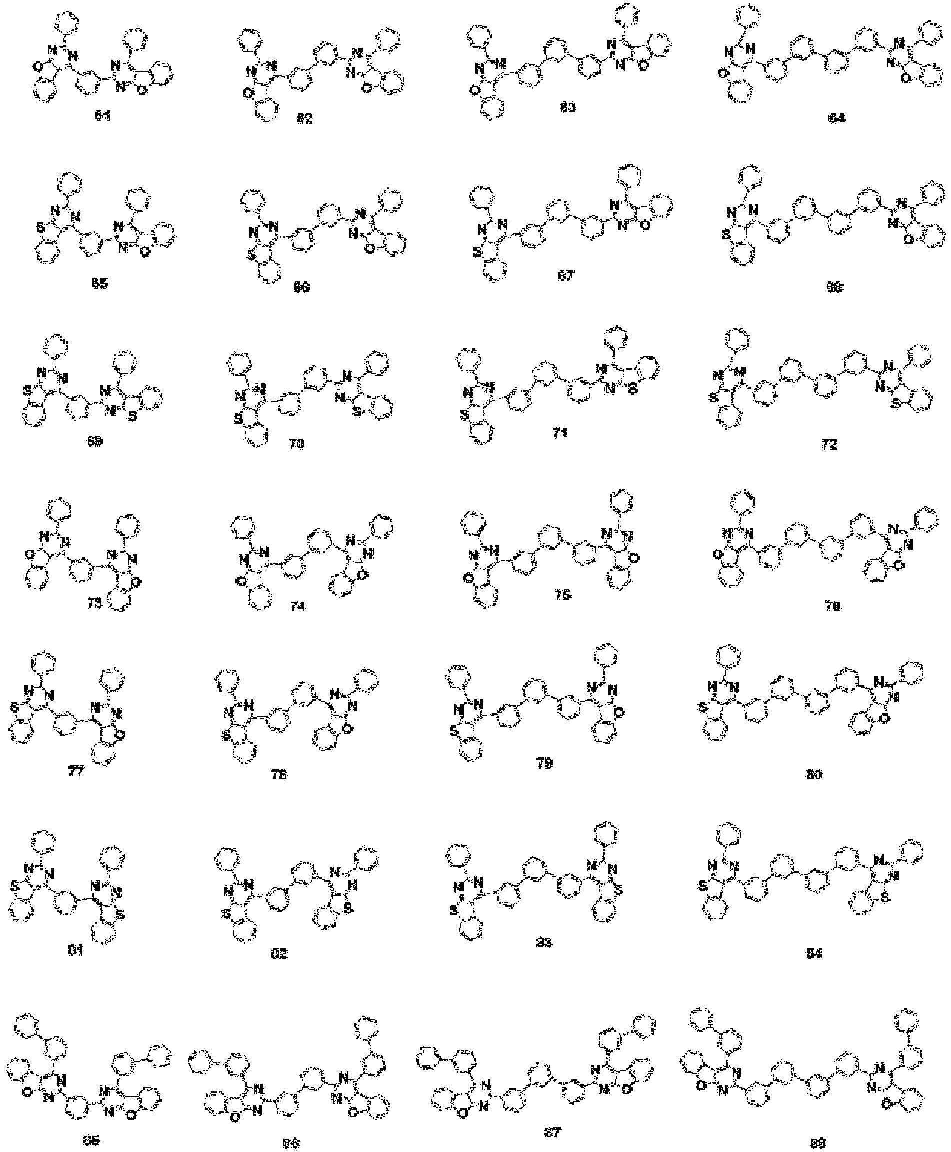

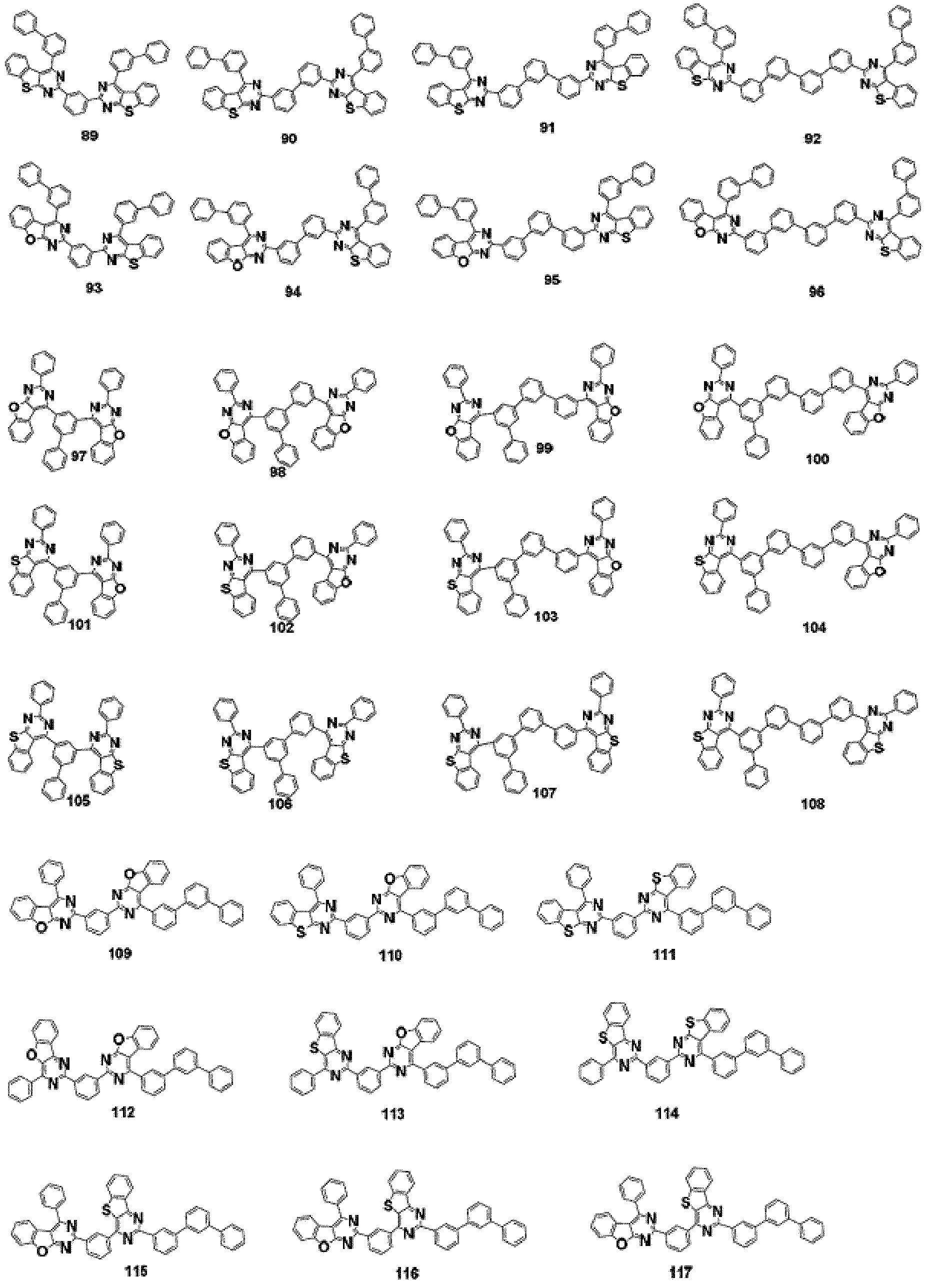

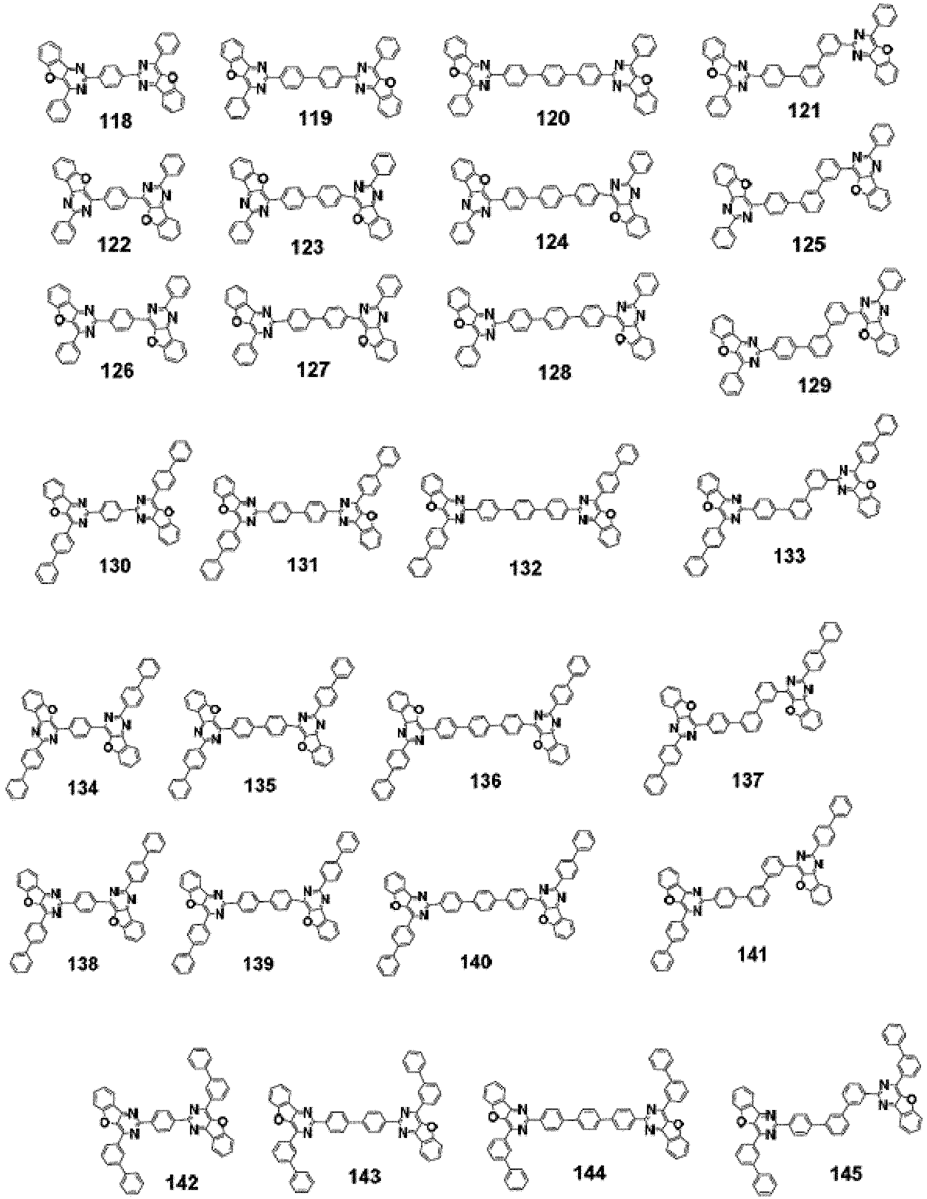

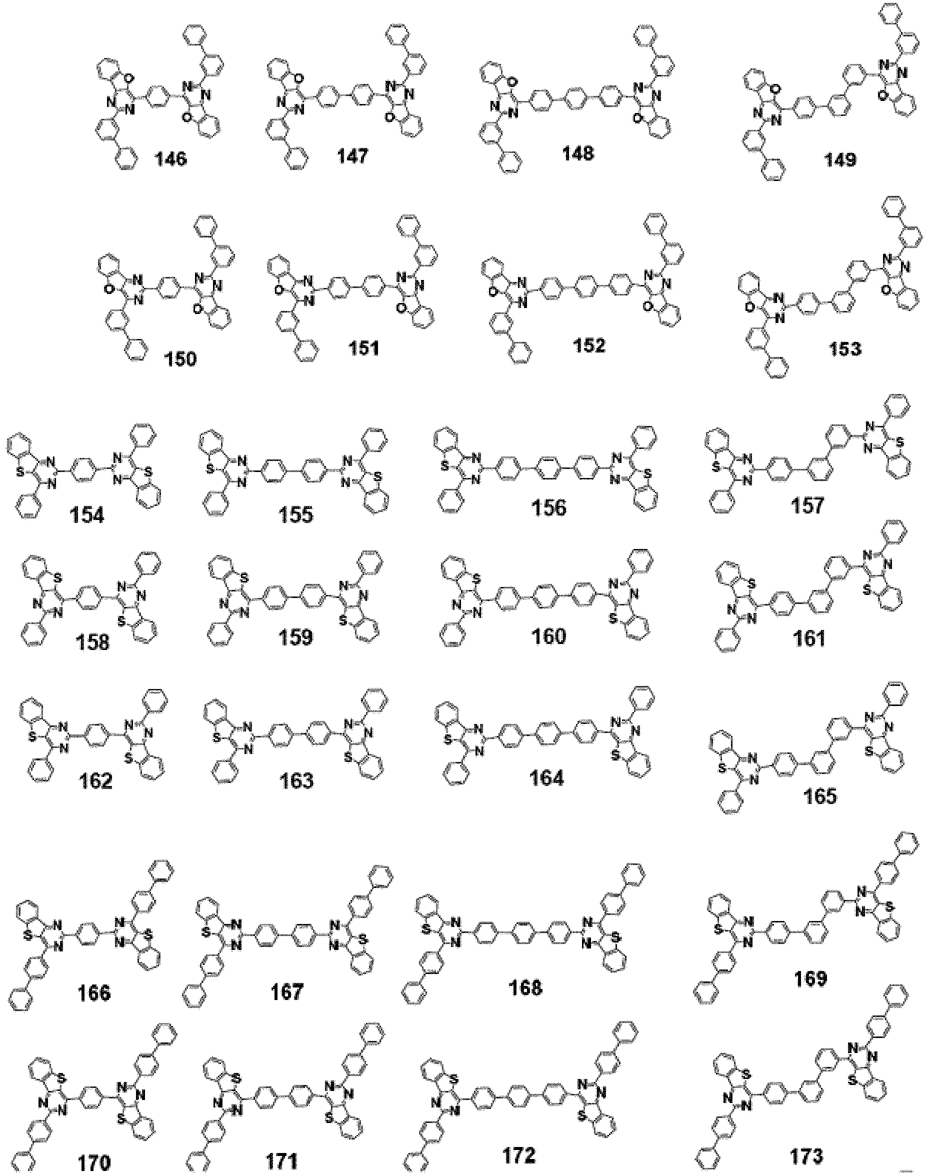

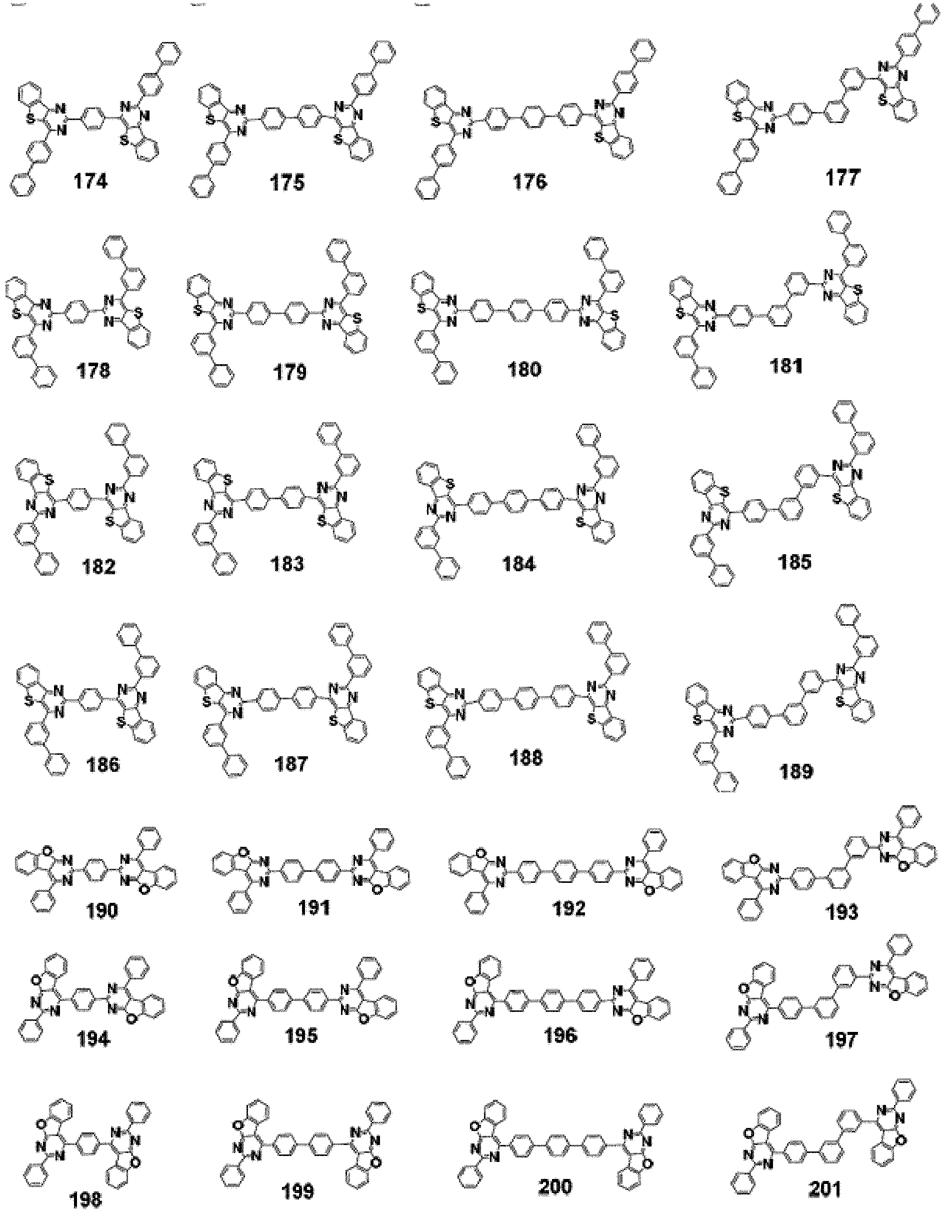

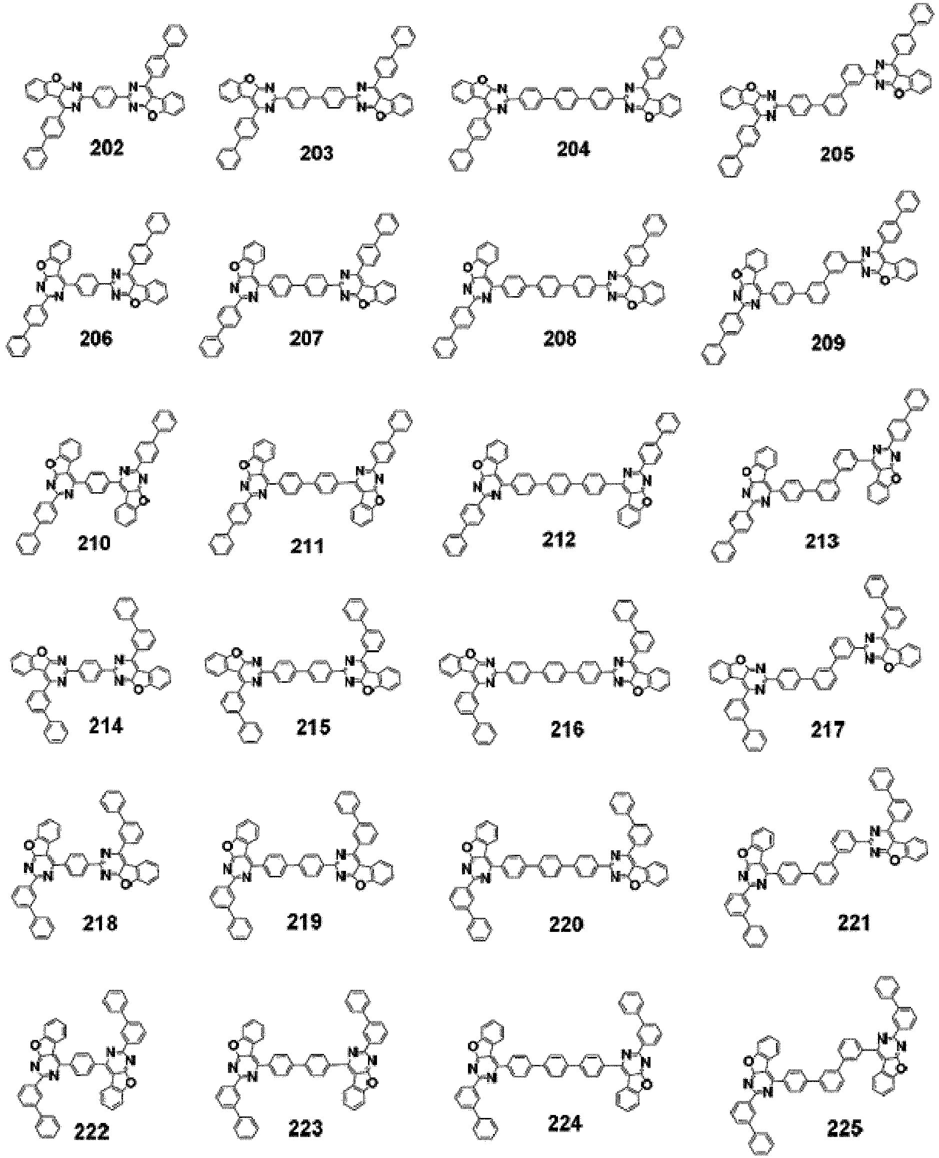

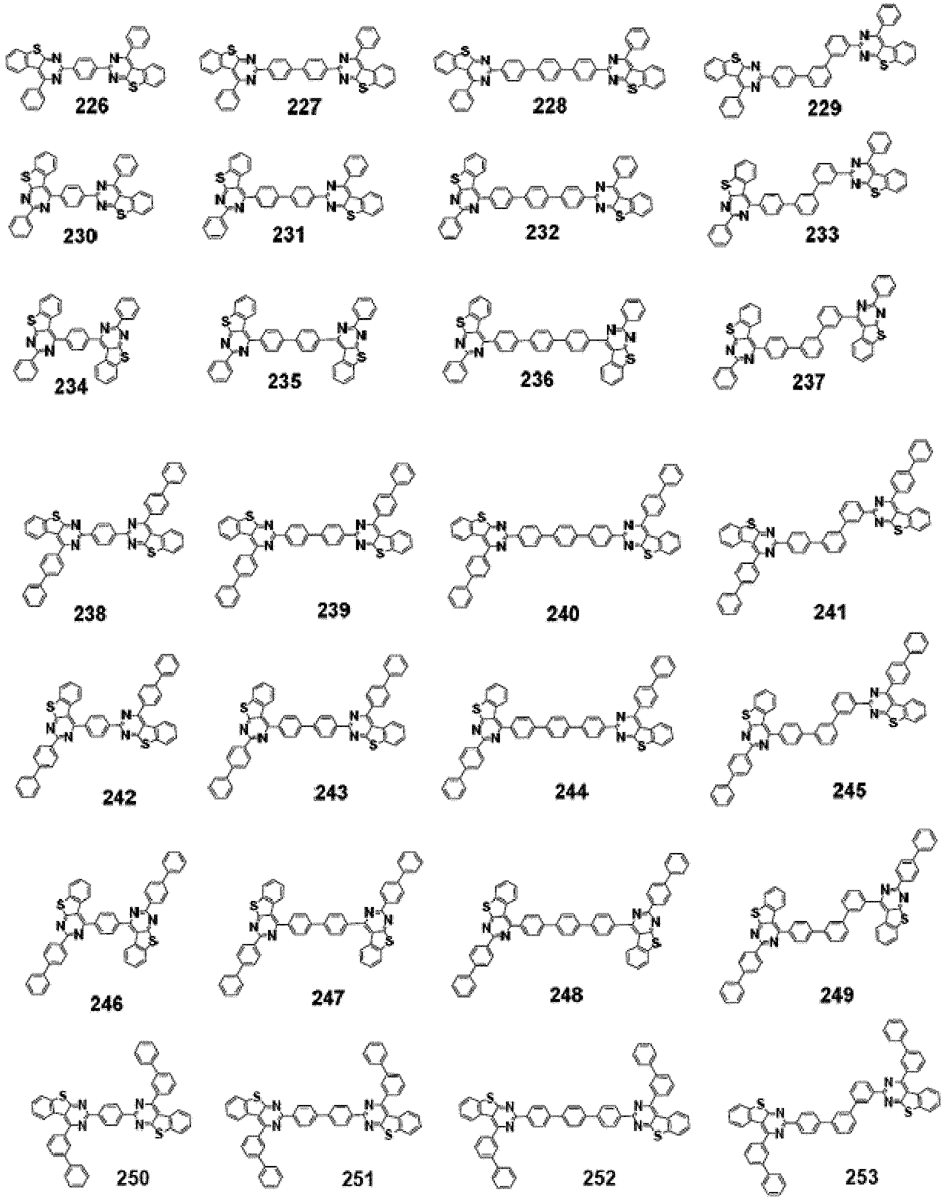

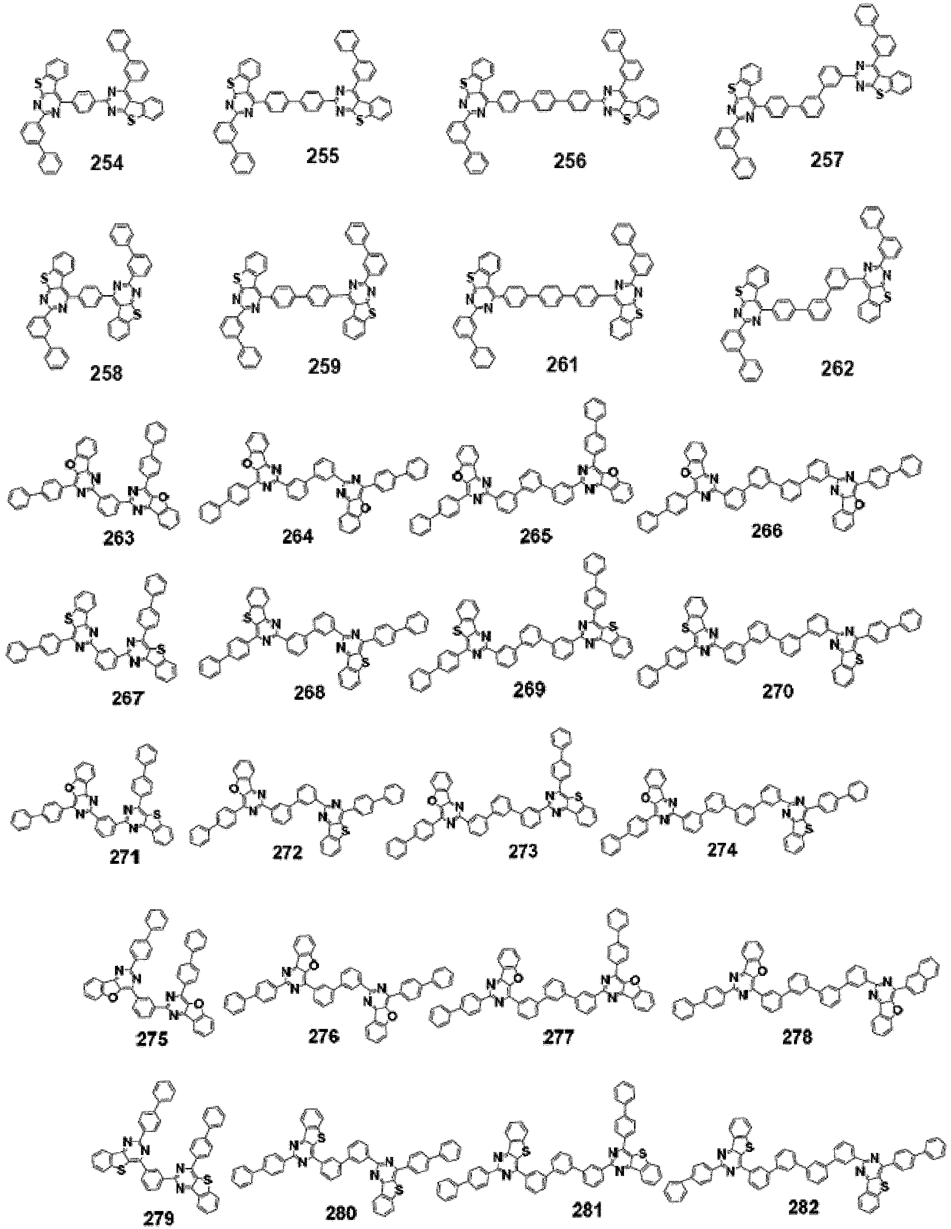

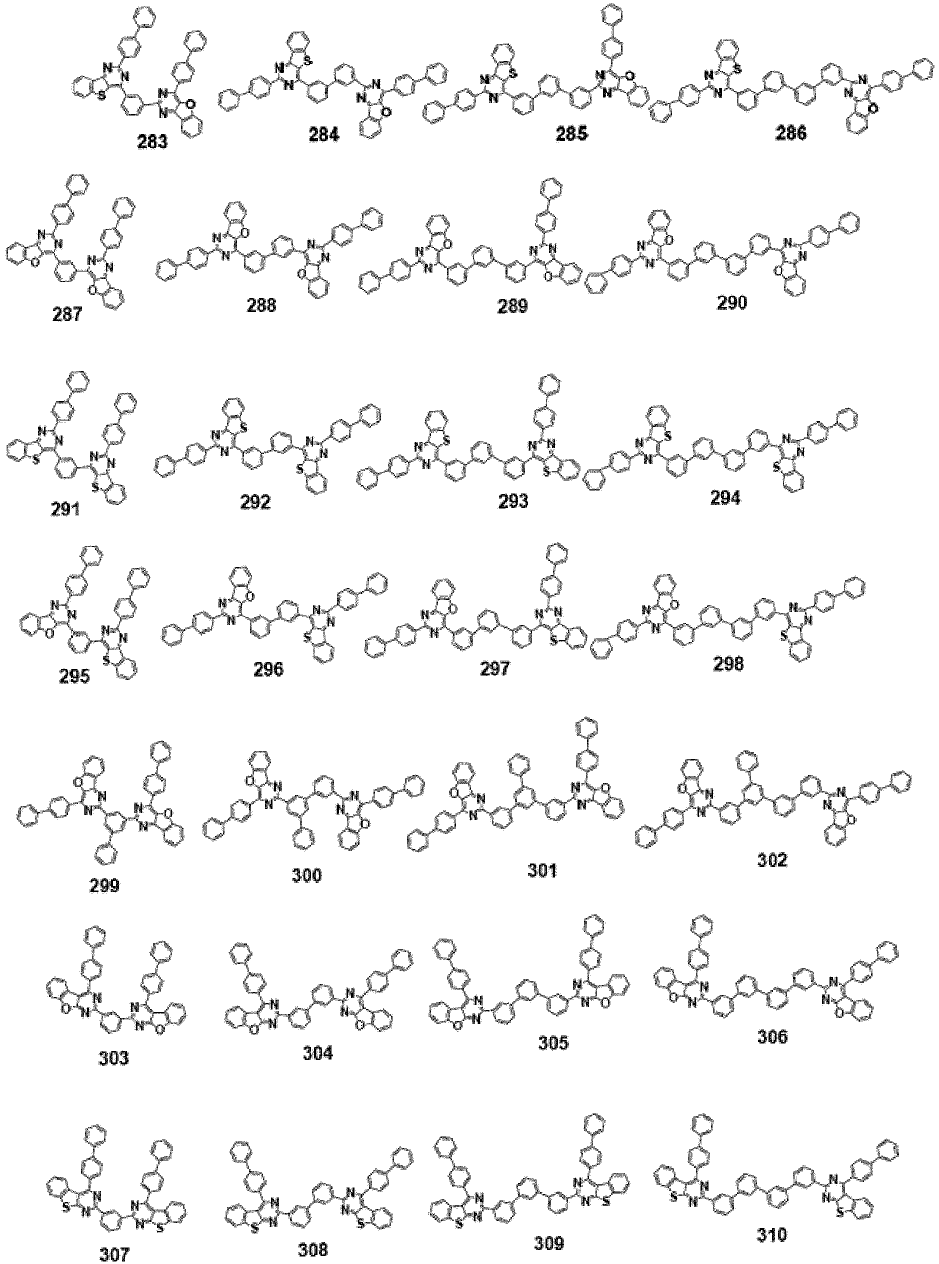

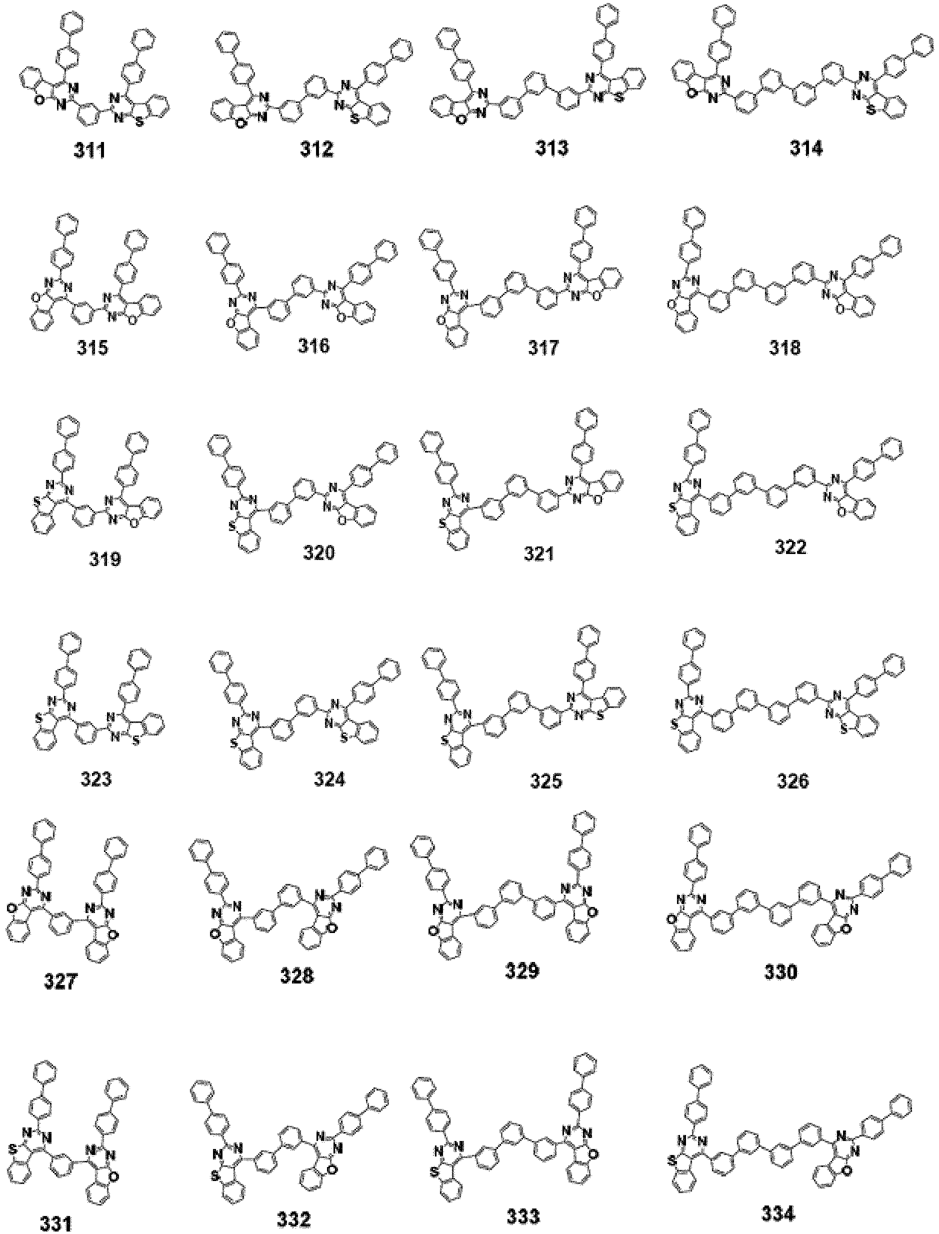

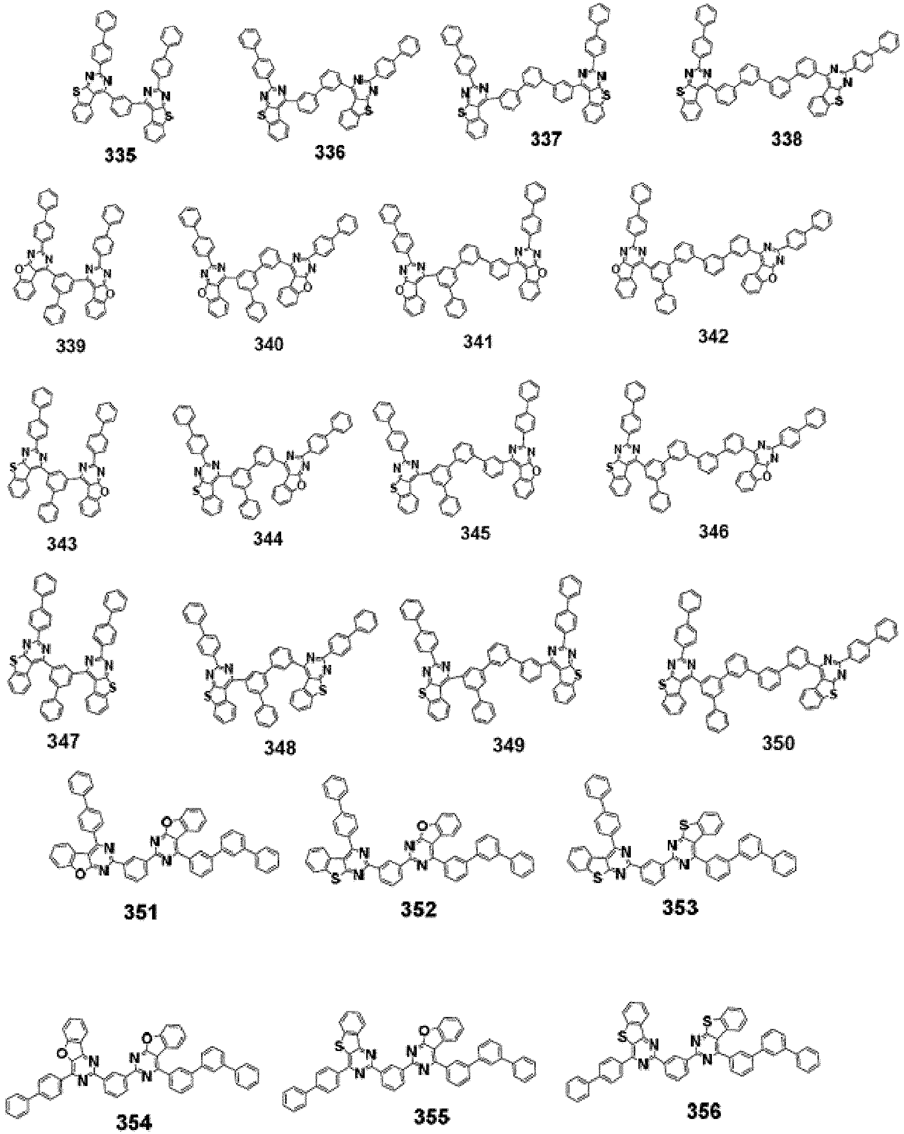

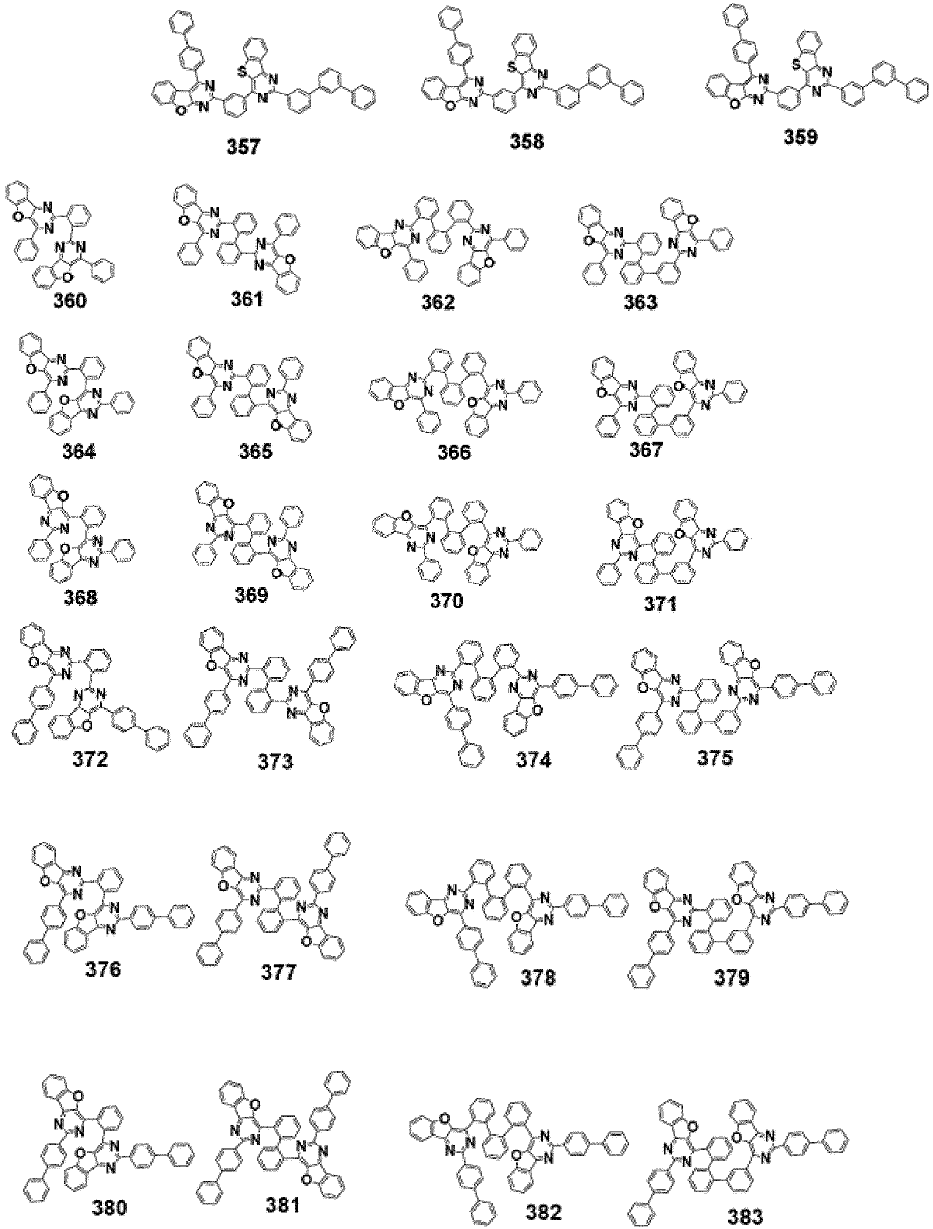

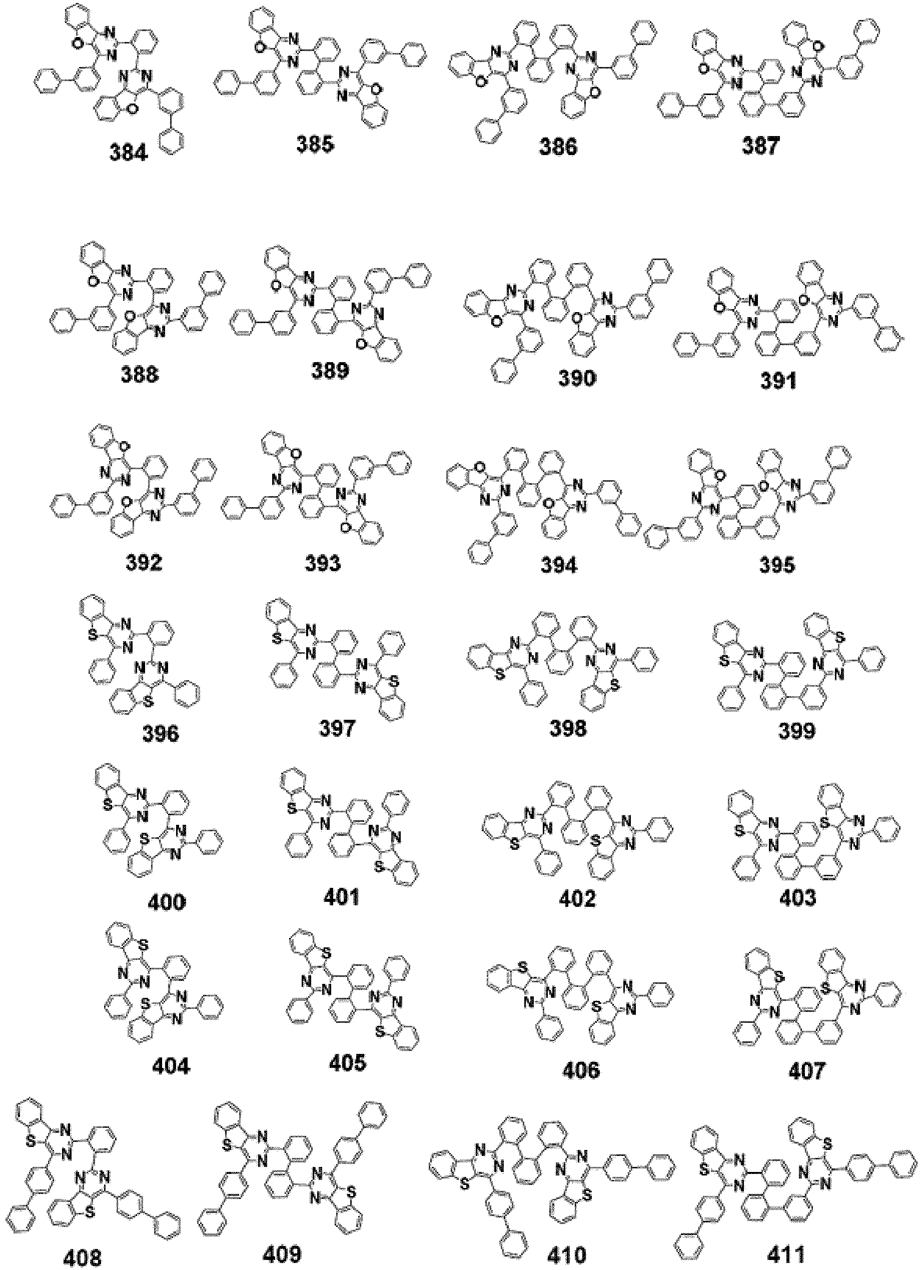

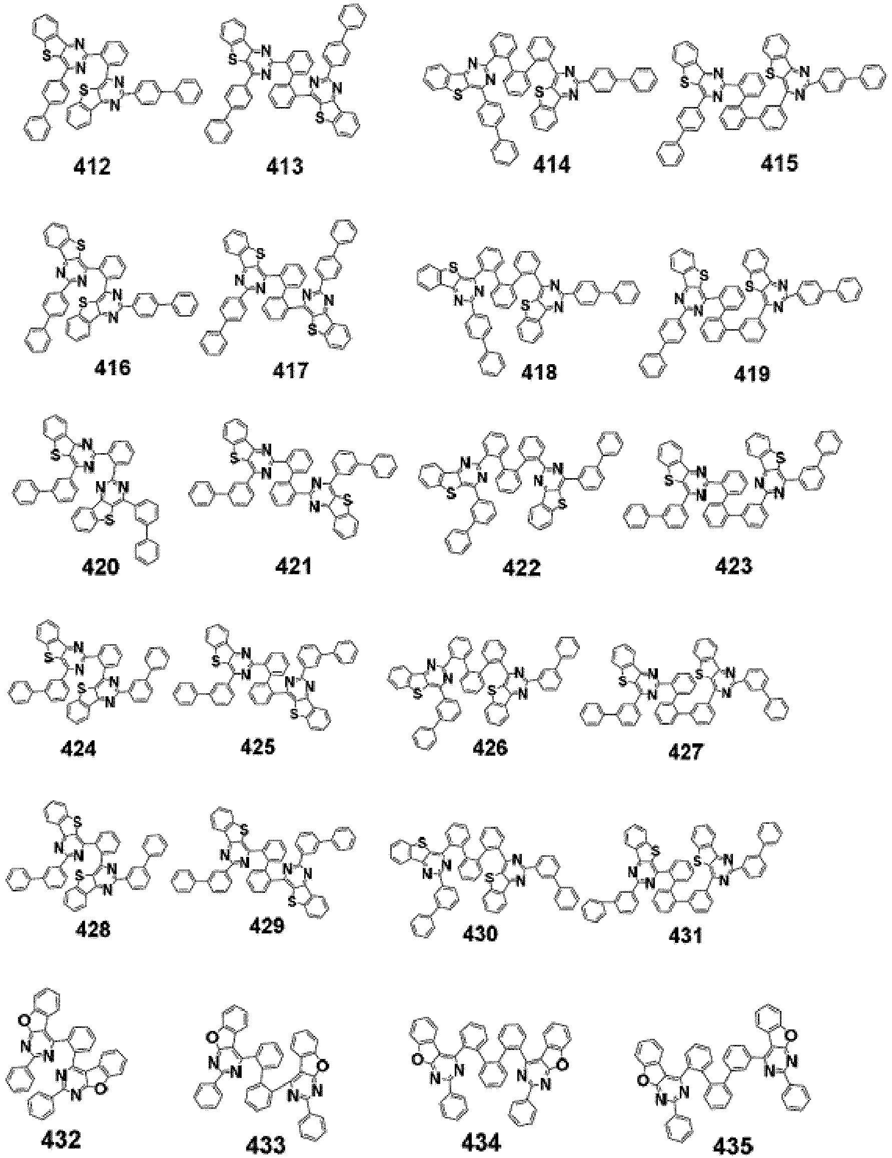

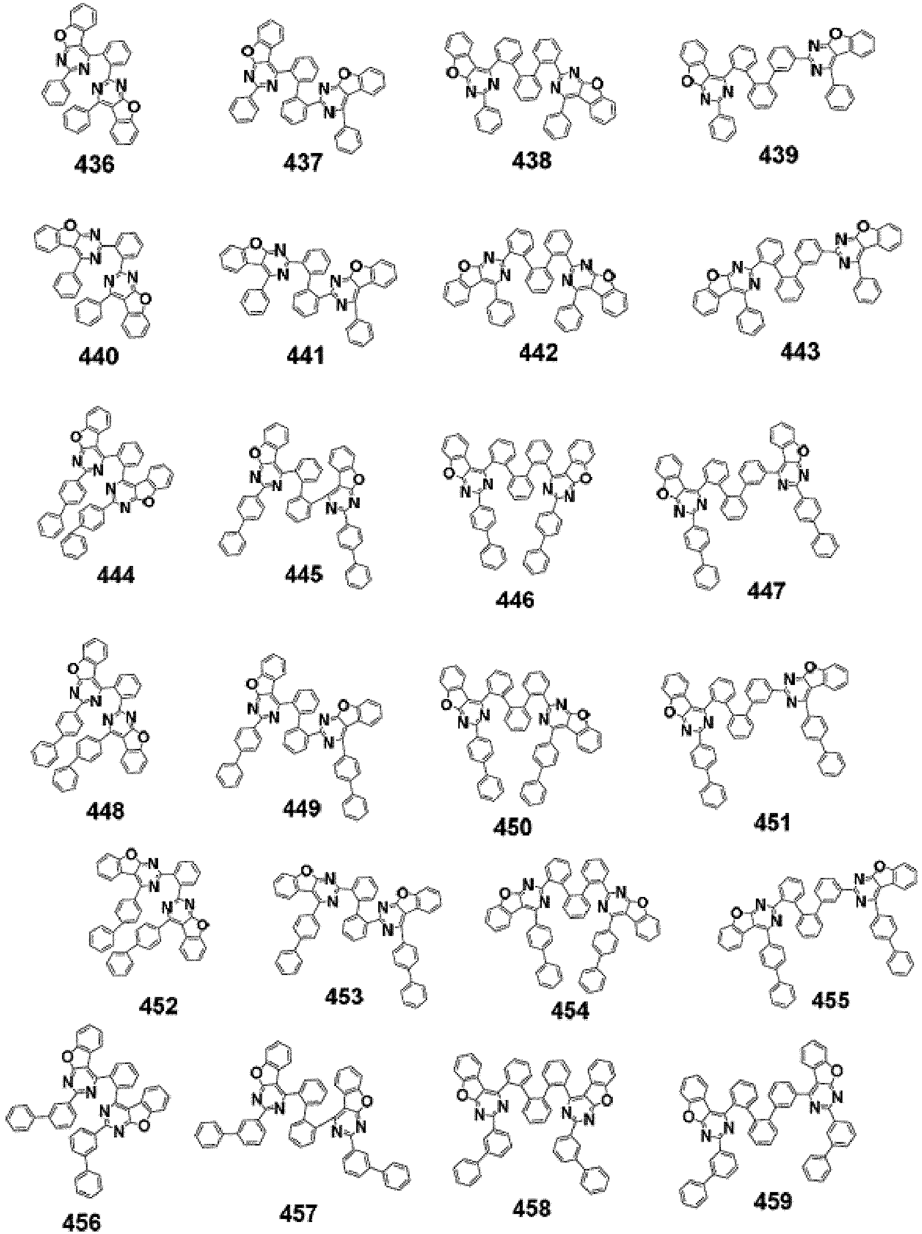

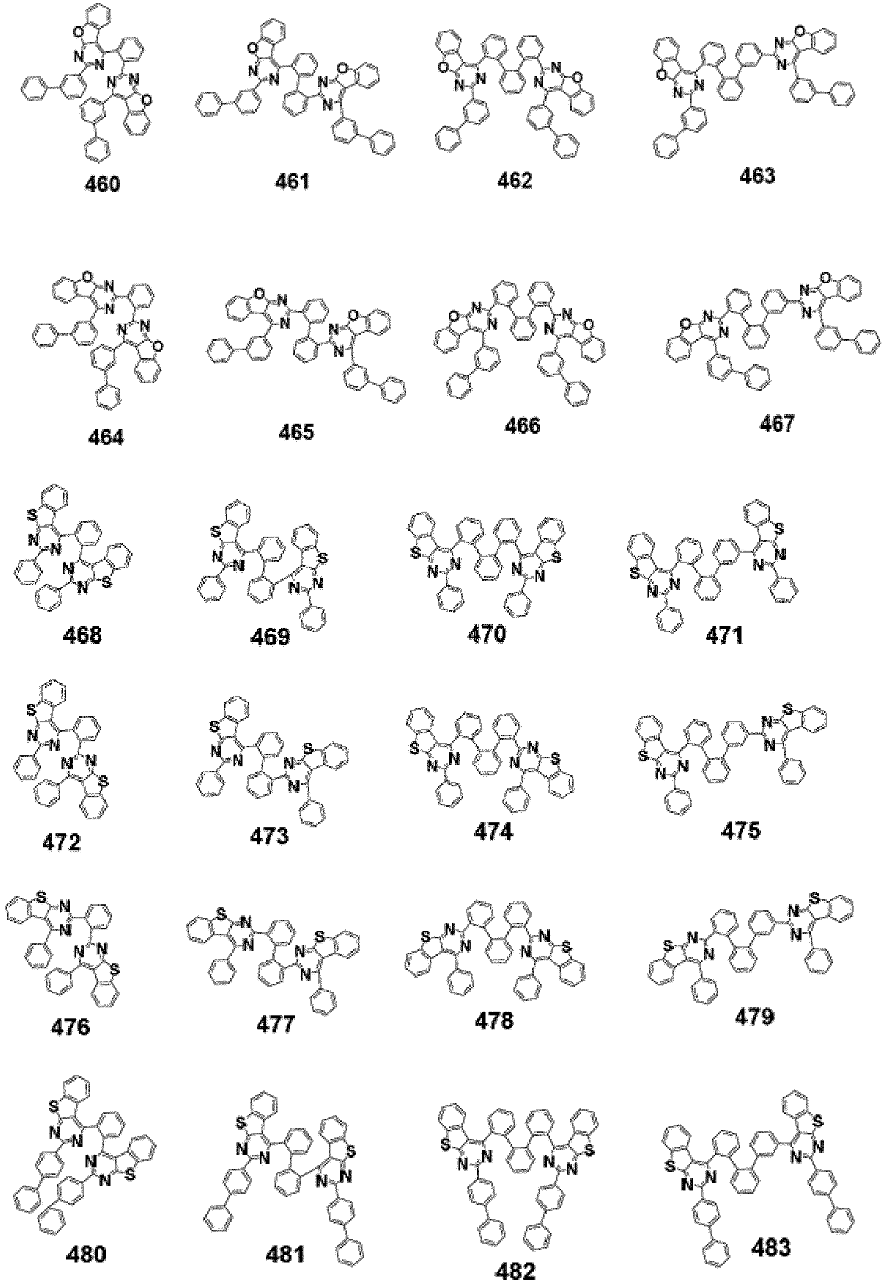

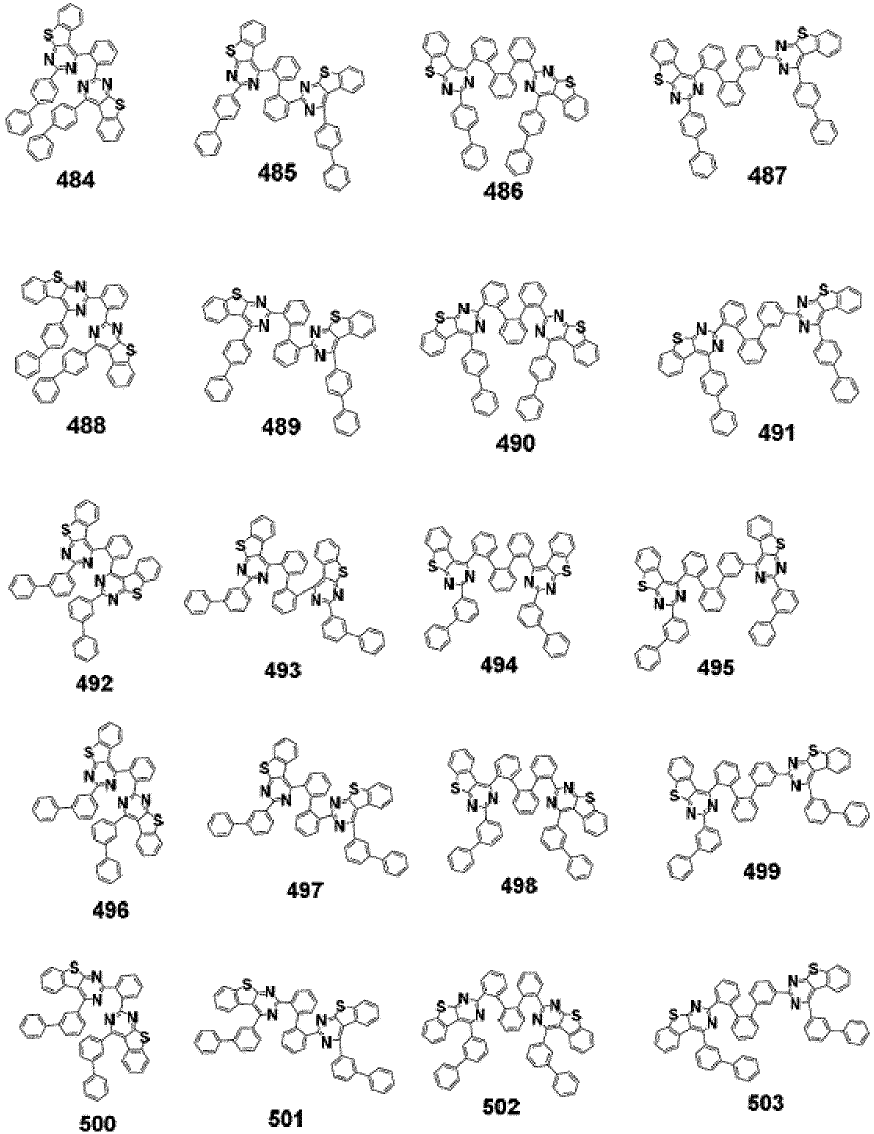

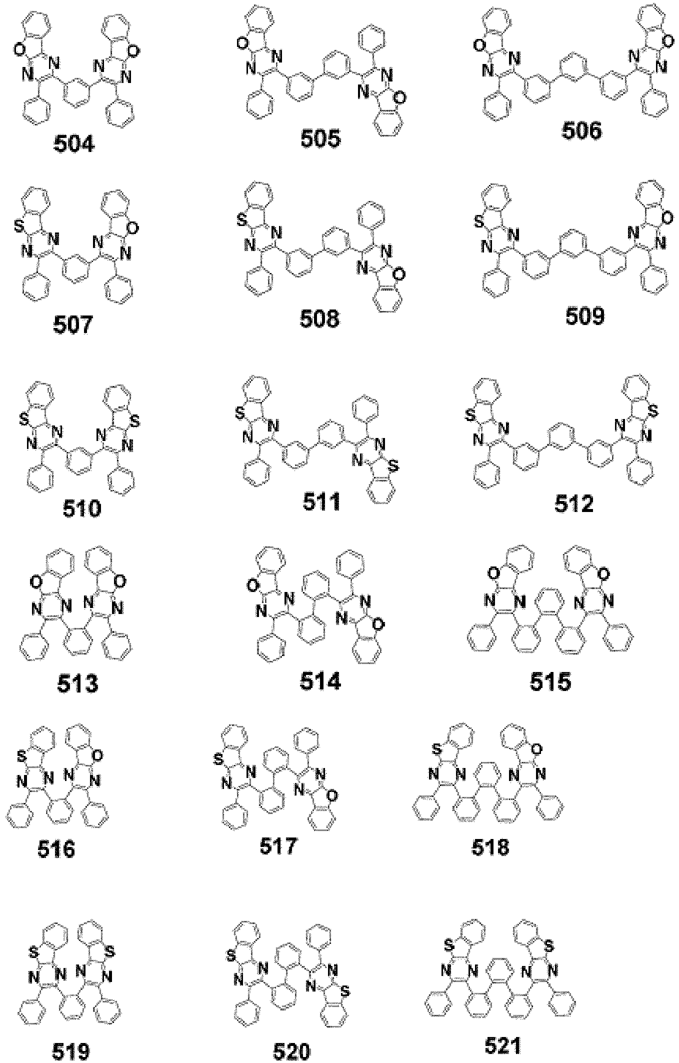

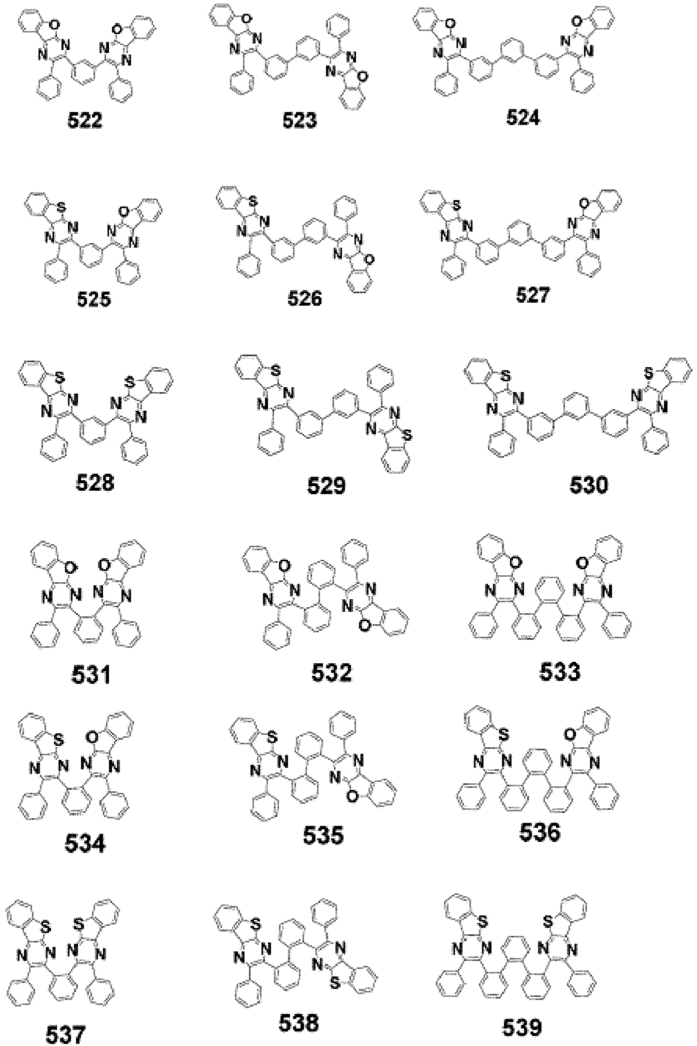

9. Dispositif optoélectronique comprenant une anode et une cathode se faisant face, et au moins une couche organique entre l'anode et la cathode, dans lequel la couche organique comprend le composé pour un dispositif optoélectronique organique selon l'une quelconque des revendications 1 à 8.

10. Dispositif optoélectronique selon la revendication 9, dans lequel la couche organique comprend une couche d'émission, et
la couche d'émission comprend le composé pour un dispositif optoélectronique organique.

11. Dispositif optoélectronique selon la revendication 10, dans lequel le composé pour un dispositif optoélectronique organique est inclus en tant qu'hôte dans une couche d'émission.

12. Dispositif optoélectronique selon la revendication 9, dans lequel la couche organique comprend au moins une couche auxiliaire sélectionnée à partir d'une couche d'injection de trous (HIL), une couche de transport de trous (HTL), une couche auxiliaire de transport de trous, une couche auxiliaire de transport d'électrons, une couche de transport d'électrons (ETL), et une couche d'injection d'électrons (EIL), et
la couche auxiliaire comprend le composé pour un dispositif optoélectronique organique.

13. Dispositif d'affichage comprenant le dispositif optoélectronique organique selon la revendication 9.
